# FASCICULE DE BREVET EUROPEEN

(11) **EP 2 488 660 B1**
(45) Date de publication et mention de la délivrance du brevet: **10.08.2016**
(21) Numéro de dépôt: 10785113.1
(22) Date de dépôt: 14.10.2010
(51) Int. Cl.: C12Q 1/04, G01N 33/68

(54) **PROCEDE DE CARACTERISATION D'AU MOINS UN MICROORGANISME PAR SPECTROMETRIE DE MASSE**
VERFAHREN ZUR CHARAKTERISIERUNG VON MINDESTENS EINEM MIKROORGANISMUS MITHILFE VON MASSENSPEKTROMETRIE
METHOD FOR CHARACTERIZING AT LEAST ONE MICROORGANISM BY MEANS OF MASS SPECTROMETRY

(30) Priorité: 15.10.2009 FR 0957218
(43) Date de publication de la demande: 22.08.2012
(73) Titulaire: Biomérieux, 69280 Marcy L'Etoile (FR)
(72) Inventeur: BEAULIEU, Corinne, F-69140 Rillieux La Pape (FR); CHARRETIER, Yannick, F-69690 Courzieu (FR); CHARRIER, Jean-Philippe, F-69160 Tassin La Demi-lune (FR); CHATELLIER, Sonia, F-01500 Amberieu En Bugey (FR); DUFOUR, Philippe, F-69300 Caluire Et Cuire (FR); FRANCESCHI, Christine, F-01800 Meximieux (FR); GIRARD, Victoria, F-69006 Lyon (FR); PONS, Sylvie, F-69290 Saint Genis Les Ollieres (FR)
(86) Numéro de dépôt international: PCT/FR2010/052181
(87) Numéro de publication internationale: WO 2011/045544

(56) Documents cités:
- WO-A1-2008/145763
- BERNARDO KATUSSEVANI ET AL: "Identification and discrimination of Staphylococcus aureus strains using matrix-assisted laser desorption/ionization-time of flight mass spectrometry", PROTEOMICS, vol. 2, no. 6, juin 2002 (2002-06), pages 747-753, XP002581202, ISSN: 1615-9853
- MAJCHERCZYK PAUL A ET AL: "The discriminatory power of MALDI-TOF mass spectrometry to differentiate between isogenic teicoplanin-susceptible and teicoplanin-resistant strains of methicillin-resistant Staphylococcus aureus.", FEMS MICROBIOLOGY LETTERS FEB 2006 LNKD- PUBMED:16448500, vol. 255, no. 2, février 2006 (2006-02), pages 233-239, XP002581222, ISSN: 0378-1097
- BERNARDO KATUSSEVANI ET AL: "Identification of Staphylococcus aureus exotoxins by combined sodium dodecyl sulfate gel electrophoresis and matrix-assisted laser desorption/ionization-time of flight mass spectrometry", PROTEOMICS, vol. 2, no. 6, juin 2002 (2002-06), pages 740-746, XP002627135, ISSN: 1615-9853
- DARE D ET AL: "Staphylococci speciation and Panton-Valentine leukocidin detection by matrix-assisted laser desorption ionisation time-of-flight mass spectrometry", INTERNATIONAL JOURNAL OF ANTIMICROBIAL AGENTS, vol. 29, no. Suppl. 2, mars 2007 (2007-03) , pages S103-S104, XP002627136, & 17TH EUROPEAN CONGRESS OF CLINICAL MICROBIOLOGY AND INFECTIOUS DISEASES/25TH INTERNATIONAL CONGRESS; MUNICH, GERMANY; MARCH 31 -APRIL 03, 2007 ISSN: 0924-8579
- MAZZEO MARIA FIORELLA ET AL: "Matrix-assisted laser desorption ionization-time of flight mass spectrometry for the discrimination of food-borne microorganisms", APPLIED AND ENVIRONMENTAL MICROBIOLOGY, vol. 72, no. 2, février 2006 (2006-02), pages 1180-1189, XP002627137,
- JOHANNA E CAMARA ET AL: "Discrimination between wild-type and ampicillin-resistant Escherichia coli by matrix-assisted laser desorption/ionization time-of-flight mass spectrometry", ANALYTICAL AND BIOANALYTICAL CHEMISTRY, SPRINGER, BERLIN, DE, vol. 389, no. 5, 12 septembre 2007 (2007-09-12), pages 1633-1638, XP019559826, ISSN: 1618-2650, DOI: DOI:10.1007/S00216-007-1558-7
- TAKAO T ET AL: "Identity of molecular structure of Shiga-like toxin I (VT1) from Escherichia coli O157 : H7 with that of Shiga toxin", MICROBIAL PATHOGENESIS, ACADEMIC PRESS LIMITED, NEW YORK, NY, US, vol. 5, no. 5, 1 novembre 1988 (1988-11-01), pages 357-369, XP023312555, ISSN: 0882-4010, DOI: DOI:10.1016/0882-4010(88)90036-8 [extrait le 1988-11-01]
- KONDO FUMIO ET AL: "Identification of Shiga toxins in Shiga toxin-producing Escherichia coli using immunoprecipitation and high-performance liquid chromatography-electrospray ionization mass spectrometry.", THE ANALYST NOV 2003 LNKD- PUBMED:14700230, vol. 128, no. 11, novembre 2003 (2003-11), pages 1360-1364, XP009145621, ISSN: 0003-2654
- LI MO ET AL: "Comparative proteomic analysis to identification of extracellular virulence factors of enterohemorrhagic Escherichia coli (EHEC) and enteropathogenic Escherichia coli (EPEC)", FASEB JOURNAL, vol. 19, no. 5, Suppl. S, Part 2, mars 2005 (2005-03), page A1388, XP009145623, & EXPERIMENTAL BIOLOGY 2005 MEETING/35TH INTERNATIONAL CONGRESS OF PHYSIOLOGICAL SCIENCES; SAN DIEGO, CA, USA; MARCH 31 -APRIL 06, 2005 ISSN: 0892-6638
- JIANG QIAN ET AL: "MALDI-TOF mass signatures for differentiation of yeast species, strain grouping and monitoring of morphogenesis markers", ANALYTICAL AND BIOANALYTICAL CHEMISTRY, SPRINGER, BERLIN, DE, vol. 392, no. 3, 9 août 2008 (2008-08-09), pages 439-449, XP019621641, ISSN: 1618-2650
- MARINACH CARINE ET AL: "MALDI-TOF MS-based drug susceptibility testing of pathogens: The example of Candida albicans and fluconazole", PROTEOMICS, vol. 9, no. 20, 11 septembre 2009 (2009-09-11), pages 4627-4631, XP002627138, ISSN: 1615-9853
- MELANSON JEREMY E ET AL: "Targeted comparative proteomics by liquid chromatography/matrix-assi sted laser desorption/ionization triple-quadrupole mass spectrometry", RAPID COMMUNICATIONS IN MASS SPECTROMETRY, vol. 20, no. 5, 2006, pages 904-910, XP002627139, ISSN: 0951-4198
- CARBONNELLE ETIENNE ET AL: "Rapid identification of Staphylococci isolated in clinical microbiology laboratories by matrix-assisted laser desorption ionization-time of flight mass spectrometry.", JOURNAL OF CLINICAL MICROBIOLOGY JUL 2007 LNKD- PUBMED:17507519, vol. 45, no. 7, juillet 2007 (2007-07), pages 2156-2161, XP002627140, ISSN: 0095-1137
- SENG PISETH ET AL: "Ongoing Revolution in Bacteriology: Routine Identification of Bacteria by Matrix-Assisted Laser Desorption Ionization Time-of-Flight Mass Spectrometry", CLINICAL INFECTIOUS DISEASES, THE UNIVERSITY OF CHICAGO PRESS, CHICAGO, IL, US, vol. 49, no. 4, 15 août 2009 (2009-08-15), pages 543-551, XP002601280, ISSN: 1058-4838, DOI: DOI:10.1086/600885 [extrait le 2009-07-07]
- VAEZZADEH ALI R ET AL: "Imaging mass spectrometry using peptide isoelectric focusing", RAPID COMMUNICATIONS IN MASS SPECTROMETRY, vol. 22, no. 17, September 2008 (2008-09), pages 2667-2676, ISSN: 0951-4198
- PIEPER R ET AL: "Comparative proteomic analysis of Staphylococcus aureus strains with differences in resistance to the cell wall-targeting antibiotic vancomycin", PROTEOMICS, WILEY - VCH VERLAG, WEINHEIM, DE, vol. 6, no. 15, 1 August 2006 (2006-08-01) , pages 4246-4258, XP008105706, ISSN: 1615-9853, DOI: 10.1002/PMIC.200500764 [retrieved on 2006-07-06]
- HEDSTROM ET AL: "Miniaturized on-line digestion system for the sequential identification and characterization of protein analytes", JOURNAL OF CHROMATOGRAPHY, ELSEVIER SCIENCE PUBLISHERS B.V, NL, vol. 1146, no. 1, 8 March 2007 (2007-03-08), pages 17-22, XP005917993, ISSN: 0021-9673, DOI: 10.1016/J.CHROMA.2006.12.072
- Anren Hu ET AL: "Identification of Microbial Mixtures by Capillary Electrophoresis/Selective Tandem Mass Spectrometry", Analytical Chemistry, vol. 77, no. 5, 1 March 2005 (2005-03-01), pages 1488-1495, XP055081155, ISSN: 0003-2700, DOI: 10.1021/ac0484427
- LANGE VINZENZ ET AL: "Targeted quantitative analysis of Streptococcus pyogenes virulence factors by multiple reaction monitoring", MOLECULAR & CELLULAR PROTEOMICS, AMERICAN SOCIETY FOR BIOCHEMISTRY AND MOLECULAR BIOLOGY, US, vol. 7, no. 8, 1 August 2008 (2008-08-01), pages 1489-1500, XP009107637, ISSN: 1535-9476, DOI: 10.1074/MCP.M800032-MCP200

### SEQUENCE LISTING

<110> bioMérieux
<120> Procédé de caractérisation d'au moins un microorganisme par spectrométrie de masse
<130> MICROSM
<150> FR 0957218
   <151> 2009-10-15
<160> 177
<170> PatentIn version 3.3
<210> 1
   <211> 508
   <212> PRT
   <213> Staphylococcus aureus
<400> 1
   Met Lys Lys Lys Asn Ile Tyr Ser Ile Arg Lys Leu Gly Val Gly Ile
   1               5                   10                  15
   Ala Ser Val Thr Leu Gly Thr Leu Leu Ile Ser Gly Gly Val Thr Pro
               20                  25                  30
   Ala Ala Asn Ala Ala Gln His Asp Glu Ala Gln Gln Asn Ala Phe Tyr
           35                  40                  45
   Gln Val Leu Asn Met Pro Asn Leu Asn Ala Asp Gln Arg Asn Gly Phe
       50                  55                  60
   Ile Gln Ser Leu Lys Asp Asp Pro Ser Gln Ser Ala Asn Val Leu Gly
   65                  70                  75                  80
   Glu Ala Gln Lys Leu Asn Asp Ser Gln Ala Pro Lys Ala Asp Ala Gln
                   85                  90                  95
   Gln Asn Lys Phe Asn Lys Asp Gln Gln Ser Ala Phe Tyr Glu Ile Leu
               100                 105                 110
   Asn Met Pro Asn Leu Asn Glu Glu Gln Arg Asn Gly Phe Ile Gln Ser
           115                 120                 125
   Leu Lys Asp Asp Pro Ser Gln Ser Thr Asn Val Leu Gly Glu Ala Lys
       130                 135                 140
   Lys Leu Asn Glu Ser Gln Ala Pro Lys Ala Asp Asn Asn Phe Asn Lys
   145                 150                 155                 160
   Glu Gln Gln Asn Ala Phe Tyr Glu Ile Leu Asn Met Pro Asn Leu Asn
                   165                 170                 175
   Glu Glu Gln Arg Asn Gly Phe Ile Gln Ser Leu Lys Asp Asp Pro Ser
   180 185 190
   Gln Ser Ala Asn Leu Leu Ala Glu Ala Lys Lys Leu Asn Glu Ser Gln
   195 200 205
   Ala Pro Lys Ala Asp Asn Lys Phe Asn Lys Glu Gln Gln Asn Ala Phe
   210 215 220
   Tyr Glu Ile Leu His Leu Pro Asn Leu Asn Glu Glu Gln Arg Asn Gly
   225 230 235 240
   Phe Ile Gln Ser Leu Lys Asp Asp Pro Ser Gln Ser Ala Asn Leu Leu
   245 250 255
   Ala Glu Ala Lys Lys Leu Asn Asp Ala Gln Ala Pro Lys Ala Asp Asn
   260 265 270
   Lys Phe Asn Lys Glu Gln Gln Asn Ala Phe Tyr Glu Ile Leu His Leu
   275 280 285
   Pro Asn Leu Thr Glu Glu Gln Arg Asn Gly Phe Ile Gln Ser Leu Lys
   290 295 300
   Asp Asp Pro Ser Val Ser Lys Glu Ile Leu Ala Glu Ala Lys Lys Leu
   305 310 315 320
   Asn Asp Ala Gln Ala Pro Lys Glu Glu Asp Asn Asn Lys Pro Gly Lys
   325 330 335
   Glu Asp Gly Asn Lys Pro Gly Lys Glu Asp Gly Asn Lys Pro Gly Lys
   340 345 350
   Glu Asp Asn Lys Lys Pro Gly Lys Glu Asp Gly Asn Lys Pro Gly Lys
   355 360 365
   Glu Asp Asn Lys Lys Pro Gly Lys Glu Asp Gly Asn Lys Pro Gly Lys
   370 375 380
   Glu Asp Gly Asn Lys Pro Gly Lys Glu Asp Gly Asn Lys Pro Gly Lys
   385 390 395 400
   Glu Asp Gly Asn Lys Pro Gly Lys Glu Asp Gly Asn Gly Val His Val
   405 410 415
   Val Lys Pro Gly Asp Thr Val Asn Asp Ile Ala Lys Ala Asn Gly Thr
   420 425 430
   Thr Ala Asp Lys Ile Ala Ala Asp Asn Lys Leu Ala Asp Lys Asn Met
   435 440 445
   Ile Lys Pro Gly Gln Glu Leu Val Val Asp Lys Lys Gln Pro Ala Asn
   450 455 460
   His Ala Asp Ala Asn Lys Ala Gln Ala Leu Pro Glu Thr Gly Glu Glu
   465 470 475 480
   Asn Pro Phe Ile Gly Thr Thr Val Phe Gly Gly Leu Ser Leu Ala Leu
   485 490 495
   Gly Ala Ala Leu Leu Ala Gly Arg Arg Arg Glu Leu
   500 505
<210> 2
   <211> 15
   <212> PRT
   <213> Staphylococcus aureus
<400> 2
   Asp Asp Pro Ser Gln Ser Ala Asn Val Leu Gly Glu Ala Gln Lys
   1 5 10 15
<210> 3
   <211> 20
   <212> PRT
   <213> Staphylococcus aureus
<400> 3
   Asp Gln Gln Ser Ala Phe Tyr Glu Ile Leu Asn Met Pro Asn Leu Asn
   1 5 10 15
   Glu Glu Gln Arg
   20
<210> 4
   <211> 14
   <212> PRT
   <213> Staphylococcus aureus
<400> 4
   Asp Asp Pro Ser Gln Ser Thr Asn Val Leu Gly Glu Ala Lys
   1 5 10
<210> 5
   <211> 20
   <212> PRT
   <213> Staphylococcus aureus
<400> 5
   Glu Gln Gln Asn Ala Phe Tyr Glu Ile Leu Asn Met Pro Asn Leu Asn
   1               5                   10                  15
   Glu Glu Gln Arg
               20
<210> 6
   <211> 14
   <212> PRT
   <213> Staphylococcus aureus
<400> 6
   Asp Asp Pro Ser Gln Ser Ala Asn Leu Leu Ala Glu Ala Lys
   1               5                   10
<210> 7
   <211> 7
   <212> PRT
   <213> Staphylococcus aureus
<400> 7
   Asp Asp Pro Ser Val Ser Lys
   1               5
<210> 8
   <211> 6
   <212> PRT
   <213> Staphylococcus aureus
<400> 8
   Ile Ala Ala Asp Asn Lys
   1               5
<210> 9
   <211> 668
   <212> PRT
   <213> Staphylococcus aureus
<400> 9
   Met Lys Lys Ile Lys Ile Val Pro Leu Ile Leu Ile Val Val Val Val
   1               5                   10                  15
   Gly Phe Gly Ile Tyr Phe Tyr Ala Ser Lys Asp Lys Glu Ile Asn Asn
               20                  25                  30
   Thr Ile Asp Ala Ile Glu Asp Lys Asn Phe Lys Gln Val Tyr Lys Asp
           35                  40                  45
   Ser Ser Tyr Ile Ser Lys Ser Asp Asn Gly Glu Val Glu Met Thr Glu
       50                  55                  60
   Arg Pro Ile Lys Ile Tyr Asn Ser Leu Gly Val Lys Asp Ile Asn Ile
   65                  70                  75                  80
   Gln Asp Arg Lys Ile Lys Lys Val Ser Lys Asn Lys Lys Arg Val Asp
                   85                  90                  95
   Ala Gln Tyr Lys Ile Lys Thr Asn Tyr Gly Asn Ile Asp Arg Asn Val
               100                 105                 110
   Gln Phe Asn Phe Val Lys Glu Asp Gly Met Trp Lys Leu Asp Trp Asp
           115                 120                 125
   His Ser Val Ile Ile Pro Gly Met Gln Lys Asp Gln Ser Ile His Ile
       130                 135                 140
   Glu Asn Leu Lys Ser Glu Arg Gly Lys Ile Leu Asp Arg Asn Asn Val
   145                 150                 155                 160
   Glu Leu Ala Asn Thr Gly Thr Ala Tyr Glu Ile Gly Ile Val Pro Lys
                   165                 170                 175
   Asn Val Ser Lys Lys Asp Tyr Lys Ala Ile Ala Lys Glu Leu Ser Ile
               180                 185                 190
   Ser Glu Asp Tyr Ile Lys Gln Gln Met Asp Gln Asn Trp Val Gln Asp
           195                 200                 205
   Asp Thr Phe Val Pro Leu Lys Thr Val Lys Lys Met Asp Glu Tyr Leu
       210                 215                 220
   Ser Asp Phe Ala Lys Lys Phe His Leu Thr Thr Asn Glu Thr Glu Ser
   225                 230                 235                 240
   Arg Asn Tyr Pro Leu Glu Lys Ala Thr Ser His Leu Leu Gly Tyr Val
                   245                 250                 255
   Gly Pro Ile Asn Ser Glu Glu Leu Lys Gln Lys Glu Tyr Lys Gly Tyr
               260                 265                 270
   Lys Asp Asp Ala Val Ile Gly Lys Lys Gly Leu Glu Lys Leu Tyr Asp
           275                 280                 285
   Lys Lys Leu Gln His Glu Asp Gly Tyr Arg Val Thr Ile Val Asp Asp
       290                 295                 300
   Asn Ser Asn Thr Ile Ala His Thr Leu Ile Glu Lys Lys Lys Lys Asp
   305                 310                 315                 320
   Gly Lys Asp Ile Gln Leu Thr Ile Asp Ala Lys Val Gln Lys Ser Ile
                   325                 330                 335
   Tyr Asn Asn Met Lys Asn Asp Tyr Gly Ser Gly Thr Ala Ile His Pro
               340                 345                 350
   Gln Thr Gly Glu Leu Leu Ala Leu Val Ser Thr Pro Ser Tyr Asp Val
           355                 360                 365
   Tyr Pro Phe Met Tyr Gly Met Ser Asn Glu Glu Tyr Asn Lys Leu Thr
       370                 375                 380
   Glu Asp Lys Lys Glu Pro Leu Leu Asn Lys Phe Gln Ile Thr Thr Ser
   385                 390                 395                 400
   Pro Gly Ser Thr Gln Lys Ile Leu Thr Ala Met Ile Gly Leu Asn Asn
                   405                 410                 415
   Lys Thr Leu Asp Asp Lys Thr Ser Tyr Lys Ile Asp Gly Lys Gly Trp
               420                 425                 430
   Gln Lys Asp Lys Ser Trp Gly Gly Tyr Asn Val Thr Arg Tyr Glu Val
           435                 440                 445
   Val Asn Gly Asn Ile Asp Leu Lys Gln Ala Ile Glu Ser Ser Asp Asn
       450                 455                 460
   Ile Phe Phe Ala Arg Val Ala Leu Glu Leu Gly Ser Lys Lys Phe Glu
   465                 470                 475                 480
   Lys Gly Met Lys Lys Leu Gly Val Gly Glu Asp Ile Pro Ser Asp Tyr
                   485                 490                 495
   Pro Phe Tyr Asn Ala Gln Ile Ser Asn Lys Asn Leu Asp Asn Glu Ile
               500                 505                 510
   Leu Leu Ala Asp Ser Gly Tyr Gly Gln Gly Glu Ile Leu Ile Asn Pro
           515                 520                 525
   Val Gln Ile Leu Ser Ile Tyr Ser Ala Leu Glu Asn Asn Gly Asn Ile
       530                 535                 540
   Asn Ala Pro His Leu Leu Lys Asp Thr Lys Asn Lys Val Trp Lys Lys
   545                 550                 555                 560
   Asn Ile Ile Ser Lys Glu Asn Ile Asn Leu Leu Thr Asp Gly Met Gln
                   565                 570                 575
   Gln Val Val Asn Lys Thr His Lys Glu Asp Ile Tyr Arg Ser Tyr Ala
               580                 585                 590
   Asn Leu Ile Gly Lys Ser Gly Thr Ala Glu Leu Lys Met Lys Gln Gly
   595 600 605
   Glu Thr Gly Arg Gln Ile Gly Trp Phe Ile Ser Tyr Asp Lys Asp Asn
   610 615 620
   Pro Asn Met Met Met Ala Ile Asn Val Lys Asp Val Gln Asp Lys Gly
   625 630 635 640
   Met Ala Ser Tyr Asn Ala Lys Ile Ser Gly Lys Val Tyr Asp Glu Leu
   645 650 655
   Tyr Glu Asn Gly Asn Lys Lys Tyr Asp Ile Asp Glu
   660 665
<210> 10
   <211> 8
   <212> PRT
   <213> Staphylococcus aureus
<400> 10
   Ile Tyr Asn Ser Leu Gly Val Lys
   1 5
<210> 11
   <211> 7
   <212> PRT
   <213> Staphylococcus aureus
<400> 11
   Asp Ile Asn Ile Gln Asp Arg
   1 5
<210> 12
   <211> 10
   <212> PRT
   <213> Staphylococcus aureus
<400> 12
   Glu Leu Ser Ile Ser Glu Asp Tyr Ile Lys
   1 5 10
<210> 13
   <211> 12
   <212> PRT
   <213> Staphylococcus aureus
<400> 13
   Phe Gln Ile Thr Thr Ser Pro Gly Ser Thr Gln Lys
   1 5 10
<210> 14
   <211> 11
   <212> PRT
   <213> Staphylococcus aureus
<400> 14
   Ile Leu Thr Ala Met Ile Gly Leu Asn Asn Lys
   1               5                   10
<210> 15
   <211> 11
   <212> PRT
   <213> Staphylococcus aureus
<400> 15
   Tyr Glu Val Val Asn Gly Asn Ile Asp Leu Lys
   1               5                   10
<210> 16
   <211> 8
   <212> PRT
   <213> Staphylococcus aureus
<400> 16
   Val Ala Leu Glu Leu Gly Ser Lys
   1               5
<210> 17
   <211> 8
   <212> PRT
   <213> Staphylococcus aureus
<400> 17
   Ser Tyr Ala Asn Leu Ile Gly Lys
   1               5
<210> 18
   <211> 315
   <212> PRT
   <213> Staphylococcus aureus
<400> 18
   Met Ile Phe Met Val Lys Lys Arg Leu Leu Ala Ala Thr Leu Ser Leu
   1               5                   10                  15
   Gly Ile Ile Thr Pro Ile Ala Thr Ser Phe His Glu Ser Lys Ala Asp
               20                  25                  30
   Asn Asn Ile Glu Asn Ile Gly Asp Gly Ala Glu Val Val Lys Arg Thr
           35                  40                  45
   Glu Asp Thr Ser Ser Asp Lys Trp Gly Val Thr Gln Asn Ile Gln Val
       50                  55                  60
   Asp Phe Val Lys Asp Lys Lys Tyr Asn Lys Asp Ala Leu Ile Leu Lys
   65                  70                  75                  80
   Met Gln Gly Phe Ile Asn Ser Lys Thr Thr Tyr Tyr Asn Tyr Lys Asn
                   85                  90                  95
   Thr Asp His Ile Lys Ala Met Arg Trp Pro Phe Gln Tyr Asn Ile Gly
               100                 105                 110
   Leu Lys Thr Asn Asp Pro Asn Val Asp Leu Ile Asn Tyr Leu Pro Lys
           115                 120                 125
   Asn Lys Ile Asp Ser Val Asn Val Ser Gln Thr Leu Gly Tyr Asn Ile
       130                 135                 140
   Gly Gly Asn Phe Asn Ser Gly Pro Ser Thr Gly Gly Asn Gly Ser Phe
   145                 150                 155                 160
   Asn Tyr Ser Lys Thr Ile Ser Tyr Asn Gln Gln Asn Tyr Ile Ser Glu
                   165                 170                 175
   Val Glu Arg Gln Asn Ser Lys Ser Val Gln Trp Gly Ile Lys Ala Asn
               180                 185                 190
   Ser Phe Ile Thr Ser Leu Gly Lys Met Ser Gly His Asp Pro Asn Leu
           195                 200                 205
   Phe Val Gly Tyr Lys Pro Tyr Ser Gln Asn Pro Arg Asp Tyr Phe Val
       210                 215                 220
   Pro Asp Asn Glu Leu Pro Pro Leu Val His Ser Gly Phe Asn Pro Ser
   225                 230                 235                 240
   Phe Ile Ala Thr Val Ser His Glu Lys Gly Ser Gly Asp Thr Ser Glu
                   245                 250                 255
   Phe Glu Ile Thr Tyr Gly Arg Asn Met Asp Val Thr His Ala Thr Arg
               260                 265                 270
   Arg Thr Thr His Tyr Gly Asn Ser Tyr Leu Glu Gly Ser Arg Ile His
           275                 280                 285
   Asn Ala Phe Val Asn Arg Asn Tyr Thr Val Lys Tyr Glu Val Asn Trp
       290                 295                 300
   Lys Thr His Glu Ile Lys Val Lys Gly His Asn
   305                 310                 315
<210> 19
   <211> 14
   <212> PRT
   <213> Staphylococcus aureus
<400> 19
   Thr Asn Asp Pro Asn Val Asp Leu Ile Asn Tyr Leu Pro Lys
   1               5                   10
<210> 20
   <211> 7
   <212> PRT
   <213> Staphylococcus aureus
<400> 20
   Ser Val Gln Trp Gly Ile Lys
   1               5
<210> 21
   <211> 10
   <212> PRT
   <213> Staphylococcus aureus
<400> 21
   Ala Asn Ser Phe Ile Thr Ser Leu Gly Lys
   1               5                   10
<210> 22
   <211> 325
   <212> PRT
   <213> Staphylococcus aureus
<400> 22
   Met Lys Lys Ile Val Lys Ser Ser Val Val Thr Ser Ile Ala Leu Leu
   1               5                   10                  15
   Leu Leu Ser Asn Thr Val Asp Ala Ala Gln His Ile Thr Pro Val Ser
               20                  25                  30
   Glu Lys Lys Val Asp Asp Lys Ile Thr Leu Tyr Lys Thr Thr Ala Thr
           35                  40                  45
   Ser Asp Ser Asp Lys Leu Lys Ile Ser Gln Ile Leu Thr Phe Asn Phe
       50                  55                  60
   Ile Lys Asp Lys Ser Tyr Asp Lys Asp Thr Leu Ile Leu Lys Ala Ala
   65                  70                  75                  80
   Gly Asn Ile Tyr Ser Gly Tyr Thr Lys Pro Asn Pro Lys Asp Thr Ile
                   85                  90                  95
   Ser Ser Gln Phe Tyr Trp Gly Ser Lys Tyr Asn Ile Ser Ile Asn Ser
               100                 105                 110
   Asp Ser Asn Asp Ser Val Asn Val Val Asp Tyr Ala Pro Lys Asn Gln
           115                 120                 125
   Asn Glu Glu Phe Gln Val Gln Gln Thr Val Gly Tyr Ser Tyr Gly Gly
       130                 135                 140
   Asp Ile Asn Ile Ser Asn Gly Leu Ser Gly Gly Gly Asn Gly Ser Lys
   145                 150                 155                 160
   Ser Phe Ser Glu Thr Ile Asn Tyr Lys Gln Glu Ser Tyr Arg Thr Ser
                   165                 170                 175
   Leu Asp Lys Arg Thr Asn Phe Lys Lys Ile Gly Trp Asp Val Glu Ala
               180                 185                 190
   His Lys Ile Met Asn Asn Gly Trp Gly Pro Tyr Gly Arg Asp Ser Tyr
           195                 200                 205
   His Ser Thr Tyr Gly Asn Glu Met Phe Leu Gly Ser Arg Gln Ser Asn
       210                 215                 220
   Leu Asn Ala Gly Gln Asn Phe Leu Glu Tyr His Lys Met Pro Val Leu
   225                 230                 235                 240
   Ser Arg Gly Asn Phe Asn Pro Glu Phe Ile Gly Val Leu Ser Arg Lys
                   245                 250                 255
   Gln Asn Ala Ala Lys Lys Ser Lys Ile Thr Val Thr Tyr Gln Arg Glu
               260                 265                 270
   Met Asp Arg Tyr Thr Asn Phe Trp Asn Gln Leu His Trp Ile Gly Asn
           275                 280                 285
   Asn Tyr Lys Asp Glu Asn Arg Ala Thr His Thr Ser Ile Tyr Glu Val
       290                 295                 300
   Asp Trp Glu Asn His Thr Val Lys Leu Ile Asp Thr Gln Ser Lys Glu
   305                 310                 315                 320
   Lys Asn Pro Met Ser
                   325
<210> 23
   <211> 6
   <212> PRT
   <213> Staphylococcus aureus
<400> 23
   Met Pro Val Leu Ser Arg
   1               5
<210> 24
   <211> 13
   <212> PRT
   <213> Staphylococcus aureus
<400> 24
   Gly Asn Phe Asn Pro Glu Phe Ile Gly Val Leu Ser Arg
   1               5                   10
<210> 25
   <211> 59
   <212> PRT
   <213> Staphylococcus aureus
<400> 25
   Met Ala Lys Leu Gln Ile Thr Leu Thr Arg Ser Val Ile Gly Arg Pro
   1               5                   10                  15
   Glu Thr Gln Arg Lys Thr Val Glu Ala Leu Gly Leu Lys Lys Thr Asn
               20                  25                  30
   Ser Ser Val Val Val Glu Asp Asn Pro Ala Ile Arg Gly Gln Ile Asn
           35                  40                  45
   Lys Val Lys His Leu Val Thr Val Glu Glu Lys
       50                  55
<210> 26
   <211> 7
   <212> PRT
   <213> Staphylococcus aureus
<400> 26
   Leu Gln Ile Thr Leu Thr Arg
   1               5
<210> 27
   <211> 14
   <212> PRT
   <213> Staphylococcus aureus
<400> 27
   Thr Asn Ser Ser Val Val Val Glu Asp Asn Pro Ala Ile Arg
   1 5 10
<210> 28
   <211> 49
   <212> PRT
   <213> Staphylococcus aureus
<400> 28
   Met Arg Val Asn Val Thr Leu Ala Cys Thr Glu Cys Gly Asp Arg Asn
   1 5 10 15
   Tyr Ile Thr Thr Lys Asn Lys Arg Asn Asn Pro Glu Arg Val Glu Met
   20 25 30
   Lys Lys Phe Cys Ser Arg Glu Asn Lys Gln Thr Leu His Arg Glu Thr
   35 40 45
   Lys
<210> 29
   <211> 13
   <212> PRT
   <213> Staphylococcus aureus
<400> 29
   Val Asn Val Thr Leu Ala Cys Thr Glu Cys Gly Asp Arg
   1 5 10
<210> 30
   <211> 6
   <212> PRT
   <213> Staphylococcus aureus
<400> 30
   Asn Tyr Ile Thr Thr Lys
   1 5
<210> 31
   <211> 267
   <212> PRT
   <213> Staphylococcus aureus
<400> 31
   Met Lys Lys Ile Ala Thr Ala Thr Ile Ala Thr Ala Gly Phe Ala Thr
   1               5                   10                  15
   Ile Ala Ile Ala Ser Gly Asn Gln Ala His Ala Ser Glu Gln Asp Asn
               20                  25                  30
   Tyr Gly Tyr Asn Pro Asn Asp Pro Thr Ser Tyr Ser Tyr Thr Tyr Thr
           35                  40                  45
   Ile Asp Ala Gln Gly Asn Tyr His Tyr Thr Trp Lys Gly Asn Trp His
       50                  55                  60
   Pro Ser Gln Leu Asn Gln Asp Asn Gly Tyr Tyr Ser Tyr Tyr Tyr Tyr
   65                  70                  75                  80
   Asn Gly Tyr Asn Asn Tyr Asn Asn Tyr Asn Asn Gly Tyr Ser Tyr Asn
                   85                  90                  95
   Asn Tyr Ser Arg Tyr Asn Asn Tyr Ser Asn Asn Asn Gln Ser Tyr Asn
               100                 105                 110
   Tyr Asn Asn Tyr Asn Ser Tyr Asn Thr Asn Ser Tyr Arg Thr Gly Gly
           115                 120                 125
   Leu Gly Ala Ser Tyr Ser Thr Ser Ser Asn Asn Val Gln Val Thr Thr
       130                 135                 140
   Thr Met Ala Pro Ser Ser Asn Gly Arg Ser Ile Ser Ser Gly Tyr Thr
   145                 150                 155                 160
   Ser Gly Arg Asn Leu Tyr Thr Ser Gly Gln Cys Thr Tyr Tyr Val Phe
                   165                 170                 175
   Asp Arg Val Gly Gly Lys Ile Gly Ser Thr Trp Gly Asn Ala Ser Asn
               180                 185                 190
   Trp Ala Asn Ala Ala Ala Arg Ala Gly Tyr Thr Val Asn Asn Thr Pro
           195                 200                 205
   Lys Ala Gly Ala Ile Met Gln Thr Thr Gln Gly Ala Tyr Gly His Val
       210                 215                 220
   Ala Tyr Val Glu Ser Val Asn Ser Asn Gly Ser Val Arg Val Ser Glu
   225                 230                 235                 240
   Met Asn Tyr Gly Tyr Gly Pro Gly Val Val Thr Ser Arg Thr Ile Ser
                   245                 250                 255
   Ala Ser Gln Ala Ala Gly Tyr Asn Phe Ile His
               260                 265
<210> 32
   <211> 10
   <212> PRT
   <213> Staphylococcus aureus
<400> 32
   Ala Gly Tyr Thr Val Asn Asn Thr Pro Lys
   1               5                   10
<210> 33
   <211> 64
   <212> PRT
   <213> Staphylococcus aureus
<400> 33
   Met Ala Asp Glu Ser Lys Phe Glu Gln Ala Lys Gly Asn Val Lys Glu
   1               5                   10                  15
   Thr Val Gly Asn Val Thr Asp Asn Lys Asn Leu Glu Asn Glu Gly Lys
               20                  25                  30
   Glu Asp Lys Ala Ser Gly Lys Ala Lys Glu Phe Val Glu Asn Ala Lys
           35                  40                  45
   Glu Lys Ala Thr Asp Phe Ile Asp Lys Val Lys Gly Asn Lys Gly Glu
       50                  55                  60
<210> 34
   <211> 9
   <212> PRT
   <213> Staphylococcus aureus
<400> 34
   Glu Phe Val Glu Asn Ala Lys Glu Lys
   1               5
<210> 35
   <211> 9
   <212> PRT
   <213> Staphylococcus aureus
<400> 35
   Ala Thr Asp Phe Ile Asp Lys Val Lys
   1               5
<210> 36
   <211> 1248
   <212> PRT
   <213> Staphylococcus aureus
<400> 36
   Met Ala Lys Lys Phe Asn Tyr Lys Leu Pro Ser Met Val Ala Leu Thr
   1               5                   10                  15
   Leu Val Gly Ser Ala Val Thr Ala His Gln Val Gln Ala Ala Glu Thr
               20                  25                  30
   Thr Gln Asp Gln Thr Thr Asn Lys Asn Val Leu Asp Ser Asn Lys Val
           35                  40                  45
   Lys Ala Thr Thr Glu Gln Ala Lys Ala Glu Val Lys Asn Pro Thr Gln
       50                  55                  60
   Asn Ile Ser Gly Thr Gln Val Tyr Gln Asp Pro Ala Ile Val Gln Pro
   65                  70                  75                  80
   Lys Thr Ala Asn Asn Lys Thr Gly Asn Ala Gln Val Ser Gln Lys Val
                   85                  90                  95
   Asp Thr Ala Gln Val Asn Gly Asp Thr Arg Ala Asn Gln Ser Ala Thr
               100                 105                 110
   Thr Asn Asn Thr Gln Pro Val Ala Lys Ser Thr Ser Thr Thr Ala Pro
           115                 120                 125
   Lys Thr Asn Thr Asn Val Thr Asn Ala Gly Tyr Ser Leu Val Asp Asp
       130                 135                 140
   Glu Asp Asp Asn Ser Glu His Gln Ile Asn Pro Glu Leu Ile Lys Ser
   145                 150                 155                 160
   Ala Ala Lys Pro Ala Ala Leu Glu Thr Gln Tyr Lys Ala Ala Ala Pro
                   165                 170                 175
   Lys Ala Lys Thr Glu Ala Thr Pro Lys Val Thr Thr Phe Ser Ala Ser
               180                 185                 190
   Ala Gln Pro Arg Ser Val Ala Ala Thr Pro Lys Thr Ser Leu Pro Lys
           195                 200                 205
   Tyr Lys Pro Gln Val Asn Ser Ser Ile Asn Asp Tyr Ile Arg Lys Asn
       210                 215                 220
   Asn Leu Lys Ala Pro Lys Ile Glu Glu Asp Tyr Thr Ser Tyr Phe Pro
   225                 230                 235                 240
   Lys Tyr Ala Tyr Arg Asn Gly Val Gly Arg Pro Glu Gly Ile Val Val
                   245                 250                 255
   His Asp Thr Ala Asn Asp Arg Ser Thr Ile Asn Gly Glu Ile Ser Tyr
               260                 265                 270
   Met Lys Asn Asn Tyr Gln Asn Ala Phe Val His Ala Phe Val Asp Gly
           275                 280                 285
   Asp Arg Ile Ile Glu Thr Ala Pro Thr Asp Tyr Leu Ser Trp Gly Val
       290                 295                 300
   Gly Ala Val Gly Asn Pro Arg Phe Ile Asn Val Glu Ile Val His Thr
   305                 310                 315                 320
   His Asp Tyr Ala Ser Phe Ala Arg Ser Met Asn Asn Tyr Ala Asp Tyr
                   325                 330                 335
   Ala Ala Thr Gln Leu Gln Tyr Tyr Gly Leu Lys Pro Asp Ser Ala Glu
               340                 345                 350
   Tyr Asp Gly Asn Gly Thr Val Trp Thr His Tyr Ala Val Ser Lys Tyr
           355                 360                 365
   Leu Gly Gly Thr Asp His Ala Asp Pro His Gly Tyr Leu Arg Ser His
       370                 375                 380
   Asn Tyr Ser Tyr Asp Gln Leu Tyr Asp Leu Ile Asn Glu Lys Tyr Leu
   385                 390                 395                 400
   Ile Lys Met Gly Lys Val Ala Pro Trp Gly Thr Gln Phe Thr Thr Thr
                   405                 410                 415
   Pro Thr Thr Pro Ser Lys Pro Thr Thr Pro Ser Lys Pro Ser Thr Gly
               420                 425                 430
   Lys Leu Thr Val Ala Ala Asn Asn Gly Val Ala Gln Ile Lys Pro Thr
           435                 440                 445
   Asn Ser Gly Leu Tyr Thr Thr Val Tyr Asp Lys Thr Gly Lys Ala Thr
       450                 455                 460
   Asn Glu Val Gln Lys Thr Phe Ala Val Ser Lys Thr Ala Thr Leu Gly
   465                 470                 475                 480
   Asn Gln Lys Phe Tyr Leu Val Gln Asp Tyr Asn Ser Gly Asn Lys Phe
                   485                 490                 495
   Gly Trp Val Lys Glu Gly Asp Val Val Tyr Asn Thr Ala Lys Ser Pro
               500                 505                 510
   Val Asn Val Asn Gln Ser Tyr Ser Ile Lys Ser Gly Thr Lys Leu Tyr
           515                 520                 525
   Thr Val Pro Trp Gly Thr Ser Lys Gln Val Ala Gly Ser Val Ser Gly
       530                 535                 540
   Ser Gly Asn Gln Thr Phe Lys Ala Ser Lys Gln Gln Gln Ile Asp Lys
   545                 550                 555                 560
   Ser Ile Tyr Leu Tyr Gly Ser Val Asn Gly Lys Ser Gly Trp Val Ser
                   565                 570                 575
   Lys Ala Tyr Leu Val Asp Thr Ala Lys Pro Thr Pro Thr Pro Ile Pro
   580 585 590
   Lys Pro Ser Thr Pro Thr Thr Asn Asn Lys Leu Thr Val Ser Ser Leu
   595 600 605
   Asn Gly Val Ala Gln Ile Asn Ala Lys Asn Asn Gly Leu Phe Thr Thr
   610 615 620
   Val Tyr Asp Lys Thr Gly Lys Pro Thr Lys Glu Val Gln Lys Thr Phe
   625 630 635 640
   Ala Val Thr Lys Glu Ala Ser Leu Gly Gly Asn Lys Phe Tyr Leu Val
   645 650 655
   Lys Asp Tyr Asn Ser Pro Thr Leu Ile Gly Trp Val Lys Gln Gly Asp
   660 665 670
   Val Ile Tyr Asn Asn Ala Lys Ser Pro Val Asn Val Met Gln Thr Tyr
   675 680 685
   Thr Val Lys Pro Gly Thr Lys Leu Tyr Ser Val Pro Trp Gly Thr Tyr
   690 695 700
   Lys Gln Glu Ala Gly Ala Val Ser Gly Thr Gly Asn Gln Thr Phe Lys
   705 710 715 720
   Ala Thr Lys Gln Gln Gln Ile Asp Lys Ser Ile Tyr Leu Phe Gly Thr
   725 730 735
   Val Asn Gly Lys Ser Gly Trp Val Ser Lys Ala Tyr Leu Ala Val Pro
   740 745 750
   Ala Ala Pro Lys Lys Ala Val Ala Gln Pro Lys Thr Ala Val Lys Ala
   755 760 765
   Tyr Thr Val Thr Lys Pro Gln Thr Thr Gln Thr Val Ser Lys Ile Ala
   770 775 780
   Gln Val Lys Pro Asn Asn Thr Gly Ile Arg Ala Ser Val Tyr Glu Lys
   785 790 795 800
   Thr Ala Lys Asn Gly Ala Lys Tyr Ala Asp Arg Thr Phe Tyr Val Thr
   805 810 815
   Lys Glu Arg Ala His Gly Asn Glu Thr Tyr Val Leu Leu Asn Asn Thr
   820 825 830
   Ser His Asn Ile Pro Leu Gly Trp Phe Asn Val Lys Asp Leu Asn Val
   835 840 845
   Gln Asn Leu Gly Lys Glu Val Lys Thr Thr Gln Lys Tyr Thr Val Asn
       850                 855                 860
   Lys Ser Asn Asn Gly Leu Ser Met Val Pro Trp Gly Thr Lys Asn Gln
   865                 870                 875                 880
   Val Ile Leu Thr Gly Asn Asn Ile Ala Gln Gly Thr Phe Asn Ala Thr
                   885                 890                 895
   Lys Gln Val Ser Val Gly Lys Asp Val Tyr Leu Tyr Gly Thr Ile Asn
               900                 905                 910
   Asn Arg Thr Gly Trp Val Asn Ala Lys Asp Leu Thr Ala Pro Thr Ala
           915                 920                 925
   Val Lys Pro Thr Thr Ser Ala Ala Lys Asp Tyr Asn Tyr Thr Tyr Val
       930                 935                 940
   Ile Lys Asn Gly Asn Gly Tyr Tyr Tyr Val Thr Pro Asn Ser Asp Thr
   945                 950                 955                 960
   Ala Lys Tyr Ser Leu Lys Ala Phe Asn Glu Gln Pro Phe Ala Val Val
                   965                 970                 975
   Lys Glu Gln Val Ile Asn Gly Gln Thr Trp Tyr Tyr Gly Lys Leu Ser
               980                 985                 990
   Asn Gly Lys Leu Ala Trp Ile Lys  Ser Thr Asp Leu Ala  Lys Glu Leu
           995                 1000                 1005
   Ile Lys  Tyr Asn Gln Thr Gly  Met Thr Leu Asn Gln  Val Ala Gln
       1010                 1015                 1020
   Ile Gln  Ala Gly Leu Gln Tyr  Lys Pro Gln Val Gln  Arg Val Pro
       1025                 1030                 1035
   Gly Lys  Trp Thr Asp Ala Asn  Phe Asn Asp Val Lys  His Ala Met
       1040                 1045                 1050
   Asp Thr  Lys Arg Leu Ala Gln  Asp Pro Ala Leu Lys  Tyr Gln Phe
       1055                 1060                 1065
   Leu Arg  Leu Asp Gln Pro Gln  Asn Ile Ser Ile Asp  Lys Ile Asn
       1070                 1075                 1080
   Gln Phe  Leu Lys Gly Lys Gly  Val Leu Glu Asn Gln  Gly Ala Ala
       1085                 1090                 1095
   Phe Asn Lys Ala Ala Gln Met Tyr Gly Ile Asn Glu Val Tyr Leu
   1100 1105 1110
   Ile Ser His Ala Leu Leu Glu Thr Gly Asn Gly Thr Ser Gln Leu
   1115 1120 1125
   Ala Lys Gly Ala Asp Val Val Asn Asn Lys Val Val Thr Asn Ser
   1130 1135 1140
   Asn Thr Lys Tyr His Asn Val Phe Gly Ile Ala Ala Tyr Asp Asn
   1145 1150 1155
   Asp Pro Leu Arg Glu Gly Ile Lys Tyr Ala Lys Gln Ala Gly Trp
   1160 1165 1170
   Asp Thr Val Ser Lys Ala Ile Val Gly Gly Ala Lys Phe Ile Gly
   1175 1180 1185
   Asn Ser Tyr Val Lys Ala Gly Gln Asn Thr Leu Tyr Lys Met Arg
   1190 1195 1200
   Trp Asn Pro Ala His Pro Gly Thr His Gln Tyr Ala Thr Asp Val
   1205 1210 1215
   Asp Trp Ala Asn Ile Asn Ala Lys Ile Ile Lys Gly Tyr Tyr Asp
   1220 1225 1230
   Lys Ile Gly Glu Val Gly Lys Tyr Phe Asp Ile Pro Gln Tyr Lys
   1235 1240 1245
<210> 37
   <211> 10
   <212> PRT
   <213> Staphylococcus aureus
<400> 37
   Leu Tyr Ser Val Pro Trp Gly Thr Tyr Lys
   1 5 10
<210> 38
   <211> 10
   <212> PRT
   <213> Staphylococcus aureus
<400> 38
   Ala Tyr Leu Ala Val Pro Ala Ala Pro Lys
   1 5 10
<210> 39
   <211> 394
   <212> PRT
   <213> Staphylococcus aureus
<400> 39
   Met Ala Lys Glu Lys Phe Asp Arg Ser Lys Glu His Ala Asn Ile Gly
   1               5                   10                  15
   Thr Ile Gly His Val Asp His Gly Lys Thr Thr Leu Thr Ala Ala Ile
               20                  25                  30
   Ala Thr Val Leu Ala Lys Asn Gly Asp Ser Val Ala Gln Ser Tyr Asp
           35                  40                  45
   Met Ile Asp Asn Ala Pro Glu Glu Lys Glu Arg Gly Ile Thr Ile Asn
       50                  55                  60
   Thr Ser His Ile Glu Tyr Gln Thr Asp Lys Arg His Tyr Ala His Val
   65                  70                  75                  80
   Asp Cys Pro Gly His Ala Asp Tyr Val Lys Asn Met Ile Thr Gly Ala
                   85                  90                  95
   Ala Gln Met Asp Gly Gly Ile Leu Val Val Ser Ala Ala Asp Gly Pro
               100                 105                 110
   Met Pro Gln Thr Arg Glu His Ile Leu Leu Ser Arg Asn Val Gly Val
           115                 120                 125
   Pro Ala Leu Val Val Phe Leu Asn Lys Val Asp Met Val Asp Asp Glu
       130                 135                 140
   Glu Leu Leu Glu Leu Val Glu Met Glu Val Arg Asp Leu Leu Ser Glu
   145                 150                 155                 160
   Tyr Asp Phe Pro Gly Asp Asp Val Pro Val Ile Ala Gly Ser Ala Leu
                   165                 170                 175
   Lys Ala Leu Glu Gly Asp Ala Gln Tyr Glu Glu Lys Ile Leu Glu Leu
               180                 185                 190
   Met Glu Ala Val Asp Thr Tyr Ile Pro Thr Pro Glu Arg Asp Ser Asp
           195                 200                 205
   Lys Pro Phe Met Met Pro Val Glu Asp Val Phe Ser Ile Thr Gly Arg
       210                 215                 220
   Gly Thr Val Ala Thr Gly Arg Val Glu Arg Gly Gln Ile Lys Val Gly
   225                 230                 235                 240
   Glu Glu Val Glu Ile Ile Gly Leu His Asp Thr Ser Lys Thr Thr Val
   245 250 255
   Thr Gly Val Glu Met Phe Arg Lys Leu Leu Asp Tyr Ala Glu Ala Gly
   260 265 270
   Asp Asn Ile Gly Ala Leu Leu Arg Gly Val Ala Arg Glu Asp Val Gln
   275 280 285
   Arg Gly Gln Val Leu Ala Ala Pro Gly Ser Ile Thr Pro His Thr Glu
   290 295 300
   Phe Lys Ala Glu Val Tyr Val Leu Ser Lys Asp Glu Gly Gly Arg His
   305 310 315 320
   Thr Pro Phe Phe Ser Asn Tyr Arg Pro Gln Phe Tyr Phe Arg Thr Thr
   325 330 335
   Asp Val Thr Gly Val Val His Leu Pro Glu Gly Thr Glu Met Val Met
   340 345 350
   Pro Gly Asp Asn Val Glu Met Thr Val Glu Leu Ile Ala Pro Ile Ala
   355 360 365
   Ile Glu Asp Gly Thr Arg Phe Ser Ile Arg Glu Gly Gly Arg Thr Val
   370 375 380
   Gly Ser Gly Val Val Thr Glu Ile Ile Lys
   385 390
<210> 40
   <211> 12
   <212> PRT
   <213> Staphylococcus aureus
<400> 40
   Thr Val Gly Ser Gly Val Val Thr Glu Ile Ile Lys
   1 5 10
<210> 41
   <211> 233
   <212> PRT
   <213> Staphylococcus aureus
<400> 41
   Met Lys Lys Thr Ile Met Ala Ser Ser Leu Ala Val Ala Leu Gly Val
   1               5                   10                  15
   Thr Gly Tyr Ala Ala Gly Thr Gly His Gln Ala His Ala Ala Glu Val
               20                  25                  30
   Asn Val Asp Gln Ala His Leu Val Asp Leu Ala His Asn His Gln Asp
           35                  40                  45
   Gln Leu Asn Ala Ala Pro Ile Lys Asp Gly Ala Tyr Asp Ile His Phe
       50                  55                  60
   Val Lys Asp Gly Phe Gln Tyr Asn Phe Thr Ser Asn Gly Thr Thr Trp
   65                  70                  75                  80
   Ser Trp Ser Tyr Glu Ala Ala Asn Gly Gln Thr Ala Gly Phe Ser Asn
                   85                  90                  95
   Val Ala Gly Ala Asp Tyr Thr Thr Ser Tyr Asn Gln Gly Ser Asn Val
               100                 105                 110
   Gln Ser Val Ser Tyr Asn Ala Gln Ser Ser Asn Ser Asn Val Glu Ala
           115                 120                 125
   Val Ser Ala Pro Thr Tyr His Asn Tyr Ser Thr Ser Thr Thr Ser Ser
       130                 135                 140
   Ser Val Arg Leu Ser Asn Gly Asn Thr Ala Gly Ala Thr Gly Ser Ser
   145                 150                 155                 160
   Ala Ala Gln Ile Met Ala Gln Arg Thr Gly Val Ser Ala Ser Thr Trp
                   165                 170                 175
   Ala Ala Ile Ile Ala Arg Glu Ser Asn Gly Gln Val Asn Ala Tyr Asn
               180                 185                 190
   Pro Ser Gly Ala Ser Gly Leu Phe Gln Thr Met Pro Gly Trp Gly Pro
           195                 200                 205
   Thr Asn Thr Val Asp Gln Gln Ile Asn Ala Ala Val Lys Ala Tyr Lys
       210                 215                 220
   Ala Gln Gly Leu Gly Ala Trp Gly Phe
   225                 230
<210> 42
   <211> 21
   <212> PRT
   <213> Staphylococcus aureus
<400> 42
   Leu Ser Asn Gly Asn Thr Ala Gly Ala Thr Gly Ser Ser Ala Ala Gln
   1               5                   10                  15
   Ile Met Ala Gln Arg
<210> 43
   <211> 86
   <212> PRT
   <213> Staphylococcus aureus
<400> 43
   Met Ala Met Thr Val Lys Lys Asp Asn Asn Glu Val Arg Ile Gln Trp
   1               5                   10                  15
   Arg Val Ala Asp Ile Lys Ile Pro Thr Ser Glu Ile Lys Asn Ile Thr
               20                  25                  30
   Gln Asp Gln Asp Ile His Ala Val Pro Lys Leu Asp Ser Lys Asp Val
           35                  40                  45
   Ser Arg Ile Gly Ser Thr Phe Gly Lys Thr Asn Arg Val Ile Ile Asp
       50                  55                  60
   Thr Glu Asp His Glu Tyr Ile Ile Tyr Thr Gln Asn Asp Gln Lys Val
   65                  70                  75                  80
   Tyr Asn Glu Leu Thr Lys
                   85
<210> 44
   <211> 13
   <212> PRT
   <213> Staphylococcus aureus
<400> 44
   Asn Ile Thr Gln Asp Gln Asp Ile His Ala Val Pro Lys
   1               5                   10
<210> 45
   <211> 8
   <212> PRT
   <213> Staphylococcus aureus
<400> 45
   Leu Asp Ser Lys Asp Val Ser Arg
   1               5
<210> 46
   <211> 11
   <212> PRT
   <213> Escherichia coli
<400> 46
   Ile Leu Glu Ile Glu Gly Leu Pro Asp Leu Lys
   1 5 10
<210> 47
   <211> 13
   <212> PRT
   <213> Escherichia coli
<400> 47
   Val Ala Asp Gly Ala Thr Val Val Ser Thr Ser Thr Arg
   1 5 10
<210> 48
   <211> 10
   <212> PRT
   <213> Escherichia coli
<400> 48
   Thr Asn Thr Thr Asp Val Ala Thr Phe Lys
   1 5 10
<210> 49
   <211> 9
   <212> PRT
   <213> Escherichia coli
<400> 49
   Leu Asp Thr Thr Gly Leu Ile Asp Arg
   1 5
<210> 50
   <211> 13
   <212> PRT
   <213> Escherichia coli
<400> 50
   Val Gly Leu Ile Ala Gly Ser Gly Gly Gly Ser Pro Arg
   1 5 10
<210> 51
   <211> 10
   <212> PRT
   <213> Escherichia coli
<400> 51
   Ala Ile Asp Phe Ser Asp Gly Tyr Tyr Lys
   1 5 10
<210> 52
   <211> 10
   <212> PRT
   <213> Escherichia coli
<400> 52
   Leu Gly Ala Trp Asp Thr Leu Ser Pro Lys
   1 5 10
<210> 53
   <211> 11
   <212> PRT
   <213> Escherichia coli
<400> 53
   Phe Gly Glu Ile Glu Glu Val Glu Leu Gly Arg
   1 5 10
<210> 54
   <211> 11
   <212> PRT
   <213> Escherichia coli
<400> 54
   Ile Asn Leu Leu Asp Asp Asn Gln Phe Thr Arg
   1 5 10
<210> 55
   <211> 10
   <212> PRT
   <213> Escherichia coli
<400> 55
   Leu Ala Thr Ala Trp Glu Gly Phe Thr Lys
   1 5 10
<210> 56
   <211> 12
   <212> PRT
   <213> Escherichia coli
<400> 56
   Asp Ser Ile Leu Glu Ala Ile Asp Ala Gly Ile Lys
   1 5 10
<210> 57
   <211> 10
   <212> PRT
   <213> Escherichia coli
<400> 57
   Phe Ala Ala Leu Glu Ala Ala Gly Val Lys
   1               5                   10
<210> 58
   <211> 10
   <212> PRT
   <213> Escherichia coli
<400> 58
   Ser Leu Ala Asp Ile Gly Glu Ala Leu Lys
   1               5                   10
<210> 59
   <211> 10
   <212> PRT
   <213> Escherichia coli
<400> 59
   Thr Glu Glu Gln Leu Ala Asn Ile Ala Arg
   1               5                   10
<210> 60
   <211> 13
   <212> PRT
   <213> Escherichia coli
<400> 60
   Ala Glu Ala Glu Gln Thr Leu Ala Ala Leu Thr Glu Lys
   1               5                   10
<210> 61
   <211> 10
   <212> PRT
   <213> Escherichia coli
<400> 61
   Ser Gly Asp Thr Leu Ser Ala Ile Ser Lys
   1               5                   10
<210> 62
   <211> 12
   <212> PRT
   <213> Escherichia coli
<400> 62
   Leu Leu Thr Gly Glu Leu Leu Thr Leu Ala Ser Arg
   1               5                   10
<210> 63
   <211> 12
   <212> PRT
   <213> Escherichia coli
<400> 63
   Ser Ala Leu Pro Ala Gly Trp Phe Ile Ala Asp Lys
   1               5                   10
<210> 64
   <211> 7
   <212> PRT
   <213> Escherichia coli
<400> 64
   Val Ala Gly Pro Leu Leu Arg
   1               5
<210> 65
   <211> 12
   <212> PRT
   <213> Escherichia coli
<400> 65
   Val Gly Tyr Ile Glu Leu Asp Leu Asn Ser Gly Lys
   1               5                   10
<210> 66
   <211> 10
   <212> PRT
   <213> Escherichia coli
<400> 66
   Val Leu Leu Cys Gly Ala Val Leu Ser Arg
   1               5                   10
<210> 67
   <211> 9
   <212> PRT
   <213> Escherichia coli
<400> 67
   Ile Ser Asp Ile Pro Glu Phe Val Arg
   1               5
<210> 68
   <211> 9
   <212> PRT
   <213> Escherichia coli
<400> 68
   Ile Glu Glu Asp Leu Leu Gly Thr Arg
   1 5
<210> 69
   <211> 9
   <212> PRT
   <213> Escherichia coli
<400> 69
   Leu Val Asp Ala Ile Asn Gln Leu Arg
   1 5
<210> 70
   <211> 12
   <212> PRT
   <213> Escherichia coli
<400> 70
   Thr Ala Leu Ala Ile Asp Ala Ile Ile Asn Gln Arg
   1 5 10
<210> 71
   <211> 12
   <212> PRT
   <213> Escherichia coli
<400> 71
   Val Val Asn Thr Leu Gly Ala Pro Ile Asp Gly Lys
   1 5 10
<210> 72
   <211> 14
   <212> PRT
   <213> Escherichia coli
<400> 72
   Ser Ala Gly Gly Ile Val Leu Thr Gly Ser Ala Ala Ala Lys
   1 5 10
<210> 73
   <211> 10
   <212> PRT
   <213> Escherichia coli
<400> 73
   Ala Val Thr Ala Ala Val Glu Glu Leu Lys
   1 5 10
<210> 74
   <211> 14
   <212> PRT
   <213> Escherichia coli
<400> 74
   Ala Leu Asp Ala Ile Ile Ala Ser Val Thr Glu Ser Leu Lys
   1               5                   10
<210> 75
   <211> 7
   <212> PRT
   <213> Escherichia coli
<400> 75
   Tyr Trp Asp Val Glu Leu Arg
   1               5
<210> 76
   <211> 9
   <212> PRT
   <213> Escherichia coli
<400> 76
   Leu Pro Gly Ile Leu Glu Leu Ser Arg
   1               5
<210> 77
   <211> 10
   <212> PRT
   <213> Escherichia coli
<400> 77
   Ser Lys Ala Thr Asn Leu Leu Tyr Thr Arg
   1               5                   10
<210> 78
   <211> 11
   <212> PRT
   <213> Escherichia coli
<400> 78
   Ser Glu Ala Leu Lys Ile Leu Asn Asn Ile Arg
   1               5                   10
<210> 79
   <211> 11
   <212> PRT
   <213> Escherichia coli
<400> 79
   Leu Phe Gly Val Thr Thr Leu Asp Ile Ile Arg
   1               5                   10
<210> 80
   <211> 11
   <212> PRT
   <213> Escherichia coli
<400> 80
   Ala Ser Leu Pro Thr Ile Glu Leu Ala Leu Lys
   1 5 10
<210> 81
   <211> 10
   <212> PRT
   <213> Escherichia coli
<400> 81
   Leu Leu Ser Asp Thr Glu Cys Leu Val Lys
   1 5 10
<210> 82
   <211> 8
   <212> PRT
   <213> Escherichia coli
<400> 82
   Ser Ile Leu Ser Glu Leu Val Arg
   1 5
<210> 83
   <211> 11
   <212> PRT
   <213> Escherichia coli
<400> 83
   Gly Gly Phe Thr Val Glu Leu Asn Gly Ile Arg
   1 5 10
<210> 84
   <211> 13
   <212> PRT
   <213> Escherichia coli
<400> 84
   Thr Gly Glu Val Pro Ala Asp Val Ala Ala Gln Ala Arg
   1 5 10
<210> 85
   <211> 10
   <212> PRT
   <213> Escherichia coli
<400> 85
   Thr Tyr Val Asp Ser Leu Asn Val Ile Arg
   1 5 10
<210> 86
   <211> 10
   <212> PRT
   <213> Escherichia coli
<400> 86
   Phe Val Thr Val Thr Ala Glu Ala Leu Arg
   1               5                   10
<210> 87
   <211> 9
   <212> PRT
   <213> Escherichia coli
<400> 87
   Ile Ser Asn Val Leu Pro Glu Tyr Arg
   1               5
<210> 88
   <211> 8
   <212> PRT
   <213> Candida albicans
<400> 88
   Ala Asp Glu Glu Phe Phe Ala Lys
   1               5
<210> 89
   <211> 9
   <212> PRT
   <213> Candida albicans
<400> 89
   Ala Phe Asp Asp Glu Ser Val Gln Lys
   1               5
<210> 90
   <211> 10
   <212> PRT
   <213> Candida albicans
<400> 90
   Ala Lys Ile Asp Val Val Asp Gln Ala Lys
   1               5                   10
<210> 91
   <211> 11
   <212> PRT
   <213> Candida albicans
<400> 91
   Ala Val Glu Val Pro Glu Gln Thr Ala Tyr Arg
   1               5                   10
<210> 92
   <211> 7
   <212> PRT
   <213> Candida albicans
<400> 92
   Cys Gly Val Ile Gln Pro Arg
   1               5
<210> 93
   <211> 12
   <212> PRT
   <213> Candida albicans
<400> 93
   Asp Leu Gln Glu Leu Ile Ala Glu Gly Asn Thr Lys
   1               5                   10
<210> 94
   <211> 9
   <212> PRT
   <213> Candida albicans
<400> 94
   Phe Ala Asp Glu Asn Phe Val Lys Arg
   1               5
<210> 95
   <211> 8
   <212> PRT
   <213> Candida albicans
<400> 95
   Phe Ala Leu Asp Thr Gly Val Lys
   1               5
<210> 96
   <211> 11
   <212> PRT
   <213> Candida albicans
<400> 96
   Gly Arg Leu Pro Glu Val Pro Val Ile Val Lys
   1               5                   10
<210> 97
   <211> 11
   <212> PRT
   <213> Candida albicans
<400> 97
   Ile Ala Glu Ala Leu Asp Ile Phe His Thr Lys
   1 5 10
<210> 98
   <211> 8
   <212> PRT
   <213> Candida albicans
<400> 98
   Ile Asp Val Val Asp Gln Ala Lys
   1 5
<210> 99
   <211> 18
   <212> PRT
   <213> Candida albicans
<400> 99
   Ile Glu Glu Glu Leu Gly Ser Glu Ala Ile Tyr Ala Gly Lys Asp Phe
   1 5 10 15
   Gln Lys
<210> 100
   <211> 13
   <212> PRT
   <213> Candida albicans
<400> 100
   Ile Glu Ser Phe Gly Ser Gly Ser Gly Ala Thr Ser Lys
   1 5 10
<210> 101
   <211> 8
   <212> PRT
   <213> Candida albicans
<400> 101
   Leu Ala Ser Leu Pro Phe Gln Lys
   1 5
<210> 102
   <211> 11
   <212> PRT
   <213> Candida albicans
<400> 102
   Leu Asp Ala Thr Asp Asp Val Val Ala Val Lys
   1 5 10
<210> 103
   <211> 8
   <212> PRT
   <213> Candida albicans
<400> 103
   Asn Val Glu His Leu Val Glu Lys
   1 5
<210> 104
   <211> 9
   <212> PRT
   <213> Candida albicans
<400> 104
   Ser Gly Ile Ala Ala Gly Val Asn Lys
   1 5
<210> 105
   <211> 15
   <212> PRT
   <213> Candida albicans
<400> 105
   Ser Gly Val Asp Tyr Val Ile Glu Ser Thr Gly Val Phe Thr Lys
   1 5 10 15
<210> 106
   <211> 9
   <212> PRT
   <213> Candida albicans
<400> 106
   Ser Leu Asn Glu Phe Leu Ala Asp Lys
   1 5
<210> 107
   <211> 11
   <212> PRT
   <213> Candida albicans
<400> 107
   Ser Gln Glu Thr Phe Asp Ala Asn Val Ala Arg
   1 5 10
<210> 108
   <211> 8
   <212> PRT
   <213> Candida albicans
<400> 108
   Ser Ser Ser Ser Thr Thr Lys Lys
   1 5
<210> 109
   <211> 8
   <212> PRT
   <213> Candida albicans
<400> 109
   Ser Thr Leu Asp Pro Val Gly Lys
   1 5
<210> 110
   <211> 11
   <212> PRT
   <213> Candida albicans
<400> 110
   Ser Val Glu Leu Phe Gln Gln Ala Val Ala Lys
   1 5 10
<210> 111
   <211> 9
   <212> PRT
   <213> Candida albicans
<400> 111
   Thr Ala Asn Asp Val Leu Glu Leu Arg
   1 5
<210> 112
   <211> 19
   <212> PRT
   <213> Candida albicans
<400> 112 Pro Thr Arg
<210> 113
   <211> 10
   <212> PRT
   <213> Candida albicans
<400> 113
   Val Val Ala Ile Asp Gly Gly Asp Glu Lys
   1 5 10
<210> 114
   <211> 9
   <212> PRT
   <213> Candida albicans
<400> 114
   Trp Thr Asp Asn Leu Leu Pro Ala Arg
   1 5
<210> 115
   <211> 18
   <212> PRT
   <213> Candida albicans
<400> 115
   Tyr Lys Gly Glu Val Thr Ala Ser Gly Asp Asp Leu Val Ile Asp Gly
   1 5 10 15
   His Lys
<210> 116
   <211> 7
   <212> PRT
   <213> Candida albicans
<400> 116
   Tyr Val Leu Asp Thr Ser Lys
   1 5
<210> 117
   <211> 10
   <212> PRT
   <213> Candida albicans
<400> 117
   Ala Val Ile Tyr Asp Cys Pro Asn Ser Arg
   1 5 10
<210> 118
   <211> 16
   <212> PRT
   <213> Candida albicans
<400> 118
   Gly His Tyr Val Leu Val Ser Pro Gly Tyr Ala His Thr Ser Glu Arg
   1 5 10 15
<210> 119
   <211> 10
   <212> PRT
   <213> Candida albicans
<400> 119
   Gly Val Ile Tyr Asp Cys Pro Asn Ser Arg
   1 5 10
<210> 120
   <211> 13
   <212> PRT
   <213> Candida albicans
<400> 120
   Gly Val Ser Ser Pro Tyr Leu Pro Phe Gly Gly Gly Arg
   1               5                   10
<210> 121
   <211> 13
   <212> PRT
   <213> Candida albicans
<400> 121
   Gly Val Ser Ser Pro Tyr Leu Pro Phe Ser Gly Gly Lys
   1               5                   10
<210> 122
   <211> 13
   <212> PRT
   <213> Candida albicans
<400> 122
   Gly Val Ser Ser Pro Tyr Leu Pro Phe Ser Gly Gly Arg
   1               5                   10
<210> 123
   <211> 10
   <212> PRT
   <213> Candida albicans
<400> 123
   Ala Ala Val Gly Asp Ile Leu Gln Ser Arg
   1               5                   10
<210> 124
   <211> 7
   <212> PRT
   <213> Candida albicans
<400> 124
   Ile Thr Pro Asp Asp Leu Arg
   1               5
<210> 125
   <211> 7
   <212> PRT
   <213> Candida albicans
<400> 125
   Thr Gly Trp Asp Ile Leu Lys
   1               5
<210> 126
   <211> 263
   <212> PRT
   <213> Escherichia coli
<400> 126
   His Pro Glu Thr Leu Val Lys Val Lys Asp Ala Glu Asp Lys Leu Gly
   1               5                   10                  15
   Ala Arg Val Gly Tyr Ile Glu Leu Asp Leu Asn Ser Gly Lys Ile Leu
               20                  25                  30
   Glu Ser Phe Arg Pro Glu Glu Arg Phe Pro Met Met Ser Thr Phe Lys
           35                  40                  45
   Val Leu Leu Cys Gly Ala Val Leu Ser Arg Val Asp Ala Gly Gln Glu
       50                  55                  60
   Gln Leu Gly Arg Arg Ile His Tyr Ser Gln Asn Asp Leu Val Glu Tyr
   65                  70                  75                  80
   Ser Pro Val Thr Glu Lys His Leu Thr Asp Gly Met Thr Val Arg Glu
                   85                  90                  95
   Leu Cys Ser Ala Ala Ile Thr Met Ser Asp Asn Thr Ala Ala Asn Leu
               100                 105                 110
   Leu Leu Thr Thr Ile Gly Gly Pro Lys Glu Leu Thr Ala Phe Leu His
           115                 120                 125
   Asn Met Gly Asp His Val Thr Arg Leu Asp Arg Trp Glu Pro Glu Leu
       130                 135                 140
   Asn Glu Ala Ile Pro Asn Asp Glu Arg Asp Thr Thr Met Pro Ala Ala
   145                 150                 155                 160
   Met Ala Thr Thr Leu Arg Lys Leu Leu Thr Gly Glu Leu Leu Thr Leu
                   165                 170                 175
   Ala Ser Arg Gln Gln Leu Ile Asp Trp Met Glu Ala Asp Lys Val Ala
               180                 185                 190
   Gly Pro Leu Leu Arg Ser Ala Leu Pro Ala Gly Trp Phe Ile Ala Asp
           195                 200                 205
   Lys Ser Gly Val Gly Glu Arg Gly Ser Arg Gly Ile Ile Ala Ala Leu
       210                 215                 220
   Gly Pro Asp Gly Lys Pro Ser Arg Ile Val Val Ile Tyr Thr Thr Gly
   225                 230                 235                 240
   Ser Gln Ala Thr Met Asp Glu Arg Asn Arg Gln Ile Ala Glu Ile Gly
                   245                 250                 255
   Ala Ser Leu Ile Lys His Trp
               260
<210> 127
   <211> 865
   <212> PRT
   <213> Escherichia coli
<400> 127
   Met Leu Glu Glu Tyr Arg Lys His Val Ala Glu Arg Ala Ala Glu Gly
   1               5                   10                  15
   Ile Ala Pro Lys Pro Leu Asp Ala Asn Gln Met Ala Ala Leu Val Glu
               20                  25                  30
   Leu Leu Lys Asn Pro Pro Ala Gly Glu Glu Glu Phe Leu Leu Asp Leu
           35                  40                  45
   Leu Thr Asn Arg Val Pro Pro Gly Val Asp Glu Ala Ala Tyr Val Lys
       50                  55                  60
   Ala Gly Phe Leu Ala Ala Ile Ala Lys Gly Glu Ala Lys Ser Pro Leu
   65                  70                  75                  80
   Leu Thr Pro Glu Lys Ala Ile Glu Leu Leu Gly Thr Met Gln Gly Gly
                   85                  90                  95
   Tyr Asn Ile His Pro Leu Ile Asp Ala Leu Asp Asp Ala Lys Leu Ala
               100                 105                 110
   Pro Ile Ala Ala Lys Ala Leu Ser His Thr Leu Leu Met Phe Asp Asn
           115                 120                 125
   Phe Tyr Asp Val Glu Glu Lys Ala Lys Ala Gly Asn Glu Tyr Ala Lys
       130                 135                 140
   Gln Val Met Gln Ser Trp Ala Asp Ala Glu Trp Phe Leu Asn Arg Pro
   145                 150                 155                 160
   Ala Leu Ala Glu Lys Leu Thr Val Thr Val Phe Lys Val Thr Gly Glu
                   165                 170                 175
   Thr Asn Thr Asp Asp Leu Ser Pro Ala Pro Asp Ala Trp Ser Arg Pro
               180                 185                 190
   Asp Ile Pro Leu His Ala Leu Ala Met Leu Lys Asn Ala Arg Glu Gly
           195                 200                 205
   Ile Glu Pro Asp Gln Pro Gly Val Val Gly Pro Ile Lys Gln Ile Glu
       210                 215                 220
   Ala Leu Gln Gln Lys Gly Phe Pro Leu Ala Tyr Val Gly Asp Val Val
   225                 230                 235                 240
   Gly Thr Gly Ser Ser Arg Lys Ser Ala Thr Asn Ser Val Leu Trp Phe
                   245                 250                 255
   Met Gly Asp Asp Ile Pro His Val Pro Asn Lys Arg Gly Gly Gly Leu
               260                 265                 270
   Cys Leu Gly Gly Lys Ile Ala Pro Ile Phe Phe Asn Thr Met Glu Asp
           275                 280                 285
   Ala Gly Ala Leu Pro Ile Glu Val Asp Val Ser Asn Leu Asn Met Gly
       290                 295                 300
   Asp Val Ile Asp Val Tyr Pro Tyr Lys Gly Glu Val Arg Asn His Glu
   305 310 315 320
   Thr Gly Glu Leu Leu Ala Thr Phe Glu Leu Lys Thr Asp Val Leu Ile
   325 330 335
   Asp Glu Val Arg Ala Gly Gly Arg Ile Pro Leu Ile Ile Gly Arg Gly
   340 345 350
   Leu Thr Thr Lys Ala Arg Glu Ala Leu Gly Leu Pro His Ser Asp Val
   355 360 365
   Phe Arg Gln Ala Lys Asp Val Ala Glu Ser Asp Arg Gly Phe Ser Leu
   370 375 380
   Ala Gln Lys Met Val Gly Arg Ala Cys Gly Val Lys Gly Ile Arg Pro
   385 390 395 400
   Gly Ala Tyr Cys Glu Pro Lys Met Thr Ser Val Gly Ser Gln Asp Thr
   405 410 415
   Thr Gly Pro Met Thr Arg Asp Glu Leu Lys Asp Leu Ala Cys Leu Gly
   420 425 430
   Phe Ser Ala Asp Leu Val Met Gln Ser Phe Cys His Thr Ala Ala Tyr
   435 440 445
   Pro Lys Pro Val Asp Val Asn Thr His His Thr Leu Pro Asp Phe Ile
   450 455 460
   Met Asn Arg Gly Gly Val Ser Leu Arg Pro Gly Asp Gly Val Ile His
   465 470 475 480
   Ser Trp Leu Asn Arg Met Leu Leu Pro Asp Thr Val Gly Thr Gly Gly
   485 490 495
   Asp Ser His Thr Arg Phe Pro Ile Gly Ile Ser Phe Pro Ala Gly Ser
   500 505 510
   Gly Leu Val Ala Phe Ala Ala Ala Thr Gly Val Met Pro Leu Asp Met
   515 520 525
   Pro Glu Ser Val Leu Val Arg Phe Lys Gly Lys Met Gln Pro Gly Ile
   530 535 540
   Thr Leu Arg Asp Leu Val His Ala Ile Pro Leu Tyr Ala Ile Lys Gln
   545 550 555 560
   Gly Leu Leu Thr Val Glu Lys Lys Gly Lys Lys Asn Ile Phe Ser Gly
                   565                 570                 575
   Arg Ile Leu Glu Ile Glu Gly Leu Pro Asp Leu Lys Val Glu Gln Ala
               580                 585                 590
   Phe Glu Leu Thr Asp Ala Ser Ala Glu Arg Ser Ala Ala Gly Cys Thr
           595                 600                 605
   Ile Lys Leu Asn Lys Glu Pro Ile Ile Glu Tyr Leu Asn Ser Asn Ile
       610                 615                 620
   Val Leu Leu Lys Trp Met Ile Ala Glu Gly Tyr Gly Asp Arg Arg Thr
   625                 630                 635                 640
   Leu Glu Arg Arg Ile Gln Gly Met Glu Lys Trp Leu Ala Asn Pro Glu
                   645                 650                 655
   Leu Leu Glu Ala Asp Ala Asp Ala Glu Tyr Ala Ala Val Ile Asp Ile
               660                 665                 670
   Asp Leu Ala Asp Ile Lys Glu Pro Ile Leu Cys Ala Pro Asn Asp Pro
           675                 680                 685
   Asp Asp Ala Arg Pro Leu Ser Ala Val Gln Gly Glu Lys Ile Asp Glu
       690                 695                 700
   Val Phe Ile Gly Ser Cys Met Thr Asn Ile Gly His Phe Arg Ala Ala
   705                 710                 715                 720
   Gly Lys Leu Leu Asp Ala His Lys Gly Gln Leu Pro Thr Arg Leu Trp
                   725                 730                 735
   Val Ala Pro Pro Thr Arg Met Asp Ala Ala Gln Leu Thr Glu Glu Gly
               740                 745                 750
   Tyr Tyr Ser Val Phe Gly Lys Ser Gly Ala Arg Ile Glu Ile Pro Gly
           755                 760                 765
   Cys Ser Leu Cys Met Gly Asn Gln Ala Arg Val Ala Asp Gly Ala Thr
       770                 775                 780
   Val Val Ser Thr Ser Thr Arg Asn Phe Pro Asn Arg Leu Gly Thr Gly
   785                 790                 795                 800
   Ala Asn Val Phe Leu Ala Ser Ala Glu Leu Ala Ala Val Ala Ala Leu
                   805                 810                 815
   Ile Gly Lys Leu Pro Thr Pro Glu Glu Tyr Gln Thr Tyr Val Ala Gln
   820 825 830
   Val Asp Lys Thr Ala Val Asp Thr Tyr Arg Tyr Leu Asn Phe Asn Gln
   835 840 845
   Leu Ser Gln Tyr Thr Glu Lys Ala Asp Gly Val Ile Phe Gln Thr Ala
   850 855 860
   Val
   865
<210> 128
   <211> 348
   <212> PRT
   <213> Escherichia coli
<400> 128
   Met Glu Phe Phe Lys Lys Thr Ala Leu Ala Ala Leu Val Met Gly Phe
   1               5                   10                  15
   Ser Gly Ala Ala Leu Ala Leu Pro Asn Ile Thr Ile Leu Ala Thr Gly
               20                  25                  30
   Gly Thr Ile Ala Gly Gly Gly Asp Ser Ala Thr Lys Ser Asn Tyr Thr
           35                  40                  45
   Val Gly Lys Val Gly Val Glu Asn Leu Val Asn Ala Val Pro Gln Leu
       50                  55                  60
   Lys Asp Ile Ala Asn Val Lys Gly Glu Gln Val Val Asn Ile Gly Ser
   65                  70                  75                  80
   Gln Asp Met Asn Asp Asn Val Trp Leu Thr Leu Ala Lys Lys Ile Asn
                   85                  90                  95
   Thr Asp Cys Asp Lys Thr Asp Gly Phe Val Ile Thr His Gly Thr Asp
               100                 105                 110
   Thr Met Glu Glu Thr Ala Tyr Phe Leu Asp Leu Thr Val Lys Cys Asp
           115                 120                 125
   Lys Pro Val Val Met Val Gly Ala Met Arg Pro Ser Thr Ser Met Ser
       130                 135                 140
   Ala Asp Gly Pro Phe Asn Leu Tyr Asn Ala Val Val Thr Ala Ala Asp
   145                 150                 155                 160
   Lys Ala Ser Ala Asn Arg Gly Val Leu Val Val Met Asn Asp Thr Val
                   165                 170                 175
   Leu Asp Gly Arg Asp Val Thr Lys Thr Asn Thr Thr Asp Val Ala Thr
               180                 185                 190
   Phe Lys Ser Val Asn Tyr Gly Pro Leu Gly Tyr Ile His Asn Gly Lys
           195                 200                 205
   Ile Asp Tyr Gln Arg Thr Pro Ala Arg Lys His Thr Ser Asp Thr Pro
       210                 215                 220
   Phe Asp Val Ser Lys Leu Asn Glu Leu Pro Lys Val Gly Ile Val Tyr
   225                 230                 235                 240
   Asn Tyr Ala Asn Ala Ser Asp Leu Pro Ala Lys Ala Leu Val Asp Ala
                   245                 250                 255
   Gly Tyr Asp Gly Ile Val Ser Ala Gly Val Gly Asn Gly Asn Leu Tyr
               260                 265                 270
   Lys Ser Val Phe Asp Thr Leu Ala Thr Ala Ala Lys Thr Gly Thr Ala
           275                 280                 285
   Val Val Arg Ser Ser Arg Val Pro Thr Gly Ala Thr Thr Gln Asp Ala
       290                 295                 300
   Glu Val Asp Asp Ala Lys Tyr Gly Phe Val Ala Ser Gly Thr Leu Asn
   305                 310                 315                 320
   Pro Gln Lys Ala Arg Val Leu Leu Gln Leu Ala Leu Thr Gln Thr Lys
                   325                 330                 335
   Asp Pro Gln Gln Ile Gln Gln Ile Phe Asn Gln Tyr
               340                 345
<210> 129
   <211> 406
   <212> PRT
   <213> Escherichia coli
<400> 129
   Met Lys Arg Ala Val Ile Thr Gly Leu Gly Ile Val Ser Ser Ile Gly
   1               5                   10                  15
   Asn Asn Gln Gln Glu Val Leu Ala Ser Leu Arg Glu Gly Arg Ser Gly
               20                  25                  30
   Ile Thr Phe Ser Gln Glu Leu Lys Asp Ser Gly Met Arg Ser His Val
           35                  40                  45
   Trp Gly Asn Val Lys Leu Asp Thr Thr Gly Leu Ile Asp Arg Lys Val
       50                  55                  60
   Val Arg Phe Met Ser Asp Ala Ser Ile Tyr Ala Phe Leu Ser Met Glu
   65                  70                  75                  80
   Gln Ala Ile Ala Asp Ala Gly Leu Ser Pro Glu Ala Tyr Gln Asn Asn
                   85                  90                  95
   Pro Arg Val Gly Leu Ile Ala Gly Ser Gly Gly Gly Ser Pro Arg Phe
               100                 105                 110
   Gln Val Phe Gly Ala Asp Ala Met Arg Gly Pro Arg Gly Leu Lys Ala
           115                 120                 125
   Val Gly Pro Tyr Val Val Thr Lys Ala Met Ala Ser Gly Val Ser Ala
       130                 135                 140
   Cys Leu Ala Thr Pro Phe Lys Ile His Gly Val Asn Tyr Ser Ile Ser
   145                 150                 155                 160
   Ser Ala Cys Ala Thr Ser Ala His Cys Ile Gly Asn Ala Val Glu Gln
                   165                 170                 175
   Ile Gln Leu Gly Lys Gln Asp Ile Val Phe Ala Gly Gly Gly Glu Glu
               180                 185                 190
   Leu Cys Trp Glu Met Ala Cys Glu Phe Asp Ala Met Gly Ala Leu Ser
           195                 200                 205
   Thr Lys Tyr Asn Asp Thr Pro Glu Lys Ala Ser Arg Thr Tyr Asp Ala
       210                 215                 220
   His Arg Asp Gly Phe Val Ile Ala Gly Gly Gly Gly Met Val Val Val
   225                 230                 235                 240
   Glu Glu Leu Glu His Ala Leu Ala Arg Gly Ala His Ile Tyr Ala Glu
                   245                 250                 255
   Ile Val Gly Tyr Gly Ala Thr Ser Asp Gly Ala Asp Met Val Ala Pro
               260                 265                 270
   Ser Gly Glu Gly Ala Val Arg Cys Met Lys Met Ala Met His Gly Val
           275                 280                 285
   Asp Thr Pro Ile Asp Tyr Leu Asn Ser His Gly Thr Ser Thr Pro Val
       290                 295                 300
   Gly Asp Val Lys Glu Leu Ala Ala Ile Arg Glu Val Phe Gly Asp Lys
   305                 310                 315                 320
   Ser Pro Ala Ile Ser Ala Thr Lys Ala Met Thr Gly His Ser Leu Gly
   325 330 335
   Ala Ala Gly Val Gln Glu Ala Ile Tyr Ser Leu Leu Met Leu Glu His
   340 345 350
   Gly Phe Ile Ala Pro Ser Ile Asn Ile Glu Glu Leu Asp Glu Gln Ala
   355 360 365
   Ala Gly Leu Asn Ile Val Thr Glu Thr Thr Asp Arg Glu Leu Thr Thr
   370 375 380
   Val Met Ser Asn Ser Phe Gly Phe Gly Gly Thr Asn Ala Thr Leu Val
   385 390 395 400
   Met Arg Lys Leu Lys Asp
   405
<210> 130
   <211> 248
   <212> PRT
   <213> Escherichia coli
<400> 130
   Met Lys Ser Val Leu Lys Val Ser Leu Ala Ala Leu Thr Leu Ala Phe
   1 5 10 15
   Ala Val Ser Ser His Ala Ala Asp Lys Lys Leu Val Val Ala Thr Asp
   20 25 30
   Thr Ala Phe Val Pro Phe Glu Phe Lys Gln Gly Asp Lys Tyr Val Gly
   35 40 45
   Phe Asp Val Asp Leu Trp Ala Ala Ile Ala Lys Glu Leu Lys Leu Asp
   50 55 60
   Tyr Glu Leu Lys Pro Met Asp Phe Ser Gly Ile Ile Pro Ala Leu Gln
   65 70 75 80
   Thr Lys Asn Val Asp Leu Ala Leu Ala Gly Ile Thr Ile Thr Asp Glu
   85 90 95
   Arg Lys Lys Ala Ile Asp Phe Ser Asp Gly Tyr Tyr Lys Ser Gly Leu
   100 105 110
   Leu Val Met Val Lys Ala Asn Asn Asn Asp Val Lys Ser Val Lys Asp
   115 120 125
   Leu Asp Gly Lys Val Val Ala Val Lys Ser Gly Thr Gly Ser Val Asp
       130                 135                 140
   Tyr Ala Lys Ala Asn Ile Lys Thr Lys Asp Leu Arg Gln Phe Pro Asn
   145                 150                 155                 160
   Ile Asp Asn Ala Tyr Met Glu Leu Gly Thr Asn Arg Ala Asp Ala Val
                   165                 170                 175
   Leu His Asp Thr Pro Asn Ile Leu Tyr Phe Ile Lys Thr Ala Gly Asn
               180                 185                 190
   Gly Gln Phe Lys Ala Val Gly Asp Ser Leu Glu Ala Gln Gln Tyr Gly
           195                 200                 205
   Ile Ala Phe Pro Lys Gly Ser Asp Glu Leu Arg Asp Lys Val Asn Gly
       210                 215                 220
   Ala Leu Lys Thr Leu Arg Glu Asn Gly Thr Tyr Asn Glu Ile Tyr Lys
   225                 230                 235                 240
   Lys Trp Phe Gly Thr Glu Pro Lys
                   245
<210> 131
   <211> 257
   <212> PRT
   <213> Escherichia coli
<400> 131
   Met Ala Arg Lys Trp Leu Asn Leu Phe Ala Gly Ala Ala Leu Ser Phe
   1               5                   10                  15
   Ala Val Ala Gly Asn Ala Leu Ala Asp Glu Gly Lys Ile Thr Val Phe
               20                  25                  30
   Ala Ala Ala Ser Leu Thr Asn Ala Met Gln Asp Ile Ala Thr Gln Phe
           35                  40                  45
   Lys Lys Glu Lys Gly Val Asp Val Val Ser Ser Phe Ala Ser Ser Ser
       50                  55                  60
   Thr Leu Ala Arg Gln Ile Glu Ala Gly Ala Pro Ala Asp Leu Phe Ile
   65                  70                  75                  80
   Ser Ala Asp Gln Lys Trp Met Asp Tyr Ala Val Asp Lys Lys Ala Ile
                   85                  90                  95
   Asp Thr Ala Thr Arg Gln Thr Leu Leu Gly Asn Ser Leu Val Val Val
               100                 105                 110
   Ala Pro Lys Ala Ser Val Gln Lys Asp Phe Thr Ile Asp Ser Lys Thr
   115 120 125
   Asn Trp Thr Ser Leu Leu Asn Gly Gly Arg Leu Ala Val Gly Asp Pro
   130 135 140
   Glu His Val Pro Ala Gly Ile Tyr Ala Lys Glu Ala Leu Gln Lys Leu
   145 150 155 160
   Gly Ala Trp Asp Thr Leu Ser Pro Lys Leu Ala Pro Ala Glu Asp Val
   165 170 175
   Arg Gly Ala Leu Ala Leu Val Glu Arg Asn Glu Ala Pro Leu Gly Ile
   180 185 190
   Val Tyr Gly Ser Asp Ala Val Ala Ser Lys Gly Val Lys Val Val Ala
   195 200 205
   Thr Phe Pro Glu Asp Ser His Lys Lys Val Glu Tyr Pro Val Ala Val
   210 215 220
   Val Glu Gly His Asn Asn Ala Thr Val Lys Ala Phe Tyr Asp Tyr Leu
   225 230 235 240
   Lys Gly Pro Gln Ala Ala Glu Ile Phe Lys Arg Tyr Gly Phe Thr Ile
   245 250 255
   Lys
<210> 132
   <211> 630
   <212> PRT
   <213> Escherichia coli
<400> 132
   Met Ala Ile Glu Ile Lys Val Pro Asp Ile Gly Ala Asp Glu Val Glu
   1 5 10 15
   Ile Thr Glu Ile Leu Val Lys Val Gly Asp Lys Val Glu Ala Glu Gln
   20 25 30
   Ser Leu Ile Thr Val Glu Gly Asp Lys Ala Ser Met Glu Val Pro Ser
   35 40 45
   Pro Gln Ala Gly Ile Val Lys Glu Ile Lys Val Ser Val Gly Asp Lys
   50 55 60
   Thr Gln Thr Gly Ala Leu Ile Met Ile Phe Asp Ser Ala Asp Gly Ala
   65 70 75 80
   Ala Asp Ala Ala Pro Ala Gln Ala Glu Glu Lys Lys Glu Ala Ala Pro
   85 90 95
   Ala Ala Ala Pro Ala Ala Ala Ala Ala Lys Asp Val Asn Val Pro Asp
   100 105 110
   Ile Gly Ser Asp Glu Val Glu Val Thr Glu Ile Leu Val Lys Val Gly
   115 120 125
   Asp Lys Val Glu Ala Glu Gln Ser Leu Ile Thr Val Glu Gly Asp Lys
   130 135 140
   Ala Ser Met Glu Val Pro Ala Pro Phe Ala Gly Thr Val Lys Glu Ile
   145 150 155 160
   Lys Val Asn Val Gly Asp Lys Val Ser Thr Gly Ser Leu Ile Met Val
   165 170 175
   Phe Glu Val Ala Gly Glu Ala Gly Ala Ala Ala Pro Ala Ala Lys Gln
   180 185 190
   Glu Ala Ala Pro Ala Ala Ala Pro Ala Pro Ala Ala Gly Val Lys Glu
   195 200 205
   Val Asn Val Pro Asp Ile Gly Gly Asp Glu Val Glu Val Thr Glu Val
   210 215 220
   Met Val Lys Val Gly Asp Lys Val Ala Ala Glu Gln Ser Leu Ile Thr
   225 230 235 240
   Val Glu Gly Asp Lys Ala Ser Met Glu Val Pro Ala Pro Phe Ala Gly
   245 250 255
   Val Val Lys Glu Leu Lys Val Asn Val Gly Asp Lys Val Lys Thr Gly
   260 265 270
   Ser Leu Ile Met Ile Phe Glu Val Glu Gly Ala Ala Pro Ala Ala Ala
   275 280 285
   Pro Ala Lys Gln Glu Ala Ala Ala Pro Ala Pro Ala Ala Lys Ala Glu
   290 295 300
   Ala Pro Ala Ala Ala Pro Ala Ala Lys Ala Glu Gly Lys Ser Glu Phe
   305 310 315 320
   Ala Glu Asn Asp Ala Tyr Val His Ala Thr Pro Leu Ile Arg Arg Leu
   325 330 335
   Ala Arg Glu Phe Gly Val Asn Leu Ala Lys Val Lys Gly Thr Gly Arg
   340 345 350
   Lys Gly Arg Ile Leu Arg Glu Asp Val Gln Ala Tyr Val Lys Glu Ala
   355 360 365
   Ile Lys Arg Ala Glu Ala Ala Pro Ala Ala Thr Gly Gly Gly Ile Pro
   370 375 380
   Gly Met Leu Pro Trp Pro Lys Val Asp Phe Ser Lys Phe Gly Glu Ile
   385 390 395 400
   Glu Glu Val Glu Leu Gly Arg Ile Gln Lys Ile Ser Gly Ala Asn Leu
   405 410 415
   Ser Arg Asn Trp Val Met Ile Pro His Val Thr His Phe Asp Lys Thr
   420 425 430
   Asp Ile Thr Glu Leu Glu Ala Phe Arg Lys Gln Gln Asn Glu Glu Ala
   435 440 445
   Ala Lys Arg Lys Leu Asp Val Lys Ile Thr Pro Val Val Phe Ile Met
   450 455 460
   Lys Ala Val Ala Ala Ala Leu Glu Gln Met Pro Arg Phe Asn Ser Ser
   465 470 475 480
   Leu Ser Glu Asp Gly Gln Arg Leu Thr Leu Lys Lys Tyr Ile Asn Ile
   485 490 495
   Gly Val Ala Val Asp Thr Pro Asn Gly Leu Val Val Pro Val Phe Lys
   500 505 510
   Asp Val Asn Lys Lys Gly Ile Ile Glu Leu Ser Arg Glu Leu Met Thr
   515 520 525
   Ile Ser Lys Lys Ala Arg Asp Gly Lys Leu Thr Ala Gly Glu Met Gln
   530 535 540
   Gly Gly Cys Phe Thr Ile Ser Ser Ile Gly Gly Leu Gly Thr Thr His
   545 550 555 560
   Phe Ala Pro Ile Val Asn Ala Pro Glu Val Ala Ile Leu Gly Val Ser
   565 570 575
   Lys Ser Ala Met Glu Pro Val Trp Asn Gly Lys Glu Phe Val Pro Arg
   580 585 590
   Leu Met Leu Pro Ile Ser Leu Ser Phe Asp His Arg Val Ile Asp Gly
   595 600 605
   Ala Asp Gly Ala Arg Phe Ile Thr Ile Ile Asn Asn Thr Leu Ser Asp
   610 615 620
   Ile Arg Arg Leu Val Met
   625 630
<210> 133
   <211> 367
   <212> PRT
   <213> Escherichia coli
<400> 133
   Met Lys Val Lys Val Leu Ser Leu Leu Val Pro Ala Leu Leu Val Ala
   1 5 10 15
   Gly Ala Ala Asn Ala Ala Glu Val Tyr Asn Lys Asp Gly Asn Lys Leu
   20 25 30
   Asp Leu Tyr Gly Lys Val Asp Gly Leu His Tyr Phe Ser Asp Asp Lys
   35 40 45
   Ser Val Asp Gly Asp Gln Thr Tyr Met Arg Leu Gly Phe Lys Gly Glu
   50 55 60
   Thr Gln Val Thr Asp Gln Leu Thr Gly Tyr Gly Gln Trp Glu Tyr Gln
   65 70 75 80
   Ile Gln Gly Asn Ser Ala Glu Asn Glu Asn Asn Ser Trp Thr Arg Val
   85 90 95
   Ala Phe Ala Gly Leu Lys Phe Gln Asp Val Gly Ser Phe Asp Tyr Gly
   100 105 110
   Arg Asn Tyr Gly Val Val Tyr Asp Val Thr Ser Trp Thr Asp Val Leu
   115 120 125
   Pro Glu Phe Gly Gly Asp Thr Tyr Gly Ser Asp Asn Phe Met Gln Gln
   130 135 140
   Arg Gly Asn Gly Phe Ala Thr Tyr Arg Asn Thr Asp Phe Phe Gly Leu
   145 150 155 160
   Val Asp Gly Leu Asn Phe Ala Val Gln Tyr Gln Gly Lys Asn Gly Ser
   165 170 175
   Val Ser Gly Glu Gly Met Thr Asn Asn Gly Arg Glu Ala Leu Arg Gln
   180 185 190
   Asn Gly Asp Gly Val Gly Gly Ser Ile Thr Tyr Asp Tyr Glu Gly Phe
   195 200 205
   Gly Ile Gly Ala Ala Val Ser Ser Ser Lys Arg Thr Asp Asp Gln Asn
   210 215 220
   Ser Pro Leu Tyr Ile Gly Asn Gly Asp Arg Ala Glu Thr Tyr Thr Gly
   225 230 235 240
   Gly Leu Lys Tyr Asp Ala Asn Asn Ile Tyr Leu Ala Ala Gln Tyr Thr
   245 250 255
   Gln Thr Tyr Asn Ala Thr Arg Val Gly Ser Leu Gly Trp Ala Asn Lys
   260 265 270
   Ala Gln Asn Phe Glu Ala Val Ala Gln Tyr Gln Phe Asp Phe Gly Leu
   275 280 285
   Arg Pro Ser Leu Ala Tyr Leu Gln Ser Lys Gly Lys Asn Leu Gly Val
   290 295 300
   Ile Asn Gly Arg Asn Tyr Asp Asp Glu Asp Ile Leu Lys Tyr Val Asp
   305 310 315 320
   Val Gly Ala Thr Tyr Tyr Phe Asn Lys Asn Met Ser Thr Tyr Val Asp
   325 330 335
   Tyr Lys Ile Asn Leu Leu Asp Asp Asn Gln Phe Thr Arg Asp Ala Gly
   340 345 350
   Ile Asn Thr Asp Asn Ile Val Ala Leu Gly Leu Val Tyr Gln Phe
   355 360 365
<210> 134
   <211> 760
   <212> PRT
   <213> Escherichia coli
<400> 134
   Met Ser Glu Leu Asn Glu Lys Leu Ala Thr Ala Trp Glu Gly Phe Thr
   1               5                   10                  15
   Lys Gly Asp Trp Gln Asn Glu Val Asn Val Arg Asp Phe Ile Gln Lys
               20                  25                  30
   Asn Tyr Thr Pro Tyr Glu Gly Asp Glu Ser Phe Leu Ala Gly Ala Thr
           35                  40                  45
   Glu Ala Thr Thr Thr Leu Trp Asp Lys Val Met Glu Gly Val Lys Leu
       50                  55                  60
   Glu Asn Arg Thr His Ala Pro Val Asp Phe Asp Thr Ala Val Ala Ser
   65                  70                  75                  80
   Thr Ile Thr Ser His Asp Ala Gly Tyr Ile Asn Lys Gln Leu Glu Lys
                   85                  90                  95
   Ile Val Gly Leu Gln Thr Glu Ala Pro Leu Lys Arg Ala Leu Ile Pro
               100                 105                 110
   Phe Gly Gly Ile Lys Met Ile Glu Gly Ser Cys Lys Ala Tyr Asn Arg
           115                 120                 125
   Glu Leu Asp Pro Met Ile Lys Lys Ile Phe Thr Glu Tyr Arg Lys Thr
       130                 135                 140
   His Asn Gln Gly Val Phe Asp Val Tyr Thr Pro Asp Ile Leu Arg Cys
   145                 150                 155                 160
   Arg Lys Ser Gly Val Leu Thr Gly Leu Pro Asp Ala Tyr Gly Arg Gly
                   165                 170                 175
   Arg Ile Ile Gly Asp Tyr Arg Arg Val Ala Leu Tyr Gly Ile Asp Tyr
               180                 185                 190
   Leu Met Lys Asp Lys Leu Ala Gln Phe Thr Ser Leu Gln Ala Asp Leu
           195                 200                 205
   Glu Asn Gly Val Asn Leu Glu Gln Thr Ile Arg Leu Arg Glu Glu Ile
       210                 215                 220
   Ala Glu Gln His Arg Ala Leu Gly Gln Met Lys Glu Met Ala Ala Lys
   225                 230                 235                 240
   Tyr Gly Tyr Asp Ile Ser Gly Pro Ala Thr Asn Ala Gln Glu Ala Ile
                   245                 250                 255
   Gln Trp Thr Tyr Phe Gly Tyr Leu Ala Ala Val Lys Ser Gln Asn Gly
               260                 265                 270
   Ala Ala Met Ser Phe Gly Arg Thr Ser Thr Phe Leu Asp Val Tyr Ile
           275                 280                 285
   Glu Arg Asp Leu Lys Ala Gly Lys Ile Thr Glu Gln Glu Ala Gln Glu
       290                 295                 300
   Met Val Asp His Leu Val Met Lys Leu Arg Met Val Arg Phe Leu Arg
   305                 310                 315                 320
   Thr Pro Glu Tyr Asp Glu Leu Phe Ser Gly Asp Pro Ile Trp Ala Thr
                   325                 330                 335
   Glu Ser Ile Gly Gly Met Gly Leu Asp Gly Arg Thr Leu Val Thr Lys
               340                 345                 350
   Asn Ser Phe Arg Phe Leu Asn Thr Leu Tyr Thr Met Gly Pro Ser Pro
           355                 360                 365
   Glu Pro Asn Met Thr Ile Leu Trp Ser Glu Lys Leu Pro Leu Asn Phe
       370                 375                 380
   Lys Lys Phe Ala Ala Lys Val Ser Ile Asp Thr Ser Ser Leu Gln Tyr
   385                 390                 395                 400
   Glu Asn Asp Asp Leu Met Arg Pro Asp Phe Asn Asn Asp Asp Tyr Ala
                   405                 410                 415
   Ile Ala Cys Cys Val Ser Pro Met Ile Val Gly Lys Gln Met Gln Phe
               420                 425                 430
   Phe Gly Ala Arg Ala Asn Leu Ala Lys Thr Met Leu Tyr Ala Ile Asn
           435                 440                 445
   Gly Gly Val Asp Glu Lys Leu Lys Met Gln Val Gly Pro Lys Ser Glu
       450                 455                 460
   Pro Ile Lys Gly Asp Val Leu Asn Tyr Asp Glu Val Met Glu Arg Met
   465                 470                 475                 480
   Asp His Phe Met Asp Trp Leu Ala Lys Gln Tyr Ile Thr Ala Leu Asn
                   485                 490                 495
   Ile Ile His Tyr Met His Asp Lys Tyr Ser Tyr Glu Ala Ser Leu Met
               500                 505                 510
   Ala Leu His Asp Arg Asp Val Ile Arg Thr Met Ala Cys Gly Ile Ala
           515                 520                 525
   Gly Leu Ser Val Ala Ala Asp Ser Leu Ser Ala Ile Lys Tyr Ala Lys
       530                 535                 540
   Val Lys Pro Ile Arg Asp Glu Asp Gly Leu Ala Ile Asp Phe Glu Ile
   545                 550                 555                 560
   Glu Gly Glu Tyr Pro Gln Phe Gly Asn Asn Asp Pro Arg Val Asp Asp
                   565                 570                 575
   Leu Ala Val Asp Leu Val Glu Arg Phe Met Lys Lys Ile Gln Lys Leu
               580                 585                 590
   His Thr Tyr Arg Asp Ala Ile Pro Thr Gln Ser Val Leu Thr Ile Thr
           595                 600                 605
   Ser Asn Val Val Tyr Gly Lys Lys Thr Gly Asn Thr Pro Asp Gly Arg
       610                 615                 620
   Arg Ala Gly Ala Pro Phe Gly Pro Gly Ala Asn Pro Met His Gly Arg
   625                 630                 635                 640
   Asp Gln Lys Gly Ala Val Ala Ser Leu Thr Ser Val Ala Lys Leu Pro
                   645                 650                 655
   Phe Ala Tyr Ala Lys Asp Gly Ile Ser Tyr Thr Phe Ser Ile Val Pro
               660                 665                 670
   Asn Ala Leu Gly Lys Asp Asp Glu Val Arg Lys Thr Asn Leu Ala Gly
           675                 680                 685
   Leu Met Asp Gly Tyr Phe His His Glu Ala Ser Ile Glu Gly Gly Gln
       690                 695                 700
   His Leu Asn Val Asn Val Met Asn Arg Glu Met Leu Leu Asp Ala Met
   705                 710                 715                 720
   Glu Asn Pro Glu Lys Tyr Pro Gln Leu Thr Ile Arg Val Ser Gly Tyr
                   725                 730                 735
   Ala Val Arg Phe Asn Ser Leu Thr Lys Glu Gln Gln Gln Asp Val Ile
               740                 745                 750
   Thr Arg Thr Phe Thr Gln Ser Met
           755                 760
<210> 135
   <211> 289
   <212> PRT
   <213> Escherichia coli
<400> 135
   Met Ser Ile Leu Ile Asp Lys Asn Thr Lys Val Ile Cys Gln Gly Phe
   1               5                   10                  15
   Thr Gly Ser Gln Gly Thr Phe His Ser Glu Gln Ala Ile Ala Tyr Gly
               20                  25                  30
   Thr Lys Met Val Gly Gly Val Thr Pro Gly Lys Gly Gly Thr Thr His
           35                  40                  45
   Leu Gly Leu Pro Val Phe Asn Thr Val Arg Glu Ala Val Ala Ala Thr
       50                  55                  60
   Gly Ala Thr Ala Ser Val Ile Tyr Val Pro Ala Pro Phe Cys Lys Asp
   65                  70                  75                  80
   Ser Ile Leu Glu Ala Ile Asp Ala Gly Ile Lys Leu Ile Ile Thr Ile
                   85                  90                  95
   Thr Glu Gly Ile Pro Thr Leu Asp Met Leu Thr Val Lys Val Lys Leu
               100                 105                 110
   Asp Glu Ala Gly Val Arg Met Ile Gly Pro Asn Cys Pro Gly Val Ile
           115                 120                 125
   Thr Pro Gly Glu Cys Lys Ile Gly Ile Gln Pro Gly His Ile His Lys
       130                 135                 140
   Pro Gly Lys Val Gly Ile Val Ser Arg Ser Gly Thr Leu Thr Tyr Glu
   145                 150                 155                 160
   Ala Val Lys Gln Thr Thr Asp Tyr Gly Phe Gly Gln Ser Thr Cys Val
                   165                 170                 175
   Gly Ile Gly Gly Asp Pro Ile Pro Gly Ser Asn Phe Ile Asp Ile Leu
               180                 185                 190
   Glu Met Phe Glu Lys Asp Pro Gln Thr Glu Ala Ile Val Met Ile Gly
           195                 200                 205
   Glu Ile Gly Gly Ser Ala Glu Glu Glu Ala Ala Ala Tyr Ile Lys Glu
       210                 215                 220
   His Val Thr Lys Pro Val Val Gly Tyr Ile Ala Gly Val Thr Ala Pro
   225                 230                 235                 240
   Lys Gly Lys Arg Met Gly His Ala Gly Ala Ile Ile Ala Gly Gly Lys
                   245                 250                 255
   Gly Thr Ala Asp Glu Lys Phe Ala Ala Leu Glu Ala Ala Gly Val Lys
               260                 265                 270
   Thr Val Arg Ser Leu Ala Asp Ile Gly Glu Ala Leu Lys Thr Val Leu
           275                 280                 285
   Lys
<210> 136
   <211> 94
   <212> PRT
   <213> Escherichia coli
<400> 136
   Met Ser Lys Cys Ser Ala Asp Glu Thr Pro Val Cys Cys Cys Met Asp
   1               5                   10                  15
   Val Gly Thr Ile Met Asp Asn Ser Asp Cys Thr Ala Ser Tyr Ser Arg
               20                  25                  30
   Val Phe Ala Asn Arg Ala Glu Ala Glu Gln Thr Leu Ala Ala Leu Thr
           35                  40                  45
   Glu Lys Ala Arg Ser Val Glu Ser Glu Pro Cys Lys Ile Thr Pro Thr
       50                  55                  60
   Phe Thr Glu Glu Ser Asp Gly Val Arg Leu Asp Ile Asp Phe Thr Phe
   65                  70                  75                  80
   Ala Cys Glu Ala Glu Met Leu Ile Phe Gln Leu Gly Leu Arg
                   85                  90
<210> 137
   <211> 149
   <212> PRT
   <213> Escherichia coli
<400> 137
   Met Gly Leu Phe Asn Phe Val Lys Asp Ala Gly Glu Lys Leu Trp Asp
   1               5                   10                  15
   Ala Val Thr Gly Gln His Asp Lys Asp Asp Gln Ala Lys Lys Val Gln
               20                  25                  30
   Glu His Leu Asn Lys Thr Gly Ile Pro Asp Ala Asp Lys Val Asn Ile
           35                  40                  45
   Gln Ile Ala Asp Gly Lys Ala Thr Val Thr Gly Asp Gly Leu Ser Gln
       50                  55                  60
   Glu Ala Lys Glu Lys Ile Leu Val Ala Val Gly Asn Ile Ser Gly Ile
   65                  70                  75                  80
   Ala Ser Val Asp Asp Gln Val Lys Thr Ala Thr Pro Ala Thr Ala Ser
                   85                  90                  95
   Gln Phe Tyr Thr Val Lys Ser Gly Asp Thr Leu Ser Ala Ile Ser Lys
               100                 105                 110
   Gln Val Tyr Gly Asn Ala Asn Leu Tyr Asn Lys Ile Phe Glu Ala Asn
           115                 120                 125
   Lys Pro Met Leu Lys Ser Pro Asp Lys Ile Tyr Pro Gly Gln Val Leu
       130                 135                 140
   Arg Ile Pro Glu Glu
   145
<210> 138
   <211> 478
   <212> PRT
   <213> Escherichia coli
<400> 138
   Met Ser Asn Asn Ile Arg Ile Glu Glu Asp Leu Leu Gly Thr Arg Glu
   1               5                   10                  15
   Val Pro Ala Asp Ala Tyr Tyr Gly Val His Thr Leu Arg Ala Ile Glu
               20                  25                  30
   Asn Phe Tyr Ile Ser Asn Asn Lys Ile Ser Asp Ile Pro Glu Phe Val
           35                  40                  45
   Arg Gly Met Val Met Val Lys Lys Ala Ala Ala Met Ala Asn Lys Glu
       50                  55                  60
   Leu Gln Thr Ile Pro Lys Ser Val Ala Asn Ala Ile Ile Ala Ala Cys
   65                  70                  75                  80
   Asp Glu Val Leu Asn Asn Gly Lys Cys Met Asp Gln Phe Pro Val Asp
                   85                  90                  95
   Val Tyr Gln Gly Gly Ala Gly Thr Ser Val Asn Met Asn Thr Asn Glu
               100                 105                 110
   Val Leu Ala Asn Ile Gly Leu Glu Leu Met Gly His Gln Lys Gly Glu
           115                 120                 125
   Tyr Gln Tyr Leu Asn Pro Asn Asp His Val Asn Lys Cys Gln Ser Thr
       130                 135                 140
   Asn Asp Ala Tyr Pro Thr Gly Phe Arg Ile Ala Val Tyr Ser Ser Leu
   145                 150                 155                 160
   Ile Lys Leu Val Asp Ala Ile Asn Gln Leu Arg Glu Gly Phe Glu Arg
                   165                 170                 175
   Lys Ala Val Glu Phe Gln Asp Ile Leu Lys Met Gly Arg Thr Gln Leu
               180                 185                 190
   Gln Asp Ala Val Pro Met Thr Leu Gly Gln Glu Phe Arg Ala Phe Ser
           195                 200                 205
   Ile Leu Leu Lys Glu Glu Val Lys Asn Ile Gln Arg Thr Ala Glu Leu
       210                 215                 220
   Leu Leu Glu Val Asn Leu Gly Ala Thr Ala Ile Gly Thr Gly Leu Asn
   225                 230                 235                 240
   Thr Pro Lys Glu Tyr Ser Pro Leu Ala Val Lys Lys Leu Ala Glu Val
                   245                 250                 255
   Thr Gly Phe Pro Cys Val Pro Ala Glu Asp Leu Ile Glu Ala Thr Ser
               260                 265                 270
   Asp Cys Gly Ala Tyr Val Met Val His Gly Ala Leu Lys Arg Leu Ala
           275                 280                 285
   Val Lys Met Ser Lys Ile Cys Asn Asp Leu Arg Leu Leu Ser Ser Gly
       290                 295                 300
   Pro Arg Ala Gly Leu Asn Glu Ile Asn Leu Pro Glu Leu Gln Ala Gly
   305                 310                 315                 320
   Ser Ser Ile Met Pro Ala Lys Val Asn Pro Val Val Pro Glu Val Val
                   325                 330                 335
   Asn Gln Val Cys Phe Lys Val Ile Gly Asn Asp Thr Thr Val Thr Met
               340                 345                 350
   Ala Ala Glu Ala Gly Gln Leu Gln Leu Asn Val Met Glu Pro Val Ile
           355                 360                 365
   Gly Gln Ala Met Phe Glu Ser Val His Ile Leu Thr Asn Ala Cys Tyr
       370                 375                 380
   Asn Leu Leu Glu Lys Cys Ile Asn Gly Ile Thr Ala Asn Lys Glu Val
   385                 390                 395                 400
   Cys Glu Gly Tyr Val Tyr Asn Ser Ile Gly Ile Val Thr Tyr Leu Asn
                   405                 410                 415
   Pro Phe Ile Gly His His Asn Gly Asp Ile Val Gly Lys Ile Cys Ala
   420 425 430
   Glu Thr Gly Lys Ser Val Arg Glu Val Val Leu Glu Arg Gly Leu Leu
   435 440 445
   Thr Glu Ala Glu Leu Asp Asp Ile Phe Ser Val Gln Asn Leu Met His
   450 455 460
   Pro Ala Tyr Lys Ala Lys Arg Tyr Thr Asp Glu Ser Glu Gln
   465 470 475
<210> 139
   <211> 513
   <212> PRT
   <213> Escherichia coli
<400> 139
   Met Gln Leu Asn Ser Thr Glu Ile Ser Glu Leu Ile Lys Gln Arg Ile
   1               5                   10                  15
   Ala Gln Phe Asn Val Val Ser Glu Ala His Asn Glu Gly Thr Ile Val
               20                  25                  30
   Ser Val Ser Asp Gly Val Ile Arg Ile His Gly Leu Ala Asp Cys Met
           35                  40                  45
   Gln Gly Glu Met Ile Ser Leu Pro Gly Asn Arg Tyr Ala Ile Ala Leu
       50                  55                  60
   Asn Leu Glu Arg Asp Ser Val Gly Ala Val Val Met Gly Pro Tyr Ala
   65                  70                  75                  80
   Asp Leu Ala Glu Gly Met Lys Val Lys Cys Thr Gly Arg Ile Leu Glu
                   85                  90                  95
   Val Pro Val Gly Arg Gly Leu Leu Gly Arg Val Val Asn Thr Leu Gly
               100                 105                 110
   Ala Pro Ile Asp Gly Lys Gly Pro Leu Asp His Asp Gly Phe Ser Ala
           115                 120                 125
   Val Glu Ala Ile Ala Pro Gly Val Ile Glu Arg Gln Ser Val Asp Gln
       130                 135                 140
   Pro Val Gln Thr Gly Tyr Lys Ala Val Asp Ser Met Ile Pro Ile Gly
   145                 150                 155                 160
   Arg Gly Gln Arg Glu Leu Ile Ile Gly Asp Arg Gln Thr Gly Lys Thr
                   165                 170                 175
   Ala Leu Ala Ile Asp Ala Ile Ile Asn Gln Arg Asp Ser Gly Ile Lys
               180                 185                 190
   Cys Ile Tyr Val Ala Ile Gly Gln Lys Ala Ser Thr Ile Ser Asn Val
           195                 200                 205
   Val Arg Lys Leu Glu Glu His Gly Ala Leu Ala Asn Thr Ile Val Val
       210                 215                 220
   Val Ala Thr Ala Ser Glu Ser Ala Ala Leu Gln Tyr Leu Ala Pro Tyr
   225                 230                 235                 240
   Ala Gly Cys Ala Met Gly Glu Tyr Phe Arg Asp Arg Gly Glu Asp Ala
                   245                 250                 255
   Leu Ile Ile Tyr Asp Asp Leu Ser Lys Gln Ala Val Ala Tyr Arg Gln
               260                 265                 270
   Ile Ser Leu Leu Leu Arg Arg Pro Pro Gly Arg Glu Ala Phe Pro Gly
           275                 280                 285
   Asp Val Phe Tyr Leu His Ser Arg Leu Leu Glu Arg Ala Ala Arg Val
       290                 295                 300
   Asn Ala Glu Tyr Val Glu Ala Phe Thr Lys Gly Glu Val Lys Gly Lys
   305                 310                 315                 320
   Thr Gly Ser Leu Thr Ala Leu Pro Ile Ile Glu Thr Gln Ala Gly Asp
                   325                 330                 335
   Val Ser Ala Phe Val Pro Thr Asn Val Ile Ser Ile Thr Asp Gly Gln
               340                 345                 350
   Ile Phe Leu Glu Thr Asn Leu Phe Asn Ala Gly Ile Arg Pro Ala Val
           355                 360                 365
   Asn Pro Gly Ile Ser Val Ser Arg Val Gly Gly Ala Ala Gln Thr Lys
       370                 375                 380
   Ile Met Lys Lys Leu Ser Gly Gly Ile Arg Thr Ala Leu Ala Gln Tyr
   385                 390                 395                 400
   Arg Glu Leu Ala Ala Phe Ser Gln Phe Ala Ser Asp Leu Asp Asp Ala
                   405                 410                 415
   Thr Arg Lys Gln Leu Asp His Gly Gln Lys Val Thr Glu Leu Leu Lys
               420                 425                 430
   Gln Lys Gln Tyr Ala Pro Met Ser Val Ala Gln Gln Ser Leu Val Leu
   435 440 445
   Phe Ala Ala Glu Arg Gly Tyr Leu Ala Asp Val Glu Leu Ser Lys Ile
   450 455 460
   Gly Ser Phe Glu Ala Ala Leu Leu Ala Tyr Val Asp Arg Asp His Ala
   465 470 475 480
   Pro Leu Met Gln Glu Ile Asn Gln Thr Gly Gly Tyr Asn Asp Glu Ile
   485 490 495
   Glu Gly Lys Leu Lys Gly Ile Leu Asp Ser Phe Lys Ala Thr Gln Ser
   500 505 510
   Trp
<210> 140
   <211> 97
   <212> PRT
   <213> Escherichia coli
<400> 140
<210> 141
   <211> 548
   <212> PRT
   <213> Escherichia coli
<400> 141
   Met Ala Ala Lys Asp Val Lys Phe Gly Asn Asp Ala Arg Val Lys Met
   1               5                   10                  15
   Leu Arg Gly Val Asn Val Leu Ala Asp Ala Val Lys Val Thr Leu Gly
               20                  25                  30
   Pro Lys Gly Arg Asn Val Val Leu Asp Lys Ser Phe Gly Ala Pro Thr
           35                  40                  45
   Ile Thr Lys Asp Gly Val Ser Val Ala Arg Glu Ile Glu Leu Glu Asp
       50                  55                  60
   Lys Phe Glu Asn Met Gly Ala Gln Met Val Lys Glu Val Ala Ser Lys
   65                  70                  75                  80
   Ala Asn Asp Ala Ala Gly Asp Gly Thr Thr Thr Ala Thr Val Leu Ala
                   85                  90                  95
   Gln Ala Ile Ile Thr Glu Gly Leu Lys Ala Val Ala Ala Gly Met Asn
               100                 105                 110
   Pro Met Asp Leu Lys Arg Gly Ile Asp Lys Ala Val Thr Ala Ala Val
           115                 120                 125
   Glu Glu Leu Lys Ala Leu Ser Val Pro Cys Ser Asp Ser Lys Ala Ile
       130                 135                 140
   Ala Gln Val Gly Thr Ile Ser Ala Asn Ser Asp Glu Thr Val Gly Lys
   145                 150                 155                 160
   Leu Ile Ala Glu Ala Met Asp Lys Val Gly Lys Glu Gly Val Ile Thr
                   165                 170                 175
   Val Glu Asp Gly Thr Gly Leu Gln Asp Glu Leu Asp Val Val Glu Gly
               180                 185                 190
   Met Gln Phe Asp Arg Gly Tyr Leu Ser Pro Tyr Phe Ile Asn Lys Pro
           195                 200                 205
   Glu Thr Gly Ala Val Glu Leu Glu Ser Pro Phe Ile Leu Leu Ala Asp
       210                 215                 220
   Lys Lys Ile Ser Asn Ile Arg Glu Met Leu Pro Val Leu Glu Ala Val
   225                 230                 235                 240
   Ala Lys Ala Gly Lys Pro Leu Leu Ile Ile Ala Glu Asp Val Glu Gly
                   245                 250                 255
   Glu Ala Leu Ala Thr Leu Val Val Asn Thr Met Arg Gly Ile Val Lys
   260 265 270
   Val Ala Ala Val Lys Ala Pro Gly Phe Gly Asp Arg Arg Lys Ala Met
   275 280 285
   Leu Gln Asp Ile Ala Thr Leu Thr Gly Gly Thr Val Ile Ser Glu Glu
   290 295 300
   Ile Gly Met Glu Leu Glu Lys Ala Thr Leu Glu Asp Leu Gly Gln Ala
   305 310 315 320
   Lys Arg Val Val Ile Asn Lys Asp Thr Thr Thr Ile Ile Asp Gly Val
   325 330 335
   Gly Glu Glu Ala Ala Ile Gln Gly Arg Val Ala Gln Ile Arg Gln Gln
   340 345 350
   Ile Glu Glu Ala Thr Ser Asp Tyr Asp Arg Glu Lys Leu Gln Glu Arg
   355 360 365
   Val Ala Lys Leu Ala Gly Gly Val Ala Val Ile Lys Val Gly Ala Ala
   370 375 380
   Thr Glu Val Glu Met Lys Glu Lys Lys Ala Arg Val Glu Asp Ala Leu
   385 390 395 400
   His Ala Thr Arg Ala Ala Val Glu Glu Gly Val Val Ala Gly Gly Gly
   405 410 415
   Val Ala Leu Ile Arg Val Ala Ser Lys Leu Ala Asp Leu Arg Gly Gln
   420 425 430
   Asn Glu Asp Gln Asn Val Gly Ile Lys Val Ala Leu Arg Ala Met Glu
   435 440 445
   Ala Pro Leu Arg Gln Ile Val Leu Asn Cys Gly Glu Glu Pro Ser Val
   450 455 460
   Val Ala Asn Thr Val Lys Gly Gly Asp Gly Asn Tyr Gly Tyr Asn Ala
   465 470 475 480
   Ala Thr Glu Glu Tyr Gly Asn Met Ile Asp Met Gly Ile Leu Asp Pro
   485 490 495
   Thr Lys Val Thr Arg Ser Ala Leu Gln Tyr Ala Ala Ser Val Ala Gly
   500 505 510
   Leu Met Ile Thr Thr Glu Cys Met Val Thr Asp Leu Pro Lys Asn Asp
   515 520 525
   Ala Ala Asp Leu Gly Ala Ala Gly Gly Met Gly Gly Met Gly Gly Met
   530 535 540
   Gly Gly Met Met
   545
<210> 142
   <211> 90
   <212> PRT
   <213> Escherichia coli
<400> 142
   Met Asn Lys Ser Gln Leu Ile Asp Lys Ile Ala Ala Gly Ala Asp Ile
   1 5 10 15
   Ser Lys Ala Ala Ala Gly Arg Ala Leu Asp Ala Ile Ile Ala Ser Val
   20 25 30
   Thr Glu Ser Leu Lys Glu Gly Asp Asp Val Ala Leu Val Gly Phe Gly
   35 40 45
   Thr Phe Ala Val Lys Glu Arg Ala Ala Arg Thr Gly Arg Asn Pro Gln
   50 55 60
   Thr Gly Lys Glu Ile Thr Ile Ala Ala Ala Lys Val Pro Ser Phe Arg
   65 70 75 80
   Ala Gly Lys Ala Leu Lys Asp Ala Val Asn
   85 90
<210> 143
   <211> 466
   <212> PRT
   <213> Escherichia coli
<400> 143
   Met Asp Lys Lys Gln Val Thr Asp Leu Arg Ser Glu Leu Leu Asp Ser
   1               5                   10                  15
   Arg Phe Gly Ala Lys Ser Ile Ser Thr Ile Ala Glu Ser Lys Arg Phe
               20                  25                  30
   Pro Leu His Glu Met Arg Asp Asp Val Ala Phe Gln Ile Ile Asn Asp
           35                  40                  45
   Glu Leu Tyr Leu Asp Gly Asn Ala Arg Gln Asn Leu Ala Thr Phe Cys
       50                  55                  60
   Gln Thr Trp Asp Asp Glu Asn Val His Lys Leu Met Asp Leu Ser Ile
   65 70 75 80
   Asn Lys Asn Trp Ile Asp Lys Glu Glu Tyr Pro Gln Ser Ala Ala Ile
   85 90 95
   Asp Leu Arg Cys Val Asn Met Val Ala Asp Leu Trp His Ala Pro Ala
   100 105 110
   Pro Lys Asn Gly Gln Ala Val Gly Thr Asn Thr Ile Gly Ser Ser Glu
   115 120 125
   Ala Cys Met Leu Gly Gly Met Ala Met Lys Trp Arg Trp Arg Lys Arg
   130 135 140
   Met Glu Ala Ala Gly Lys Pro Thr Asp Lys Pro Asn Leu Val Cys Gly
   145 150 155 160
   Pro Val Gln Ile Cys Trp His Lys Phe Ala Arg Tyr Trp Asp Val Glu
   165 170 175
   Leu Arg Glu Ile Pro Met Arg Pro Gly Gln Leu Phe Met Asp Pro Lys
   180 185 190
   Arg Met Ile Glu Ala Cys Asp Glu Asn Thr Ile Gly Val Val Pro Thr
   195 200 205
   Phe Gly Val Thr Tyr Thr Gly Asn Tyr Glu Phe Pro Gln Pro Leu His
   210 215 220
   Asp Ala Leu Asp Lys Phe Gln Ala Asp Thr Gly Ile Asp Ile Asp Met
   225 230 235 240
   His Ile Asp Ala Ala Ser Gly Gly Phe Leu Ala Pro Phe Val Ala Pro
   245 250 255
   Asp Ile Val Trp Asp Phe Arg Leu Pro Arg Val Lys Ser Ile Ser Ala
   260 265 270
   Ser Gly His Lys Phe Gly Leu Ala Pro Leu Gly Cys Gly Trp Val Ile
   275 280 285
   Trp Arg Asp Glu Glu Ala Leu Pro Gln Glu Leu Val Phe Asn Val Asp
   290 295 300
   Tyr Leu Gly Gly Gln Ile Gly Thr Phe Ala Ile Asn Phe Ser Arg Pro
   305 310 315 320
   Ala Gly Gln Val Ile Ala Gln Tyr Tyr Glu Phe Leu Arg Leu Gly Arg
   325 330 335
   Glu Gly Tyr Thr Lys Val Gln Asn Ala Ser Tyr Gln Val Ala Ala Tyr
   340 345 350
   Leu Ala Asp Glu Ile Ala Lys Leu Gly Pro Tyr Glu Phe Ile Cys Thr
   355 360 365
   Gly Arg Pro Asp Glu Gly Ile Pro Ala Val Cys Phe Lys Leu Lys Asp
   370 375 380
   Gly Glu Asp Pro Gly Tyr Thr Leu Tyr Asp Leu Ser Glu Arg Leu Arg
   385 390 395 400
   Leu Arg Gly Trp Gln Val Pro Ala Phe Thr Leu Gly Gly Glu Ala Thr
   405 410 415
   Asp Ile Val Val Met Arg Ile Met Cys Arg Arg Gly Phe Glu Met Asp
   420 425 430
   Phe Ala Glu Leu Leu Leu Glu Asp Tyr Lys Ala Ser Leu Lys Tyr Leu
   435 440 445
   Ser Asp His Pro Lys Leu Gln Gly Ile Ala Gln Gln Asn Ser Phe Lys
   450 455 460
   His Thr
   465
<210> 144
   <211> 588
   <212> PRT
   <213> Escherichia coli
<400> 144
   Met Lys Leu Pro Val Arg Glu Phe Asp Ala Val Val Ile Gly Ala Gly
   1               5                   10                  15
   Gly Ala Gly Met Arg Ala Ala Leu Gln Ile Ser Gln Ser Gly Gln Thr
               20                  25                  30
   Cys Ala Leu Leu Ser Lys Val Phe Pro Thr Arg Ser His Thr Val Ser
           35                  40                  45
   Ala Gln Gly Gly Ile Thr Val Ala Leu Gly Asn Thr His Glu Asp Asn
       50                  55                  60
   Trp Glu Trp His Met Tyr Asp Thr Val Lys Gly Ser Asp Tyr Ile Gly
   65                  70                  75                  80
   Asp Gln Asp Ala Ile Glu Tyr Met Cys Lys Thr Gly Pro Glu Ala Ile
                   85                  90                  95
   Leu Glu Leu Glu His Met Gly Leu Pro Phe Ser Arg Leu Asp Asp Gly
               100                 105                 110
   Arg Ile Tyr Gln Arg Pro Phe Gly Gly Gln Ser Lys Asn Phe Gly Gly
           115                 120                 125
   Glu Gln Ala Ala Arg Thr Ala Ala Ala Ala Asp Arg Thr Gly His Ala
       130                 135                 140
   Leu Leu His Thr Leu Tyr Gln Gln Asn Leu Lys Asn His Thr Thr Ile
   145                 150                 155                 160
   Phe Ser Glu Trp Tyr Ala Leu Asp Leu Val Lys Asn Gln Asp Gly Ala
                   165                 170                 175
   Val Val Gly Cys Thr Ala Leu Cys Ile Glu Thr Gly Glu Val Val Tyr
               180                 185                 190
   Phe Lys Ala Arg Ala Thr Val Leu Ala Thr Gly Gly Ala Gly Arg Ile
           195                 200                 205
   Tyr Gln Ser Thr Thr Asn Ala His Ile Asn Thr Gly Asp Gly Val Gly
       210                 215                 220
   Met Ala Ile Arg Ala Gly Val Pro Val Gln Asp Met Glu Met Trp Gln
   225                 230                 235                 240
   Phe His Pro Thr Gly Ile Ala Gly Ala Gly Val Leu Val Thr Glu Gly
                   245                 250                 255
   Cys Arg Gly Glu Gly Gly Tyr Leu Leu Asn Lys His Gly Glu Arg Phe
               260                 265                 270
   Met Glu Arg Tyr Ala Pro Asn Ala Lys Asp Leu Ala Gly Arg Asp Val
           275                 280                 285
   Val Ala Arg Ser Ile Met Ile Glu Ile Arg Glu Gly Arg Gly Cys Asp
       290                 295                 300
   Gly Pro Trp Gly Pro His Ala Lys Leu Lys Leu Asp His Leu Gly Lys
   305                 310                 315                 320
   Glu Val Leu Glu Ser Arg Leu Pro Gly Ile Leu Glu Leu Ser Arg Thr
                   325                 330                 335
   Phe Ala His Val Asp Pro Val Lys Glu Pro Ile Pro Val Ile Pro Thr
               340                 345                 350
   Cys His Tyr Met Met Gly Gly Ile Pro Thr Lys Val Thr Gly Gln Ala
           355                 360                 365
   Leu Thr Val Asn Glu Lys Gly Glu Asp Val Val Val Pro Gly Leu Phe
       370                 375                 380
   Ala Val Gly Glu Ile Ala Cys Val Ser Val His Gly Ala Asn Arg Leu
   385                 390                 395                 400
   Gly Gly Asn Ser Leu Leu Asp Leu Val Val Phe Gly Arg Ala Ala Gly
                   405                 410                 415
   Leu His Leu Gln Glu Ser Ile Ala Glu Gln Gly Ala Leu Arg Asp Ala
               420                 425                 430
   Ser Glu Ser Asp Val Glu Ala Ser Leu Asp Arg Leu Asn Arg Trp Asn
           435                 440                 445
   Asn Asn Arg Asn Gly Glu Asp Pro Val Ala Ile Arg Lys Ala Leu Gln
       450                 455                 460
   Glu Cys Met Gln His Asn Phe Ser Val Phe Arg Glu Gly Asp Ala Met
   465                 470                 475                 480
   Ala Lys Gly Leu Glu Gln Leu Lys Val Ile Arg Glu Arg Leu Lys Asn
                   485                 490                 495
   Ala Arg Leu Asp Asp Thr Ser Ser Glu Phe Asn Thr Gln Arg Val Glu
               500                 505                 510
   Cys Leu Glu Leu Asp Asn Leu Met Glu Thr Ala Tyr Ala Thr Ala Val
           515                 520                 525
   Ser Ala Asn Phe Arg Thr Glu Ser Arg Gly Ala His Ser Arg Phe Asp
       530                 535                 540
   Phe Pro Asp Arg Asp Asp Glu Asn Trp Leu Cys His Ser Leu Tyr Leu
   545                 550                 555                 560
   Pro Glu Ser Glu Ser Met Thr Arg Arg Ser Val Asn Met Glu Pro Lys
                   565                 570                 575
   Leu Arg Pro Ala Phe Pro Pro Lys Ile Arg Thr Tyr
               580                 585
<210> 145
   <211> 167
   <212> PRT
   <213> Escherichia coli
<400> 145
<210> 146
   <211> 137
   <212> PRT
   <213> Escherichia coli
<400> 146
   Met Ser Glu Ala Leu Lys Ile Leu Asn Asn Ile Arg Thr Leu Arg Ala
   1 5 10 15
   Gln Ala Arg Glu Cys Thr Leu Glu Thr Leu Glu Glu Met Leu Glu Lys
   20 25 30
   Leu Glu Val Val Val Asn Glu Arg Arg Glu Glu Glu Ser Ala Ala Ala
   35 40 45
   Ala Glu Val Glu Glu Arg Thr Arg Lys Leu Gln Gln Tyr Arg Glu Met
   50 55 60
   Leu Ile Ala Asp Gly Ile Asp Pro Asn Glu Leu Leu Asn Ser Leu Ala
   65 70 75 80
   Ala Val Lys Ser Gly Thr Lys Ala Lys Arg Ala Gln Arg Pro Ala Lys
   85 90 95
   Tyr Ser Tyr Val Asp Glu Asn Gly Glu Thr Lys Thr Trp Thr Gly Gln
   100 105 110
   Gly Arg Thr Pro Ala Val Ile Lys Lys Ala Met Asp Glu Gln Gly Lys
   115 120 125
   Ser Leu Asp Asp Phe Leu Ile Lys Gln
   130 135
<210> 147
   <211> 312
   <212> PRT
   <213> Escherichia coli
<400> 147
   Met Lys Val Ala Val Leu Gly Ala Ala Gly Gly Ile Gly Gln Ala Leu
   1               5                   10                  15
   Ala Leu Leu Leu Lys Thr Gln Leu Pro Ser Gly Ser Glu Leu Ser Leu
               20                  25                  30
   Tyr Asp Ile Ala Pro Val Thr Pro Gly Val Ala Val Asp Leu Ser His
           35                  40                  45
   Ile Pro Thr Ala Val Lys Ile Lys Gly Phe Ser Gly Glu Asp Ala Thr
       50                  55                  60
   Pro Ala Leu Glu Gly Ala Asn Val Val Leu Ile Ser Ala Gly Val Ala
   65                  70                  75                  80
   Arg Lys Pro Gly Met Asp Arg Ser Asp Leu Phe Asn Val Asn Ala Gly
                   85                  90                  95
   Ile Val Lys Asn Leu Val Gln Gln Val Ala Lys Thr Cys Pro Lys Ala
               100                 105                 110
   Cys Ile Gly Ile Ile Thr Asn Pro Val Asn Thr Thr Val Ala Ile Ala
           115                 120                 125
   Ala Glu Val Leu Lys Lys Ala Gly Val Tyr Asp Lys Asn Lys Leu Phe
       130                 135                 140
   Gly Val Thr Thr Leu Asp Ile Ile Arg Ser Asn Thr Phe Val Ala Glu
   145                 150                 155                 160
   Leu Lys Gly Lys Gln Pro Gly Glu Val Glu Val Pro Val Ile Gly Gly
                   165                 170                 175
   His Ser Gly Val Thr Ile Leu Pro Leu Leu Ser Gln Val Pro Gly Val
               180                 185                 190
   Ser Phe Thr Glu Gln Glu Val Ala Asp Leu Thr Lys Arg Ile Gln Asn
           195                 200                 205
   Ala Gly Thr Glu Val Val Glu Ala Lys Ala Gly Gly Gly Ser Ala Thr
       210                 215                 220
   Leu Ser Met Gly Gln Ala Ala Ala Arg Phe Gly Leu Ser Leu Val Arg
   225                 230                 235                 240
   Ala Leu Gln Gly Glu Gln Gly Val Val Glu Cys Ala Tyr Val Glu Gly
                   245                 250                 255
   Asp Gly Gln Tyr Ala Arg Phe Phe Ser Gln Pro Leu Leu Leu Gly Lys
               260                 265                 270
   Asn Gly Val Glu Glu Arg Lys Ser Ile Gly Thr Leu Ser Ala Phe Glu
           275                 280                 285
   Lys Asn Ala Leu Glu Gly Met Leu Asp Thr Leu Lys Lys Asp Ile Ala
       290                 295                 300
   Leu Gly Glu Glu Phe Val Asn Lys
   305                 310
<210> 148
   <211> 387
   <212> PRT
   <213> Escherichia coli
<400> 148
   Met Ser Val Ile Lys Met Thr Asp Leu Asp Leu Ala Gly Lys Arg Val
   1               5                   10                  15
   Phe Ile Arg Ala Asp Leu Asn Val Pro Val Lys Asp Gly Lys Val Thr
               20                  25                  30
   Ser Asp Ala Arg Ile Arg Ala Ser Leu Pro Thr Ile Glu Leu Ala Leu
           35                  40                  45
   Lys Gln Gly Ala Lys Val Met Val Thr Ser His Leu Gly Arg Pro Thr
       50                  55                  60
   Glu Gly Glu Tyr Asn Glu Glu Phe Ser Leu Leu Pro Val Val Asn Tyr
   65                  70                  75                  80
   Leu Lys Asp Lys Leu Ser Asn Pro Val Arg Leu Val Lys Asp Tyr Leu
                   85                  90                  95
   Asp Gly Val Asp Val Ala Glu Gly Glu Leu Val Val Leu Glu Asn Val
               100                 105                 110
   Arg Phe Asn Lys Gly Glu Lys Lys Asp Asp Glu Thr Leu Ser Lys Lys
           115                 120                 125
   Tyr Ala Ala Leu Cys Asp Val Phe Val Met Asp Ala Phe Gly Thr Ala
       130                 135                 140
   His Arg Ala Gln Ala Ser Thr His Gly Ile Gly Lys Phe Ala Asp Val
   145                 150                 155                 160
   Ala Cys Ala Gly Pro Leu Leu Ala Ala Glu Leu Asp Ala Leu Gly Lys
                   165                 170                 175
   Ala Leu Lys Glu Pro Ala Arg Pro Met Val Ala Ile Val Gly Gly Ser
               180                 185                 190
   Lys Val Ser Thr Lys Leu Thr Val Leu Asp Ser Leu Ser Lys Ile Ala
           195                 200                 205
   Asp Gln Leu Ile Val Gly Gly Gly Ile Ala Asn Thr Phe Ile Ala Ala
       210                 215                 220
   Gln Gly His Asp Val Gly Lys Ser Leu Tyr Glu Ala Asp Leu Val Asp
   225                 230                 235                 240
   Glu Ala Lys Arg Leu Leu Ser Thr Cys Asn Ile Pro Val Pro Ser Asp
                   245                 250                 255
   Val Arg Val Ala Thr Glu Phe Ser Glu Thr Ala Pro Ala Thr Leu Lys
               260                 265                 270
   Ser Val Asn Asp Val Lys Ala Asp Glu Gln Ile Leu Asp Ile Gly Asp
           275                 280                 285
   Ala Ser Ala Gln Glu Leu Ala Glu Ile Leu Lys Asn Ala Lys Thr Ile
   290 295 300
   Leu Trp Asn Gly Pro Val Gly Val Phe Glu Phe Pro Asn Phe Arg Lys
   305 310 315 320
   Gly Thr Glu Ile Val Ala Asn Ala Ile Ala Asp Ser Glu Ala Phe Ser
   325 330 335
   Ile Ala Gly Gly Gly Asp Thr Leu Ala Ala Ile Asp Leu Phe Gly Ile
   340 345 350
   Ala Asp Lys Ile Ser Tyr Ile Ser Thr Gly Gly Gly Ala Phe Leu Glu
   355 360 365
   Phe Val Glu Gly Lys Val Leu Pro Ala Val Ala Met Leu Glu Glu Arg
   370 375 380
   Ala Lys Lys
   385
<210> 149
   <211> 237
   <212> PRT
   <213> Escherichia coli
<400> 149
<210> 150
   <211> 201
   <212> PRT
   <213> Escherichia coli
<400> 150
<210> 151
   <211> 557
   <212> PRT
   <213> Escherichia coli
<400> 151
   Met Thr Glu Ser Phe Ala Gln Leu Phe Glu Glu Ser Leu Lys Glu Ile
   1               5                   10                  15
   Glu Thr Arg Pro Gly Ser Ile Val Arg Gly Val Val Val Ala Ile Asp
               20                  25                  30
   Lys Asp Val Val Leu Val Asp Ala Gly Leu Lys Ser Glu Ser Ala Ile
           35                  40                  45
   Pro Ala Glu Gln Phe Lys Asn Ala Gln Gly Glu Leu Glu Ile Gln Val
       50                  55                  60
   Gly Asp Glu Val Asp Val Ala Leu Asp Ala Val Glu Asp Gly Phe Gly
   65                  70                  75                  80
   Glu Thr Leu Leu Ser Arg Glu Lys Ala Lys Arg His Glu Ala Trp Ile
                   85                  90                  95
   Thr Leu Glu Lys Ala Tyr Glu Asp Ala Glu Thr Val Thr Gly Val Ile
               100                 105                 110
   Asn Gly Lys Val Lys Gly Gly Phe Thr Val Glu Leu Asn Gly Ile Arg
           115                 120                 125
   Ala Phe Leu Pro Gly Ser Leu Val Asp Val Arg Pro Val Arg Asp Thr
       130                 135                 140
   Leu His Leu Glu Gly Lys Glu Leu Glu Phe Lys Val Ile Lys Leu Asp
   145                 150                 155                 160
   Gln Lys Arg Asn Asn Val Val Val Ser Arg Arg Ala Val Ile Glu Ser
                   165                 170                 175
   Glu Asn Ser Ala Glu Arg Asp Gln Leu Leu Glu Asn Leu Gln Glu Gly
               180                 185                 190
   Met Glu Val Lys Gly Ile Val Lys Asn Leu Thr Asp Tyr Gly Ala Phe
           195                 200                 205
   Val Asp Leu Gly Gly Val Asp Gly Leu Leu His Ile Thr Asp Met Ala
       210                 215                 220
   Trp Lys Arg Val Lys His Pro Ser Glu Ile Val Asn Val Gly Asp Glu
   225                 230                 235                 240
   Ile Thr Val Lys Val Leu Lys Phe Asp Arg Glu Arg Thr Arg Val Ser
                   245                 250                 255
   Leu Gly Leu Lys Gln Leu Gly Glu Asp Pro Trp Val Ala Ile Ala Lys
               260                 265                 270
   Arg Tyr Pro Glu Gly Thr Lys Leu Thr Gly Arg Val Thr Asn Leu Thr
           275                 280                 285
   Asp Tyr Gly Cys Phe Val Glu Ile Glu Glu Gly Val Glu Gly Leu Val
       290                 295                 300
   His Val Ser Glu Met Asp Trp Thr Asn Lys Asn Ile His Pro Ser Lys
   305                 310                 315                 320
   Val Val Asn Val Gly Asp Val Val Glu Val Met Val Leu Asp Ile Asp
                   325                 330                 335
   Glu Glu Arg Arg Arg Ile Ser Leu Gly Leu Lys Gln Cys Lys Ala Asn
               340                 345                 350
   Pro Trp Gln Gln Phe Ala Glu Thr His Asn Lys Gly Asp Arg Val Glu
           355                 360                 365
   Gly Lys Ile Lys Ser Ile Thr Asp Phe Gly Ile Phe Ile Gly Leu Asp
       370                 375                 380
   Gly Gly Ile Asp Gly Leu Val His Leu Ser Asp Ile Ser Trp Asn Val
   385                 390                 395                 400
   Ala Gly Glu Glu Ala Val Arg Glu Tyr Lys Lys Gly Asp Glu Ile Ala
                   405                 410                 415
   Ala Val Val Leu Gln Val Asp Ala Glu Arg Glu Arg Ile Ser Leu Gly
               420                 425                 430
   Val Lys Gln Leu Ala Glu Asp Pro Phe Asn Asn Trp Val Ala Leu Asn
           435                 440                 445
   Lys Lys Gly Ala Ile Val Thr Gly Lys Val Thr Ala Val Asp Ala Lys
       450                 455                 460
   Gly Ala Thr Val Glu Leu Ala Asp Gly Val Glu Gly Tyr Leu Arg Ala
   465                 470                 475                 480
   Ser Glu Ala Ser Arg Asp Arg Val Glu Asp Ala Thr Leu Val Leu Ser
                   485                 490                 495
   Val Gly Asp Glu Val Glu Ala Lys Phe Thr Gly Val Asp Arg Lys Asn
               500                 505                 510
   Arg Ala Ile Ser Leu Ser Val Arg Ala Lys Asp Glu Ala Asp Glu Lys
           515                 520                 525
   Asp Ala Ile Ala Thr Val Asn Lys Gln Glu Asp Ala Asn Phe Ser Asn
       530                 535                 540
   Asn Ala Met Ala Glu Ala Phe Lys Ala Ala Lys Gly Glu
   545                 550                 555
<210> 152
   <211> 128
   <212> PRT
   <213> Escherichia coli
<400> 152
   Met Ser Lys Thr Ile Ala Thr Glu Asn Ala Pro Ala Ala Ile Gly Pro
   1               5                   10                  15
   Tyr Val Gln Gly Val Asp Leu Gly Asn Met Ile Ile Thr Ser Gly Gln
               20                  25                  30
   Ile Pro Val Asn Pro Lys Thr Gly Glu Val Pro Ala Asp Val Ala Ala
           35                  40                  45
   Gln Ala Arg Gln Ser Leu Asp Asn Val Lys Ala Ile Val Glu Ala Ala
       50                  55                  60
   Gly Leu Lys Val Gly Asp Ile Val Lys Thr Thr Val Phe Val Lys Asp
   65                  70                  75                  80
   Leu Asn Asp Phe Ala Thr Val Asn Ala Thr Tyr Glu Ala Phe Phe Thr
                   85                  90                  95
   Glu His Asn Ala Thr Phe Pro Ala Arg Ser Cys Val Glu Val Ala Arg
               100                 105                 110
   Leu Pro Lys Asp Val Lys Ile Glu Ile Glu Ala Ile Ala Val Arg Arg
           115                 120                 125
<210> 153
   <211> 315
   <212> PRT
   <213> Escherichia coli
<400> 153
<210> 154
   <211> 319
   <212> PRT
   <213> Escherichia coli
<400> 154
   Met Lys Cys Ile Leu Phe Lys Trp Val Leu Cys Leu Leu Leu Gly Phe
   1               5                   10                  15
   Ser Ser Val Ser His Ser Arg Glu Phe Thr Ile Asp Phe Ser Thr Gln
               20                  25                  30
   Gln Ser Tyr Val Ser Ser Leu Asn Ser Ile Arg Thr Glu Ile Ser Thr
           35                  40                  45
   Pro Leu Glu His Ile Ser Gln Gly Thr Thr Ser Val Ser Val Ile Asn
       50                  55                  60
   His Thr Pro Pro Gly Ser Tyr Phe Ala Val Asp Ile Arg Gly Leu Asp
   65                  70                  75                  80
   Val Tyr Gln Ala Arg Phe Asp His Leu Arg Leu Ile Ile Glu Gln Asn
                   85                  90                  95
   Asn Leu Tyr Val Ala Gly Phe Val Asn Thr Ala Thr Asn Thr Phe Tyr
   100 105 110
   Arg Phe Ser Asp Phe Thr His Ile Ser Val Pro Gly Val Thr Thr Val
   115 120 125
   Ser Met Thr Thr Asp Ser Ser Tyr Thr Thr Leu Gln Arg Val Ala Ala
   130 135 140
   Leu Glu Arg Ser Gly Met Gln Ile Ser Arg His Ser Leu Val Ser Ser
   145 150 155 160
   Tyr Leu Ala Leu Val Glu Phe Ser Gly Asn Thr Met Thr Arg Asp Ala
   165 170 175
   Ser Arg Ala Val Leu Arg Phe Val Thr Val Thr Ala Glu Ala Leu Arg
   180 185 190
   Phe Arg Gln Ile Gln Arg Glu Phe Arg Gln Ala Leu Ser Glu Thr Ala
   195 200 205
   Pro Val Tyr Thr Met Thr Pro Gly Asp Val Asp Leu Thr Leu Asn Trp
   210 215 220
   Gly Arg Ile Ser Asn Val Leu Pro Glu Tyr Arg Gly Glu Asp Gly Val
   225 230 235 240
   Arg Val Gly Arg Ile Ser Phe Asn Asn Ile Ser Ala Ile Leu Gly Thr
   245 250 255
   Val Ala Val Ile Leu Asn Cys His His Gln Gly Ala Arg Ser Val Arg
   260 265 270
   Ala Val Asn Glu Asp Ser Gln Pro Glu Cys Gln Ile Thr Gly Asp Arg
   275 280 285
   Pro Val Ile Lys Ile Asn Asn Thr Leu Trp Glu Ser Asn Thr Ala Ala
   290 295 300
   Ala Phe Leu Asn Arg Lys Ser Gln Phe Leu Tyr Thr Thr Gly Lys
   305 310 315
<210> 155
   <211> 350
   <212> PRT
   <213> Candida albicans
<400> 155
   Met Ser Glu Gln Ile Pro Lys Thr Gln Lys Ala Val Val Phe Asp Thr
   1               5                   10                  15
   Asn Gly Gly Gln Leu Val Tyr Lys Asp Tyr Pro Val Pro Thr Pro Lys
               20                  25                  30
   Pro Asn Glu Leu Leu Ile His Val Lys Tyr Ser Gly Val Cys His Thr
           35                  40                  45
   Asp Leu His Ala Arg Lys Gly Asp Trp Pro Leu Ala Thr Lys Leu Pro
       50                  55                  60
   Leu Val Gly Gly His Glu Gly Ala Gly Val Val Val Gly Met Gly Glu
   65                  70                  75                  80
   Asn Val Lys Gly Trp Lys Ile Gly Asp Phe Ala Gly Ile Lys Trp Leu
                   85                  90                  95
   Asn Gly Ser Cys Met Ser Cys Glu Phe Cys Gln Gln Gly Ala Glu Pro
               100                 105                 110
   Asn Cys Gly Glu Ala Asp Leu Ser Gly Tyr Thr His Asp Gly Ser Phe
           115                 120                 125
   Glu Gln Tyr Ala Thr Ala Asp Ala Val Gln Ala Ala Lys Ile Pro Ala
       130                 135                 140
   Gly Thr Asp Leu Ala Asn Val Ala Pro Ile Leu Cys Ala Gly Val Thr
   145                 150                 155                 160
   Val Tyr Lys Ala Leu Lys Thr Ala Asp Leu Ala Ala Gly Gln Trp Val
                   165                 170                 175
   Ala Ile Ser Gly Ala Gly Gly Gly Leu Gly Ser Leu Ala Val Gln Tyr
               180                 185                 190
   Ala Arg Ala Met Gly Leu Arg Val Val Ala Ile Asp Gly Gly Asp Glu
           195                 200                 205
   Lys Gly Glu Phe Val Lys Ser Leu Gly Ala Glu Ala Tyr Val Asp Phe
       210                 215                 220
   Thr Lys Asp Lys Asp Ile Val Glu Ala Val Lys Lys Ala Thr Asp Gly
   225                 230                 235                 240
   Gly Pro His Gly Ala Ile Asn Val Ser Val Ser Glu Lys Ala Ile Asp
                   245                 250                 255
   Gln Ser Val Glu Tyr Val Arg Pro Leu Gly Lys Val Val Leu Val Gly
   260 265 270
   Leu Pro Ala His Ala Lys Val Thr Ala Pro Val Phe Asp Ala Val Val
   275 280 285
   Lys Ser Ile Glu Ile Lys Gly Ser Tyr Val Gly Asn Arg Lys Asp Thr
   290 295 300
   Ala Glu Ala Ile Asp Phe Phe Ser Arg Gly Leu Ile Lys Cys Pro Ile
   305 310 315 320
   Lys Ile Val Gly Leu Ser Asp Leu Pro Glu Val Phe Lys Leu Met Glu
   325 330 335
   Glu Gly Lys Ile Leu Gly Arg Tyr Val Leu Asp Thr Ser Lys
   340 345 350
<210> 156
   <211> 359
   <212> PRT
   <213> Candida albicans
<400> 156
   Met Ala Pro Pro Ala Val Leu Ser Lys Ser Gly Val Ile Tyr Gly Lys
   1               5                   10                  15
   Asp Val Lys Asp Leu Phe Asp Tyr Ala Gln Glu Lys Gly Phe Ala Ile
               20                  25                  30
   Pro Ala Ile Asn Val Thr Ser Ser Ser Thr Val Val Ala Ala Leu Glu
           35                  40                  45
   Ala Ala Arg Asp Asn Lys Ala Pro Ile Ile Leu Gln Thr Ser Gln Gly
       50                  55                  60
   Gly Ala Ala Tyr Phe Ala Gly Lys Gly Val Asp Asn Lys Asp Gln Ala
   65                  70                  75                  80
   Ala Ser Ile Ala Gly Ser Ile Ala Ala Ala His Tyr Ile Arg Ala Ile
                   85                  90                  95
   Ala Pro Thr Tyr Gly Ile Pro Val Val Leu His Thr Asp His Cys Ala
               100                 105                 110
   Lys Lys Leu Leu Pro Trp Phe Asp Gly Met Leu Lys Ala Asp Glu Glu
           115                 120                 125
   Phe Phe Ala Lys Thr Gly Thr Pro Leu Phe Ser Ser His Met Leu Asp
       130                 135                 140
   Leu Ser Glu Glu Thr Asp Asp Glu Asn Ile Ala Thr Cys Ala Lys Tyr
   145 150 155 160
   Phe Glu Arg Met Ala Lys Met Gly Gln Trp Leu Glu Met Glu Ile Gly
   165 170 175
   Ile Thr Gly Gly Glu Glu Asp Gly Val Asn Asn Glu His Val Glu Lys
   180 185 190
   Asp Ala Leu Tyr Thr Ser Pro Glu Thr Val Phe Ala Val Tyr Glu Ser
   195 200 205
   Leu His Lys Ile Ser Pro Asn Phe Ser Ile Ala Ala Ala Phe Gly Asn
   210 215 220
   Val His Gly Val Tyr Lys Pro Gly Asn Val Gln Leu Arg Pro Glu Ile
   225 230 235 240
   Leu Gly Asp His Gln Val Tyr Ala Lys Lys Gln Ile Gly Thr Asp Ala
   245 250 255
   Lys His Pro Leu Tyr Leu Val Phe His Gly Gly Ser Gly Ser Thr Gln
   260 265 270
   Glu Glu Phe Asn Thr Ala Ile Lys Asn Gly Val Val Lys Val Asn Leu
   275 280 285
   Asp Thr Asp Cys Gln Tyr Ala Tyr Leu Thr Gly Ile Arg Asp Tyr Val
   290 295 300
   Thr Asn Lys Ile Glu Tyr Leu Lys Ala Pro Val Gly Asn Pro Glu Gly
   305 310 315 320
   Ala Asp Lys Pro Asn Lys Lys Tyr Phe Asp Pro Arg Val Trp Val Arg
   325 330 335
   Glu Gly Glu Lys Thr Met Ser Lys Arg Ile Ala Glu Ala Leu Asp Ile
   340 345 350
   Phe His Thr Lys Gly Gln Leu
   355
<210> 157
   <211> 528
   <212> PRT
   <213> Candida albicans
<400> 157
   Met Ala Ile Val Glu Thr Val Ile Asp Gly Ile Asn Tyr Phe Leu Ser
   1               5                   10                  15
   Leu Ser Val Thr Gln Gln Ile Ser Ile Leu Leu Gly Val Pro Phe Val
               20                  25                  30
   Tyr Asn Leu Val Trp Gln Tyr Leu Tyr Ser Leu Arg Lys Asp Arg Ala
           35                  40                  45
   Pro Leu Val Phe Tyr Trp Ile Pro Trp Phe Gly Ser Ala Ala Ser Tyr
       50                  55                  60
   Gly Gln Gln Pro Tyr Glu Phe Phe Glu Ser Cys Arg Gln Lys Tyr Gly
   65                  70                  75                  80
   Asp Val Phe Ser Phe Met Leu Leu Gly Lys Ile Met Thr Val Tyr Leu
                   85                  90                  95
   Gly Pro Lys Gly His Glu Phe Val Phe Asn Ala Lys Leu Ser Asp Val
               100                 105                 110
   Ser Ala Glu Asp Ala Tyr Lys His Leu Thr Thr Pro Val Phe Gly Lys
           115                 120                 125
   Gly Val Ile Tyr Asp Cys Pro Asn Ser Arg Leu Met Glu Gln Lys Lys
       130                 135                 140
   Phe Ala Lys Phe Ala Leu Thr Thr Asp Ser Phe Lys Arg Tyr Val Pro
   145                 150                 155                 160
   Lys Ile Arg Glu Glu Ile Leu Asn Tyr Phe Val Thr Asp Glu Ser Phe
                   165                 170                 175
   Lys Leu Lys Glu Lys Thr His Gly Val Ala Asn Val Met Lys Thr Gln
               180                 185                 190
   Pro Glu Ile Thr Ile Phe Thr Ala Ser Arg Ser Leu Phe Gly Asp Glu
           195                 200                 205
   Met Arg Arg Ile Phe Asp Arg Ser Phe Ala Gln Leu Tyr Ser Asp Leu
       210                 215                 220
   Asp Lys Gly Phe Thr Pro Ile Asn Phe Val Phe Pro Asn Leu Pro Leu
   225                 230                 235                 240
   Pro His Tyr Trp Arg Arg Asp Ala Ala Gln Lys Lys Ile Ser Ala Thr
                   245                 250                 255
   Tyr Met Lys Glu Ile Lys Ser Arg Arg Glu Arg Gly Asp Ile Asp Pro
               260                 265                 270
   Asn Arg Asp Leu Ile Asp Ser Leu Leu Ile His Ser Thr Tyr Lys Asp
           275                 280                 285
   Gly Val Lys Met Thr Asp Gln Glu Ile Ala Asn Leu Leu Ile Gly Ile
       290                 295                 300
   Leu Met Gly Gly Gln His Thr Ser Ala Ser Thr Ser Ala Trp Phe Leu
   305                 310                 315                 320
   Leu His Leu Gly Glu Lys Pro His Leu Gln Asp Val Ile Tyr Gln Glu
                   325                 330                 335
   Val Val Glu Leu Leu Lys Glu Lys Gly Gly Asp Leu Asn Asp Leu Thr
               340                 345                 350
   Tyr Glu Asp Leu Gln Lys Leu Pro Ser Val Asn Asn Thr Ile Lys Glu
           355                 360                 365
   Thr Leu Arg Met His Met Pro Leu His Ser Ile Phe Arg Lys Val Thr
       370                 375                 380
   Asn Pro Leu Arg Ile Pro Glu Thr Asn Tyr Ile Val Pro Lys Gly His
   385                 390                 395                 400
   Tyr Val Leu Val Ser Pro Gly Tyr Ala His Thr Ser Glu Arg Tyr Phe
                   405                 410                 415
   Asp Asn Pro Glu Asp Phe Asp Pro Thr Arg Trp Asp Thr Ala Ala Ala
               420                 425                 430
   Lys Ala Asn Ser Val Ser Phe Asn Ser Ser Asp Glu Val Asp Tyr Gly
           435                 440                 445
   Phe Gly Lys Val Ser Lys Gly Val Ser Ser Pro Tyr Leu Pro Phe Gly
       450                 455                 460
   Gly Gly Arg His Arg Cys Ile Gly Glu Gln Phe Ala Tyr Val Gln Leu
   465                 470                 475                 480
   Gly Thr Ile Leu Thr Thr Phe Val Tyr Asn Leu Arg Trp Thr Ile Asp
                   485                 490                 495
   Gly Tyr Lys Val Pro Asp Pro Asp Tyr Ser Ser Met Val Val Leu Pro
               500                 505                 510
   Thr Glu Pro Ala Glu Ile Ile Trp Glu Lys Arg Glu Thr Cys Met Phe
           515                 520                 525
<210> 158
   <211> 743
   <212> PRT
   <213> Candida albicans
<400> 158
   Met Leu Lys Thr Asp Ser Leu Asp Phe His Ser Tyr Leu Pro Pro Tyr
   1               5                   10                  15
   Arg Ser Leu Ile Asn Pro Asn Ala Arg Tyr Asp Tyr Arg Thr His Ser
               20                  25                  30
   Leu Ile Pro Leu Thr Gln Asn Asp Leu Asn Leu Leu Arg Ile Ala Phe
           35                  40                  45
   Gln Lys Lys Lys Glu Ala Pro Pro Ser Ala Phe Lys Met Lys Tyr Lys
       50                  55                  60
   Ser Leu Leu Ser Asp Val Ser Arg Thr Ile Ser Met Arg Leu Ser Asn
   65                  70                  75                  80
   Ser Asn Leu Leu Ser Ser Ser Ser Ala Asn Asn Asn Asn Val Leu Leu
                   85                  90                  95
   Ser Pro Pro Pro Ser Ser Ser Ser Thr Leu Ser Thr Pro Cys Gly Asn
               100                 105                 110
   Ile Leu Asn Arg Ala Gly Thr Thr Ser Ser Asn Ile Ser Lys Ile Asn
           115                 120                 125
   Asn Leu Ser Gln Asn Gln Thr Gln Asn Gln Leu Pro Leu Phe Pro Ala
       130                 135                 140
   Glu Leu His Ile Lys Asn Leu Pro Val Glu Ile Leu Asp Tyr Ile Phe
   145                 150                 155                 160
   Tyr Leu Val Asp Asp Asn Leu Asp Tyr Lys Ser Cys Met Tyr Thr Cys
                   165                 170                 175
   Lys Leu Phe Tyr Phe Leu Ala Lys Pro Tyr Tyr Tyr Glu Asn Leu Val
               180                 185                 190
   Phe Thr Ser Thr Tyr Arg Phe Ala Gln Phe Val Thr Tyr Leu Arg Val
           195                 200                 205
   Asn Ser Glu Val Gly Gln Tyr Val Gln Ser Ile Asp Leu Ser Gly Ile
       210                 215                 220
   Lys Pro Gly Tyr Asp Glu Asp Glu Gln Glu Glu Gly Gln Glu Glu Asn
   225                 230                 235                 240
   Ala Glu Asn Gly Glu Glu Glu Asn Gly Gly Gly Ala Arg Asp Pro Gln
                   245                 250                 255
   Tyr Leu Leu Gly Glu Ile Ala Asp Asn Pro His His Glu Arg Val Asp
               260                 265                 270
   Gln Phe Pro Arg Gly Lys Ile Leu Ala Gly Trp Arg Asp Trp Lys Phe
           275                 280                 285
   Lys Asn Asn Pro Leu Tyr Thr Ile His Pro Ser Pro Ser Leu Thr Lys
       290                 295                 300
   Ile Ala Ser Asn Ser Gln Phe Ser Asn Val Ser Ser Lys Ser Ser Arg
   305                 310                 315                 320
   Ser Thr Ser Ser Lys Ser Ser Ser Ser Thr Thr Lys Lys Phe Val Lys
                   325                 330                 335
   Pro Phe Arg Tyr Phe Lys Ser Arg Lys Arg Lys Met Ser Tyr Ser Gly
               340                 345                 350
   Thr Thr Lys Leu Glu Arg Lys Ser Pro Arg Leu Glu Gln Leu Gln Leu
           355                 360                 365
   Asp Gln Tyr Ser Ser Asn Trp Asn Lys Arg Val Asn Ser His Pro Leu
       370                 375                 380
   Ile Asn Lys Phe Leu Leu His Tyr Ser Thr Ser Lys Asp Leu Pro Ile
   385                 390                 395                 400
   Gly Tyr Ile Leu His Met Ile Asn Leu Cys Pro Asn Ile Val Ser Leu
                   405                 410                 415
   Asn Leu Gly Asn Leu Ser Leu Ser Thr Asp Tyr Glu Ile Ser Arg Ser
               420                 425                 430
   Thr Ile His Lys Tyr Gln Asn Phe Asp Leu Ile Asn Asn Tyr Pro Lys
           435                 440                 445
   Asp Leu Ile Tyr Lys Val Asp Asn Ile Met Arg Leu Asn Asp Val Asp
       450                 455                 460
   Asp Val Tyr Ser Ile Asp Gly Ser Ile Leu Arg Phe Gly Asn Ile Asn
   465                 470                 475                 480
   Ser Gly Ser Ser Gly Ser Asn Trp Glu Arg Asn Gly Ser Ser Ser Asn
                   485                 490                 495
   Asn Arg Ile Leu Phe Lys Ser Asn Gln Ser Ile Ala Ser Thr Ala Ser
               500                 505                 510
   Ser Val Tyr Ser Val Thr Thr Phe Ser Lys Pro Ile Arg Lys Tyr Asn
           515                 520                 525
   Ser Leu Leu Pro Pro Leu Pro Gln Thr Val Ala Asp Ile Ser Tyr Leu
       530                 535                 540
   Asn Lys Gly Asp Gly Lys Val Tyr Leu Ser Asp Leu Asn Leu Lys Glu
   545                 550                 555                 560
   Ile Asn Ser Ala Tyr Leu Lys Lys Ile Asn Glu Asp Glu Ile Leu Ser
                   565                 570                 575
   Ala Ile Ile Asn Val His Gly Lys Arg Leu Ile Glu Tyr Asp Thr Ser
               580                 585                 590
   Leu Tyr Gln Ile Pro Lys Pro Leu Asn Val Asp Ile Ala Gly Thr Leu
           595                 600                 605
   Lys Tyr Ile Asn Leu Ser Ser Met Ile Trp Leu Asn Arg Lys Leu Ile
       610                 615                 620
   Glu Lys Phe Leu Thr Arg Leu Leu Thr Lys Lys Ser Pro Glu Leu Asp
   625                 630                 635                 640
   Met Tyr Gly Ile Cys Tyr Thr Asp Glu Phe Phe Asp Ser Asp Glu Gln
                   645                 650                 655
   Glu Ser Asp Asp Asp Tyr Glu Asp Ser Asp Asp Glu Glu Gln Arg Gln
               660                 665                 670
   Cys Pro Ile Ile Tyr Lys Gln Asn Leu Val Ile Asp Phe Thr Asp Ser
           675                 680                 685
   Gly Met Tyr Lys Ser Leu Pro Trp Ala Lys Arg Ile Asp Leu Asn Ser
       690                 695                 700
   Phe Glu Gly Cys Gln Leu Ala Asn Lys Ile Ile Asn Asn Asp Leu Met
   705                 710                 715                 720
   Thr Pro Gln Glu Gln Ala Leu Arg Arg Glu Arg Arg Arg Arg Gly Ala
                   725                 730                 735
   Ile Ala Ala Asn Tyr Leu Ala
               740
<210> 159
   <211> 213
   <212> PRT
   <213> Candida albicans
<400> 159
   Met Ser Phe Ser Asp Phe Ser Lys Val Glu Ser Ile Lys Ser Leu Asn
   1               5                   10                  15
   Glu Phe Leu Ala Asp Lys Ser Tyr Ile Asp Gly Thr Thr Ala Thr Gln
               20                  25                  30
   Ala Asp Val Thr Val Tyr Lys Ala Phe Gln Lys Glu Phe Pro Gln Phe
           35                  40                  45
   Thr Arg Trp Phe Asn His Ile Ala Ser Phe Thr Glu Glu Phe Glu Asp
       50                  55                  60
   Leu Pro Ala Gly Lys Ala Pro Ala Ala Ser Gly Ser Ala Ala Ala Ala
   65                  70                  75                  80
   Ala Glu Glu Glu Asp Asp Glu Asp Val Asp Leu Phe Gly Ser Asp Asp
                   85                  90                  95
   Glu Val Asp Glu Glu Ala Glu Lys Leu Lys Gln Gln Arg Leu Ala Glu
               100                 105                 110
   Tyr Ala Ala Lys Lys Ala Ala Lys Gly Pro Lys Pro Ala Ala Lys Ser
           115                 120                 125
   Ile Val Thr Leu Asp Val Lys Pro Trp Asp Asp Glu Thr Asp Leu Asp
       130                 135                 140
   Glu Leu Leu Thr Asn Val Lys Ala Ile Glu Met Glu Gly Leu Thr Trp
   145                 150                 155                 160
   Gly Ala His Gln Trp Ile Pro Val Gly Phe Gly Ile Lys Lys Leu Gln
                   165                 170                 175
   Ile Asn Leu Val Val Glu Asp Ala Leu Val Ser Leu Asp Asp Leu Gln
               180                 185                 190
   Ala Ala Val Glu Glu Asp Glu Asp His Val Gln Ser Thr Asp Ile Ala
           195                 200                 205
   Ala Met Gln Lys Leu
       210
<210> 160
   <211> 440
   <212> PRT
   <213> Candida albicans
<400> 160
<210> 161
   <211> 331
   <212> PRT
   <213> Candida albicans
<400> 161
   Met Ala Ile Lys Ile Gly Ile Asn Gly Phe Gly Arg Ile Gly Arg Leu
   1               5                   10                  15
   Val Leu Arg Val Ala Leu Gly Arg Lys Asp Ile Glu Val Val Ala Val
               20                  25                  30
   Asn Asp Pro Phe Ile Ala Pro Asp Tyr Ala Ala Tyr Met Phe Lys Tyr
           35                  40                  45
   Asp Ser Thr His Gly Arg Tyr Lys Gly Glu Val Thr Ala Ser Gly Asp
       50                  55                  60
   Asp Leu Val Ile Asp Gly His Lys Ile Lys Val Phe Gln Glu Arg Asp
   65                  70                  75                  80
   Pro Ala Asn Ile Pro Trp Gly Lys Ser Gly Val Asp Tyr Val Ile Glu
                   85                  90                  95
   Ser Thr Gly Val Phe Thr Lys Val Glu Gly Ala Gln Lys His Ile Asp
               100                 105                 110
   Ala Gly Ala Lys Lys Val Ile Ile Thr Ala Pro Ser Ala Asp Ala Pro
           115                 120                 125
   Met Phe Val Val Gly Val Asn Glu Asp Lys Tyr Thr Pro Asp Leu Lys
       130                 135                 140
   Ile Ile Ser Asn Ala Ser Cys Thr Thr Asn Cys Leu Ala Pro Leu Ala
   145                 150                 155                 160
   Lys Val Val Asn Asp Thr Phe Gly Ile Glu Glu Gly Leu Met Thr Thr
                   165                 170                 175
   Val His Ser Ile Thr Ala Thr Gln Lys Thr Val Asp Gly Pro Ser His
               180                 185                 190
   Lys Asp Trp Arg Gly Gly Arg Thr Ala Ser Gly Asn Ile Ile Pro Ser
           195                 200                 205
   Ser Thr Gly Ala Ala Lys Ala Val Gly Lys Val Ile Pro Glu Leu Asn
       210                 215                 220
   Gly Lys Leu Thr Gly Met Ser Leu Arg Leu Pro Thr Thr Asp Val Ser
   225                 230                 235                 240
   Val Val Asp Leu Thr Val Arg Leu Lys Lys Ala Ala Ser Tyr Glu Glu
                   245                 250                 255
   Ile Ala Pro Ala Ile Lys Lys Ala Ser Glu Gly Pro Leu Lys Gly Val
               260                 265                 270
   Leu Gly Tyr Thr Glu Asp Ala Val Val Ser Thr Asp Phe Leu Gly Ser
           275                 280                 285
   Ser Tyr Ser Ser Ile Phe Asp Glu Lys Ala Gly Ile Leu Leu Ser Pro
       290                 295                 300
   Thr Phe Val Lys Leu Ile Ser Trp Tyr Asp Asn Glu Tyr Gly Tyr Ser
   305                 310                 315                 320
   Thr Lys Val Val Asp Leu Leu Glu His Val Ala
                   325                 330
<210> 162
   <211> 656
   <212> PRT
   <213> Candida albicans
<400> 162
   Met Ser Lys Ala Val Gly Ile Asp Leu Gly Thr Thr Tyr Ser Cys Val
   1               5                   10                  15
   Ala His Phe Ala Asn Asp Arg Val Glu Ile Ile Ala Asn Asp Gln Gly
               20                  25                  30
   Asn Arg Thr Thr Pro Ser Phe Val Ala Phe Thr Asp Thr Glu Arg Leu
           35                  40                  45
   Ile Gly Asp Ala Ala Lys Asn Gln Ala Ala Met Asn Pro Ala Asn Thr
       50                  55                  60
   Val Phe Asp Ala Lys Arg Leu Ile Gly Arg Lys Phe Asp Asp Pro Glu
   65                  70                  75                  80
   Val Ile Asn Asp Ala Lys His Phe Pro Phe Lys Val Ile Asp Lys Ala
                   85                  90                  95
   Gly Lys Pro Val Ile Gln Val Glu Tyr Lys Gly Glu Thr Lys Thr Phe
               100                 105                 110
   Ser Pro Glu Glu Ile Ser Ser Met Val Leu Thr Lys Met Lys Glu Ile
           115                 120                 125
   Ala Glu Gly Tyr Leu Gly Ser Thr Val Lys Asp Ala Val Val Thr Val
       130                 135                 140
   Pro Ala Tyr Phe Asn Asp Ser Gln Arg Gln Ala Thr Lys Asp Ala Gly
   145                 150                 155                 160
   Thr Ile Ala Gly Leu Asn Val Leu Arg Ile Ile Asn Glu Pro Thr Ala
                   165                 170                 175
   Ala Ala Ile Ala Tyr Gly Leu Asp Lys Lys Gly Ser Arg Gly Glu His
               180                 185                 190
   Asn Val Leu Ile Phe Asp Leu Gly Gly Gly Thr Phe Asp Val Ser Leu
           195                 200                 205
   Leu Ala Ile Asp Glu Gly Ile Phe Glu Val Lys Ala Thr Ala Gly Asp
       210                 215                 220
   Thr His Leu Gly Gly Glu Asp Phe Asp Asn Arg Leu Val Asn Phe Phe
   225                 230                 235                 240
   Ile Gln Glu Phe Lys Arg Lys Asn Lys Lys Asp Ile Ser Thr Asn Gln
                   245                 250                 255
   Arg Ala Leu Arg Arg Leu Arg Thr Ala Cys Glu Arg Ala Lys Arg Thr
               260                 265                 270
   Leu Ser Ser Ser Ala Gln Thr Ser Ile Glu Ile Asp Ser Leu Tyr Glu
           275                 280                 285
   Gly Ile Asp Phe Tyr Thr Ser Ile Thr Arg Ala Arg Phe Glu Glu Leu
       290                 295                 300
   Cys Ala Asp Leu Phe Arg Ser Thr Leu Asp Pro Val Gly Lys Val Leu
   305                 310                 315                 320
   Ala Asp Ala Lys Ile Asp Lys Ser Gln Val Glu Glu Ile Val Leu Val
                   325                 330                 335
   Gly Gly Ser Thr Arg Ile Pro Lys Ile Gln Lys Leu Val Ser Asp Phe
               340                 345                 350
   Phe Asn Gly Lys Glu Leu Asn Lys Ser Ile Asn Pro Asp Glu Ala Val
           355                 360                 365
   Ala Tyr Gly Ala Ala Val Gln Ala Ala Ile Leu Thr Gly Asp Thr Ser
       370                 375                 380
   Ser Lys Thr Gln Asp Ile Leu Leu Leu Asp Val Ala Pro Leu Ser Leu
   385                 390                 395                 400
   Gly Ile Glu Thr Ala Gly Gly Ile Met Thr Lys Leu Ile Pro Arg Asn
                   405                 410                 415
   Ser Thr Ile Pro Thr Lys Lys Ser Glu Thr Phe Ser Thr Tyr Ala Asp
               420                 425                 430
   Asn Gln Pro Gly Val Leu Ile Gln Val Phe Glu Gly Glu Arg Ala Lys
               435                 440                 445
   Thr Lys Asp Asn Asn Leu Leu Gly Lys Phe Glu Leu Ser Gly Ile Pro
       450                 455                 460
   Pro Ala Pro Arg Gly Val Pro Gln Ile Glu Val Thr Phe Asp Ile Asp
   465                 470                 475                 480
   Ala Asn Gly Ile Leu Asn Val Ser Ala Leu Glu Lys Gly Thr Gly Lys
                   485                 490                 495
   Thr Gln Lys Ile Thr Ile Thr Asn Asp Lys Gly Arg Leu Ser Lys Glu
               500                 505                 510
   Glu Ile Asp Lys Met Val Ser Glu Ala Glu Lys Phe Lys Glu Glu Asp
           515                 520                 525
   Glu Lys Glu Ala Ala Arg Val Gln Ala Lys Asn Gln Leu Glu Ser Tyr
       530                 535                 540
   Ala Tyr Ser Leu Lys Asn Thr Ile Asn Asp Gly Glu Met Lys Asp Lys
   545                 550                 555                 560
   Ile Gly Ala Asp Asp Lys Glu Lys Leu Thr Lys Ala Ile Asp Glu Thr
                   565                 570                 575
   Ile Ser Trp Leu Asp Ala Ser Gln Ala Ala Ser Thr Glu Glu Tyr Glu
               580                 585                 590
   Asp Lys Arg Lys Glu Leu Glu Ser Val Ala Asn Pro Ile Ile Ser Gly
           595                 600                 605
   Ala Tyr Gly Ala Ala Gly Gly Ala Pro Gly Gly Ala Gly Gly Phe Pro
       610                 615                 620
   Gly Ala Gly Gly Phe Pro Gly Gly Ala Pro Gly Ala Gly Gly Pro Gly
   625                 630                 635                 640
   Gly Ala Thr Gly Gly Glu Ser Ser Gly Pro Thr Val Glu Glu Val Asp
                   645                 650                 655
<210> 163
   <211> 613
   <212> PRT
   <213> Candida albicans
<400> 163
   Met Ala Asp Gly Val Phe Gln Gly Ala Ile Gly Ile Asp Leu Gly Thr
   1               5                   10                  15
   Thr Tyr Ser Cys Val Ala Thr Tyr Asp Ser Ala Val Glu Ile Ile Ala
               20                  25                  30
   Asn Glu Gln Gly Asn Arg Val Thr Pro Ser Phe Val Ala Phe Thr Ser
           35                  40                  45
   Glu Glu Arg Leu Ile Gly Asp Ala Ala Lys Asn Gln Ala Ala Leu Asn
       50                  55                  60
   Pro Lys Asn Thr Val Phe Asp Ala Lys Arg Leu Ile Gly Arg Ala Phe
   65                  70                  75                  80
   Asp Asp Glu Ser Val Gln Lys Asp Ile Lys Ser Trp Pro Phe Lys Val
                   85                  90                  95
   Val Glu Ser Asn Gly Gln Pro Leu Ile Glu Val Glu Tyr Leu Asp Glu
               100                 105                 110
   Thr Lys Thr Phe Ser Pro Gln Glu Ile Ser Ser Met Val Leu Thr Lys
           115                 120                 125
   Met Lys Glu Ile Ala Glu Ala Lys Ile Gly Lys Lys Val Glu Lys Ala
       130                 135                 140
   Val Val Thr Val Pro Ala Tyr Phe Asn Asp Ala Gln Arg Gln Ala Thr
   145                 150                 155                 160
   Lys Asp Ala Gly Ala Ile Ala Gly Leu Asn Val Leu Arg Ile Ile Asn
                   165                 170                 175
   Glu Pro Thr Ala Ala Ala Ile Ala Tyr Gly Leu Gly Ala Gly Lys Ser
               180                 185                 190
   Glu Lys Glu Arg His Val Leu Ile Phe Asp Leu Gly Gly Gly Thr Phe
           195                 200                 205
   Asp Val Ser Leu Leu Asn Ile Thr Gly Gly Val Phe Thr Val Lys Ala
       210                 215                 220
   Thr Ala Gly Asp Thr His Leu Gly Gly Gln Asp Phe Asp Thr Asn Leu
   225                 230                 235                 240
   Leu Glu His Phe Lys Lys Glu Phe Gln Lys Lys Thr Gly Asn Asp Ile
                   245                 250                 255
   Ser Ser Asp Ala Arg Ala Leu Arg Arg Leu Arg Thr Ala Cys Glu Arg
               260                 265                 270
   Ala Lys Arg Ser Leu Ser Ser Gly Thr Gln Thr Thr Val Glu Ile Asp
           275                 280                 285
   Ser Leu Phe Asp Gly Glu Asp Phe Ser Ala Asn Ile Thr Arg Ala Arg
       290                 295                 300
   Phe Glu Asp Ile Asn Ser Ala Leu Phe Lys Ser Thr Leu Glu Pro Val
   305                 310                 315                 320
   Glu Gln Val Leu Glu Asp Ala Lys Ile Ser Lys Ser Gln Val Asp Glu
                   325                 330                 335
   Val Val Leu Val Gly Gly Ser Thr Arg Ile Pro Lys Val Gln Lys Leu
               340                 345                 350
   Leu Ser Asp Phe Phe Asp Gly Lys Gln Leu Glu Lys Ser Ile Asn Pro
           355                 360                 365
   Asp Glu Ala Val Ala Tyr Gly Ala Ala Val Gln Gly Ala Ile Leu Thr
       370                 375                 380
   Gly Gln Ser Thr Asn Asp Asp Thr Lys Asp Leu Leu Leu Leu Asp Val
   385                 390                 395                 400
   Ile Pro Leu Ser Leu Gly Val Ala Met Gln Gly Asn Val Leu Ala Pro
                   405                 410                 415
   Val Val Pro Arg Asn Thr Thr Val Pro Thr Ile Lys Arg Arg Thr Phe
               420                 425                 430
   Thr Thr Val Ala Asp His Gln Thr Thr Val Gln Phe Pro Val Tyr Gln
           435                 440                 445
   Gly Glu Arg Val Asn Cys Ser Glu Asn Thr Leu Leu Gly Glu Phe Asp
       450                 455                 460
   Leu Lys Asn Ile Pro Pro Met Gln Ala Gly Glu Pro Val Leu Glu Ala
   465                 470                 475                 480
   Ile Phe Glu Val Asp Ala Asn Gly Ile Leu Lys Val Thr Ala Val Glu
                   485                 490                 495
   Lys Ser Thr Gly Arg Ser Ala Asn Ile Thr Ile Ser Asn Ser Ile Gly
               500                 505                 510
   Arg Leu Ser Thr Glu Glu Ile Glu Lys Met Ile Ser Asp Ala Glu Lys
           515                 520                 525
   Phe Lys Ser Ser Asp Asp Ala Phe Ala Lys Arg His Glu Gln Lys Gln
   530 535 540
   Lys Leu Glu Ala Tyr Val Ala Ser Val Glu Ser Thr Val Thr Asp Pro
   545 550 555 560
   Val Leu Ser Ala Lys Leu Lys Lys Ser Ala Lys Asp Lys Ile Glu Ala
   565 570 575
   Ala Leu Ser Asp Ala Leu Gln Thr Leu Glu Ile Glu Glu Ser Ser Ala
   580 585 590
   Asp Asp Tyr Arg Lys Ala Glu Leu Ala Leu Lys Arg Ala Val Thr Lys
   595 600 605
   Gly Met Ala Thr Arg
   610
<210> 164
   <211> 92
   <212> PRT
   <213> Candida albicans
<400> 164
   Phe Thr Ile Pro Pro Asn His Glu Met Ile Phe Thr Thr Asp Asp Ala
   1 5 10 15
   Tyr Lys Thr Lys Cys Asp Asp Lys Val Met Ile Ile Asp Tyr Lys Asn
   20 25 30
   Ile Thr Lys Val Ile Ala Pro Gly Lys Ile Ile Tyr Val Asp Asp Gly
   35 40 45
   Val Leu Ser Phe Glu Val Ile Ser Val Asp Asp Gln Gln Thr Leu Lys
   50 55 60
   Val Arg Ser Leu Asn Ala Gly Met Ile Ser Ser His Lys Thr Ala Asn
   65 70 75 80
   Asp Val Leu Glu Leu Arg Val Leu Ser Thr Ser Gly
   85 90
<210> 165
   <211> 460
   <212> PRT
   <213> Candida albicans
<400> 165
   Met Leu Phe Leu Leu Phe Leu Leu Ile Thr Pro Ile Tyr Ala Gly Leu
   1               5                   10                  15
   Ile Phe Pro Thr Lys Pro Ser Ser Asp Pro Phe Tyr Asn Pro Pro Lys
               20                  25                  30
   Gly Phe Glu Lys Ala Ala Val Gly Asp Ile Leu Gln Ser Arg Glu Thr
           35                  40                  45
   Pro Lys Ser Ile Thr Gly Arg Phe Ala Pro Leu Lys Ile Gln Asn Ser
       50                  55                  60
   Trp Gln Leu Leu Val Arg Ser Glu Asp Ser Phe Gly Asn Pro Asn Ala
   65                  70                  75                  80
   Ile Val Thr Thr Val Ile Glu Pro Val Asn Ala Asp Pro Ser Lys Ile
                   85                  90                  95
   Ala Ser Tyr Gln Val Phe Glu Asp Ala Ala Lys Ala Asp Cys Ala Pro
               100                 105                 110
   Ser Tyr Ala Leu Gln Phe Gly Ser Asp Leu Thr Thr Phe Val Thr Gln
           115                 120                 125
   Ala Glu Met Tyr Leu Met Ala Pro Leu Leu Asp Gln Gly Tyr Tyr Val
       130                 135                 140
   Val Ser Pro Asp Tyr Glu Gly Pro Lys Ser Thr Phe Thr Ile Gly Lys
   145                 150                 155                 160
   Gln Ser Gly Gln Ala Val Leu Asn Ser Ile Arg Ala Thr Leu Lys Ser
                   165                 170                 175
   Ser Lys Ile Thr Asn Ile Lys Glu Asp Ala Lys Val Val Met Trp Gly
               180                 185                 190
   Tyr Ser Gly Gly Ser Leu Ala Ser Gly Trp Ala Ala Ala Leu Gln Pro
           195                 200                 205
   Ser Tyr Ala Pro Glu Leu Ser Ser Ser Leu Leu Gly Ala Ala Leu Gly
       210                 215                 220
   Gly Phe Val Thr Asn Ile Thr Ala Thr Ala Gln Ala Ala Asp Gly Thr
   225                 230                 235                 240
   Val Phe Ala Gly Ile Val Ala Asn Ala Leu Gly Gly Val Ala Asn Glu
                   245                 250                 255
   Tyr Pro Glu Phe Lys Ser Ile Leu Gln Ser Asp Thr Asp Lys Lys Ser
               260                 265                 270
   Val Phe Asp Glu Phe Asp Ser His Cys Leu Ala Asp Gly Val Ile Asp
               275                 280                 285
   Tyr Ile Asn Thr Ser Phe Leu Thr Gly Asp Asn Lys Ile Phe Lys Thr
       290                 295                 300
   Gly Trp Asp Ile Leu Lys Ser Pro Thr Ile Ala Lys Ile Val Glu Asp
   305                 310                 315                 320
   Asn Gly Leu Val Tyr Gln Lys Gln Leu Val Pro Lys Ile Pro Ile Phe
                   325                 330                 335
   Val Tyr His Gly Ser Ile Asp Gln Ile Val Pro Ile Val Asn Val Lys
               340                 345                 350
   Lys Thr Tyr Gln Asn Trp Cys Glu Gly Gly Ile Ser Ser Leu Glu Phe
           355                 360                 365
   Ala Glu Asp Gly Thr Asn Gly His Leu Thr Glu Thr Val Val Gly Ala
       370                 375                 380
   Pro Ala Ala Leu Thr Trp Ile Ile Asp Arg Phe Asn Gly Lys Gln Thr
   385                 390                 395                 400
   Val Ser Gly Cys Gln His Asp Lys Arg Leu Ser Asn Phe Gln Tyr Pro
                   405                 410                 415
   Asn Ile Ser Ser Ser Ile Leu Lys Tyr Phe Lys Val Ala Leu Asp Thr
               420                 425                 430
   Met Met Ser Asn Gly Leu Gly Ser Asp Ile Gln Lys Asp Lys Ile Thr
           435                 440                 445
   Pro Asp Asp Leu Arg Lys Phe Leu Leu Gly Gly Trp
       450                 455                 460
<210> 166
   <211> 151
   <212> PRT
   <213> Candida albicans
<400> 166
   Met Ser Ser Asp Trp Asp Ser Val Thr Ile Ile Gly Gln Lys Ala Arg
   1               5                   10                  15
   Val Gly Gly Gly Gly Pro Arg Glu Asn Val Ala Lys Thr Ser Ser Gln
               20                  25                  30
   Leu Asn Ala Ala Arg Arg Ala Gly Leu Val Val Gly Thr Glu Lys Lys
           35                  40                  45
   Tyr Gly Thr Ala Asn Thr Lys Ser Asn Pro Glu Gly Gln Arg Leu Thr
       50                  55                  60
   Lys Leu Asp Ala Thr Asp Asp Val Val Ala Val Lys Lys Val Asp Val
   65                  70                  75                  80
   Ser Val Gly Lys Ala Ile Gln Gln Ala Arg Gln Glu Lys Lys Leu Thr
                   85                  90                  95
   Gln Lys Glu Leu Ala Thr Lys Val Asn Glu Lys Pro Asn Val Ile Asn
               100                 105                 110
   Asp Tyr Glu Ala Gly Arg Ala Ile Pro Asn Gln Gln Leu Leu Ala Lys
           115                 120                 125
   Leu Glu Arg Ala Leu Gly Val Lys Leu Arg Gly Lys Asn Ile Gly Glu
       130                 135                 140
   Pro Leu Phe Ala Lys Lys Lys
   145                 150
<210> 167
   <211> 124
   <212> PRT
   <213> Candida albicans
<400> 167
   Met Ser Val Asp Phe Asn Ala Val Ala Thr Glu Phe Cys Asn Phe Tyr
   1               5                   10                  15
   Tyr Asn Gln Phe Asp Ser Asp Arg Ser Gln Leu Gly Asn Leu Tyr Arg
               20                  25                  30
   Asn Glu Ser Met Leu Thr Phe Glu Thr Ser Gln Leu Gln Gly Ala Arg
           35                  40                  45
   Asp Ile Val Glu Lys Leu Ala Ser Leu Pro Phe Gln Lys Val Ala His
       50                  55                  60
   Arg Ile Ser Thr Leu Asp Ala Gln Pro Ala Ser Ala Asn Gly Asp Ile
   65                  70                  75                  80
   Leu Val Met Val Thr Gly Glu Leu Leu Ile Asp Glu Glu Gln Asn Ala
                   85                  90                  95
   Gln Arg Tyr Ser Gln Val Phe His Leu Ile Pro Asp Asn Gly Ser Tyr
               100                 105                 110
   Tyr Val Phe Asn Asp Ile Phe Arg Leu Asn Tyr Ser
                       115                 120
<210> 168
   <211> 417
   <212> PRT
   <213> Candida albicans
<400> 168
   Met Ser Leu Ser Asn Lys Leu Ser Val Lys Asp Leu Asp Val Ala Gly
   1               5                   10                  15
   Lys Arg Val Phe Ile Arg Val Asp Phe Asn Val Pro Leu Asp Gly Lys
               20                  25                  30
   Thr Ile Thr Asn Asn Gln Arg Ile Val Ala Ala Leu Pro Thr Ile Lys
           35                  40                  45
   Tyr Val Glu Glu His Lys Pro Lys Tyr Ile Val Leu Ala Ser His Leu
       50                  55                  60
   Gly Arg Pro Asn Gly Glu Arg Asn Asp Lys Tyr Ser Leu Ala Pro Val
   65                  70                  75                  80
   Ala Thr Glu Leu Glu Lys Leu Leu Gly Gln Lys Val Thr Phe Leu Asn
                   85                  90                  95
   Asp Cys Val Gly Pro Glu Val Thr Lys Ala Val Glu Asn Ala Lys Asp
               100                 105                 110
   Gly Glu Ile Phe Leu Leu Glu Asn Leu Arg Tyr His Ile Glu Glu Glu
           115                 120                 125
   Gly Ser Ser Lys Asp Lys Asp Gly Lys Lys Val Lys Ala Asp Pro Glu
       130                 135                 140
   Ala Val Lys Lys Phe Arg Gln Glu Leu Thr Ser Leu Ala Asp Val Tyr
   145                 150                 155                 160
   Ile Asn Asp Ala Phe Gly Thr Ala His Arg Ala His Ser Ser Met Val
                   165                 170                 175
   Gly Leu Glu Val Pro Gln Arg Ala Ala Gly Phe Leu Met Ser Lys Glu
               180                 185                 190
   Leu Glu Tyr Phe Ala Lys Ala Leu Glu Asn Pro Glu Arg Pro Phe Leu
           195                 200                 205
   Ala Ile Leu Gly Gly Ala Lys Val Ser Asp Lys Ile Gln Leu Ile Asp
       210                 215                 220
   Asn Leu Leu Asp Lys Val Asp Met Leu Ile Val Gly Gly Gly Met Ala
   225 230 235 240
   Phe Thr Phe Lys Lys Ile Leu Asn Lys Met Pro Ile Gly Asp Ser Leu
   245 250 255
   Phe Asp Glu Ala Gly Ala Lys Asn Val Glu His Leu Val Glu Lys Ala
   260 265 270
   Lys Lys Asn Asn Val Glu Leu Ile Leu Pro Val Asp Phe Val Thr Ala
   275 280 285
   Asp Lys Phe Asp Lys Asp Ala Lys Thr Ser Ser Ala Thr Asp Ala Glu
   290 295 300
   Gly Ile Pro Asp Asn Trp Met Gly Leu Asp Cys Gly Pro Lys Ser Val
   305 310 315 320
   Glu Leu Phe Gln Gln Ala Val Ala Lys Ala Lys Thr Ile Val Trp Asn
   325 330 335
   Gly Pro Pro Gly Val Phe Glu Phe Glu Lys Phe Ala Asn Gly Thr Lys
   340 345 350
   Ser Leu Leu Asp Ala Ala Val Lys Ser Ala Glu Asn Gly Asn Ile Val
   355 360 365
   Ile Ile Gly Gly Gly Asp Thr Ala Thr Val Ala Lys Lys Tyr Gly Val
   370 375 380
   Val Glu Lys Leu Ser His Val Ser Thr Gly Gly Gly Ala Ser Leu Glu
   385 390 395 400
   Leu Leu Glu Gly Lys Asp Leu Pro Gly Val Val Ala Leu Ser Asn Lys
   405 410 415
   Asn
<210> 169
   <211> 162
   <212> PRT
   <213> Candida albicans
<400> 169
   Met Ser Thr Val Tyr Phe Asp Val Ser Ala Asp Gly Gln Lys Leu Gly
   1               5                   10                  15
   Lys Ile Thr Phe Lys Leu Tyr Asp Asp Val Val Pro Lys Thr Ala Glu
               20                  25                  30
   Asn Phe Arg Ala Leu Cys Thr Gly Glu Lys Gly Phe Gly Tyr Lys Gly
           35                  40                  45
   Ser Ile Phe His Arg Val Ile Pro Gln Phe Met Leu Gln Gly Gly Asp
       50                  55                  60
   Phe Thr Asn Phe Asn Gly Thr Gly Gly Lys Ser Ile Tyr Gly Thr Lys
   65                  70                  75                  80
   Phe Ala Asp Glu Asn Phe Val Lys Arg His Asp Arg Pro Gly Leu Leu
                   85                  90                  95
   Ser Met Ala Asn Ala Gly Pro Asn Thr Asn Gly Ser Gln Phe Phe Ile
               100                 105                 110
   Thr Thr Val Pro Cys Pro Trp Leu Asp Gly Lys His Val Val Phe Gly
           115                 120                 125
   Glu Val Thr Asp Gly Leu Asp Ile Val Lys Lys Ile Glu Ser Phe Gly
       130                 135                 140
   Ser Gly Ser Gly Ala Thr Ser Lys Lys Ile Val Ile Glu Glu Ser Gly
   145                 150                 155                 160
   Gln Leu
<210> 170
   <211> 202
   <212> PRT
   <213> Candida albicans
<400> 170
   Met Ala Ile Ser Lys Asn Leu Pro Leu Leu Asn Asn His Phe Arg Lys
   1               5                   10                  15
   His Trp Gln Glu Arg Val Arg Val His Phe Asp Gln Ala Gly Lys Lys
               20                  25                  30
   Ala Ser Arg Arg Gln Ser Arg Leu Arg Lys Ala Ala Lys Ile Ala Pro
           35                  40                  45
   Arg Pro Ile Asp Ala Leu Arg Pro Val Val Arg Ala Pro Thr Val Lys
       50                  55                  60
   Tyr Asn Arg Lys Val Arg Ala Gly Arg Gly Phe Thr Leu Ala Glu Leu
   65                  70                  75                  80
   Lys Ala Val Gly Ile Ala Pro Lys Tyr Ala Arg Thr Ile Gly Ile Ser
                   85                  90                  95
   Val Asp His Arg Arg Gln Asn Lys Ser Gln Glu Thr Phe Asp Ala Asn
               100                 105                 110
   Val Ala Arg Leu Gln Glu Tyr Lys Ser Lys Leu Val Ile Phe Asp Lys
           115                 120                 125
   Lys Thr Lys Ala Ser Glu Val Ala Ser Phe Glu Gln Val Asp Val Ser
       130                 135                 140
   Ala Thr Phe Pro Val Glu Gln Pro Ala Pro Glu Ser Gly Leu Arg Ala
   145                 150                 155                 160
   Val Glu Val Pro Glu Gln Thr Ala Tyr Arg Thr Leu Arg Leu Ala Arg
                   165                 170                 175
   Asn Glu Lys Lys Tyr Lys Gly Ile Arg Glu Lys Arg Ala Lys Glu Lys
               180                 185                 190
   Ala Glu Ala Glu Ala Glu Lys Ala Lys Lys
           195                 200
<210> 171
   <211> 62
   <212> PRT
   <213> Candida albicans
<400> 171
   Glu Lys Lys Asp Glu Tyr Leu Ser Lys Ser Ser Ala Ser Ala Ala Pro
   1               5                   10                  15
   Val Ile Asp Thr Leu Ala His Gly Tyr Gly Lys Val Leu Gly Lys Gly
               20                  25                  30
   Arg Leu Pro Glu Val Pro Val Ile Val Lys Ala Arg Phe Val Ser Lys
           35                  40                  45
   Leu Ala Glu Glu Lys Ser Glu Ser Leu Val Val Leu Ser Asn
       50                  55                  60
<210> 172
   <211> 99
   <212> PRT
   <213> Candida albicans
<400> 172
   Met Ala Lys Ser Gly Ile Ala Ala Gly Val Asn Lys Gly Arg Lys Thr
   1               5                   10                  15
   Thr Ala Lys Glu Val Ala Pro Lys Ile Ser Tyr Arg Lys Gly Ala Ser
               20                  25                  30
   Ser Gln Arg Thr Val Phe Val Arg Ser Ile Val Lys Glu Val Ala Gly
           35                  40                  45
   Leu Ala Pro Tyr Glu Arg Arg Leu Ile Glu Leu Ile Arg Asn Ala Gly
       50                  55                  60
   Glu Lys Arg Ala Lys Lys Leu Ala Lys Lys Arg Leu Gly Thr His Lys
   65                  70                  75                  80
   Arg Ala Leu Arg Lys Val Glu Glu Met Thr Gln Val Ile Ala Glu Ser
                   85                  90                  95
   Arg Arg His
<210> 173
   <211> 111
   <212> PRT
   <213> Candida albicans
<400> 173
   Met Lys Tyr Leu Ala Ala Tyr Leu Leu Leu Val Gln Gly Gly Asn Thr
   1               5                   10                  15
   Ser Pro Ser Ala Ser Asp Ile Thr Ala Leu Leu Glu Ser Val Gly Val
               20                  25                  30
   Glu Ala Glu Glu Ser Arg Leu Gln Ala Leu Leu Lys Asp Leu Glu Gly
           35                  40                  45
   Lys Asp Leu Gln Glu Leu Ile Ala Glu Gly Asn Thr Lys Leu Ala Ser
       50                  55                  60
   Val Pro Ser Gly Gly Ala Ala Ala Gly Gly Ala Ser Ala Ser Thr Gly
   65                  70                  75                  80
   Ala Ala Ala Gly Gly Ala Ala Glu Ala Glu Glu Glu Lys Glu Glu Glu
                   85                  90                  95
   Ala Lys Glu Glu Ser Asp Asp Asp Met Gly Phe Gly Leu Phe Asp
               100                 105                 110
<210> 174
   <211> 130
   <212> PRT
   <213> Candida albicans
<400> 174
   Met Thr Arg Thr Ser Val Leu Ala Asp Ala Leu Asn Ala Ile Asn Asn
   1               5                   10                  15
   Ala Glu Lys Thr Gly Lys Arg Gln Val Leu Ile Arg Pro Ser Ser Lys
               20                  25                  30
   Val Ile Ile Lys Phe Leu Thr Val Met Gln Lys His Gly Tyr Ile Gly
           35                  40                  45
   Glu Phe Glu Tyr Ile Asp Asp His Arg Ser Gly Lys Ile Val Val Gln
       50                  55                  60
   Leu Asn Gly Arg Leu Asn Lys Cys Gly Val Ile Gln Pro Arg Phe Asn
   65                  70                  75                  80
   Val Lys Ile Asn Asp Ile Glu Arg Trp Thr Asp Asn Leu Leu Pro Ala
                   85                  90                  95
   Arg Gln Phe Gly Tyr Val Ile Leu Thr Thr Ser Ala Gly Ile Met Asp
               100                 105                 110
   His Glu Glu Ala Arg Arg Lys His Val Ser Gly Lys Ile Leu Gly Phe
           115                 120                 125
   Val Tyr
       130
<210> 175
   <211> 248
   <212> PRT
   <213> Candida albicans
<400> 175
   Met Ala Arg Gln Phe Phe Val Gly Gly Asn Phe Lys Ala Asn Gly Thr
   1               5                   10                  15
   Lys Gln Gln Ile Thr Ser Ile Ile Asp Asn Leu Asn Lys Ala Asp Leu
               20                  25                  30
   Pro Lys Asp Val Glu Val Val Ile Cys Pro Pro Ala Leu Tyr Leu Gly
           35                  40                  45
   Leu Ala Val Glu Gln Asn Lys Gln Pro Thr Val Ala Ile Gly Ala Gln
       50                  55                  60
   Asn Val Phe Asp Lys Ser Cys Gly Ala Phe Thr Gly Glu Thr Cys Ala
   65                  70                  75                  80
   Ser Gln Ile Leu Asp Val Gly Ala Ser Trp Thr Leu Thr Gly His Ser
                   85                  90                  95
   Glu Arg Arg Thr Ile Ile Lys Glu Ser Asp Glu Phe Ile Ala Glu Lys
               100                 105                 110
   Thr Lys Phe Ala Leu Asp Thr Gly Val Lys Val Ile Leu Cys Ile Gly
           115                 120                 125
   Glu Thr Leu Glu Glu Arg Lys Gly Gly Val Thr Leu Asp Val Cys Ala
       130                 135                 140
   Arg Gln Leu Asp Ala Val Ser Lys Ile Val Ser Asp Trp Ser Asn Ile
   145                 150                 155                 160
   Val Val Ala Tyr Glu Pro Val Trp Ala Ile Gly Thr Gly Leu Ala Ala
                   165                 170                 175
   Thr Pro Glu Asp Ala Glu Glu Thr His Lys Gly Ile Arg Ala His Leu
               180                 185                 190
   Ala Lys Ser Ile Gly Ala Glu Gln Ala Glu Lys Thr Arg Ile Leu Tyr
           195                 200                 205
   Gly Gly Ser Val Asn Gly Lys Asn Ala Lys Asp Phe Lys Asp Lys Ala
       210                 215                 220
   Asn Val Asp Gly Phe Leu Val Gly Gly Ala Ser Leu Lys Pro Glu Phe
   225                 230                 235                 240
   Val Asp Ile Ile Lys Ser Arg Leu
                   245
<210> 176
   <211> 663
   <212> PRT
   <213> Escherichia coli
<400> 176
   Met Ser Ser Arg Lys Glu Leu Ala Asn Ala Ile Arg Ala Leu Ser Met
   1               5                   10                  15
   Asp Ala Val Gln Lys Ala Lys Ser Gly His Pro Gly Ala Pro Met Gly
               20                  25                  30
   Met Ala Asp Ile Ala Glu Val Leu Trp Arg Asp Phe Leu Lys His Asn
           35                  40                  45
   Pro Gln Asn Pro Ser Trp Ala Asp Arg Asp Arg Phe Val Leu Ser Asn
       50                  55                  60
   Gly His Gly Ser Met Leu Ile Tyr Ser Leu Leu His Leu Thr Gly Tyr
   65                  70                  75                  80
   Asp Leu Pro Met Glu Glu Leu Lys Asn Phe Arg Gln Leu His Ser Lys
                   85                  90                  95
   Thr Pro Gly His Pro Glu Val Gly Tyr Thr Ala Gly Val Glu Thr Thr
               100                 105                 110
   Thr Gly Pro Leu Gly Gln Gly Ile Ala Asn Ala Val Gly Met Ala Ile
           115                 120                 125
   Ala Glu Lys Thr Leu Ala Ala Gln Phe Asn Arg Pro Gly His Asp Ile
       130                 135                 140
   Val Asp His Tyr Thr Tyr Ala Phe Met Gly Asp Gly Cys Met Met Glu
   145                 150                 155                 160
   Gly Ile Ser His Glu Val Cys Ser Leu Ala Gly Thr Leu Lys Leu Gly
                   165                 170                 175
   Lys Leu Ile Ala Phe Tyr Asp Asp Asn Gly Ile Ser Ile Asp Gly His
               180                 185                 190
   Val Glu Gly Trp Phe Thr Asp Asp Thr Ala Met Arg Phe Glu Ala Tyr
           195                 200                 205
   Gly Trp His Val Ile Arg Asp Ile Asp Gly His Asp Ala Ala Ser Ile
       210                 215                 220
   Lys Arg Ala Val Glu Glu Ala Arg Ala Val Thr Asp Lys Pro Ser Leu
   225                 230                 235                 240
   Leu Met Cys Lys Thr Ile Ile Gly Phe Gly Ser Pro Asn Lys Ala Gly
                   245                 250                 255
   Thr His Asp Ser His Gly Ala Pro Leu Gly Asp Ala Glu Ile Ala Leu
               260                 265                 270
   Thr Arg Glu Gln Leu Gly Trp Lys Tyr Ala Pro Phe Glu Ile Pro Ser
           275                 280                 285
   Glu Ile Tyr Ala Gln Trp Asp Ala Lys Glu Ala Gly Gln Ala Lys Glu
       290                 295                 300
   Ser Ala Trp Asn Glu Lys Phe Ala Ala Tyr Ala Lys Ala Tyr Pro Gln
   305                 310                 315                 320
   Glu Ala Ala Glu Phe Thr Arg Arg Met Lys Gly Glu Met Pro Ser Asp
                   325                 330                 335
   Phe Asp Ala Lys Ala Lys Glu Phe Ile Ala Lys Leu Gln Ala Asn Pro
               340                 345                 350
   Ala Lys Ile Ala Ser Arg Lys Ala Ser Gln Asn Ala Ile Glu Ala Phe
           355                 360                 365
   Gly Pro Leu Leu Pro Glu Phe Leu Gly Gly Ser Ala Asp Leu Ala Pro
       370                 375                 380
   Ser Asn Leu Thr Leu Trp Ser Gly Ser Lys Ala Ile Asn Glu Asp Ala
   385                 390                 395                 400
   Ala Gly Asn Tyr Ile His Tyr Gly Val Arg Glu Phe Gly Met Thr Ala
                   405                 410                 415
   Ile Ala Asn Gly Ile Ser Leu His Gly Gly Phe Leu Pro Tyr Thr Ser
               420                 425                 430
   Thr Phe Leu Met Phe Val Glu Tyr Ala Arg Asn Ala Val Arg Met Ala
           435                 440                 445
   Ala Leu Met Lys Gln Arg Gln Val Met Val Tyr Thr His Asp Ser Ile
       450                 455                 460
   Gly Leu Gly Glu Asp Gly Pro Thr His Gln Pro Val Glu Gln Val Ala
   465                 470                 475                 480
   Ser Leu Arg Val Thr Pro Asn Met Ser Thr Trp Arg Pro Cys Asp Gln
                   485                 490                 495
   Val Glu Ser Ala Val Ala Trp Lys Tyr Gly Val Glu Arg Gln Asp Gly
               500                 505                 510
   Pro Thr Ala Leu Ile Leu Ser Arg Gln Asn Leu Ala Gln Gln Glu Arg
           515                 520                 525
   Thr Glu Glu Gln Leu Ala Asn Ile Ala Arg Gly Gly Tyr Val Leu Lys
       530                 535                 540
   Asp Cys Ala Gly Gln Pro Glu Leu Ile Phe Ile Ala Thr Gly Ser Glu
   545                 550                 555                 560
   Val Glu Leu Ala Val Ala Ala Tyr Glu Lys Leu Thr Ala Glu Gly Val
                   565                 570                 575
   Lys Ala Arg Val Val Ser Met Pro Ser Thr Asp Ala Phe Asp Lys Gln
                   580                 585                 590
   Asp Ala Ala Tyr Arg Glu Ser Val Leu Pro Lys Ala Val Thr Ala Arg
               595                 600                 605
   Val Ala Val Glu Ala Gly Ile Ala Asp Tyr Trp Tyr Lys Tyr Val Gly
       610                 615                 620
   Leu Asn Gly Ala Ile Val Gly Met Thr Thr Phe Gly Glu Ser Ala Pro
   625                 630                 635                 640
   Ala Glu Leu Leu Phe Glu Glu Phe Gly Phe Thr Val Asp Asn Val Val
                   645                 650                 655
   Ala Lys Ala Lys Glu Leu Leu
                   660
<210> 177
   <211> 16
   <212> PRT
   <213> Candida albicans
<400> 177
   Gly His Tyr Val Leu Val Phe Pro Gly Tyr Ala His Thr Ser Glu Arg
   1               5                   10                  15

## Description

La présente invention concerne le domaine de la microbiologie. Plus précisément, l'invention concerne la caractérisation de microorganismes issus d'un échantillon en utilisant la spectrométrie de masse.

Depuis la découverte des microbes par Pasteur, les microorganismes sont étudiés par microscopie et analyses biochimiques. Ces méthodes traditionnelles sont souvent longues et fastidieuses et des alternatives analytiques ont très tôt été recherchées. C'est ainsi que l'analyse de bactéries par spectrométrie de masse a été initiée dès 1975 par J. Anhalt et C. Fenselau [1].

Ces travaux préliminaires ont été suivis par l'étude en chromatographie en phase gazeuse couplée à la spectrométrie de masse (GC-MS) d'acides gras de la paroi des microorganismes [2]. Cette méthode a été popularisée sous l'appellation anglo-saxonne de FAME pour Fatty Acid Methyl Ester. Elle constitue actuellement une méthode de référence pour les études taxonomiques. Son utilisation reste cependant limitée à certains laboratoires spécialisés maîtrisant le traitement de l'échantillon par saponification, hydrolyse et dérivation.

En 1996, les travaux de M. Claydon et al [3] ainsi que de T. Krishnamurthy et P. Ross [4] ont montré la possibilité d'identifier différentes espèces bactériennes avec un spectromètre de masse de type MALDI-TOF (acronyme de l'anglais Matrix Assisted Laser Desorption Ionization - Time Of Flight). L'analyse associe l'acquisition d'un spectre de masse et l'interprétation d'un logiciel expert. Elle est extrêmement simple et peut être effectuée en quelques minutes. Cependant elle ne se diffuse que depuis très peu de temps dans les laboratoires d'analyses médicales [5]. Son utilisation clinique est actuellement limitée à l'identification d'espèces bactériennes et de levures. Elle n'est utilisée ni pour le typage, ni pour l'identification des résistances aux antimicrobiens, ni pour l'analyse de la virulence.

Or la caractérisation des microorganismes est fondamentale tant dans le domaine clinique que le domaine industriel. Ainsi, par exemple, l'identification de résistances aux antimicrobiens tels que les antibiotiques, et la détection de facteurs de virulence sont des éléments essentiels pour assurer une prise en charge optimale des patients. De même, le typage est crucial pour les études épidémiologiques et pour assurer la lutte contre les maladies nosocomiales.

D'autres procédés de spectrométrie de masse, notamment en tandem, ont été proposés pour répondre à ces besoins. A titre d'exemple, il est possible de citer les travaux de C. Fenselau et al. pour l'identification de β-Lactamase avec un quadripôle-TOF (Q-TOF) [6], ceux de D. Ding et al. pour la détection d'entérotoxine staphylococcique C2 (facteur de virulence SEC2) avec un triple quadripôle [7], ou encore ceux de R. Everley et al. pour le typage de *Clostridium* avec un Q-TOF [8].

Cependant ces résultats de recherche ne sont pas applicables à une utilisation clinique de routine. Ils ont été obtenus avec des instruments de recherche nécessitant un personnel hautement qualifié. Les temps d'analyse, souvent supérieurs à une heure par échantillon, sont incompatibles avec la charge de travail d'un laboratoire d'analyse microbiologique. Enfin, les données obtenues par les différentes équipes répondent à une question spécifique, mais pas simultanément à l'ensemble des besoins cliniques.

Par ailleurs, Lange Vinzenz et al ont décrit l'analyse quantitative ciblée de facteurs de virulence chez Streptococcus pyogenes en spectrométrie de masse de type MRM [34].

Plus récemment S. Hofstadler et al. ont proposé un procédé répondant à l'ensemble des besoins cliniques [9]. Ils ont associé une amplification du génome microbien par PCR à une détection des produits de PCR par électrospray-TOF (ESI-TOF). Ce procédé est maintenant totalement automatisé [10]. Toutefois, il nécessite une amplification par PCR avec les défauts inhérents à la biologie moléculaire, à savoir coût des sondes, rendement d'extraction, etc.

Dans ce contexte, l'objectif de la présente invention est de proposer un procédé de caractérisation des microorganismes, à savoir identification et détermination des propriétés de typage, résistance à au moins un antimicrobien et facteur de virulence, qui permette de palier les inconvénients des procédés de l'art antérieur, à savoir fournir un procédé peu coûteux, sans réactifs spécifiques à chaque espèce, notamment par rapport aux procédés de biologie moléculaire, donnant un résultat en un temps court, inférieur à une heure, et utilisable en clinique de routine, sans nécessiter un personnel hautement qualifié. De plus, l'ensemble du procédé de caractérisation des microorganismes peut être avantageusement réalisé avec un même spectromètre de masse, ce qui simplifie l'instrumentation du laboratoire d'analyse microbiologique.

A cette fin, l'invention propose un nouveau procédé de caractérisation d'au moins un microorganisme issu d'un échantillon, comprenant l'identification dudit au moins un microorganisme et la détermination des propriétés de typage, résistance potentielle à au moins un antimicrobien et facteur de virulence, caractérisé en ce que la détermination des propriétés de typage, résistance à au moins un antimicrobien et facteur de virulence pour ledit au moins un microorganisme est mise en oeuvre par spectrométrie de masse en utilisant des protéines, peptides et/ou métabolites en tant que marqueurs desdites propriétés de typage, résistance à au moins un antimicrobien et facteur de virulence.

Les microorganismes qui peuvent être caractérisés par le procédé de l'invention sont tous des microorganismes, pathogènes ou non, rencontrés tant dans l'industrie que dans la clinique. Ce peut être des bactéries, des virus, des protozoaires ou des levures.

Par marqueurs des propriétés de typage, résistance à au moins un antimicrobien et facteur de virulence, on entend des molécules, d'origine protéique ou métabolique, qui sont caractéristiques desdites propriétés.

Par typage d'un microorganisme, on entend la différentiation de plusieurs souches au sein d'une même espèce. Le typage a une valeur épidémiologique, le clinicien sait si la souche isolée chez le patient provient de la même source que d'autres souches apparemment identiques et isolées chez d'autres patients ou dans l'environnement. Cela permet ainsi de mettre en évidence un foyer infectieux au sein d'un hôpital ou lors d'une intoxication alimentaire. A titre d'exemples non limitatifs de marqueurs de propriétés de typage chez les bactéries, on peut citer des peptides présentant des mutations caractéristiques tels que les produits de transcription des gènes *adk, fum*C, *gyr*B, *icd, mdh, pur*A et *rec*A d' *Escherichia coli,* et ceux des gènes *arc, aro*E, *glp*F, *gmk*, *pta, tpi* et *yqi*L de *Staphylococcus aureus.* A titre d'exemples non limitatifs de marqueurs de propriétés de typage chez les protozoaires, on peut citer les produits du gène chitinase d'*Entamoeba histolytica* et *E. dispar*. A titre d'exemples non limitatifs de marqueurs de propriétés de typage chez les virus, on peut citer les produits du gène polymérase du virus de l'immunodéficience humaine. Enfin, à titre d'exemples non limitatifs de marqueurs de propriétés de typage chez les levures, on peut citer les produits de transcription des fragments de gènes *aat*1a, *acc*1, *adp*1, *mpi*b, *sya*1, *vps*13, et *zwf*1b de *Candida albicans.*

Par détermination de la résistance à au moins un antimicrobien, on entend la détermination de la susceptibilité d'un microorganisme à être détruit par un antimicrobien. Ainsi, si le microorganisme est une bactérie, l'antimicrobien contre lequel il peut développer une résistance est un antibiotique, si c'est un protozoaire, l'antimicrobien est un anti-parasitaire, si c'est un virus, l'antimicrobien est un antiviral, et, si c'est une levure, l'antimicrobien est un anti-fongique Les protéines impliquées dans les mécanismes de résistance vont différer selon la famille et l'espèce. A titre d'exemples non limitatifs de marqueurs de résistance à au moins un antibiotique utiles chez les bactéries, on peut citer les produits de transcription du gène *mec*A de *Staphylococcus aureus,* conférant une résistance à la Méticilline, et permettant d'indiquer si les souches sont méthicillino-résistantes (souches MRSA) ou bien méthillicino-sensibles (souches MSSA). On peut également citer la protéine TEM-2 qui permet d'indiquer si les souches d' *Escherichia coli* sont résistantes aux pénicillines mais sensibles à d'autres classes d'antibiotiques type céphalosporines ou carbapénèmes. Un autre marqueur est l'enzyme appelée KPC (pour *Klebsiella Pneumoniae* Carbapenemase) qui confère une résistance aux carbapénèmes. Un autre exemple de marqueur de résistance pour *Staphylococcus aureus* est le profil métabolique représentatif de la résistance à la vancomycine tel que décrit par Alexander E. et al dans le poster « Metabolomics-based approach to antibiotic resistance in *Staphylococcus aureus*» présenté au congrès de l'ASMS, 2009. A titre d'exemple non limitatif de marqueurs de résistance à au moins un anti-parasitaire utile chez les protozoaires, on peut citer la superoxide dismutase contenant du fer (Fe-SOD) et la péroxyrédoxine dont l'expression accrue confère une résistance au métronidazole. A titre d'exemple non limitatif de marqueur de résistance à au moins un antiviral utile chez les virus, on peut citer les mutations de l'enzyme transcriptase inverse du virus de l'immunodéficience humaine, conférant une sensibilité diminuée aux inhibiteurs nucléosidiques de la transcriptase inverse. Enfin, à titre d'exemple non limitatif de marqueurs de résistance à au moins un antifongique utile chez les levures, on peut citer la mutation de l'enzyme 1-3-b-D-glucane synthase de *Candida albicans,* conférant une sensibilité diminuée aux échinocandines. Pour autre exemple, on peut évoquer la résistance aux antifongiques azolés chez *Candida albicans,* en particulier la résistance au fluconazole. La cible du fluconazole est une enzyme, la lanostérol déméthylase, impliquée dans la synthèse de l'ergostérol, constituant principal de la paroi fongique. La résistance au fluconazole peut être associée à l'apparition de mutations ponctuelles dans le gène erg11 codant pour la lanostérol déméthylase.

Il convient de noter que les marqueurs spécifiques de la résistance sont également utilisables comme marqueurs de typage, comme mis en évidence par la Demanderesse.

Par détermination de la virulence d'un microorganisme, on entend l'évaluation du caractère pathogène, nocif et violent du microorganisme. A titre d'exemples non limitatifs de marqueur de virulence chez les bactéries, on peut citer la PVL (Panton-Valentine Leucocidine), toxine cytolytique à deux composantes synergiques (LukFet LukS), présente chez *Staphylococcus aureus,* qui est l'une des toxines les plus virulentes causant des atteintes cutanées, de la cellulite extensive, de l'ostéomyélite et des pneumonies nécrosantes, et est impliquée dans des surinfections virales. D'autres exemples comprennent l'Autolysine et la Pneumolysine présents chez *Streptococcus pneumoniae,* espèce responsable d'infections des voies respiratoires, de méningites et de bactériémies, ainsi que les toxines A et B de *Clostridium difficile,* bactérie commensale de l'intestin, lesquelles provoquent soit l'altération de la perméabilité de l'épithélium intestinal (toxine A), soit s'attaquent directement aux cellules de l'épithélium (toxine B), soit diminuent dans le temps le transit intestinal et l'absorption intestinale, provoquant une diarrhée (action combinée des toxines A et B). On peut aussi citer comme exemple les Shiga toxines Stx1 et Stx2 présentes chez *Escherichia coli.* Ces deux cytotoxines sont considérés comme des facteurs de virulence importants des *Escherichia coli* entérohémorragiques. Elles sont responsables de complications comme la colite hémorragique ou le syndrome hémolytique-urémique. A titre d'exemple non limitatif de marqueur de virulence chez les protozoaires, on peut citer des antioxydants (Fe-hydrogénase 2, peroxiredoxine, superoxyde dismutase) présents chez *Entarnaoeba histolytica,* espèce responsable de dysenterie, abcès hépatique. A titre d'exemple non limitatif de marqueur de virulence chez les virus, on peut citer le variant de la protéine Nef chez le virus de l'immunodéficience humaine de type 1, type plus pathogène chez l'être humain. Enfin, à titre d'exemple non limitatif de marqueur de virulence chez les levures, on peut citer la lipase 8 chez *Candida albicans,* espèce responsable de candidoses superficielles mais aussi de candidoses septicémiques et disséminées.

Il convient de noter que les marqueurs spécifiques de la virulence sont également utilisables comme marqueur de typage, comme mis en évidence par la Demanderesse.

Le procédé de l'invention peut être mis en oeuvre pour caractériser des bactéries, ledit antimicrobien étant alors un antibiotique, ce qui constitue un mode de réalisation de l'invention. Ainsi, par exemple, à titre de bactéries pouvant caractérisées selon le procédé de l'invention, on peut citer :
- *Escherichia coli* en utilisant TEM-2 comme marqueur de résistance et de typage, ainsi que Shiga toxins, OmpA comme marqueur de virulence et de typage.
- *Enterococcus faecalis* et *faecium* en utilisant VanA et VanB pour la résistance et le typage, ainsi que ESP (Enterococcal Surface Protein) pour la virulence et le typage, ou bien
- *Staphylococcus aureus* en utilisant la protéine appelée Immunoglobulin G-binding protéine A (également appelée protéine A) pour le typage, la protéine PBP2a pour la résistance, voire le typage, ainsi que la protéine PVL pour la virulence, voire également le typage.

A titre d'autres microorganismes pouvant être caractérisés selon le procédé de l'invention, on peut citer :
- *Candida albicans* en utilisant l'enzyme 1-3-b-D-glucane synthase ou bien l'enzyme lanostérol déméthylase comme marqueur de résistance et de typage, ainsi que la lipase 8 comme marqueur de virulence et de typage.

L'échantillon sur lequel le procédé de l'invention peut être mis en oeuvre est tout échantillon susceptible de contenir un microorganisme cible. L'échantillon peut être d'origine biologique, soit animale, végétale ou humaine. Il peut alors correspondre à un prélèvement de fluide biologique (sang total, sérum, plasma, urine, liquide céphalo-rachidien, sécrétion organique, par exemple), un prélèvement tissulaire ou des cellules isolées. Ce prélèvement peut être utilisé tel quel dans la mesure où les marqueurs de caractérisation des microorganismes sont disponibles dans l'échantillon testé, ou bien il peut subir préalablement à l'analyse, une préparation de type enrichissement, extraction, concentration, purification, culture, selon des méthodes connues de l'homme du métier.

L'échantillon peut être d'origine industrielle, soit, selon une liste non exhaustive un prélèvement d'air, un prélèvement d'eau, un prélèvement effectué sur une surface, une pièce ou un produit manufacturé, un produit d'origine alimentaire. Parmi les échantillons d'origine alimentaire, on peut citer de façon non exhaustive un échantillon de produit lacté (yaourts, fromages,), de viande, de poisson, d'oeuf, de fruit, de légume, d'eau, de boisson (lait, jus de fruits, soda, etc). Ces échantillons d'origine alimentaire peuvent aussi provenir de sauces ou de plats élaborés. Un échantillon alimentaire peut enfin être issu d'une alimentation destinée aux animaux, telle que notamment des farines animales.

Lorsque les marqueurs de caractérisation des microorganismes sont d'origine protéique, en amont de la détection par spectrométrie de masse, l'échantillon à analyser est préférentiellement traité au préalable pour générer des peptides à partir de l'ensemble des protéines présentes dans l'échantillon pour fragmenter ces protéines en peptides, par exemple par digestion avec une enzyme protéolytique (protéase), ou par action d'un réactif chimique. En effet, le clivage des protéines peut être fait par un traitement physico-chimique, par un traitement biologique ou par une combinaison des deux traitements. Parmi les traitements utilisables, on peut citer le traitement par des radicaux hydroxyle, notamment avec de l'H₂O₂. Le traitement par les radicaux hydroxyle provoque une coupure des liaisons peptidiques qui se fait de manière aléatoire sur n'importe quelle liaison peptidique de la protéine. La concentration en radicaux hydroxyle conditionne le nombre de clivages opérés et donc la longueur des fragments peptidiques obtenus. D'autres traitements chimiques peuvent également être utilisés comme, par exemple, le traitement au bromure de cyanogène (CNBr) qui scinde spécifiquement les liaisons peptidiques au niveau du groupe carboxylique des résidus méthionyle. Il est également possible de réaliser un clivage acide partiel au niveau des résidus aspartyle par chauffage à 1000°C d'une solution de protéines dans de l'acide trifluoroacétique.

Le traitement des protéines par digestion enzymatique est néanmoins préféré par rapport au traitement physico-chimique car il préserve davantage la structure des protéines, et est plus facile à contrôler. Par « digestion enzymatique », on entend l'action simple ou combinée d'une ou de plusieurs enzymes dans des conditions de réaction appropriées. Les enzymes effectuant la protéolyse, appelées protéases, coupent les protéines à des endroits spécifiques. Chaque protéase reconnaît généralement une séquence d'acides aminés au sein desquels elle effectue toujours la même coupure. Certaines protéases reconnaissent un seul acide aminé ou une séquence de deux acides aminés entre lesquels elles opèrent un clivage, d'autres protéases ne reconnaissent que des séquences plus longues. Ces protéases peuvent être des endoprotéases ou des exoprotéases. Parmi les protéases connues on peut citer, comme décrit dans WO2005/098071 :
- les enzymes spécifiques comme la trypsine qui scinde la liaison peptidique au niveau du groupe carboxylique des résidus Arg et Lys, l'endolysine qui clive la liaison peptidique du groupe -CO des lysines, la chymotrypsine qui hydrolyse la liaison peptidique au niveau du groupe carboxylique des résidus aromatiques (Phe, Tyr et Trp), la pepsine qui coupe au niveau du groupe NH₂ des résidus aromatiques (Phe, Tyr et Trp), la protéase V8 de la souche V8 de *Staphylococcus aureus* qui clive la liaison peptidique au niveau du groupe carboxylique du résidu Glu ;
- les enzymes non-spécifiques comme la thermolysine provenant de la bactérie *Bacillus thermoproteolyticus* qui hydrolyse la liaison peptidique du groupe NH₂ des acides aminés hydrophobes (Xaa-Leu, Xaa-Ile, Xaa-Phe), la subtilisine et la pronase qui sont des protéases bactériennes qui hydrolysent pratiquement toutes les liaisons et peuvent transformer les protéines en oligopeptides dans des conditions de réaction contrôlées (concentration en enzyme et durée de réaction).

Plusieurs protéases peuvent être utilisées de façon simultanée, si leurs modes d'action sont compatibles, ou elles peuvent être utilisées de façon successive. Dans le cadre de l'invention, la digestion de l'échantillon est, de préférence, réalisée par action d'une enzyme protéase, par exemple la trypsine.

La génération de peptides à l'aide d'un réactif chimique ou d'une protéase, peut être obtenu par simple réaction en solution. Elle peut également être mise en oeuvre avec un four à micro-ondes [11], ou sous pression [12], ou bien encore avec un dispositif à ultrasons [13]. Dans ces trois derniers cas, le protocole sera beaucoup plus rapide.

Parmi les peptides ainsi obtenus, les peptides spécifiques de la protéine, sont nommés peptides protéotypiques. Ce sont eux qui seront dosés par spectrométrie de masse.

Selon un mode de réalisation de l'invention, les marqueurs de caractérisation sont des protéines du microorganisme à caractériser. En particulier, lesdites protéines sont digérées en peptides, de préférence par une enzyme, de préférence encore par la trypsine.

De même, l'échantillon contenant des marqueurs de caractérisation d'origine protéique peut également être préalablement traité à des fins de purification. Lorsque les marqueurs sont d'origine protéique, ce traitement préalable de purification peut être mis en oeuvre avant ou après l'étape de génération de peptides tels que décrits précédemment.

Le traitement préalable de purification d'échantillon est largement connu de l'homme du métier et pourra notamment mettre en oeuvre des techniques de centrifugation, de filtration, d'électrophorèse ou de chromatographie. Ces techniques séparatives peuvent être utilisées seules ou combinées entre elles pour obtenir une séparation multidimensionnelle. Par exemple, une chromatographie multidimensionnelle peut être utilisée en associant une séparation par chromatographie d'échange d'ions à une chromatographie en phase inverse, comme décrit par T. Fortin et al. [14], ou H. Keshishian et al. [15]. Dans ces publications, le milieu chromatographique peut être en colonne ou en cartouche (extraction en phase solide).

La fraction électrophorétique ou chromatographique (ou le temps de rétention en chromatographie mono ou multidimensionnelle) des peptides protéotypiques est caractéristique de chaque peptide et la mise en oeuvre de ces techniques permet donc de sélectionner le ou les peptides protéotypiques à doser. Un tel fractionnement des peptides générés permet d'accroître la spécificité du dosage ultérieur par spectrométrie de masse.

Une alternative aux techniques d'électrophorèse ou de chromatographie, pour le fractionnement des peptides, consiste à purifier spécifiquement les N-glycopeptides ([16] et demande de brevet WO 2008/066629). Néanmoins, une telle purification ne permet que la quantification des peptides ayant subi une modification post-traductionnelle de type N-glycosylation. Or toutes les protéines ne sont pas glycosylées, ce qui limite donc son utilisation.

La spectrométrie de masse à mettre en oeuvre dans le procédé de l'invention est largement connue de l'homme du métier comme un outil puissant pour l'analyse et la détection de différents types de molécules. De façon générale, tout type de molécule pouvant être ionisée peut être détecté en fonction de sa masse moléculaire à l'aide d'un spectromètre de masse. Selon la nature de la molécule à détecter, d'origine protéique ou métabolique, certaines technologies de spectrométrie de masse peuvent être plus adaptées. Néanmoins, quelque soit la méthode de spectrométrie de masse utilisée pour la détection, cette dernière comprend une étape d'ionisation de la molécule cible en ions dits moléculaires, dans le cas présent une étape d'ionisation des marqueurs de caractérisation, et une étape de séparation des ions moléculaires obtenus en fonction de leur masse.

Tous les spectromètres de masse comportent donc :
i) une source d'ionisation destinée à ioniser les marqueurs présents dans l'échantillon à analyser, c'est-à-dire à conférer une charge positive ou négative à ces marqueurs;
ii) un analyseur de masse destiné à séparer les marqueurs ionisés, ou ions moléculaires, en fonction de leur ratio masse sur charge (m /z) ;
iii) un détecteur destiné à mesurer le signal produit soit directement par les ions moléculaires, soit par des ions produits à partir des ions moléculaires, comme détaillés ci-après.

L'étape d'ionisation nécessaire pour la mise en oeuvre d'une spectrométrie de masse peut être mise en oeuvre par tout procédé connu de l'homme du métier. La source d'ionisation permet d'amener les molécules à doser sous un état gazeux et ionisé. Une source d'ionisation peut être utilisée soit en mode positif pour étudier les ions positifs, soit en mode négatif pour étudier les ions négatifs. Plusieurs types de sources existent et seront utilisés en fonction du résultat recherché et des molécules analysées. On peut citer, notamment :
- l'ionisation électronique (EI), l'ionisation chimique (CI) et la désorption-ionisation chimique (DCI)
- le bombardement par atomes rapides (FAB), atomes métastables (MAB) ou ions (SIMS, LSIMS)
- le couplage plasma inductif (ICP)
- l'ionisation chimique à pression atmosphérique (APCI) et la photoionisation à pression atmosphérique (APPI)
- l'électronébulisation ou électrospray (ESI)
- la désorption-ionisation laser assistée par matrice (MALDI), activée par une surface (SELDI) ou sur silicium (DIOS)
- l'ionisation-désorption par interaction avec espèces métastables (DART)

Notamment, l'ionisation peut être mise en oeuvre comme suit : l'échantillon contenant les molécules cibles est introduit dans une source d'ionisation, où les molécules sont ionisées à l'état gazeux et ainsi transformées en ions moléculaires qui correspondent aux molécules initiales. Une source d'ionisation de type électrospray (ESI pour ElectroSpray Ionisation) permet d'ioniser une molécule tout en la faisant passer d'un état liquide à un état gazeux. Les ions moléculaires obtenus correspondent alors aux molécules présentes à l'état liquide, avec en mode positif un, deux, voire trois protons supplémentaires ou plus et sont donc porteurs de une, deux, voire trois charges ou plus. Par exemple, lorsque la molécule cible est une protéine, une ionisation des peptides protéotypiques obtenus après fractionnement de la protéine cible, grâce à une source de type électrospray fonctionnant en mode positif, conduit à des ions polypeptidiques à l'état gazeux, avec un, deux, voire trois protons supplémentaires ou plus et qui sont donc porteurs de une, deux, voire trois charges ou plus, et permet un passage d'un état liquide à un état gazeux [17]. Ce type de source est particulièrement bien adapté, lorsque les molécules cibles ou peptides protéotypiques obtenus sont préalablement séparées par chromatographie liquide en phase inverse. Néanmoins, le rendement d'ionisation des molécules présentes dans l'échantillon peut varier en fonction de la concentration et de la nature des différentes espèces en présence. Ce phénomène se traduit par un effet matrice bien connu de l'homme de l'art.

Une source d'ionisation MALDI permettra d'ioniser des molécules, à partir d'un échantillon à l'état solide.

L'analyseur de masse dans lequel est mis en oeuvre l'étape de séparation des marqueurs ionisés en fonction de leur rapport masse/charge (m/z) est tout analyseur de masse connu de l'homme du métier. On peut citer les analyseurs basse résolution, du type quadripôle ou quadrupôle (Q), piège à ions 3D (IT) ou linéaire (LIT), également appelés trappe ionique, et les analyseurs haute résolution, permettant de mesurer la masse exacte des analytes et qui utilisent notamment le secteur magnétique couplé à un secteur électrique, le temps de vol (TOF).

La séparation des ions moléculaires en fonction de leur ratio m/z peut être mise en oeuvre une seule fois (spectrométrie de masse simple ou MS), ou bien plusieurs séparations MS successives peuvent être menées. Lorsque deux séparations MS successives sont réalisées, l'analyse est appelée MS/MS ou MS². Lorsque trois séparations MS successives sont réalisées, l'analyse est appelée MS/MS/MS ou MS³ et plus généralement, lorsque n séparations MS successives sont réalisées, l'analyse est appelée MSⁿ.

Parmi les techniques mettant en oeuvre plusieurs séparations successives, les modes SRM (Selected Reaction Monitoring) en cas de détection ou dosage d'une seule molécule cible, ou bien MRM (Multiple Reaction Monitoring) en cas de détection ou dosage de plusieurs molécules cibles, sont des utilisations particulières de séparation MS². De même le mode MRM³ est une utilisation particulière de séparation en MS/MS/MS. On parle alors de spectrométrie de masse ciblée.

Dans le cas d'une détection en mode MS simple, c'est le rapport masse/charge des ions moléculaires obtenus qui est corrélé à la molécule cible à détecter.

Dans le cas d'une détection en mode MS/MS, essentiellement deux étapes sont ajoutées, par rapport à un dosage MS qui sont :
i) une fragmentation des ions moléculaires, alors appelés ions précurseurs, pour donner des ions dit ions fragments de 1^{ère} génération, et
ii) une séparation des ions dit ions fragments de 1^{ère} génération en fonction de leur masse (m/z)₂, le rapport (m/z)₁ correspondant au rapport (m/z) des ions précurseurs.

C'est alors le rapport masse/charge des ions fragments de 1^{ère} génération ainsi obtenus qui est corrélé à la molécule cible à détecter. Par ion fragment de première génération, on entend un ion issu de l'ion précurseur, suite à une étape de fragmentation et dont le rapport masse sur charge m/z est différent de l'ion précurseur.

Les couples (m/z)₁ et (m/z)₂ sont baptisés transitions et sont représentatifs des ions caractéristiques à détecter.

Le choix des ions caractéristiques qui sont détectés pour être corrélés à la molécule cible est effectué par l'homme du métier selon les méthodes standards. Leur sélection conduira avantageusement aux dosages les plus sensibles, les plus spécifiques et les plus robustes possibles, en termes de reproductibilité et fiabilité. Dans les méthodes développées pour la sélection de peptides protéotypiques (m/z)₁, et de fragment de première génération (m/z)₂, le choix est essentiellement basé sur l'intensité de la réponse. Pour plus de détails, on pourra se référer à V. Fusaro et al. [18]. Des logiciels commerciaux, tels que les logiciels MIDAS et MRM Pilote d'Applied Biosytems ou encore MRMaid [19] pourront être utilisés par l'homme de l'art pour lui permettre de prédire tous les couples de transitions possibles. Il pourra également être fait appel à une base de données nommée PeptideAtlas, construite par F. Desiere et al. [20] pour compiler l'ensemble des transitions MRM de peptides décrites par la communauté scientifique. Cette base PeptideAtlas est disponible en accès libre sur internet. Pour des molécules non protéiques, il est également possible d'utiliser des bases de données, telles que par exemple celle accessible au travers du logiciel Cliquid de la société Applied Biosytems (Etats Unis d'Amérique).

Une approche alternative pour sélectionner les peptides protéotypiques, (m/z)₁ et (m/z)₂, consiste à utiliser les spectres de fragmentation MS/MS obtenus à l'occasion d'autres travaux. Ces travaux peuvent être, par exemple, les phases de découverte et d'identification des biomarqueurs par analyse protéomique. Cette approche a été proposée par Thermo Scientific lors de réunion utilisateurs [19]. Elle permet de générer une liste de transitions candidates à partir des peptides identifiés expérimentalement par le logiciel SIEVE (Thermo Scientific). Certains critères ont été détaillés par J. Mead et al. [19] pour le choix des ions (m/z)₁ et (m/z)₂ et sont détaillés ci-après :
- Les peptides avec des sites de clivage interne, c'est-à-dire avec de la Lysine ou de l'Arginine interne, doivent être évités, sauf si la Lysine ou l'Arginine est suivie par de la Proline,
- Les peptides avec de l'Asparagine ou de la Glutamine doivent être évités car ils peuvent se désaminer,
- Les peptides avec de la Glutamine ou de l'Acide Glutamique en N-terminal doivent être évités car ils peuvent se cycliser spontanément,
- Les peptides avec de la Méthionine doivent être évités car ils peuvent être oxydés,
- Les peptides avec de la Cystéine doivent être évités car ils peuvent être modifiés de façon non reproductible lors d'une éventuelle étape de dénaturation, réduction et blocage des fonctions thiols,
- Les peptides avec de la Proline peuvent être considérés comme favorables parce qu'ils produisent généralement des fragments intenses en MS/MS avec un seul pic très majoritaire. Cependant, un seul fragment très majoritaire ne permet pas de valider l'identité de la transition dans un mélange complexe. En effet, seule la présence simultanée de plusieurs fragments caractéristiques permet de vérifier que l'ion précurseur recherché est bien détecté,
- Les peptides ayant une Proline adjacente au C-terminal (position n-1) ou en seconde position par rapport au C-terminal (position n-2) sont à éviter car, dans ce cas, la taille du peptide fragment de première génération est généralement considérée comme trop petite pour être suffisamment spécifique,
- La sélection de fragments ayant une masse supérieure au précurseur est à privilégier pour favoriser la spécificité. Pour cela, il faut sélectionner un ion précurseur dichargé et sélectionner l'ion fragment de première génération le plus intense ayant une masse supérieure au précurseur, c'est à dire un ion fragment de première génération monochargé.

La fragmentation des ions précurseurs sélectionnés est mise en oeuvre dans une cellule de fragmentation telle que les modèles de type triple quadripôle [21], ou de type trappe ionique [22], ou encore de type temps de vol (TOF) [23], lesquels permettent également la séparation des ions. La ou les fragmentations seront classiquement réalisées par collision avec un gaz inerte tel que l'argon ou l'azote, au sein d'un champ électrique, par photo-excitation ou photodissociation à l'aide d'une source lumineuse intense, collision avec des électrons ou espèces radicalaires, par application d'une différence de potentiel, par exemple dans un tube de temps de vol, ou par tout autre mode d'activation. Les caractéristiques du champ électrique conditionnent l'intensité et la nature de la fragmentation. Ainsi, le champ électrique appliqué en présence d'un gaz inerte, par exemple dans un quadripôle, conditionne l'énergie de collision apportée aux ions. Cette énergie de collision sera optimisée, par l'homme du métier, pour accroître la sensibilité de la transition à doser. A titre d'exemple, il est possible de faire varier l'énergie de collision entre 5 et 180 e⁻V en q2 dans un spectromètre de masse AB SCIEX QTRAP® 5500 de la société Applied Biosystems (Foster City, Etats Unis d'Amérique). De même, la durée de l'étape de collision et l'énergie d'excitation au sein, par exemple, d'une trappe ionique seront optimisées, par l'homme du métier, pour conduire au dosage le plus sensible. A titre d'exemple, il est possible de faire varier cette durée, baptisée temps d'excitation, entre 0,010 et 50 ms et l'énergie d'excitation entre 0 et 1 (unité arbitraire) en Q3 dans un spectromètre de masse AB SCIEX QTRAP® 5500 de la société Applied Biosystems.

Enfin, la détection des ions caractéristiques sélectionnés se fait de façon classique, notamment grâce à un détecteur et à un système de traitement. Le détecteur collecte les ions et produit un signal électrique dont l'intensité dépend de la quantité d'ions collectée. Le signal obtenu est ensuite amplifié pour qu'il puisse être traité informatiquement. Un ensemble informatique de traitement des données permet de transformer les informations reçues par le détecteur en spectre de masse.

Le principe du mode SRM, ou encore du mode MRM, est de sélectionner spécifiquement un ion précurseur, de le fragmenter, puis de sélectionner spécifiquement l'un de ses ions fragments. Pour de telles applications, des dispositifs du type triple quadripôle ou des hybrides triple quadripôle à trappe ionique sont généralement utilisés.

Dans le cas d'un dispositif triple quadripôle (Qlq2Q3) utilisé en mode MS², en vue du dosage ou de la détection d'une protéine cible, le premier quadripôle (Q1) permet de filtrer les ions moléculaires, correspondant aux peptides protéotypiques caractéristiques de la protéine à doser et obtenus lors d'une étape antérieure de digestion, en fonction de leur ratio masse sur charge (m/z). Seuls les peptides ayant le ratio masse/charge du peptide protéotypique recherché, ratio appelé (m/z)₁, sont transmis dans le deuxième quadripôle (q2) et jouent le rôle d'ions précurseurs pour la fragmentation ultérieure. L'analyseur q2 permet de fragmenter les peptides de ratio masse/charge (m/z)₁ en ions fragments de première génération. La fragmentation est généralement obtenue par collision des peptides précurseurs avec un gaz inerte, comme de l'azote ou de l'argon dans q2. Les ions fragments de première génération sont transmis dans un troisième quadripôle (Q3) qui filtre les ions fragments de première génération en fonction d'un ratio masse sur charge spécifique, ratio appelé (m/z)₂. Seuls les ions fragments de première génération ayant le ratio masse/charge d'un fragment caractéristique du peptide protéotypique recherché (m/z)₂ sont transmis dans le détecteur pour être détectés, voire quantifiés.

Ce mode de fonctionnement présente une double sélectivité, en relation avec la sélection de l'ion précurseur d'une part et de la sélection de l'ion fragment de première génération d'autre part. La spectrométrie de masse en mode SRM ou MRM est donc avantageuse pour la quantification

Lorsque la spectrométrie de masse mise en oeuvre dans le procédé divulgué est une spectrométrie de masse en tandem (MS², MS³, MS⁴ ou MS⁵), plusieurs analyseurs de masse peuvent être couplés entre eux. Par exemple, un premier analyseur sépare les ions, une cellule de collision permet de fragmenter les ions, et un second analyseur sépare les ions fragments. Certains analyseurs, comme les pièges à ions ou le FT-ICR, constituent plusieurs analyseurs en un et permettent de fragmenter les ions et d'analyser les fragments directement.

Le procédé divulgué comprend une ou plusieurs des caractéristiques suivantes :
- la spectrométrie de masse, mise en oeuvre pour les propriétés de typage, de résistance potentielle à au moins un antimicrobien et facteur de virulence, est une spectrométrie de type MS/MS, ce qui a pour avantage de générer un fragment spécifique de la molécule à détecter ou à quantifier, et ainsi d'apporter une grande spécificité à la méthode de dosage ;
- la spectrométrie MS/MS est de la MRM, ce qui a pour avantage d'utiliser un temps de cycle d'analyse dans le spectromètre de masse de quelques dizaines de millisecondes, ce qui permet de détecter ou de quantifier avec une grande sensibilité, et de façon multiplexée, un grand nombre de molécules différentes ;
- la détermination des propriétés de typage, résistance à un antimicrobien et facteur de virulence est mise en oeuvre dans le même appareil de spectrométrie de masse, simultanément, ce qui a pour avantage de réduire le temps d'analyse et le coût de l'instrument, cela facilite également le traitement et le rendu des résultats.

Outre la détermination des propriétés de typage, résistance à un antimicrobien et facteur de virulence, il convient d'identifier le ou les microorganismes présents dans l'échantillon à tester.

Les procédés d'identification de microorganismes sont largement connus de l'homme du métier, comme décrit par exemple par Murray P. R. el al. dans Manuel of Clinical Microbiology, 2007, 9ème édition, et en particulier dans le Vol. I, Section III, chapitres 15 et 16 pour les bactéries et levures, Vol. II, Section VI, chapitre 82 pour les virus, et Vol. II, Section X, chapitre 135 pour les protozoaires. A titre d'exemple de procédés classiques d'identification, on peut citer la détermination du profil biologique, en utilisant par exemple les cartes d'identification du Vitek 2 (bioMérieux), ou bien encore en utilisant des techniques de biologie moléculaire avec des critères d'identification basés sur l'étude de la présence de certains gènes, sur l'étude de leur séquence.

L'identification peut être mise en oeuvre directement à partir de l'échantillon dans lequel on effectue l'identification, ou bien les microorganismes contenus dans l'échantillon peuvent être cultivés par des méthodes bien connues de l'homme du métier avec des milieux de culture et des conditions de culture optimales adaptées en fonction des espèces de microorganismes à rechercher, comme décrit par Murray P.R. et al. dans Manuel of Clinical Microbiology, 2007, 9ème édition, Vol. I, Section III, chapitre 14, et en particulier dans le Vol. I, Section IV, chapitre 21 pour les bactéries, Vol. II, Section VI, chapitre 81 pour les virus, Vol. II, Section VIII, chapitre 117 pour les levures, et Vol. II, Section X, chapitre 134 pour les protozoaires.

Ainsi, de façon générale, dans le cas d'une identification par une méthode biochimique d'une bactérie dans un prélèvement, il faut d'abord l'obtenir en culture pure, par exemple après ensemencement sur gélose. La biologie moléculaire (PCR) peut dans certains cas s'appliquer directement à l'échantillon à analyser.

Au lieu de cultiver les microorganismes, ces derniers peuvent être concentrés par capture directement dans l'échantillon au moyen de surfaces actives. Un tel procédé a été décrit par W.-J. Chen et al. [11] qui ont capturé différentes espèces bactériennes à l'aide de billes magnétiques avec une surface activée au Fe₃O₄/TiO₂. Une capture par d'autres moyens est également possible, telle qu'une capture par des lectines [24], ou par des anticorps [25], ou encore par de la Vancomycine [26]. La capture permet de concentrer les microorganismes et ainsi de réduire ou même de supprimer l'étape de culture. Il s'en suit un gain de temps considérable.

L'identification peut également être mise en oeuvre par spectrométrie de masse, selon les techniques décrites précédemment, de préférence par MS, par MS/MS, ou bien par MS suivie d'une spectrométrie de type MS/MS, ce qui constitue un mode de réalisation de l'invention. Dans ce cas également, l'échantillon peut être soumis préalablement à une étape de culture telle qu'un ensemencement sur gélose.

L'utilisation d'un procédé d'identification par MS est avantageux car il peut être réalisé en quelques minutes et qu'il nécessite un spectromètre de masse avec un seul analyseur, c'est à dire un instrument moins complexe qu'un spectromètre de masse en tandem utilisé en MS/MS.

L'utilisation d'un procédé d'identification par MS suivi d'une spectrométrie de type MS/MS est également avantageuse. Elle permet de s'assurer de l'identité des ions observé en MS, ce qui augmente la spécificité de l'analyse.

L'utilisation d'un procédé d'identification par MS/MS de type MRM présente l'avantage d'être plus sensible et plus simple que les approches MS puis MS/MS traditionnelle. Ce procédé ne nécessite ni logiciel performant pour traiter l'information entre l'acquisition du spectre MS et du spectre MS/MS, ni changement du réglage des paramètres machines pour enchaîner des spectres MS puis MS/MS.

Le procédé d'identification par MS peut être mis en oeuvre avec une source électrospray sur échantillon brut, comme décrit par S. Vaidyanathan et al. [27] ou encore par R. Everley et al. [8] après séparation chromatographique. Différentes gammes de m/z permettent alors d'identifier les microorganismes. S. Vaidyanathan et al. ont utilisé une fenêtre entre 200 et 2000 Th et R. Everley et al. une fenêtre entre 620 et 2450 Th. Les spectres de masse peuvent également être déconvolués pour accéder à la masse des protéines indépendamment de leur état de charge. R. Everley et al. ont ainsi exploité les masses entre environ 5 000 et 50 000 Da. De façon alternative, le procédé d'identification par MS peut être également mis en oeuvre à l'aide d'un MALDI-TOF, comme décrit par Claydon et al [3] et T. Krishnamurthy et P. Ross [4]. L'analyse associe l'acquisition d'un spectre de masse et l'interprétation d'un logiciel expert. Elle est extrêmement simple et peut être effectuée en quelques minutes. Ce procédé d'identification se répand actuellement dans les laboratoires d'analyse médicale [28].

L'identification de bactéries par MS puis MS/MS via leurs protéines présentes dans l'échantillon, a été largement appliquée par de nombreuses équipes. A titre d'exemple, il est possible de citer les travaux récents de Mânes N. et al. [29] qui ont étudié le peptidome de *Salmonella enterica,* ou les travaux de R. Nandakumar et al. [30] ou de L. Hernychova et al. [31] qui ont étudié le protéome de bactéries après digestion des protéines avec de la trypsine. L'approche classique consiste à i) acquérir un spectre MS, ii) sélectionner successivement chaque ion précurseur observé sur le spectre MS avec un signal intense, iii) fragmenter successivement chaque ion précurseur et acquérir son spectre MS/MS, iv) interroger des bases de données protéiques telles que SWISS-PROT ou NCBI, aux travers de logiciels tels que Mascot (Matrix Science, Londres, Royaume-Uni) ou SEQUEST (Thermo Scientific, Waltham, Etats-Unis d'Amérique), pour identifier le peptide ayant une forte probabilité de correspondre au spectre MS/MS observé. Cette méthode peut conduire à l'identification d'un microorganisme si une protéine ou un peptide caractéristique de l'espèce est identifié.

Selon encore un autre mode de réalisation, l'identification dudit au moins un microorganisme est mis en oeuvre par un procédé classique d'identification et le procédé de l'invention comprend une étape supplémentaire de confirmation de l'identification dudit au moins un microorganisme, laquelle étape de confirmation est mise en oeuvre par spectrométrie de masse, selon les techniques décrites précédemment pour l'identification de microorganismes.

Selon un mode de réalisation particulier, la spectrométrie de masse de l'étape de confirmation est un spectrométrie de masse de type MS/MS, de préférence une MRM.

Un des avantages de l'utilisation de la spectrométrie de masse réside en ce qu'elle est particulièrement utile pour quantifier des molécules, dans le cas présent les marqueurs des propriétés de typage, résistance à au moins un antimicrobien. Pour ce faire, on utilise l'intensité de courant détectée, laquelle est proportionnelle à la quantité de molécule cible. L'intensité de courant ainsi mesurée pourra servir de mesure quantitative permettant de déterminer la quantité de molécule cible présente, laquelle est caractérisée par son expression en unités du Système International (SI) de type mol/m³ ou kg/m³, ou par les multiples ou sous-multiples de ces unités, ou par les dérivées usuelles des unités SI, y compris leurs multiples ou sous-multiples. A titre d'exemple non limitatif, les unités telles que ng/ml ou fmol/l sont des unités caractérisant une mesure quantitative.

Un calibrage est néanmoins nécessaire pour pouvoir corréler l'aire du pic mesurée, correspondant à l'intensité de courant induit par les ions détectés, à la quantité de molécule cible à doser. Pour cela, les calibrages classiquement utilisés en spectrométrie de masse pourront être mis en oeuvre, dans le cadre de l'invention. Les dosages MRM sont classiquement calibrés à l'aide de standards externes ou, de préférence, à l'aide de standards internes tels que décrits par T. Fortin et al. [14]. Dans le cas où la molécule cible est un peptide protéotypique, permettant de doser une protéine d'intérêt, la corrélation entre la mesure quantitative et la quantité de peptide protéotypique cible, et par la suite de protéine d'intérêt, est obtenue en étalonnant le signal mesuré par rapport à un signal étalon pour lequel la quantité à doser est connue. L'étalonnage peut être réalisé au moyen d'une courbe d'étalonnage, par exemple obtenue par injections successives de peptide protéotypique étalon à différentes concentrations (étalonnage externe), ou de façon préférentielle, par étalonnage interne en utilisant un peptide lourd, comme standard interne, par exemple conformément aux méthodes AQUA, QconCAT ou PSAQ détaillées ci-après. Par « peptide lourd », on entend un peptide correspondant au peptide protéotypique, mais dans lequel un ou plusieurs atomes de carbone 12 (¹²C) est (sont) remplacé(s) par du carbone 13 (¹³C), et/ou un ou plusieurs atomes d'azote 14 (¹⁴N) est (sont) remplacé(s) par de l'azote 15 (¹⁵N).

L'utilisation de peptides lourds, comme standards internes (AQUA), a également été proposée dans la demande de brevet US 2004/0229283. Le principe est de synthétiser artificiellement des peptides protéotypiques avec des acides aminés comportant des isotopes plus lourds que les isotopes naturels usuels. De tels acides aminés sont obtenus, par exemple, en remplaçant certains des atomes de carbone 12 (¹²C) par du carbone 13 (¹³C), ou en replaçant certains des atomes d'azote 14 (¹⁴N) par de l'azote 15 (¹⁵N). Le peptide artificiel (AQUA) ainsi synthétisé a rigoureusement les mêmes propriétés physicochimiques que le peptide naturel (à l'exception d'une masse plus élevée). Il est généralement ajouté, à une concentration donnée, à l'échantillon, en amont du dosage par spectroscopie de masse, par exemple entre le traitement entrainant le clivage des protéines de l'échantillon d'intérêt et le fractionnement des peptides obtenus après l'étape de traitement. De ce fait, le peptide AQUA est co-purifié avec le peptide naturel à doser, lors du fractionnement des peptides. Les deux peptides sont donc injectés simultanément dans le spectromètre de masse, pour le dosage. Ils subissent alors les mêmes rendements d'ionisation dans la source. La comparaison des aires de pic des peptides naturels et AQUA, dont la concentration est connue, permet de calculer la concentration du peptide naturel et de remonter ainsi à la concentration de la protéine à doser. Une variante de la technique AQUA a été proposée par J.-M. Pratt et al. [32] sous le nom de QconCat. Cette variante est également décrite dans la demande de brevet WO 2006/128492. Elle consiste à concaténer différents peptides AQUA et à produire le polypeptide artificiel sous forme de protéine recombinante lourde. La protéine recombinante est synthétisée avec des acides aminés comportant des isotopes lourds. De cette façon, il est possible d'obtenir un standard pour calibrer le dosage simultané de plusieurs protéines à moindre coût. Le standard QconCAT est ajouté dès le début, en amont du traitement entrainant le clivage des protéines et avant les étapes de fractionnement des protéines, de dénaturation, de réduction puis de blocage des fonctions thiols des protéines, si celles-ci sont présentes. Le standard QconCAT subit donc le même cycle de traitement entrainant le clivage des protéines que la protéine naturelle, ce qui permet de tenir compte du rendement de l'étape de traitement entrainant le clivage des protéines. En effet, le traitement, notamment par digestion, de la protéine naturelle peut ne pas être complet. Dans ce cas l'utilisation d'un standard AQUA conduirait à sous-estimer la quantité de protéine naturelle. Pour un dosage absolu, il peut donc être important de tenir compte des rendements de traitement entrainant le clivage des protéines. Cependant, V. Brun et al. [33] ont montré que, parfois, les standards QconQAT ne reproduisaient pas exactement le rendement de traitement notamment par digestion de la protéine naturelle, sans doute du fait d'une conformation tridimensionnelle différente de la protéine QconCAT.

V. Brun et al. [33] ont alors proposé d'utiliser une méthode baptisée PSAQ et décrite dans la demande de brevet WO 2008/145763. Dans ce cas, le standard interne est une protéine recombinante, ayant la même séquence que la protéine naturelle mais synthétisée avec des acides aminés lourds. La synthèse est réalisée ex-vivo avec des acides aminés lourds. Ce standard a rigoureusement les mêmes propriétés physicochimiques que la protéine naturelle (à l'exception d'une masse plus élevée). Il est ajouté dès le début, avant l'étape de fractionnement des protéines, lorsque cette dernière est présente. Il est donc co-purifié avec la protéine native, lors de l'étape de fractionnement des protéines. Il présente le même rendement de traitement, notamment par digestion, que la protéine native. Le peptide lourd obtenu après clivage est également co-purifié avec le peptide naturel, si une étape de fractionnement des peptides est réalisée. Les deux peptides sont donc injectés simultanément dans le spectromètre de masse, pour être dosé quantitativement. Ils subissent alors les mêmes rendements d'ionisation dans la source. La comparaison des aires de pic des peptides naturels et des peptides de référence dans la méthode PSAQ permet de calculer la concentration de la protéine à doser en tenant compte de la totalité des étapes du procédé de dosage.

L'ensemble de ces techniques, à savoir AQUA, QconCAT ou PSAQ ou toute autre technique de calibrage, utilisée dans des dosages par spectrométrie de masse et en particulier dans les dosages MRM ou MS, pourront être mises en oeuvre pour effectuer le calibrage, dans le cadre de l'invention.

Selon un mode de réalisation préféré, le procédé de l'invention permet la caractérisation de *Staphylococcus aureus.*

En particulier, la caractérisation de *Staphylococcus aureus* met en oeuvre au moins un peptide comme suit :
1. pour le typage :
au moins un peptide appartenant à la protéine A de séquence SEQ ID N° 1 suivante : lesdits peptides étant choisis, de préférence, parmi les peptides de séquence SEQ ID N°2, 3, 4, 5, 6, 7 et 8 tels que définis ci-après :

| Peptide SEQ ID N° | Séquence d'acides aminés | Localisation dans SEQ ID N°1 |
|---|---|---|
| 2 | DDPSQSANVLGEAQK | 70-84 |
| 3 | DQQSAFYEILNMPNLNEEQR | 103-122 |
| 4 | DDPSQSTNVLGEAK | 131-144 |
| 5 | EQQNAFYEILNMPNLNEEQR | 161-180 |
| 6 | DDPSQSANLLAEAK | 189-202 |
| 7 | DDPSVSK | 305-311 |
| 8 | IAADNK | 437-442 |

2. Pour la résistance potentielle à au moins un antibiotique
au moins un peptide appartenant à la protéine PBP2a de séquence SEQ ID N° 9 suivante : lesdits peptides étant choisis, de préférence, parmi les peptides de séquence SEQ ID N°10 à 17 tels que définis ci-après :

| Peptide SEQ ID N° | Séquence d'acides aminés | Localisation dans SEQ ID N°9 |
|---|---|---|
| 10 | IYNSLGVK | 69-76 |
| 11 | DINIQDR | 77-83 |
| 12 | ELSISEDYIK | 189-198 |
| 13 | FQITTSPGSTQK | 395-406 |
| 14 | ILTAMIGLNNK | 407-417 |
| 15 | YEVVNGNIDLK | 446-456 |
| 16 | VALELGSK | 470-477 |
| 17 | SYANLIGK | 590-597 |

3. Pour la virulence :
au moins un peptide appartenant à la protéine PVL, sous-unités LukS et LukF, de séquences SEQ ID N° 18 et 22 suivantes, respectivement :
SEQ ID N° 18 :
SEQ ID N° 22 : lesdits peptides étant choisis, de préférence, parmi les peptides de séquence SEQ ID N°19, 20, 21, 23 et 24 tels que définis ci-après :

| Peptide SEQ ID N° | Séquence d'acides aminés | Localisation dans SEQ ID N°18 |
|---|---|---|
| 19 | TNDPNVDLINYLPK | 115-128 |
| 20 | SVQWGIK | 184-190 |
| 21 | ANSFITSLGK | 191-200 |

| Peptide SEQ ID N° | Séquence d'acides aminés | Localisation dans SEQ ID N°22 |
|---|---|---|
| 23 | MPVLSR | 237-242 |
| 24 | GNFNPEFIGVLSR | 243-255 |

4. Pour l'identification:
au moins un peptide appartenant à la protéine 50S ribosomal protein L30 (RL30), 50S ribosomal protein L331 (RL331), Staphylococcal secretory antigen ssaA2 (SSAA2), UPF0337 protein SA0772 (Y772), Bifunctional autolysin (ATL), Elongation factor Tu (EFTU), Probable transglycolase isaA (ISAA) et UPF0457 protein SA1975.1 (Y197A), de séquences SEQ ID N° 25, 28, 31, 33, 36, 39, 41 et 43 suivantes, respectivement :
SEQ ID N° 25 :
SEQ ID N° 28 : MRVNVTLACTECGDRNYITTKNKRNNPERVEMKKFCSRENKQTLHRETK
SEQ ID N° 31 :
SEQ ID N° 33 :
SEQ ID N° 36 :
SEQ ID N° 39 :
SEQ ID N° 41 :
SEQ ID N° 43 : lesdits peptides étant choisis, de préférence, parmi les peptides de séquence SEQ ID N°26, 27, 29, 30, 32, 34, 35, 37, 38, 40, 42, 44 et 45 tels que définis ci-après :

| Peptide SEQ ID N° | Séquence d'acides aminés | Localisation dans SEQ ID N°25 |
|---|---|---|
| 26 | LQITLTR | 4-10 |
| 27 | TNSSWVEDNPAIR | 31-34 |

| Peptide SEQ ID N° | Séquence d'acides aminés | Localisation dans SEQ ID N°28 |
|---|---|---|
| 29 | VNVTLACTECGDR | 3-15 |
| 30 | NYITTK | 16-21 |

| Peptide SEQ ID N° | Séquence d'acides aminés | Localisation dans SEQ ID N°31 |
|---|---|---|
| 32 | AGYTVNNTPK | 200-209 |

| Peptide SEQ ID N° | Séquence d'acides aminés | Localisation dans SEQ ID N°33 |
|---|---|---|
| 34 | EFVENAKEK | 42-50 |
| 35 | ATDFIDKVK | 51-59 |

| Peptide SEQ ID N° | Séquence d'acides aminés | Localisation dans SEQ ID N°36 |
|---|---|---|
| 37 | LYSVPWGTYK | 696-705 |
| 38 | AYLAVPAAPK | 747-756 |

| Peptide SEQ ID N° | Séquence d'acides aminés | Localisation dans SEQ ID N°39 |
|---|---|---|
| 40 | TVGSGVVTEIIK | 383-394 |

| Peptide SEQ ID N° | Séquence d'acides aminés | Localisation dans SEQ ID N°41 |
|---|---|---|
| 42 | LSNGNTAGATGSSAAQ-IMAQR | 148-168 |

| Peptide SEQ ID N° | Séquence d'acides aminés | Localisation dans SEQ ID N°43 |
|---|---|---|
| 44 | NITQDQDIHAVPK | 30-42 |
| 45 | LDSKDVSR | 43-50 |

Il convient de noter, comme indiqué précédemment, que, pour le typage, le procédé de l'invention peut également utiliser au moins un peptide de séquence SEQ ID N° : 10, 11, 12, 13, 14, 15, 16, 17, 19, 20, 21, 23, 24, 26, 27, 29, 30, 32, 34, 35, 37, 38, 40, 42, 44 et 45, lesquels sont utiles pour la détermination de la résistance potentielle à au moins un antibiotique ou de la virulence, comme indiqué précédemment.

Bien entendu, par au moins un peptide, on entend au moins un, au moins deux, au moins trois, au moins quatre, au moins cinq, au moins 6 ou plus peptides représentatifs du marqueur qu'on veut détecter. De préférence, on utilisera au moins deux, voire au moins trois, voire au moins quatre peptides par caractéristique.

Selon un autre mode de réalisation préféré, le procédé de l'invention permet la caractérisation d'*Escherichia coli.*

En particulier, la caractérisation d'*Escherichia coli* met en oeuvre au moins un peptide comme suit :
1. pour le typage :
au moins un peptide appartenant à la protéine Aspartate ammonia-lyase (ASPA), la sous-unité alpha de l'ATP synthase (ATPA), la chaperonine 10 kDa (CH10), la chaperonine 60 kDa (CH60), la DNA-binding protein HU-beta (DBHB), la Glutamate decarboxylase (DCEB), Succinate dehydrogenase flavoprotein subunit (DHSA), la DNA protection dring starvation protein (DPS), la DNA-binding protein H-NS (HNS), la Malate dehydrogenase (MDH), la Phosphoglycerate kinase (PGK), la Phosphoribosylaminoimidazole-succinocarboxamide synthase (PUR7), la 50S ribosomal protein L4 (RL4), la 30S ribosomal protein S1 (RS1), la UPF0076 protein yjgF (YJGF), de séquences SEQ ID N° 138 à 152 suivantes, respectivement :
SEQ ID N° 138
SEQ ID N° 139
SEQ ID N° 140
SEQ ID N° 141
SEQ ID N° 142
SEQ ID N° 143
SEQ ID N° 144
SEQ ID N° 145
SEQ ID N° 146
SEQ ID N° 147
SEQ ID N° 148
SEQ ID N° 149
SEQ ID N° 150
SEQ ID N° 151
SEQ ID N° 152 lesdits peptides étant choisis, de préférence, parmi les peptides de séquence SEQ ID N°67 à 84 tels que définis ci-après :

| Peptide SEQ ID N° | Séquence d'acides aminés | Localisation dans SEQ ID N°138 |
|---|---|---|
| 67 | ISDIPEFVR | 41-49 |
| 68 | IEEDLLGTR | 7-15 |
| 69 | LVDAINQLR | 163-171 |

| Peptide SEQ ID N° | Séquence d'acides aminés | Localisation dans SEQ ID N°139 |
|---|---|---|
| 70 | TALAIDAIINQR | 176-187 |
| 71 | WNTLGAPIDGK | 107-118 |

| Peptide SEQ ID N° | Séquence d'acides aminés | Localisation dans SEQ ID N°140 |
|---|---|---|
| 72 | SAGGIVLTGSAAAK | 21-34 |

| Peptide SEQ ID N° | Séquence d'acides aminés | Localisation dans SEQ ID N°141 |
|---|---|---|
| 73 | AVTAAVEELK | 123-132 |

| Peptide SEQ ID N° | Séquence d'acides aminés | Localisation dans SEQ ID N°142 |
|---|---|---|
| 74 | ALDAIIASVTESLK | 24-37 |

| Peptide SEQ ID N° | Séquence d'acides aminés | Localisation dans SEQ ID N°143 |
|---|---|---|
| 75 | YWDVELR | 172-178 |

| Peptide SEQ ID N° | Séquence d'acides aminés | Localisation dans SEQ ID N°144 |
|---|---|---|
| 76 | LPGILELSR | 327-335 |

| Peptide SEQ ID N° | Séquence d'acides aminés | Localisation dans SEQ ID N°145 |
|---|---|---|
| 77 | SKATNLLYTR | 9-18 |

| Peptide SEQ ID N° | Séquence d'acides aminés | Localisation dans SEQ ID N°146 |
|---|---|---|
| 78 | SEALKILNNIR | 2-12 |

| Peptide SEQ ID N° | Séquence d'acides aminés | Localisation dans SEQ ID N°147 |
|---|---|---|
| 79 | LFGVTTLDIIR | 145-155 |

| Peptide SEQ ID N° | Séquence d'acides aminés | Localisation dans SEQ ID N°148 |
|---|---|---|
| 80 | ASLPTIELALK | 39-49 |

| Peptide SEQ ID N° | Séquence d'acides aminés | Localisation dans SEQ ID N°149 |
|---|---|---|
| 81 | LLSDTECLVK | 73-82 |

| Peptide SEQ ID N° | Séquence d'acides aminés | Localisation dans SEQ ID N°150 |
|---|---|---|
| 82 | SILSELVR | 107-114 |

| Peptide SEQ ID N° | Séquence d'acides aminés | Localisation dans SEQ ID N°151 |
|---|---|---|
| 83 | GGFTVELNGIR | 118-128 |

| Peptide SEQ ID N° | Séquence d'acides aminés | Localisation dans SEQ ID N°152 |
|---|---|---|
| 84 | TGEVPADVAAQAR | 39-51 |

2. Pour la résistance potentielle à au moins un antibiotique
au moins un peptide appartenant à la protéine TEM-2 beta-lactamase (TEM-2) de séquence SEQ ID N° 126 suivante : lesdits peptides étant choisis, de préférence, parmi les peptides de séquence SEQ ID N°62 à 66 tels que définis ci-après :

| Peptide SEQ ID N° | Séquence d'acides aminés | Localisation dans SEQ ID N°126 |
|---|---|---|
| 62 | LLTGELLTLASR | 168-179 |
| 63 | SALPAGWFIADK | 198-209 |
| 64 | VAGPLLR | 191-197 |
| 65 | VGYIELDLNSGK | 19-30 |
| 66 | VLLCGAVLSR | 49-58 |

3. Pour la virulence :
au moins un peptide appartenant à la protéine Shiga toxine 1 sous-unité A (STX1A), la protéine Shiga toxine 2 sous-unité A (STX2A) ou aux deux, de séquences SEQ ID N° 153 et 154, respectivement :
SEQ ID N° 153
SEQ ID N° 154 lesdits peptides étant choisis, de préférence, parmi les peptides de séquence SEQ ID N°85, 86 et 87 tels que définis ci-après :

| Peptide SEQ ID N° | Séquence d'acides aminés | Localisation dans SEQ ID N°153 |
|---|---|---|
| 85 | TYVDSLNVIR | 34-43 |
| 86 | FVTVTAEALR | 183-192 |

| Peptide SEQ ID N° | Séquence d'acides aminés | Localisation dans SEQ ID N° 154 |
|---|---|---|
| 86 | FVTVTAEALR | 183-192 |
| 87 | ISNVLPEYR | 227-235 |

4. Pour l'identification:
au moins un peptide appartenant à Aconitate hydratase 2 (ACON2) L-asparaginase 2 (ASPG2), 3-oxoacyl-[acyl carrier-protein] synthase 1 (FABB), Glutamine-binding periplasmic protein (GLNH), Molybdate-binding periplasmic protein (MODA), Dihydrolipoyllysine-residue acetyltransferase component of pyruvate dehydrogenase complex (ODP2), Outer membrane protein C (OMPC), Formate acetyltransferase 1 (PFLB), Succinyl-CoA ligase [ADP-forming] subunit alpha (SUCD), Transketolase 1 (TKT1), UPF0381 protein yfcZ (YFCZ), Uncharacterized protein ygaU (YGAU), de séquences SEQ ID N° 127 à 135, 176, 136, 137 suivantes, respectivement :
SEQ ID N° 127
SEQ ID N° 128
SEQ ID N° 129
SEQ ID N° 130
SEQ ID N° 131
SEQ ID N° 132
SEQ ID N° 133
SEQ ID N° 134
SEQ ID N° 135
SEQ ID N° 176
SEQ ID N° 136
SEQ ID N° 137 lesdits peptides étant choisis, de préférence, parmi les peptides de séquence SEQ ID N°46 à 61 tels que définis ci-après :

| Peptide SEQ ID N° | Séquence d'acides aminés | Localisation dans SEQ ID N°127 |
|---|---|---|
| 46 | ILEIEGLPDLK | 578-588 |
| 47 | VADGATVVSTSTR | 779-791 |

| Peptide SEQ ID N° | Séquence d'acides aminés | Localisation dans SEQ ID N°128 |
|---|---|---|
| 48 | TNTTDVATFK | 185-194 |

| Peptide SEQ ID N° | Séquence d'acides aminés | Localisation dans SEQ ID N°129 |
|---|---|---|
| 49 | LDTTGLIDR | 54-62 |
| 50 | VGLIAGSGGGSPR | 99-111 |

| Peptide SEQ ID N° | Séquence d'acides aminés | Localisation dans SEQ ID N°130 |
|---|---|---|
| 51 | AIDFSDGYYK | 100-109 |

| Peptide SEQ ID N° | Séquence d'acides aminés | Localisation dans SEQ ID N°131 |
|---|---|---|
| 52 | LGAWDTLSPK | 160-169 |

| Peptide SEQ ID N° | Séquence d'acides aminés | Localisation dans SEQ ID N°132 |
|---|---|---|
| 53 | FGEIEEVELGR | 397-407 |

| Peptide SEQ ID N° | Séquence d'acides aminés | Localisation dans SEQ ID N°133 |
|---|---|---|
| 54 | INLLDDNQFTR | 339-349 |

| Peptide SEQ ID N° | Séquence d'acides aminés | Localisation dans SEQ ID N°134 |
|---|---|---|
| 55 | LATAWEGFTK | 8-17 |

| Peptide SEQ ID N° | Séquence d'acides aminés | Localisation dans SEQ ID N°135 |
|---|---|---|
| 56 | DSILEAIDAGIK | 80-91 |
| 57 | FAALEAAGVK | 263-272 |
| 58 | SLADIGEALK | 276-285 |

| Peptide SEQ ID N° | Séquence d'acides aminés | Localisation dans SEQ ID N°176 |
|---|---|---|
| 59 | TEEQLANIAR | 529-538 |

| Peptide SEQ ID N° | Séquence d'acides aminés | Localisation dans SEQ ID N°136 |
|---|---|---|
| 60 | AEAEQTLAALTEK | 38-50 |

| Peptide SEQ ID N° | Séquence d'acides aminés | Localisation dans SEQ ID N°137 |
|---|---|---|
| 61 | SGDTLSAISK | 103-112 |

Il convient de noter, comme indiqué précédemment, que, pour le typage, le procédé de l'invention peut également utiliser au moins un peptide de séquence SEQ ID N° : 46 à 66 et 85 à 87, lesquels sont utiles pour la détermination de la résistance potentielle à au moins un antibiotique, de l'identification ou de la virulence, comme indiqué précédemment.

Selon un autre mode de réalisation préféré, le procédé de l'invention permet la caractérisation de *Candida albicans.*

En particulier, la caractérisation de *Candida albicans* met en oeuvre au moins un peptide comme suit :
1. pour le typage:
au moins un peptide appartenant à la protéine Alcohol dehydrogenase 1 (ADH1), Fructose-biphosphate aldolase (ALF), Lanosterol 14-alpha demethylase (CP51), F-box protein COS111 (CS111), Elongation factor 1-beta (EF1B), Enolase 1 (ENO1), Glyceraldehyde-3-phosphate dehydrogenase (G3P), Heat shock protein SSA1 (HSP71), Heat shock protein SSB1 (HSP75), Pyruvate kinase (KPYK), Lipase 8 (LIP8), Multiprotein-bridging factor 1 (MBF1), Nuclear transport factor 2 (NTF2), Phosphoglycerate kinase (PGK), Peptidyl-prolyl cis-trans isomerase (PPIA), 60S ribosomal protein L13 (RL13), 60S ribosomal protein L28 (RL28), 60S ribosomal protein L36 (RL36), 40S ribosomal protein S22 (RS22), Triosephosphate isomerase (TPIS) de séquences SEQ ID N° 155 à 175 suivantes, respectivement :
SEQ ID N° 155
SEQ ID N° 156
SEQ ID N° 157
SEQ ID N° 158
SEQ ID N° 159
SEQ ID N° 160
SEQ ID N° 161
SEQ ID N° 162
SEQ ID N° 163
SEQ ID N° 164
SEQ ID N° 165
SEQ ID N° 166
SEQ ID N° 167
SEQ ID N° 168
SEQ ID N° 169
SEQ ID N° 170
SEQ ID N° 171
SEQ ID N° 172
SEQ ID N° 173
SEQ ID N° 174
SEQ ID N° 175 lesdits peptides étant choisis parmi les peptides de séquence SEQ ID N°88 à 125 tels que définis ci-après :

| Peptide SEQ ID N° | Séquence d'acides aminés | Localisation dans SEQ ID N°156 |
|---|---|---|
| 88 | ADEEFFAK | 125-132 |
| 97 | IAEALDIFHTK | 346-356 |

| Peptide SEQ ID N° | Séquence d'acides aminés | Localisation dans SEQ ID N°163 |
|---|---|---|
| 89 | AFDDESVQK | 79-87 |

| Peptide SEQ ID N° | Séquence d'acides aminés | Localisation dans SEQ ID N°160 |
|---|---|---|
| 90 | AKIDWDQAK | 81-90 |
| 98 | IDWDQAK | 83-90 |
| 99 | IEEELGSEAIYAGKDFQK | 419-436 |
| 112 | VGDKIQIVGDDLTVTNPTR | 315-333 |

| Peptide SEQ ID N° | Séquence d'acides aminés | Localisation dans SEQ ID N°170 |
|---|---|---|
| 91 | AVEVPEQTAYR | 160-170 |
| 107 | SQETFDANVAR | 105-115 |

| Peptide SEQ ID N° | Séquence d'acides aminés | Localisation dans SEQ ID N°174 |
|---|---|---|
| 92 | CGVIQPR | 72-78 |
| 114 | WTDNLLPAR | 89-97 |

| Peptide SEQ ID N° | Séquence d'acides aminés | Localisation dans SEQ ID N°173 |
|---|---|---|
| 93 | DLQELIAEGNTK | 50-61 |

| Peptide SEQ ID N° | Séquence d'acides aminés | Localisation dans SEQ ID N°169 |
|---|---|---|
| 94 | FADENFVKR | 81-89 |
| 100 | IESFGSGSGATSK | 140-152 |

| Peptide SEQ ID N° | Séquence d'acides aminés | Localisation dans SEQ ID N°175 |
|---|---|---|
| 95 | FALDTGVK | 115-122 |

| Peptide SEQ ID N° | Séquence d'acides aminés | Localisation dans SEQ ID N°171 |
|---|---|---|
| 96 | GRLPEVPVIVK | 32-42 |

| Peptide SEQ ID N° | Séquence d'acides aminés | Localisation dans SEQ ID N°167 |
|---|---|---|
| 101 | LASLPFQK | 54-61 |

| Peptide SEQ ID N° | Séquence d'acides aminés | Localisation dans SEQ ID N°166 |
|---|---|---|
| 102 | LDATDDVVAVK | 66-76 |

| Peptide SEQ ID N° | Séquence d'acides aminés | Localisation dans SEQ ID N°168 |
|---|---|---|
| 103 | NVEHLVEK | 264-271 |
| 110 | SVELFQQAVAK | 319-329 |

| Peptide SEQ ID N° | Séquence dacides aminés | Localisation dans SEQ ID N°172 |
|---|---|---|
| 104 | SGIAAGVNK | 4-12 |
| 105 | SGVDYVIESTGVFTK | 89-103 |
| 115 | YKGEVTASGDDLVIDGHK | 55-72 |

| Peptide SEQ ID N° | Séquence d'acides aminés | Localisation dans SEQ ID N°158 |
|---|---|---|
| 108 | SSSSTTKK | 326-333 |

| Peptide SEQ ID N° | Séquence d'acides aminés | Localisation dans SEQ ID N°159 |
|---|---|---|
| 106 | SLNEFLADK | 14-22 |

| Peptide SEQ ID N° | Séquence d'acides aminés | Localisation dans SEQ ID N°162 |
|---|---|---|
| 109 | STLDPVGK | 311-318 |

| Peptide SEQ ID N° | Séquence d'acides aminés | Localisation dans SEQ ID N°164 |
|---|---|---|
| 111 | TANDVLELR | 78-86 |

| Peptide SEQ ID N° | Séquence d'acides aminés | Localisation dans SEQ ID N°155 |
|---|---|---|
| 113 | VVAIDGGDEK | 200-209 |
| 116 | YVLDTSK | 344-350 |

| Peptide SEQ ID N° | Séquence d'acides aminés | Localisation dans SEQ ID N°157 |
|---|---|---|
| 117 | AVIYDCPNSR | 399-414 |
| 177 | GHYVLVFPGYAHTSER | 399-414 |
| 118 | GHYVLVSPGYAHTSER | 399-414 |
| 119 | GVIYDCPNSR | 129-138 |
| 120 | GVSSPYLPFGGGR | 455-467 |
| 121 | GVSSPYLPFSGGK | 455-467 |
| 122 | GVSSPYLPFSGGR | 455-467 |

| Peptide SEQ ID N° | Séquence d'acides aminés | Localisation dans SEQ ID N°165 |
|---|---|---|
| 123 | AAVGDILQSR | 37-46 |
| 124 | ITPDDLR | 447-453 |
| 125 | TGWDILK | 304-310 |

2. Pour la résistance potentielle à au moins un antibiotique
au moins un peptide appartenant à la protéine Lanosterol 14-alpha demethylase (CP51), de séquence SEQ ID N° 157, lesdits peptides étant choisis, de préférence, parmi les peptides de séquence SEQ ID N° 117 à 122 et 177, tel que défini ci-dessus.
3. Pour la virulence :
au moins un peptide appartenant à la protéine Lipase 8 (LIP8) de séquence SEQ ID N° 163, lesdits peptides étant choisis, de préférence, parmi les peptides de séquence SEQ ID N° 123 à 125, tel que défini ci-dessus.

4. Pour l'identification:
au moins un peptide appartenant à la protéine Alcohol dehydrogenase 1 (ADH1), Fructose-biphosphate aldolase (ALF), F-box protein COS111 (CS111), Elongation factor 1-beta (EF1B), Enolase 1 (ENO1), Glyceraldehyde-3-phosphate dehydrogenase (G3P), Heat shock protein SSA1 (HSP71), Heat shock protein SSB1 (HSP75), Pyruvate kinase (KPYK), Multiprotein-bridging factor 1 (MBF1), Nuclear transport factor 2 (NTF2), Phosphoglycerate kinase (PGK), Peptidyl-prolyl cis-trans isomerase (PPIA), 60S ribosomal protein L13 (RL13), 60S ribosomal protein L28 (RL28), 60S ribosomal protein L36 (RL36), 40S ribosomal protein S22 (RS22), Triosephosphate isomerase (TPIS) de séquences SEQ ID N° 155, 156, 158 à 164, 166 à 175, lesdits peptides étant choisis, de préférence, parmi les peptides de séquence SEQ ID N°88 à 116, tel que défini ci-dessus

Les peptides utiles aux fins de l'invention, de séquence SEQ ID N°2, 3, 4, 5, 6, 7, 8, 10, 11, 12, 13, 14, 15, 16, 17, 19, 20, 21, 23, 24, 26, 27, 29, 30, 32, 34, 35, 37, 38, 40, 42, 44 à 125, 177 sont nouveaux et constituent un autre objet de l'invention.

Le procédé de l'invention et ses avantages ressortiront de la suite de la présente description relatant divers exemples non limitatifs de mise en oeuvre du procédé de l'invention.

### Exemple 1 : Identification de microorganismes à partir d'un échantillon par proril biochimique

### 1. Mise-en en culture de l'échantillon sur milieu de culture

Les milieux de culture optimaux et les conditions de culture optimales sont différents selon les espèces de microorganisme. Par défaut l'échantillon est ensemencé sur différents milieux :
∘ gélose Columbia au sang de mouton (réf bioMérieux 43041) pendant 18 à 24 h à 35°C, avec ou sans anaérobie ;
∘ gélose TSA (référence bioMérieux 43011) pendant 18 à 24 h à 37°C.

### 2. Identification des microorganismes,

L'identification est mise en oeuvre comme suit :
1. Sélection de colonies isolées
2. En respectant les conditions d'asepsie, transfert de 3,0 mL de solution saline stérile aqueuse (à 0,45-0,50 % de NaCl, de pH 4,5 à 7,0) dans un tube à essai en plastique transparent (polystyrène)
3. A l'aide d'un bâtonnet ou d'un écouvillon stérile, transfert d'un nombre suffisant de colonies identiques dans le tube de solution saline préparé à l'étape 2 et ajustement de la suspension bactérienne entre 0,50 et 0,63 McFarland avec un DENSICHEK étalonné du VITEK2
4. Positionnement du tube de suspension bactérienne et d'une carte d'identification VITEK2 sur une cassette VITEK2
5. Chargement de la cassette dans l'instrument VITEK2
6. Les opérations de remplissage, scellage, incubation et lecture sont automatiques
7. Acquisition d'un profil biochimique
8. Identification avec le système VITEK2 réalisée par comparaison à des profils biochimiques de souches connues

### Exemple 2 : Identirication de microorganismes à partir d'un échantillon par MALDI-TOF

L'identification est mise en oeuvre comme suit :
1. Transfert à l'aide d'une oese 1µl 1µl d'une portion de colonie de microorganisme obtenue selon l'exemple 1, et dépôt uniforme sur une plaque pour spectrométrie de masse par MALDI-TOF
2. Recouvrement du dépôt avec 1 µl de matrice. La matrice utilisée est une solution saturée d'HCCA (acide alpha-cyano-4-hydroxycinnamique) en solvant organique (50% d'acétonitrile et 2,5% d'acide trifluoroacétique)
3. Séchage à température ambiante
4. Introduction de la plaque dans le spectromètre de masse
5. Acquisition d'un spectre de masse
6. Comparaison du spectre obtenu avec les spectres contenus dans une base de connaissance
7. Identification du microorganisme par comparaison des pics obtenus à ceux de la base de connaissance

### Exemple 3 : Identification de microorganismes à partir d'un échantillon par ESI-MS

L'identification est mise en oeuvre comme suit :
1. Prélèvement d'une colonie de microorganisme, obtenue selon l'exemple 1, et mise en suspension dans 100µl d'eau déminéralisée.
2. Centrifugation à 3000g pendant 5 minutes.
3. Elimination du surnageant.
4. Remise en suspension dans 100µl d'eau déminéralisée.
5. Centrifugation à 3000g pendant 5 minutes.
6. Elimination du surnageant.
7. Remise en suspension dans 100µl d'un mélange Acétonitrile, eau déminéralisée, acide formique (50/50/0.1%).
8. Filtration avec un filtre de porosité 0.45 µm.
9. Injection dans un spectromètre de masse en mode MS simple.
10. Acquisition d'un spectre de masse.
11. Comparaison du spectre obtenu avec les spectres contenus dans une basse de connaissance.
12. Identification du microorganisme par référence à des spectres de référence.

### Exemple 4 : Obtention de protéines digérées à partir de microorganismes

Classiquement le protocole suivant est mis en oeuvre en 11 étapes :
1. Prélèvement d'une colonie de microorganisme, obtenue selon l'exemple 1, et mise en suspension dans 10 à 100µl d'une solution d'hydrochlorure de guanidine 6M, 50 mM Tris-HCl, pH=8,0.
2. Ajout de dithiothréitol (DTT) pour obtenir une concentration finale de 5mM.
3. Réduction pendant 20 minutes à 95°C dans un bain-marie.
4. Refroidissement des tubes à température ambiante.
5. Ajout d'iodoacétamide pour obtenir une concentration finale de 12.5 mM.
6. Alkylation pendant 40 minutes à température ambiante et à l'obscurité.
7. Dilution d'un facteur 6 avec une solution de NH₄HCO₃ 50 mM, pH=8.0 pour obtenir une concentration finale en hydrochlorure de guanidine de 1M.
8. Ajout de 1 µg de trypsine.
9. Digestion à 37°C pendant 6 heures jusqu'à une nuit.
10. Ajout d'acide formique jusqu'à un pH inférieur à 4 pour stopper la réaction.
11. Ultracentrifugation à 100 000g pendant 30 minutes.

### Exemple 5 : Caractérisation d'échantillons de S. aureus:

Après avoir établi la ou les espèces des échantillons selon l'une quelconque des méthodes décrites dans les exemples 1 à 3, les espèces listées ci-dessous sont analysées. Treize souches de *S. aureus* sont analysées pour confirmer leur identification et établir leurs caractéristiques :

| | |
|---|---|
| ID2 | ID3 |
| ID4 | ID2bis |
| ID3bis | ID4bis |
| AST7 | AST8 |
| AST13 | AST14 |
| VIR5 | VIR6 |
| VIR7 | |

La même méthode d'analyse est appliquée à des espèces n'appartenant pas à l'espèce *S. aureus* pour servir de témoin négatif :
• *E. coli*

| | |
|---|---|
| ID7 | AST2 |

• *S. pneumoniae* VIR21
• *C. difficile* VIR26

Chaque échantillon est traité selon l'exemple 4, puis un volume de 5µl de protéines digérées est injecté et analysé selon les conditions suivantes :
• Chaîne chromatographique Agilent 1100 series de la société Agilent Technologies (Massy, France).
• Colonne Waters Symmetry C18, 2,1mm de diamètre interne, 100mm de long, taille de particule de 3,5µm.
• Solvant A : H₂O + 0,1% acide formique.
• Solvant B : ACN + 0,1% acide formique.
• Gradient HPLC défini au **TABLEAU 1** ci-après :

**TABLEAU 1**

| Temps | Débit (µl) | Solvant A (%) | Solvant B (%) |
|---|---|---|---|
| 0 | 300 | 95 | 5 |
| 25 | 300 | 60 | 40 |
| 27 | 300 | 0 | 100 |
| 35 | 300 | 0 | 100 |
| 35.1 | 300 | 95 | 5 |
| 45 | 300 | 95 | 5 |

• L'éluât en sortie de colonne chromatographique est directement injecté dans la source d'ionisation du spectromètre de masse de type QTRAP® 5500 de la société Applied Biosystems (Foster City, Etats Unis d'Amérique).
• Les peptides, issues de la digestion des protéines du microorganisme, sont analysés par le spectromètre de masse en mode MRM. Seuls les peptides indiqués dans le **TABLEAU 2** sont détectés. Pour cela, le ou les fragments de première génération indiqués dans le **TABLEAU 2** sont détectés. L'application, à laquelle permet de répondre chaque transition, c'est à dire chaque peptide associé à son fragment de première génération, est précisée dans la colonne intérêt clinique du **TABLEAU 2** avec les lettres I, T, R et V. I désigne la confirmation de l'identification du microorganisme, T le typage, R la résistance à au moins un antibiotique et V la détection de facteurs de virulence.

**TABLEAU 2 :**

| Transition Numéro | Protéine | Peptide | Ion fragment de première génération | Intérêt clinique |
|---|---|---|---|---|
| 1 | protéine A | DDPSQSANVLGEAQK | y9 monochargé | T |
| 2 | protéine A | DDPSQSANVLGEAQK | y13 monochargé | T |
| 3 | protéine A | DQQSAFYEILNMPNLNEEQR | y8 monochargé | T |
| 4 | protéine A | DQQSAFYEILNMPNLNEEQR | y8 dichargé | T |
| 5 | protéine A | DDPSQSTNVLGEAK | y9 monochargé | T |
| 6 | protéine A | DDPSQSTNVLGEAK | y12 monochargé | T |
| 7 | protéine A | EQQNAFYEILNMPNLNEEQR | y8 monochargé | T |
| 8 | protéine A | EQQNAFYEILNMPNLNEEQR | y8 dichargé | T |
| 9 | protéine A | DDPSQSANLLAEAK | y9 monochargé | T |
| 10 | protéine A | DDPSQSANLLAEAK | y12 monochargé | T |
| 11 | protéine A | DDPSVSK | y5 monochargé | T |
| 12 | protéine A | DDPSVSK | y4 monochargé | T |
| 13 | protéine A | IAADNK | y4 monochargé | T |
| 14 | protéine A | IAADNK | y5 monochargé | T |
| 15 | PBP2a | IYNSLGVK | y6 monochargé | R + T |
| 16 | PBP2a | IYNSLGVK | y7 monochargé | R + T |
| 17 | PBP2a | DINIQDR | y5 monochargé | R + T |
| 18 | PBP2a | DINIQDR | y4 monochargé | R + T |
| 19 | PBP2a | ELSISEDYIK | y6 monochargé | R + T |
| 20 | PBP2a | ELSISEDYIK | y8 monochargé | R + T |
| 21 | PBP2a | FQITTSPGSTQK | y9 monochargé | R + T |
| 22 | PBP2a | FQITTSPGSTQK | y6 monochargé | R + T |
| 23 | PBP2a | ILTAMIGLNNK | y8 monochargé | R + T |
| 24 | PBP2a | ILTAMIGLNNK | y9 monochargé | R + T |
| 25 | PBP2a | YEWNGNIDLK | y8 monochargé | R + T |
| 26 | PBP2a | YEWNGNIDLK | y9 monochargé | R + T |
| 27 | PBP2a | VALELGSK | y6 monochargé | R + T |
| 28 | PBP2a | VALELGSK | y7 monochargé | R + T |
| 29 | PBP2a | SYANLIGK | y6 monochargé | R + T |
| 30 | LukS | TNDPNVDLINYLPK | y8 monochargé | V + T |
| 31 | LukS | TNDPNVDLINYLPK | y11 monochargé | V + T |
| 32 | LukS | SVQWGIK | y5 monochargé | V + T |
| 33 | LukS | SVQWGIK | y4 monochargé | V + T |
| 34 | LukS | ANSFITSLGK | y8 monochargé | V + T |
| 35 | LukS | ANSFITSLGK | y6 monochargé | V + T |
| 36 | LukF | MPVLSR | y5 monochargé | V + T |
| 37 | LukF | MPVLSR | y3 monochargé | V + T |
| 38 | LukF | GNFNPEFIGVLSR | y8 monochargé | V + T |
| 39 | LukF | GNFNPEFIGVLSR | y9 monochargé | V + T |
| 40 | RL30 | LQITLTR | y5 monochargé | I + T |
| 41 | RL30 | LQITLTR | y6 monochargé | I + T |
| 42 | RL30 | TNSSVVVEDNPAIR | y8 monochargé | I + T |
| 43 | RL30 | TNSSVVVEDNPAIR | y8 monochargé | I + T |
| 44 | RL331 | VNVTLAC[CAM]TEC[CAM]GDR | y8 monochargé | I + T |
| 45 | RL331 | NYITTK | y5 monochargé | I + T |
| 46 | RL331 | NYITTK | y4 monochargé | I + T |
| 47 | SSAA2 | AGYTVNNTPK | y6 monochargé | I + T |
| 48 | SSAA2 | AGYTVNNTPK | y7 monochargé | I + T |
| 49 | SSAA2 | AGYTVNNTPK | y8 monochargé | I + T |
| 50 | SSAA2 | AGYTVNNTPK | y9 monochargé | I + T |
| 51 | Y772 | ATDFIDKVK | y6 monochargé | I + T |
| 52 | Y772 | ATDFIDKVK | y8 monochargé | I + T |
| 53 | ATL | AYLAVPAAPK | y7 monochargé | I + T |
| 54 | ATL | AYLAVPAAPK | y8 monochargé | I + T |
| 55 | EFTU | TVGSGVVTEIIK | y7 monochargé | I + T |
| 56 | EFTU | TVGSGVVTEIIK | y8 monochargé | I + T |
| 57 | EFTU | TVGSGVVTEIIK | y9 monochargé | I + T |
| 58 | Y772 | EFVENAKEK | y6 monochargé | I + T |
| 59 | Y772 | EFVENAKEK | y7 monochargé | I + T |
| 60 | Y772 | EFVENAKEK | y8 monochargé | I + T |
| 61 | ISAA | LSNGNTAGATGSSAAQIMAQR | y7 monochargé | I + T |
| 62 | ISAA | LSNGNTAGATGSSAAQIMAQR | y8 monochargé | I + T |
| 63 | ATL | LYSVPWGTYK | y6 monochargé | I + T |
| 64 | ATL | LYSVPWGTYK | y7 monochargé | I + T |
| 65 | ATL | LYSVPWGTYK | y8 monochargé | I + T |
| 66 | Y197A | NITQDQDIHAVPK | y8 monochargé | I + T |
| 67 | Y197A | NITQDQDIHAVPK | y7 monochargé | I + T |
| 68 | Y197A | NITQDQDIHAVPK | y6 monochargé | I + T |
| 69 | Y197A | LDSKDVSR | y6 monochargé | I + T |
| 70 | Y197A | LDSKDVSR | y7 monochargé | I + T |

| | | | | |
|---|---|---|---|---|
| L'état de charge du peptide précurseur, son temps de rétention et les transitions, c'est-à-dire les rapports (m/z)₁ en Q1 et (m/z)₂ en Q3, sont indiqués dans le **TABLEAU 3.** L'énergie de collision utilisée pour fragmenter l'ion précurseur est également indiquée dans le **TABLEAU 3.** | | | | |

**TABLEAU 3 :**

| Transition Numéro | Etat de charge du précurseur | Temps de rétention | (m/z) filtré en Q1 | (m/z) filtré en Q3 | Energie de collision |
|---|---|---|---|---|---|
| 1 | 2 | 8.2 | 779.87 | 929.51 | 39 |
| 2 | 2 | 8.2 | 779.87 | 664.84 | 39 |
| 3 | 3 | 17.9 | 813.71 | 999.49 | 45 |
| 4 | 3 | 17.9 | 813.71 | 500.24 | 45 |
| 5 | 2 | 7.9 | 730.85 | 918.49 | 37 |
| 6 | 2 | 7.9 | 730.85 | 615.82 | 37 |
| 7 | 3 | 16.5 | 827.39 | 999.49 | 45 |
| 8 | 3 | 16.5 | 827.39 | 500.25 | 34 |
| 9 | 2 | 10 | 729.86 | 916.51 | 37 |
| 10 | 2 | 10 | 729.86 | 614.83 | 37 |
| 11 | 2 | 11 | 374.18 | 517.3 | 21 |
| 12 | 2 | 11 | 374.18 | 420.25 | 21 |
| 13 | 2 | 8.9 | 316.17 | 447.22 | 19 |
| 14 | 2 | 8.9 | 316.17 | 518.26 | 19 |
| 15 | 2 | 6.7 | 447.26 | 617.36 | 25 |
| 16 | 2 | 6.7 | 447.26 | 780.43 | 25 |
| 17 | 2 | 6.2 | 437.22 | 645.33 | 24 |
| 18 | 2 | 6.2 | 437.22 | 531.3 | 24 |
| 19 | 2 | 11.1 | 598.81 | 754.36 | 31 |
| 20 | 2 | 11.1 | 598.81 | 954.48 | 31 |
| 21 | 2 | 7.4 | 647.84 | 906.45 | 34 |
| 22 | 2 | 7.4 | 647.84 | 617.3 | 26 |
| 23 | 2 | 11.8 | 594.34 | 860.47 | 31 |
| 24 | 2 | 11.8 | 594.34 | 961.51 | 31 |
| 25 | 2 | 10.1 | 632.33 | 872.48 | 33 |
| 26 | 2 | 10.1 | 632.33 | 971.55 | 33 |
| 27 | 2 | 7.4 | 408.74 | 646.38 | 23 |
| 28 | 2 | 7.4 | 408.74 | 717.41 | 23 |
| 29 | 2 | 8.2 | 433.24 | 615.38 | 24 |
| 30 | 2 | 14.9 | 808.42 | 975.55 | 41 |
| 31 | 2 | 14.9 | 808.42 | 643.4 | 41 |
| 32 | 2 | 8.2 | 409.23 | 631.36 | 23 |
| 33 | 2 | 8.2 | 409.23 | 503.3 | 23 |
| 34 | 2 | 10.3 | 519.28 | 852.48 | 28 |
| 35 | 2 | 10.3 | 519.28 | 618.4 | 28 |
| 36 | 2 | 6.4 | 351.7 | 571.36 | 20 |
| 37 | 2 | 6.4 | 351.7 | 375.24 | 20 |
| 38 | 2 | 14.3 | 725.38 | 920.52 | 37 |
| 39 | 2 | 14.3 | 725.38 | 509.29 | 37 |
| 40 | 2 | 9.2 | 422.77 | 603.38 | 24 |
| 41 | 2 | 9.2 | 422.77 | 731.44 | 24 |
| 42 | 2 | 8 | 750.89 | 913.47 | 38 |
| 43 | 3 | 8 | 500.93 | 913.47 | 29 |
| 44 | 2 | 7.9 | 747.84 | 968.36 | 38 |
| 45 | 2 | 2.8 | 370.2 | 625.36 | 21 |
| 46 | 2 | 2.8 | 370.2 | 462.29 | 21 |
| 47 | 2 | 3.9 | 532.77 | 672.37 | 28 |
| 48 | 2 | 3.9 | 532.77 | 773.42 | 28.442 |
| 49 | 2 | 3.9 | 532.77 | 936.48 | 28.442 |
| 50 | 2 | 3.9 | 532.77 | 993.5 | 28.442 |
| 51 | 2 | 7.2 | 518.79 | 749.46 | 28 |
| 52 | 2 | 7.2 | 518.79 | 965.53 | 27.827 |
| 53 | 2 | 9 | 500.79 | 653.4 | 27 |
| 54 | 2 | 9 | 500.79 | 766.48 | 27.035 |
| 55 | 2 | 11.2 | 601.85 | 801.51 | 31 |
| 56 | 2 | 11.2 | 601.85 | 858.53 | 31.482 |
| 57 | 2 | 11.2 | 601.85 | 945.56 | 31.482 |
| 58 | 2 | 2.3 | 547.28 | 718.37 | 29 |
| 59 | 2 | 2.3 | 547.28 | 817.44 | 29.08 |
| 60 | 2 | 2.3 | 547.28 | 964.51 | 29.08 |
| 61 | 3 | 8.7 | 669.33 | 817.43 | 37 |
| 62 | 3 | 8.7 | 669.33 | 888.47 | 37.466 |
| 63 | 2 | 12.2 | 607.32 | 751.38 | 32 |
| 64 | 2 | 12.2 | 607.32 | 850.45 | 31.722 |
| 65 | 2 | 12.2 | 607.32 | 937.48 | 31.722 |
| 66 | 2 | 6.3 | 739.88 | 907.5 | 38 |
| 67 | 2 | 6.3 | 739.88 | 779.44 | 37.555 |
| 68 | 2 | 6.3 | 739.88 | 664.41 | 37.555 |
| 69 | 2 | 2 | 460.25 | 691.37 | 25.251 |
| 70 | 2 | 2 | 460.25 | 806.4 | 25.251 |

• Les autres paramètres machine utilisés sont les suivants :

| | |
|---|---|
| Type de balayage: | MRM |
| Polarité: | Positive |
| Source d'ionisation: | Turbo V™ (Applied BioSystems) |
| Réglage Q1: | Filtrage avec résolution unitaire |
| Réglage Q3: | Filtrage avec résolution unitaire |
| Pause inter-scan: | 5.00 msec |
| Vitesse de balayage: | 10 Da/s |
| Gaz rideau: | 50,00 psi |
| Tension de cône: | 5500,00 V |
| Température de source: | 500,00 °C |
| Gaz de nébulisation: | 50,00 psi |
| Gaz chauffant: | 40,00 psi |
| Remplissage dynamique: | activé |
| Potentiel d'orifice : | 100, 00 V |
| (en anglais declustering potential (DP)) | |
| Potentiel d'entrée avant Q0 (EP): | 6,00 V |
| Potentiel en sortie de cellule de collision : | 11 V |
| (en anglais cell exit potential (CXP)) | |
| Temps de cycle total: 1.6 sec | |

Les aires obtenues pour chacune des transitions et pour chacun des microorganismes étudiés ont été mesurées. Toutes les transitions dont l'aire est supérieure ou égale à 1000 (unité arbitraire) sont considérées comme positives et ont été notées « 1 » dans les **tableaux 4A et 4B.** Toutes les transitions dont l'aire est inférieure à 1000, sont considérées comme négatives et ont été notées 0 dans les tableaux 4A et 4B. Lorsqu'on n'a observé aucun pic de signal, on a noté la transition comme négative.

Le nombre de transition positive est ensuite sommé pour les applications I, R et V et reporté dans le **TABLEAU 5 :**

**TABLEAU 4A :**

| | ID2 | ID3 | ID4 | ID2bis | ID3bis | ID4bis | AST7 | AST8 |
|---|---|---|---|---|---|---|---|---|
| Transition | *S. aureus* | *S. aureus* | *S. aureus* | *S. aureus* | *S. aureus* | *S. aureus* | *S. Aureus* | *S. Aureus* |
| 1 | 0 | 1 | 1 | 0 | 1 | 1 | 0 | 0 |
| 2 | 0 | 1 | 1 | 0 | 1 | 1 | 0 | 0 |
| 3 | 0 | 1 | 1 | 0 | 1 | 1 | 0 | 0 |
| 4 | 0 | 1 | 1 | 0 | 1 | 1 | 0 | 0 |
| 5 | 1 | 1 | 1 | 1 | 1 | 1 | 0 | 0 |
| 6 | 1 | 1 | 1 | 1 | 1 | 1 | 0 | 1 |
| 7 | 1 | 1 | 1 | 1 | 1 | 1 | 0 | 0 |
| 8 | 1 | 1 | 1 | 1 | 1 | 1 | 0 | 0 |
| 9 | 1 | 1 | 1 | 1 | 1 | 1 | 0 | 0 |
| 10 | 1 | 1 | 1 | 1 | 1 | 1 | 0 | 0 |
| 11 | 0 | 0 | 0 | 0 | 0 | 0 | 1 | 0 |
| 12 | 0 | 0 | 0 | 0 | 0 | 0 | 0 | 0 |
| 13 | 1 | 1 | 1 | 0 | 1 | 1 | 0 | 0 |
| 14 | 1 | 1 | 1 | 1 | 1 | 1 | 1 | 0 |
| 15 | 1 | 0 | 1 | 1 | 0 | 1 | 0 | 0 |
| 16 | 0 | 0 | 1 | 0 | 0 | 1 | 0 | 0 |
| 17 | 1 | 0 | 1 | 1 | 0 | 1 | 0 | 0 |
| 18 | 1 | 0 | 1 | 1 | 0 | 1 | 0 | 0 |
| 19 | 1 | 0 | 1 | 1 | 0 | 1 | 0 | 0 |
| 20 | 1 | 0 | 1 | 1 | 0 | 1 | 0 | 0 |
| 21 | 1 | 0 | 1 | 1 | 0 | 1 | 0 | 0 |
| 22 | 1 | 0 | 1 | 1 | 0 | 1 | 0 | 0 |
| 23 | 1 | 0 | 1 | 1 | 0 | 1 | 0 | 0 |
| 24 | 1 | 0 | 1 | 1 | 0 | 1 | 0 | 0 |
| 25 | 1 | 0 | 1 | 1 | 0 | 1 | 0 | 0 |
| 26 | 1 | 0 | 1 | 1 | 0 | 1 | 0 | 0 |
| 27 | 1 | 1 | 1 | 1 | 1 | 1 | 0 | 0 |
| 28 | 1 | 0 | 1 | 1 | 0 | 1 | 0 | 0 |
| 29 | 1 | 0 | 1 | 1 | 0 | 1 | 0 | 0 |
| 30 | 0 | 0 | 0 | 0 | 0 | 0 | 0 | 0 |
| 31 | 0 | 0 | 0 | 0 | 0 | 0 | 0 | 0 |
| 32 | 0 | 0 | 0 | 0 | 0 | 0 | 0 | 0 |
| 33 | 0 | 0 | 1 | 0 | 0 | 0 | 0 | 0 |
| 34 | 0 | 0 | 0 | 0 | 0 | 0 | 0 | 0 |
| 35 | 0 | 0 | 0 | 0 | 0 | 0 | 0 | 1 |
| 36 | 0 | 0 | 0 | 0 | 0 | 0 | 1 | 1 |
| 37 | 0 | 0 | 0 | 0 | 0 | 1 | 0 | 0 |
| 38 | 0 | 0 | 0 | 0 | 0 | 1 | 0 | 0 |
| 39 | 0 | 0 | 0 | 0 | 0 | 0 | 0 | 0 |
| 40 | 1 | 1 | 1 | 1 | 1 | 1 | 1 | 1 |
| 41 | 1 | 1 | 1 | 1 | 1 | 1 | 1 | 1 |
| 42 | 1 | 1 | 1 | 1 | 1 | 1 | 1 | 1 |
| 43 | 1 | 1 | 1 | 1 | 1 | 1 | 1 | 1 |
| 44 | 1 | 1 | 1 | 1 | 1 | 1 | 1 | 1 |
| 45 | 1 | 1 | 1 | 1 | 1 | 1 | 1 | 1 |
| 46 | 1 | 1 | 1 | 1 | 1 | 1 | 1 | 1 |
| 47 | 1 | 1 | 1 | 1 | 1 | 1 | 1 | 1 |
| 48 | 1 | 1 | 1 | 1 | 1 | 1 | 1 | 1 |
| 49 | 1 | 1 | 1 | 1 | 1 | 1 | 1 | 1 |
| 50 | 1 | 1 | 1 | 1 | 1 | 1 | 1 | 1 |
| 51 | 1 | 1 | 1 | 1 | 1 | 1 | 1 | 1 |
| 52 | 1 | 1 | 1 | 1 | 1 | 1 | 1 | 1 |
| 53 | 1 | 1 | 1 | 1 | 1 | 1 | 1 | 1 |
| 54 | 1 | 1 | 1 | 1 | 1 | 1 | 1 | 1 |
| 55 | 1 | 1 | 1 | 1 | 1 | 1 | 1 | 1 |
| 56 | 1 | 1 | 1 | 1 | 1 | 1 | 1 | 1 |
| 57 | 1 | 1 | 1 | 1 | 1 | 1 | 1 | 1 |
| 58 | 1 | 1 | 1 | 1 | 1 | 1 | 1 | 1 |
| 59 | 1 | 1 | 1 | 1 | 1 | 1 | 1 | 1 |
| 60 | 1 | 1 | 1 | 1 | 1 | 1 | 1 | 1 |
| 61 | 1 | 1 | 1 | 1 | 1 | 1 | 1 | 1 |
| 62 | 1 | 1 | 1 | 1 | 1 | 1 | 1 | 1 |
| 63 | 1 | 1 | 1 | 1 | 1 | 1 | 1 | 1 |
| 64 | 1 | 1 | 1 | 1 | 1 | 1 | 0 | 0 |
| 65 | 1 | 1 | 1 | 1 | 1 | 1 | 1 | 1 |
| 66 | 1 | 1 | 1 | 1 | 1 | 1 | 1 | 1 |
| 67 | 1 | 1 | 1 | 1 | 1 | 1 | 1 | 1 |
| 68 | 1 | 1 | 1 | 1 | 1 | 1 | 1 | 1 |
| 69 | 1 | 1 | 1 | 1 | 1 | 1 | 1 | 1 |
| 70 | 1 | 1 | 1 | 1 | 1 | 1 | 1 | 1 |

**TABLEAU 4B :**

| | AST13 | AST14 | VIR5 | VIR6 | VIR7 | ID7 | AST2 | VIR21 | VIR26 |
|---|---|---|---|---|---|---|---|---|---|
| Transition | *S. aureus* | *S. aureus* | *S. aureus* | *S. aureus* | *S. aureus* | *E. coli* | *E. coli* | *S*. *pneumoniae* | *C*. *difficile* |
| 1 | 0 | 0 | 0 | 0 | 0 | 0 | 0 | 0 | 0 |
| 2 | 0 | 0 | 0 | 1 | 0 | 0 | 0 | 0 | 0 |
| 3 | 0 | 0 | 0 | 0 | 0 | 0 | 0 | 0 | 0 |
| 4 | 0 | 0 | 0 | 0 | 0 | 0 | 0 | 0 | 0 |
| 5 | 0 | 0 | 0 | 1 | 0 | 0 | 0 | 0 | 0 |
| 6 | 1 | 0 | 0 | 1 | 0 | 0 | 0 | 0 | 0 |
| 7 | 0 | 0 | 0 | 1 | 0 | 0 | 0 | 0 | 0 |
| 8 | 0 | 0 | 0 | 1 | 0 | 0 | 0 | 0 | 0 |
| 9 | 0 | 0 | 0 | 1 | 0 | 0 | 0 | 0 | 0 |
| 10 | 0 | 0 | 0 | 1 | 0 | 0 | 0 | 0 | 0 |
| 11 | 0 | 1 | 1 | 1 | 0 | 0 | 0 | 0 | 0 |
| 12 | 0 | 1 | 1 | 0 | 0 | 0 | 0 | 0 | 0 |
| 13 | 0 | 0 | 0 | 0 | 0 | 0 | 0 | 0 | 0 |
| 14 | 1 | 0 | 0 | 1 | 0 | 0 | 0 | 0 | 0 |
| 15 | 1 | 0 | 0 | 0 | 0 | 0 | 0 | 0 | 0 |
| 16 | 1 | 0 | 0 | 0 | 0 | 0 | 0 | 0 | 0 |
| 17 | 1 | 1 | 0 | 0 | 0 | 0 | 0 | 0 | 0 |
| 18 | 1 | 0 | 0 | 0 | 0 | 0 | 1 | 0 | 0 |
| 19 | 1 | 1 | 0 | 0 | 0 | 0 | 0 | 0 | 0 |
| 20 | 1 | 1 | 0 | 0 | 0 | 0 | 0 | 0 | 0 |
| 21 | 1 | 1 | 0 | 0 | 0 | 0 | 0 | 0 | 0 |
| 22 | 1 | 0 | 0 | 0 | 0 | 0 | 0 | 0 | 0 |
| 23 | 1 | 0 | 0 | 0 | 0 | 0 | 1 | 0 | 0 |
| 24 | 1 | 1 | 0 | 1 | 0 | 0 | 0 | 0 | 0 |
| 25 | 1 | 1 | 0 | 0 | 0 | 0 | 0 | 0 | 0 |
| 26 | 1 | 0 | 0 | 0 | 0 | 0 | 0 | 0 | 0 |
| 27 | 1 | 1 | 0 | 0 | 0 | 0 | 0 | 0 | 1 |
| 28 | 1 | 1 | 0 | 0 | 0 | 0 | 0 | 0 | 0 |
| 29 | 1 | 1 | 0 | 0 | 0 | 0 | 0 | 0 | 0 |
| 30 | 0 | 0 | 1 | 0 | 1 | 0 | 0 | 0 | 0 |
| 31 | 0 | 0 | 1 | 0 | 1 | 0 | 0 | 0 | 0 |
| 32 | 0 | 0 | 1 | 0 | 1 | 0 | 0 | 0 | 0 |
| 33 | 0 | 0 | 1 | 0 | 1 | 0 | 0 | 0 | 0 |
| 34 | 0 | 0 | 1 | 0 | 1 | 0 | 0 | 0 | 0 |
| 35 | 0 | 0 | 1 | 0 | 1 | 0 | 0 | 0 | 0 |
| 36 | 1 | 1 | 1 | 1 | 1 | 0 | 0 | 0 | 0 |
| 37 | 0 | 0 | 1 | 0 | 1 | 0 | 0 | 0 | 0 |
| 38 | 0 | 0 | 1 | 0 | 1 | 0 | 0 | 0 | 0 |
| 39 | 0 | 0 | 1 | 0 | 1 | 0 | 0 | 0 | 0 |
| 40 | 1 | 1 | 1 | 1 | 1 | 0 | 0 | 0 | 0 |
| 41 | 1 | 1 | 1 | 1 | 1 | 0 | 0 | 1 | 0 |
| 42 | 1 | 1 | 1 | 1 | 1 | 0 | 0 | 0 | 0 |
| 43 | 0 | 1 | 0 | 1 | 0 | 0 | 0 | 0 | 0 |
| 44 | 1 | 1 | 1 | 1 | 1 | 0 | 0 | 0 | 0 |
| 45 | 1 | 1 | 0 | 1 | 1 | 0 | 0 | 0 | 0 |
| 46 | 1 | 1 | 1 | 1 | 1 | 0 | 0 | 0 | 0 |
| 47 | 1 | 1 | 1 | 1 | 1 | 0 | 0 | 0 | 0 |
| 48 | 1 | 1 | 0 | 1 | 1 | 0 | 0 | 0 | 0 |
| 49 | 1 | 1 | 0 | 1 | 0 | 0 | 0 | 0 | 0 |
| 50 | 1 | 0 | 0 | 1 | 0 | 0 | 0 | 0 | 0 |
| 51 | 1 | 1 | 0 | 1 | 1 | 0 | 0 | 0 | 0 |
| 52 | 1 | 1 | 0 | 1 | 1 | 1 | 0 | 0 | 0 |
| 53 | 1 | 1 | 1 | 1 | 1 | 0 | 0 | 0 | 0 |
| 54 | 1 | 1 | 1 | 1 | 1 | 0 | 0 | 0 | 0 |
| 55 | 1 | 1 | 1 | 1 | 1 | 0 | 0 | 0 | 0 |
| 56 | 1 | 1 | 1 | 1 | 1 | 0 | 0 | 0 | 0 |
| 57 | 1 | 1 | 1 | 1 | 1 | 0 | 0 | 0 | 0 |
| 58 | 1 | 1 | 0 | 1 | 1 | 0 | 0 | 0 | 0 |
| 59 | 1 | 1 | 0 | 1 | 1 | 0 | 0 | 0 | 0 |
| 60 | 1 | 1 | 0 | 1 | 1 | 0 | 0 | 0 | 0 |
| 61 | 1 | 1 | 0 | 1 | 1 | 0 | 0 | 0 | 0 |
| 62 | 1 | 1 | 0 | 1 | 1 | 0 | 0 | 0 | 0 |
| 63 | 1 | 1 | 1 | 1 | 1 | 0 | 0 | 0 | 0 |
| 64 | 1 | 0 | 0 | 0 | 0 | 0 | 0 | 0 | 0 |
| 65 | 1 | 1 | 1 | 1 | 1 | 0 | 0 | 0 | 0 |
| 66 | 1 | 1 | 0 | 1 | 1 | 0 | 0 | 0 | 0 |
| 67 | 0 | 1 | 0 | 1 | 0 | 0 | 0 | 0 | 0 |
| 68 | 1 | 1 | 0 | 1 | 0 | 0 | 0 | 0 | 0 |
| 69 | 1 | 1 | 1 | 1 | 1 | 0 | 0 | 0 | 0 |
| 70 | 1 | 1 | 0 | 1 | 1 | 0 | 0 | 0 | 0 |

**TABLEAU 5 :**

| | | I | R | V |
|---|---|---|---|---|
| ID2 | *S. aureus* | 30 | 14 | 0 |
| ID3 | *S. aureus* | 30 | 1 | 0 |
| ID4 | *S. aureus* | 30 | 15 | 1 |
| ID2bis | *S. aureus* | 30 | 14 | 0 |
| ID3bis | *S. aureus* | 30 | 1 | 0 |
| ID4bis | *S. aureus* | 30 | 15 | 2 |
| AST7 | *S. aureus* | 29 | 0 | 1 |
| AST8 | *S. aureus* | 29 | 0 | 2 |
| AST13 | *S. aureus* | 28 | 15 | 1 |
| AST14 | *S. aureus* | 28 | 9 | 1 |
| VIR5 | *S. aureus* | 13 | 0 | 10 |
| VIR6 | *S. aureus* | 29 | 1 | 1 |
| VIR7 | *S. aureus* | 24 | 0 | 10 |
| ID7 | *E. coli* | 1 | 0 | 0 |
| AST2 | *E. coli* | 0 | 2 | 0 |
| VIR21 | *S. pneumoniae* | 1 | 0 | 0 |
| VIR26 | *C. difficile* | 0 | 1 | 0 |

Tous les échantillons de *S. aureus* présentent plus de 12 transitions positives dans la catégorie I. Tous ces échantillons sont donc confirmés comme appartenant bien à l'espèce *S. aureus.*

En revanche, les échantillons ID7, AST2, VIR21 et VIR26 présentent moins de 2 transitions positives dans la catégorie I, ces échantillons sont donc confirmés comme n'appartenant pas à l'espèce *S. aureus.*

Les souches ID2, ID4, ID2bis, ID4bis, AST13 et AST14 de *S. aureus* présentent plus de 8 transitions positives pour la catégorie R, elles expriment donc la Protéine Liant la Pénicilline (PLP2a), ce qui est synonyme d'un mécanisme de résistance aux Pénicillines du groupe M (e.g. Méticilline).

En revanche, les souches ID3, ID3bis, AST7, AST8, VIR5, VIR6 et VIR7 de S. *aureus* présentent moins de 3 transitions positives pour la catégorie V, elles n'expriment donc pas PLP2a. Ces souches sont donc sensibles à un antibiotique tel qu'une pénicilline du groupe M.

Les souches ID7, AST2, VIR21 et VIR26 qui n'appartiennent pas à l'espèce S. *aureus* présentent également moins de 3 transitions pour la catégorie R, elles n'expriment donc pas PLP2a, ce qui confirme la spécificité de la méthode.

Les échantillons VIR5 et VIR7 de *S. aureus* présentent plus de 9 transitions positives dans la catégorie V, elles expriment donc la protéine Panton-Valentine Leucocidine (PVL).

En revanche, les souches ID2, ID3, ID4, ID2bis, ID3bis, ID4bis, AST7, AST8, AST13, AST14 et VIR6 de *S. aureus* présentent moins de 3 transitions positives, elles n'expriment donc pas PVL. Ces souches n'ont donc pas les propriétés de virulence en lien avec PVL.

Les souches ID7, AST2, VIR21 et VIR26 qui n'appartiennent pas à l'espèce S. aureus présentent également moins de 3 transitions pour la catégorie V, elles n'expriment donc pas PVL, ce qui confirme la spécificité de la méthode.

Pour le typage, les transitions de la catégorie T de chaque souche sont comparées au transitions des autres souches considérées comme des souches de référence. En pratique une valeur 0 est attribuée lorsque les transitions entre les deux souches se classent dans la même catégorie (positive ou négative) et une valeur de 1 est attribuée lorsque les transitions entre les 2 souches se classent dans des catégories différentes (une transition positive et une transition négative). Les valeurs sont sommées pour toutes les transitions de la catégorie T de chaque couple de souche afin d'établir un score. Les scores figurent dans le **TABLEAU 6 :**

**TABLEAU 6 :**

| | ID2 | ID3 | ID4 | ID2 bis | ID3 bis | ID4 bis | AST 7 | AST 8 | AST 13 | AST 14 | VIR 5 | VIR 6 | VIR 7 | ID7 | AST 2 | VIR 21 | VIR 26 |
|---|---|---|---|---|---|---|---|---|---|---|---|---|---|---|---|---|---|
| ID2 | 0 | 17 | 6 | 1 | 17 | 7 | 24 | 24 | 10 | 18 | 51 | 18 | 38 | 52 | 51 | 52 | 52 |
| ID3 | 17 | 0 | 15 | 18 | 0 | 16 | 15 | 15 | 27 | 25 | 42 | 9 | 29 | 43 | 46 | 43 | 43 |
| ID4 | 6 | 15 | 0 | 7 | 15 | 3 | 30 | 30 | 14 | 24 | 55 | 22 | 42 | 58 | 57 | 58 | 58 |
| ID2bis | 1 | 18 | 7 | 0 | 18 | 8 | 23 | 23 | 9 | 17 | 50 | 17 | 37 | 51 | 50 | 51 | 51 |
| ID3bis | 17 | 0 | 15 | 18 | 0 | 16 | 15 | 15 | 27 | 25 | 42 | 9 | 29 | 43 | 46 | 43 | 43 |
| ID4bis | 7 | 16 | 3 | 8 | 16 | 0 | 31 | 31 | 15 | 25 | 54 | 23 | 41 | 59 | 58 | 59 | 59 |
| AST7 | 24 | 15 | 30 | 23 | 15 | 31 | 0 | 4 | 20 | 12 | 27 | 8 | 16 | 32 | 35 | 32 | 34 |
| AST8 | 24 | 15 | 30 | 23 | 15 | 31 | 4 | 0 | 20 | 14 | 27 | 10 | 14 | 32 | 35 | 32 | 34 |
| AST13 | 10 | 27 | 14 | 9 | 27 | 15 | 20 | 20 | 0 | 14 | 43 | 24 | 30 | 46 | 45 | 46 | 46 |
| AST14 | 18 | 25 | 24 | 17 | 25 | 25 | 12 | 14 | 14 | 0 | 33 | 18 | 24 | 40 | 43 | 40 | 40 |
| VIR5 | 51 | 42 | 55 | 50 | 42 | 54 | 27 | 27 | 43 | 33 | 0 | 35 | 13 | 27 | 28 | 25 | 27 |
| VIR6 | 18 | 9 | 22 | 17 | 9 | 23 | 8 | 10 | 24 | 18 | 35 | 0 | 24 | 40 | 43 | 40 | 42 |
| VIR7 | 38 | 29 | 42 | 37 | 29 | 41 | 16 | 14 | 30 | 24 | 13 | 24 | 0 | 34 | 37 | 34 | 36 |
| ID7 | 52 | 43 | 58 | 51 | 43 | 59 | 32 | 32 | 46 | 40 | 27 | 40 | 34 | - | - | - | - |
| AST2 | 51 | 46 | 57 | 50 | 46 | 58 | 35 | 35 | 45 | 43 | 28 | 43 | 37 | - | - | - | - |
| VIR21 | 52 | 43 | 58 | 51 | 43 | 59 | 32 | 32 | 46 | 40 | 25 | 40 | 34 | - | - | - | - |
| VIR26 | 52 | 43 | 58 | 51 | 43 | 59 | 34 | 34 | 46 | 40 | 27 | 42 | 36 | - | - | - | - |

Les souches qui ont un score inférieur ou égal à 4 sont de même type, les souches qui ont un score strictement supérieur à 4 sont de type différent.
Ainsi, les souches ID2 et ID2bis, ID3 et ID3bis, ID4 et ID4bis, AST7 et AST8 sont de même type. Toutes les autres souches prises deux à deux sont de type différent. Il faut noter les sommes élevées obtenues entre les souches ID7, AST2, VIR21 et VIR26, qui ne sont pas des *S. aureus,* et toutes les autres souches qui sont des *S. aureus.* Ces résultats confirment la spécificité de la méthode.

Les souches ID7, AST2, VIR21 et VIR26 ne sont bien entendu pas de même type, ce serait absurde, elles sont d'espèces différentes. Ces souches ne peuvent donc pas être comparées entre elles et aucune valeur n'est reportée dans le TABLEAU 6. De façon extrêmement intéressante, des scores supérieurs à 25, comme par exemple entre ID2 et VR7, traduisent une grande divergence entre souches. Des scores entre 15 et 25, comme entre ID2 et AST14, traduisent une divergence modérée et des scores entre 5 et 15, comme entre ID2 et ID4bis, une divergence faible.

La méthode ainsi mise en oeuvre permet donc non seulement d'établir si 2 souches sont de même type, ce qui est important pour identifier un foyer infectieux commun, mais également d'estimer la proximité de 2 souches, ce qui est extrêmement important pour les études épidémiologiques.

Cet exemple montre que, de façon très avantageuse, la présente invention permet en un temps inférieur à une heure, ce qui est très court, de confirmer l'identité d'une espèce telle que *S. aureus* et de déterminer, simultanément au sein de la même analyse, les propriétés de typage, de résistance potentielle à au moins un antibiotique et d'établir l'existence de facteur de virulence. Ces propriétés ont été établies avec le même instrument, ce qui facilite grandement l'analyse et le rendu des résultats. Enfin, les caractéristiques des bactéries sont établies à l'aide de protéines bactériennes, ce qui traduit l'existence de microorganismes vivants et viables, contrairement aux caractérisations à partir d'ADN bactérien qui peuvent être faussées par l'existence de bactéries mortes.

### Exemple 6 : Protocole de digestion de microorganismes avec une étape de dessalage

Classiquement le protocole suivant est mis en oeuvre en 17 étapes :
Etapes 1 à 10 : idem exemple 4.

11. Le volume d'échantillon est complété à 1mL avec eau/acide formique 0,1% (v/v)
12. Equilibrage des colonnes HLB Oasis Waters avec 1ml de méthanol puis 1 ml H₂O/acide formique 0,1% (v/v)
13. Dépôt de l'échantillon qui s'écoule par gravité
14. Lavage avec 1 ml H₂O/acide formique 0,1 % (v/v)
15. Elution avec 1ml d'un mélange 80% de méthanol et 20% d'eau/acide formique 0,1 % (v/v)
16. L'éluât est évaporé avec un évaporateur de type SpeedVac® SPD2010 (Thermo Electron Corporation, Waltham, Massachusetts, Etats-Unis d'Amérique), durant 2 heures, afin d'obtenir un volume d'environ 100µl.
17. L'éluât est ensuite repris dans une solution eau/acide formique 0,5% (v/v) quantité suffisante pour (QSP) 250µl

### Exemple 7 : Caractérisation d'échantillons de E coli:

Après avoir établi la ou les espèces des échantillons selon l'une quelconque des méthodes décrites dans les exemples 1 à 3, les espèces listées ci-dessous sont analysées. Quinze souches de *E coli* sont analysées pour confirmer leur identification et établir leurs caractéristiques :

| | |
|---|---|
| AST1 | AST2 |
| AST3 | AST4 |
| AST5 | VIR41 |
| VIR42 | VIR43 |
| VIR44 | VIR45 |
| ID6 | ID7 |
| ID8 | ID9 |
| ID10 | |

La même méthode d'analyse est appliquée à des espèces n'appartenant pas à l'espèce *E. coli* pour servir de témoin négatif :
- *S. aureus* VIR10
- *S. pneumoniae* VIR19
- *C. difficile* VIR28

Chaque échantillon est traité selon l'exemple 6, puis un volume de 5µl de protéines digérées est injecté et analysé selon les conditions suivantes :
• Chaîne chromatographique Agilent 1100 series de la société Agilent Technologies (Massy, France).
• Colonne Waters Symmetry C18, 2,1mm de diamètre interne, 100mm de long, taille de particule de 3,5µm.
• Solvant A : H₂O + 0,1% acide formique.
• Solvant B : ACN + 0,1% acide formique.
• Gradient HPLC défini dans le **TABLEAU 7** ci-après :

**TABLEAU 7**

| Temps (min) | Débit (µl) | Solvant A (%) | Solvant B (%) |
|---|---|---|---|
| 0 | 300 | 95 | 5 |
| 3 | 300 | 95 | 5 |
| 28 | 300 | 60 | 40 |
| 30 | 300 | 0 | 100 |
| 38 | 300 | 0 | 100 |
| 38.1 | 300 | 95 | 5 |
| 45 | 300 | 95 | 5 |

• L'éluât en sortie de colonne chromatographique est directement injecté dans la source d'ionisation du spectromètre de masse de type QTRAP® 5500 de la société Applied Biosystems (Foster City, Etats Unis d'Amérique).
• Les peptides, issues de la digestion des protéines du microorganisme, sont analysés par le spectromètre de masse en mode MRM. Seuls les peptides indiqués dans le **TABLEAU 8** sont détectés. Pour cela, le ou les fragments de première génération indiqués dans le **TABLEAU 8** sont détectés. L'application, à laquelle permet de répondre chaque transition, c'est à dire chaque peptide associé à son fragment de première génération, est précisée dans la colonne intérêt clinique du **TABLEAU 8** avec les lettres I, T, R et V. I désigne la confirmation de l'identification du microorganisme, T le typage, R la résistance à au moins un antibiotique et V la détection de facteurs de virulence.

**TABLEAU 8 :**

| Transition numéro | Protéine | Peptide | ion fragment de première génération | Intérêt clinique |
|---|---|---|---|---|
| 1 | ACON2 | ILEIEGLPDLK | y7 monochargé | I+T |
| 2 | ACON2 | ILEIEGLPDLK | y8 monochargé | I+T |
| 3 | ACON2 | ILEIEGLPDLK | y9 monochargé | I+T |
| 4 | ACON2 | VADGATVVSTSTR | y7 monochargé | I+T |
| 5 | ACON2 | VADGATVVSTSTR | y8 monochargé | I+T |
| 6 | ACON2 | VADGATVVSTSTR | y9 monochargé | I+T |
| 7 | ASPG2 | TNTTDVATFK | y6 monochargé | I+T |
| 8 | ASPG2 | TNTTDVATFK | y7 monochargé | I+T |
| 9 | ASPG2 | TNTTDVATFK | y8 monochargé | I+T |
| 10 | FABB | LDTTGLIDR | y6 monochargé | I+T |
| 11 | FABB | LDTTGLIDR | y7 monochargé | I+T |
| 12 | FABB | LDTTGLIDR | y8 monochargé | I+T |
| 13 | FABB | VGLIAGSGGGSPR | y8 monochargé | I+T |
| 14 | FABB | VGLIAGSGGGSPR | y9 monochargé | I+T |
| 15 | FABB | VGLIAGSGGGSPR | y10 monochargé | I+T |
| 16 | GLNH | AIDFSDGYYK | y6 monochargé | I+T |
| 17 | GLNH | AIDFSDGYYK | y7 monochargé | I+T |
| 18 | GLNH | AIDFSDGYYK | y8 monochargé | I+T |
| 19 | MODA | LGAWDTLSPK | y7 monochargé | I+T |
| 20 | MODA | LGAWDTLSPK | y8 monochargé | I+T |
| 21 | MODA | LGAWDTLSPK | y9 monochargé | I+T |
| 22 | ODP2 | FGEIEEVELGR | y7 monochargé | I+T |
| 23 | ODP2 | FGEIEEVELGR | y8 monochargé | I+T |
| 24 | ODP2 | FGEIEEVELGR | y9 monochargé | I+T |
| 25 | OMPC | INLLDDNQFTR | y7 monochargé | I+T |
| 26 | OMPC | INLLDDNQFTR | y8 monochargé | I+T |
| 27 | OMPC | INLLDDNQFTR | y9 monochargé | I+T |
| 28 | PFLB | LATAWEGFTK | y6 monochargé | I+T |
| 29 | PFLB | LATAWEGFTK | y7 monochargé | I+T |
| 30 | PFLB | LATAWEGFTK | y8 monochargé | I+T |
| 31 | SUCD | DSILEAIDAGIK | y8 monochargé | I+T |
| 32 | SUCD | DSILEAIDAGIK | y9 monochargé | I+T |
| 33 | SUCD | DSILEAIDAGIK | y10 monochargé | I+T |
| 34 | SUCD | FAALEAAGVK | y7 monochargé | I+T |
| 35 | SUCD | FAALEAAGVK | y8 monochargé | I+T |
| 36 | SUCD | FAALEAAGVK | y9 monochargé | I+T |
| 37 | SUCD | SLADIGEALK | y6 monochargé | I+T |
| 38 | SUCD | SLADIGEALK | y7 monochargé | I+T |
| 39 | SUCD | SLADIGEALK | y8 monochargé | I+T |
| 40 | TKT1 | TEEQLANIAR | y7 monochargé | I+T |
| 41 | TKT1 | TEEQLANIAR | y8 monochargé | I+T |
| 42 | TKT1 | TEEQLANIAR | y9 monochargé | I+T |
| 43 | YFCZ | AEAEQTLAALTEK | y8 monochargé | I+T |
| 44 | YFCZ | AEAEQTLAALTEK | y9 monochargé | I+T |
| 45 | YFCZ | AEAEQTLAALTEK | y10 monochargé | I+T |
| 46 | YGAU | SGDTLSAISK | y6 monochargé | I+T |
| 47 | YGAU | SGDTLSAISK | y7 monochargé | I+T |
| 48 | YGAU | SGDTLSAISK | y8 monochargé | I+T |
| 49 | TEM-2 | LLTGELLTLASR | y7 monochargé | R+T |
| 50 | TEM-2 | LLTGELLTLASR | y8 monochargé | R+T |
| 51 | TEM-2 | LLTGELLTLASR | y9 monochargé | R+T |
| 52 | TEM-2 | SALPAGWFIADK | y7 monochargé | R+T |
| 53 | TEM-2 | SALPAGWFIADK | y8 monochargé | R+T |
| 54 | TEM-2 | SALPAGWFIADK | y9 monochargé | R+T |
| 55 | TEM-2 | VAGPLLR | y4 monochargé | R+T |
| 56 | TEM-2 | VAGPLLR | y5 monochargé | R+T |
| 57 | TEM-2 | VAGPLLR | y6 monochargé | R+T |
| 58 | TEM-2 | VGYIELDLNSGK | y8 monochargé | R+T |
| 59 | TEM-2 | VGYIELDLNSGK | y9 monochargé | R+T |
| 60 | TEM-2 | VGYIELDLNSGK | y10 monochargé | R+T |
| 61 | TEM-2 | VLLCGAVLSR | y7 monochargé | R+T |
| 62 | TEM-2 | VLLCGAVLSR | y8 monochargé | R+T |
| 63 | TEM-2 | VLLCGAVLSR | y9 monochargé | R+T |
| 64 | ASPA | ISDIPEFVR | y5 monochargé | T |
| 65 | ASPA | ISDIPEFVR | y6 monochargé | T |
| 66 | ASPA | ISDIPEFVR | y7 monochargé | T |
| 67 | ASPA | IEEDLLGTR | y6 monochargé | T |
| 68 | ASPA | IEEDLLGTR | y7 monochargé | T |
| 69 | ASPA | IEEDLLGTR | y8 monochargé | T |
| 70 | ASPA | LVDAINQLR | y6 monochargé | T |
| 71 | ASPA | LVDAINQLR | y7 monochargé | T |
| 72 | ASPA | LVDAINQLR | y8 monochargé | T |
| 73 | ATPA | TALAIDAIINQR | y7 monochargé | T |
| 74 | ATPA | TALAIDAIINQR | y8 monochargé | T |
| 75 | ATPA | TALAIDAIINQR | y9 monochargé | T |
| 76 | ATPA | VVNTLGAPIDGK | y7 monochargé | T |
| 77 | ATPA | VVNTLGAPIDGK | y8 monochargé | T |
| 78 | ATPA | VVNTLGAPIDGK | y9 monochargé | T |
| 79 | CH10 | SAGGIVLTGSAAAK | y9 monochargé | T |
| 80 | CH10 | SAGGIVLTGSAAAK | y10 monochargé | T |
| 81 | CH10 | SAGGIVLTGSAAAK | y11 monochargé | T |
| 82 | CH60 | AVTAAVEELK | y6 monochargé | T |
| 83 | CH60 | AVTAAVEELK | y7 monochargé | T |
| 84 | CH60 | AVTAAVEELK | y8 monochargé | T |
| 85 | DBHB | ALDAIIASVTESLK | y8 monochargé | T |
| 86 | DBHB | ALDAIIASVTESLK | y9 monochargé | T |
| 87 | DBHB | ALDAIIASVTESLK | y10 monochargé | T |
| 88 | DCEB | YWDVELR | y5 monochargé | T |
| 89 | DCEB | YWDVELR | y6 monochargé | T |
| 90 | DCEB | YWDVELR | y4 monochargé | T |
| 91 | DHSA | LPGILELSR | y6 monochargé | T |
| 92 | DHSA | LPGILELSR | y7 monochargé | T |
| 93 | DHSA | LPGILELSR | y8 monochargé | T |
| 94 | DPS | SKATNLLYTR | y6 monochargé | T |
| 95 | DPS | SKATNLLYTR | y7 monochargé | T |
| 96 | DPS | SKATNLLYTR | y8 monochargé | T |
| 97 | HNS | SEALKILNNIR | y7 monochargé | T |
| 98 | HNS | SEALKILNNIR | y8 monochargé | T |
| 99 | HNS | SEALKILNNIR | y9 monochargé | T |
| 100 | MDH | LFGVTTLDIIR | y6 monochargé | T |
| 101 | MDH | LFGVTTLDIIR | y7 monochargé | T |
| 102 | MDH | LFGVTTLDIIR | y8 monochargé | T |
| 103 | PGK | ASLPTIELALK | y7 monochargé | T |
| 104 | PGK | ASLPTIELALK | y8 monochargé | T |
| 105 | PGK | ASLPTIELALK | y9 monochargé | T |
| 106 | PUR7 | LLSDTECLVK | y6 monochargé | T |
| 107 | PUR7 | LLSDTECLVK | y7 monochargé | T |
| 108 | PUR7 | LLSDTECLVK | y8 monochargé | T |
| 109 | RL4 | SILSELVR | y5 monochargé | T |
| 110 | RL4 | SILSELVR | y6 monochargé | T |
| 111 | RL4 | SILSELVR | y7 monochargé | T |
| 112 | RS1 | GGFTVELNGIR | y7 monochargé | T |
| 113 | RS1 | GGFTVELNGIR | y8 monochargé | T |
| 114 | RS1 | GGFTVELNGIR | y9 monochargé | T |
| 115 | YJGF | TGEVPADVAAQAR | y8 monochargé | T |
| 116 | YJGF | TGEVPADVAAQAR | y9 monochargé | T |
| 117 | YJGF | TGEVPADVAAQAR | y10 monochargé | T |
| 118 | stx1A | TYVDSLNVIR | y6 monochargé | V+T |
| 119 | stx1A | TYVDSLNVIR | y7 monochargé | V+T |
| 120 | stx1A | TYVDSLNVIR | y8 monochargé | V+T |
| 121 | stx1A-2A | FVTVTAEALR | y7 monochargé | V+T |
| 122 | stx1A-2A | FVTVTAEALR | y8 monochargé | V+T |
| 123 | stx2A | ISNVLPEYR | y6 monochargé | V+T |
| 124 | stx2A | ISNVLPEYR | y7 monochargé | V+T |
| 125 | stx2A | ISNVLPEYR | y8 monochargé | V+T |

L'état de charge du peptide précurseur, son temps de rétention et les transitions, c'est-à-dire les rapports (m/z)₁ en Q1 et (m/z)₂ en Q3, sont indiqués dans le **TABLEAU 9.** L'énergie de collision utilisée pour fragmenter l'ion précurseur est également indiquée dans le **TABLEAU 9.**

**TABLEAU 9 :**

| Transition Numéro | Etat de charge du précurseur | Temps de rétention | (m/z) filtré en Q1 | (m/z) filtré en Q3 | Energie de collision |
|---|---|---|---|---|---|
| 1 | 2 | 19.04 | 620.36 | 771.42 | 36 |
| 2 | 2 | 19.04 | 620.36 | 884.51 | 36 |
| 3 | 2 | 19.04 | 620.36 | 1013.55 | 36 |
| 4 | 2 | 8.93 | 632.33 | 749.42 | 37 |
| 5 | 2 | 8.93 | 632.33 | 850.46 | 37 |
| 6 | 2 | 8.93 | 632.33 | 921.5 | 37 |
| 7 | 2 | 9.99 | 549.28 | 680.36 | 32 |
| 8 | 2 | 9.99 | 549.28 | 781.41 | 32 |
| 9 | 2 | 9.99 | 549.28 | 882.46 | 32 |
| 10 | 2 | 12.89 | 502.27 | 674.38 | 30 |
| 11 | 2 | 12.89 | 502.27 | 775.43 | 30 |
| 12 | 2 | 12.89 | 502.27 | 890.46 | 30 |
| 13 | 2 | 10.63 | 564.31 | 674.32 | 33 |
| 14 | 2 | 10.63 | 564.31 | 745.36 | 33 |
| 15 | 2 | 10.63 | 564.31 | 858.44 | 33 |
| 16 | 2 | 14.12 | 589.77 | 732.32 | 34 |
| 17 | 2 | 14.12 | 589.77 | 879.39 | 34 |
| 18 | 2 | 14.12 | 589.77 | 994.42 | 34 |
| 19 | 2 | 15.09 | 544.29 | 846.44 | 32 |
| 20 | 2 | 15.09 | 544.29 | 917.47 | 32 |
| 21 | 2 | 15.09 | 544.29 | 974.49 | 32 |
| 22 | 2 | 15.61 | 639.32 | 831.42 | 37 |
| 23 | 2 | 15.61 | 639.32 | 944.5 | 37 |
| 24 | 2 | 15.61 | 639.32 | 1073.55 | 37 |
| 25 | 2 | 16.42 | 674.85 | 895.39 | 39 |
| 26 | 2 | 16.42 | 674.85 | 1008.47 | 39 |
| 27 | 2 | 16.42 | 674.85 | 1121.56 | 39 |
| 28 | 2 | 15.49 | 562.29 | 767.37 | 33 |
| 29 | 2 | 15.49 | 562.29 | 838.41 | 33 |
| 30 | 2 | 15.49 | 562.29 | 939.46 | 33 |
| 31 | 2 | 19.84 | 622.84 | 816.45 | 36 |
| 32 | 2 | 19.84 | 622.84 | 929.53 | 36 |
| 33 | 2 | 19.84 | 622.84 | 1042.61 | 36 |
| 34 | 2 | 13.35 | 488.78 | 687.4 | 29 |
| 35 | 2 | 13.35 | 488.78 | 758.44 | 29 |
| 36 | 2 | 13.35 | 488.78 | 829.48 | 29 |
| 37 | 2 | 15.56 | 508.78 | 630.38 | 30 |
| 38 | 2 | 15.56 | 508.78 | 745.41 | 30 |
| 39 | 2 | 15.56 | 508.78 | 816.45 | 30 |
| 40 | 2 | 11.4 | 572.8 | 785.46 | 34 |
| 41 | 2 | 11.4 | 572.8 | 914.51 | 34 |
| 42 | 2 | 11.4 | 572.8 | 1043.55 | 34 |
| 43 | 2 | 14.85 | 687.86 | 846.49 | 39 |
| 44 | 2 | 14.85 | 687.86 | 974.55 | 39 |
| 45 | 2 | 14.85 | 687.86 | 1103.59 | 39 |
| 46 | 2 | 9.8 | 489.76 | 618.38 | 29 |
| 47 | 2 | 9.8 | 489.76 | 719.43 | 29 |
| 48 | 2 | 9.8 | 489.76 | 834.46 | 29 |
| 49 | 2 | 19.38 | 643.89 | 773.49 | 33 |
| 50 | 2 | 19.38 | 643.89 | 902.53 | 33 |
| 51 | 2 | 19.38 | 643.89 | 959.55 | 33 |
| 52 | 2 | 18.43 | 638.34 | 836.43 | 33 |
| 53 | 2 | 18.43 | 638.34 | 907.47 | 33 |
| 54 | 2 | 18.43 | 638.34 | 1004.52 | 33 |
| 55 | 2 | 10.79 | 363.24 | 498.34 | 21 |
| 56 | 2 | 10.79 | 363.24 | 555.36 | 21 |
| 57 | 2 | 10.79 | 363.24 | 626.4 | 21 |
| 58 | 2 | 15.94 | 654.35 | 875.45 | 34 |
| 59 | 2 | 15.94 | 654.35 | 988.53 | 34 |
| 60 | 2 | 15.94 | 654.35 | 1151.59 | 34 |
| 61 | 2 | 14.8 | 544.32 | 762.39 | 29 |
| 62 | 2 | 14.8 | 544.32 | 875.48 | 29 |
| 63 | 2 | 14.8 | 544.32 | 988.56 | 29 |
| 64 | 2 | 12.8 | 538.29 | 647.35 | 29 |
| 65 | 2 | 12.8 | 538.29 | 760.44 | 29 |
| 66 | 2 | 12.8 | 538.29 | 875.46 | 29 |
| 67 | 2 | 12.9 | 523.28 | 674.38 | 28 |
| 68 | 2 | 12.9 | 523.28 | 803.43 | 28 |
| 69 | 2 | 12.9 | 523.28 | 932.47 | 28 |
| 70 | 2 | 15.31 | 521.31 | 714.43 | 28 |
| 71 | 2 | 15.31 | 521.31 | 829.45 | 28 |
| 72 | 2 | 15.31 | 521.31 | 928.52 | 28 |
| 73 | 2 | 17.37 | 649.87 | 829.45 | 34 |
| 74 | 2 | 17.37 | 649.87 | 942.54 | 34 |
| 75 | 2 | 17.37 | 649.87 | 1013.57 | 34 |
| 76 | 2 | 11.94 | 592.34 | 657.36 | 31 |
| 77 | 2 | 11.94 | 592.34 | 770.44 | 31 |
| 78 | 2 | 11.94 | 592.34 | 871.49 | 31 |
| 79 | 2 | 11.08 | 601.84 | 817.48 | 31 |
| 80 | 2 | 11.08 | 601.84 | 930.56 | 31 |
| 81 | 2 | 11.08 | 601.84 | 987.58 | 31 |
| 82 | 2 | 11.86 | 515.79 | 688.39 | 28 |
| 83 | 2 | 11.86 | 515.79 | 759.42 | 28 |
| 84 | 2 | 11.86 | 515.79 | 860.47 | 28 |
| 85 | 2 | 22.24 | 715.91 | 834.46 | 36 |
| 86 | 2 | 22.24 | 715.91 | 947.54 | 36 |
| 87 | 2 | 22.24 | 715.91 | 1060.62 | 36 |
| 88 | 2 | 15.35 | 490.75 | 631.34 | 27 |
| 89 | 2 | 15.35 | 490.75 | 817.42 | 27 |
| 90 | 2 | 15.35 | 490.75 | 516.31 | 27 |
| 91 | 2 | 17.1 | 499.31 | 730.45 | 27 |
| 92 | 2 | 17.1 | 499.31 | 787.47 | 27 |
| 93 | 2 | 17.1 | 499.31 | 884.52 | 27 |
| 94 | 2 | 10.02 | 583.83 | 779.44 | 31 |
| 95 | 2 | 10.02 | 583.83 | 880.49 | 31 |
| 96 | 2 | 10.02 | 583.83 | 951.53 | 31 |
| 97 | 2 | 14.94 | 635.88 | 870.55 | 33 |
| 98 | 2 | 14.94 | 635.88 | 983.64 | 33 |
| 99 | 2 | 14.94 | 635.88 | 1054.67 | 33 |
| 100 | 2 | 20.34 | 624.37 | 730.45 | 32 |
| 101 | 2 | 20.34 | 624.37 | 831.49 | 32 |
| 102 | 2 | 20.34 | 624.37 | 930.56 | 32 |
| 103 | 2 | 18.16 | 578.35 | 787.49 | 30 |
| 104 | 2 | 18.16 | 578.35 | 884.55 | 30 |
| 105 | 2 | 18.16 | 578.35 | 997.63 | 30 |
| 106 | 2 | 13.11 | 589.31 | 749.39 | 31 |
| 107 | 2 | 13.11 | 589.31 | 864.41 | 31 |
| 108 | 2 | 13.11 | 589.31 | 951.45 | 31 |
| 109 | 2 | 17.68 | 458.78 | 603.35 | 25 |
| 110 | 2 | 17.68 | 458.78 | 716.43 | 25 |
| 111 | 2 | 17.68 | 458.78 | 829.51 | 25 |
| 112 | 2 | 15.99 | 581.81 | 800.46 | 31 |
| 113 | 2 | 15.99 | 581.81 | 901.51 | 31 |
| 114 | 2 | 15.99 | 581.81 | 1048.58 | 31 |
| 115 | 2 | 10.34 | 642.83 | 801.42 | 33 |
| 116 | 2 | 10.34 | 642.83 | 898.47 | 33 |
| 117 | 2 | 10.34 | 642.83 | 997.54 | 33 |
| 118 | 2 | 14.85 | 590.32 | 701.43 | 31 |
| 119 | 2 | 14.85 | 590.32 | 816.46 | 31 |
| 120 | 2 | 14.85 | 590.32 | 915.53 | 31 |
| 121 | 2 | 14.15 | 553.81 | 759.44 | 29 |
| 122 | 2 | 14.15 | 553.81 | 860.48 | 29 |
| 123 | 2 | 12.9 | 545.8 | 776.43 | 29 |
| 124 | 2 | 12.9 | 545.8 | 890.47 | 29 |
| 125 | 2 | 12.9 | 545.8 | 977.51 | 29 |

• Les autres paramètres machine utilisés sont les suivants :

| | |
|---|---|
| Type de balayage: | MRM |
| MRM planifié : | oui |
| Polarité: | Positive |
| Source d'ionisation: | Turbo V™ (Applied BioSystems) |
| Réglage Q1: | Filtrage avec résolution unitaire |
| Réglage Q3: | Filtrage avec résolution unitaire |
| Pause inter-scan: | 5.00 msec |
| Vitesse de balayage: | 10 Da/s |
| Gaz rideau: | 50,00 psi |
| Tension de cône: | 5500,00 V |
| Température de source: | 550,00 °C |
| Gaz de nébulisation: | 50,00 psi |
| Gaz chauffant: | 40,00 psi |
| Remplissage dynamique: | activé |
| Potentiel d'orifice : | 100, 00 V |
| (en anglais declustering potential (DP)) | |
| Potentiel d'entrée avant Q0 (EP): | 9,00 V |
| Potentiel en sortie de cellule de collision : | 35 V |
| (en anglais cell exit potential (CXP)) | |
| Temps de cycle total: | 1.2 sec |
| Fenêtre de détection | 80 sec |

Les aires obtenues pour chacune des transitions et pour chacun des microorganismes étudiés ont été mesurées. Toutes les transitions dont l'aire est supérieure ou égale à 2500 (unité arbitraire) sont considérées comme positives et ont été notées « 1 » dans les **tableaux 10A** et **10B.** Toutes les transitions dont l'aire est inférieure à 2500, sont considérées comme négatives et ont été notées 0 dans les tableaux 10A et 10B. Lorsqu'on n'a observé aucun pic de signal, on a noté la transition comme négative.

Le nombre de transition positive est ensuite sommé pour les applications I, R et V et reporté dans le **TABLEAU 11 :**

**TABLEAU 10A :**

| Transition numéro | AST1 | AST2 | AST3 | AST4 | AST5 | VIR41 | VIR42 | VIR43 | VIR44 |
|---|---|---|---|---|---|---|---|---|---|
| 1 | 1 | 1 | 1 | 1 | 1 | 1 | 1 | 1 | 1 |
| 2 | 1 | 1 | 1 | 1 | 1 | 1 | 1 | 1 | 1 |
| 3 | 1 | 1 | 1 | 1 | 1 | 1 | 1 | 1 | 1 |
| 4 | 1 | 1 | 1 | 1 | 1 | 1 | 1 | 1 | 1 |
| 5 | 1 | 1 | 1 | 1 | 1 | 1 | 1 | 1 | 1 |
| 6 | 1 | 1 | 1 | 1 | 1 | 1 | 1 | 1 | 1 |
| 7 | 1 | 1 | 1 | 1 | 1 | 1 | 1 | 1 | 1 |
| 8 | 1 | 1 | 1 | 1 | 1 | 1 | 1 | 1 | 1 |
| 9 | 1 | 1 | 1 | 1 | 1 | 1 | 1 | 1 | 1 |
| 10 | 1 | 1 | 1 | 1 | 1 | 1 | 1 | 1 | 1 |
| 11 | 1 | 1 | 1 | 1 | 1 | 1 | 1 | 1 | 1 |
| 12 | 1 | 1 | 1 | 1 | 1 | 1 | 1 | 1 | 1 |
| 13 | 1 | 1 | 1 | 1 | 1 | 1 | 1 | 1 | 1 |
| 14 | 1 | 1 | 1 | 1 | 1 | 1 | 1 | 1 | 1 |
| 15 | 1 | 1 | 1 | 1 | 1 | 1 | 1 | 1 | 1 |
| 16 | 1 | 1 | 1 | 1 | 1 | 1 | 1 | 1 | 1 |
| 17 | 1 | 1 | 1 | 1 | 1 | 1 | 1 | 1 | 1 |
| 18 | 1 | 1 | 1 | 1 | 1 | 1 | 1 | 1 | 1 |
| 19 | 1 | 1 | 1 | 1 | 1 | 1 | 1 | 1 | 1 |
| 20 | 1 | 1 | 1 | 1 | 1 | 1 | 1 | 1 | 1 |
| 21 | 1 | 1 | 1 | 1 | 1 | 1 | 1 | 1 | 1 |
| 22 | 1 | 1 | 1 | 1 | 1 | 1 | 1 | 1 | 1 |
| 23 | 1 | 1 | 1 | 1 | 1 | 1 | 1 | 1 | 1 |
| 24 | 1 | 1 | 1 | 1 | 1 | 1 | 1 | 1 | 1 |
| 25 | 1 | 1 | 1 | 1 | 1 | 1 | 1 | 1 | 1 |
| 26 | 1 | 1 | 1 | 1 | 1 | 1 | 1 | 1 | 1 |
| 27 | 1 | 1 | 1 | 1 | 1 | 0 | 0 | 0 | 0 |
| 28 | 1 | 1 | 1 | 1 | 1 | 1 | 1 | 1 | 1 |
| 29 | 1 | 1 | 1 | 1 | 1 | 1 | 1 | 1 | 1 |
| 30 | 1 | 1 | 1 | 1 | 1 | 1 | 1 | 1 | 1 |
| 31 | 1 | 1 | 1 | 1 | 1 | 1 | 1 | 1 | 1 |
| 32 | 1 | 1 | 1 | 1 | 1 | 1 | 1 | 1 | 1 |
| 33 | 1 | 1 | 1 | 1 | 1 | 1 | 1 | 1 | 1 |
| 34 | 1 | 1 | 1 | 1 | 1 | 1 | 1 | 1 | 1 |
| 35 | 1 | 1 | 1 | 1 | 1 | 1 | 1 | 1 | 1 |
| 36 | 1 | 1 | 1 | 1 | 1 | 1 | 1 | 1 | 1 |
| 37 | 1 | 1 | 1 | 1 | 1 | 1 | 1 | 1 | 1 |
| 38 | 1 | 1 | 1 | 1 | 1 | 1 | 1 | 1 | 1 |
| 39 | 1 | 1 | 1 | 1 | 1 | 1 | 1 | 1 | 1 |
| 40 | 1 | 1 | 1 | 1 | 1 | 1 | 1 | 1 | 1 |
| 41 | 1 | 1 | 1 | 1 | 1 | 1 | 1 | 1 | 1 |
| 42 | 1 | 1 | 0 | 0 | 0 | 1 | 1 | 1 | 1 |
| 43 | 1 | 1 | 1 | 1 | 1 | 1 | 1 | 1 | 1 |
| 44 | 1 | 1 | 1 | 1 | 1 | 1 | 1 | 1 | 1 |
| 45 | 1 | 1 | 1 | 1 | 1 | 1 | 1 | 1 | 1 |
| 46 | 1 | 1 | 1 | 1 | 1 | 1 | 1 | 1 | 1 |
| 47 | 1 | 1 | 1 | 1 | 1 | 1 | 1 | 1 | 1 |
| 48 | 1 | 1 | 1 | 1 | 1 | 1 | 1 | 1 | 1 |
| 49 | 0 | 1 | 1 | 1 | 1 | 1 | 1 | 1 | 1 |
| 50 | 0 | 1 | 1 | 1 | 1 | 0 | 0 | 0 | 0 |
| 51 | 0 | 1 | 1 | 1 | 1 | 1 | 1 | 1 | 1 |
| 52 | 0 | 1 | 1 | 1 | 1 | 0 | 0 | 1 | 1 |
| 53 | 0 | 1 | 1 | 1 | 1 | 0 | 0 | 0 | 0 |
| 54 | 0 | 1 | 1 | 1 | 1 | 0 | 0 | 0 | 0 |
| 55 | 0 | 1 | 1 | 1 | 1 | 1 | 1 | 1 | 1 |
| 56 | 1 | 1 | 1 | 1 | 1 | 0 | 0 | 0 | 1 |
| 57 | 0 | 1 | 1 | 1 | 1 | 0 | 0 | 0 | 0 |
| 58 | 0 | 1 | 1 | 1 | 1 | 0 | 0 | 0 | 0 |
| 59 | 0 | 1 | 1 | 1 | 1 | 0 | 0 | 0 | 0 |
| 60 | 0 | 1 | 1 | 1 | 1 | 0 | 0 | 0 | 0 |
| 61 | 0 | 1 | 1 | 1 | 1 | 0 | 0 | 0 | 0 |
| 62 | 0 | 1 | 1 | 1 | 1 | 0 | 1 | 1 | 1 |
| 63 | 0 | 1 | 1 | 1 | 1 | 0 | 0 | 0 | 0 |
| 64 | 0 | 0 | 0 | 0 | 0 | 0 | 0 | 0 | 1 |
| 65 | 0 | 0 | 0 | 0 | 0 | 0 | 0 | 0 | 1 |
| 66 | 0 | 0 | 0 | 0 | 0 | 1 | 1 | 1 | 1 |
| 67 | 1 | 1 | 1 | 1 | 1 | 1 | 1 | 1 | 1 |
| 68 | 1 | 1 | 1 | 1 | 1 | 1 | 1 | 1 | 1 |
| 69 | 1 | 1 | 1 | 1 | 1 | 1 | 1 | 1 | 1 |
| 70 | 1 | 1 | 1 | 1 | 1 | 1 | 1 | 1 | 1 |
| 71 | 1 | 1 | 1 | 1 | 1 | 1 | 1 | 1 | 1 |
| 72 | 1 | 1 | 1 | 1 | 1 | 1 | 1 | 1 | 1 |
| 73 | 1 | 1 | 1 | 1 | 1 | 1 | 1 | 1 | 1 |
| 74 | 1 | 1 | 1 | 1 | 1 | 1 | 1 | 1 | 1 |
| 75 | 1 | 1 | 1 | 1 | 1 | 1 | 1 | 1 | 1 |
| 76 | 1 | 1 | 1 | 1 | 1 | 1 | 1 | 1 | 1 |
| 77 | 1 | 1 | 1 | 1 | 1 | 1 | 1 | 1 | 1 |
| 78 | 1 | 1 | 1 | 1 | 1 | 1 | 1 | 1 | 1 |
| 79 | 1 | 1 | 1 | 1 | 1 | 1 | 1 | 1 | 1 |
| 80 | 1 | 1 | 1 | 1 | 1 | 1 | 1 | 1 | 1 |
| 81 | 1 | 1 | 1 | 1 | 1 | 1 | 1 | 1 | 1 |
| 82 | 1 | 1 | 1 | 1 | 1 | 1 | 1 | 1 | 1 |
| 83 | 1 | 1 | 1 | 1 | 1 | 1 | 1 | 1 | 1 |
| 84 | 1 | 1 | 1 | 1 | 1 | 1 | 1 | 1 | 1 |
| 85 | 1 | 1 | 1 | 1 | 1 | 1 | 1 | 1 | 1 |
| 86 | 1 | 1 | 1 | 1 | 1 | 1 | 1 | 1 | 1 |
| 87 | 1 | 1 | 1 | 1 | 1 | 1 | 1 | 1 | 1 |
| 88 | 1 | 1 | 1 | 1 | 1 | 1 | 1 | 1 | 1 |
| 89 | 0 | 1 | 1 | 1 | 1 | 1 | 1 | 1 | 1 |
| 90 | 1 | 1 | 1 | 1 | 1 | 1 | 1 | 1 | 1 |
| 91 | 1 | 1 | 1 | 1 | 1 | 1 | 1 | 1 | 1 |
| 92 | 1 | 1 | 1 | 1 | 1 | 1 | 1 | 1 | 1 |
| 93 | 1 | 1 | 1 | 1 | 1 | 1 | 1 | 1 | 1 |
| 94 | 1 | 1 | 1 | 1 | 1 | 1 | 1 | 1 | 0 |
| 95 | 1 | 1 | 1 | 1 | 1 | 1 | 1 | 1 | 0 |
| 96 | 1 | 1 | 1 | 1 | 1 | 1 | 1 | 1 | 1 |
| 97 | 0 | 0 | 0 | 0 | 0 | 1 | 1 | 1 | 1 |
| 98 | 0 | 0 | 0 | 0 | 0 | 1 | 1 | 1 | 0 |
| 99 | 0 | 0 | 0 | 0 | 0 | 1 | 0 | 0 | 0 |
| 100 | 1 | 1 | 1 | 1 | 1 | 1 | 1 | 1 | 1 |
| 101 | 1 | 1 | 1 | 1 | 1 | 1 | 1 | 1 | 1 |
| 102 | 1 | 1 | 1 | 1 | 1 | 1 | 1 | 1 | 1 |
| 103 | 1 | 1 | 1 | 1 | 1 | 1 | 1 | 1 | 1 |
| 104 | 1 | 1 | 1 | 1 | 1 | 1 | 1 | 1 | 1 |
| 105 | 1 | 1 | 1 | 1 | 1 | 1 | 1 | 1 | 1 |
| 106 | 1 | 1 | 1 | 1 | 1 | 1 | 1 | 1 | 1 |
| 107 | 1 | 1 | 1 | 1 | 1 | 1 | 1 | 1 | 1 |
| 108 | 1 | 1 | 1 | 1 | 1 | 1 | 1 | 1 | 1 |
| 109 | 1 | 1 | 1 | 1 | 1 | 1 | 1 | 1 | 1 |
| 110 | 1 | 1 | 1 | 1 | 1 | 1 | 1 | 1 | 1 |
| 111 | 1 | 1 | 1 | 1 | 1 | 1 | 1 | 1 | 1 |
| 112 | 1 | 1 | 1 | 1 | 1 | 1 | 1 | 1 | 1 |
| 113 | 1 | 1 | 1 | 1 | 1 | 1 | 1 | 1 | 1 |
| 114 | 0 | 0 | 0 | 0 | 0 | 0 | 0 | 0 | 1 |
| 115 | 1 | 1 | 1 | 1 | 1 | 1 | 1 | 1 | 1 |
| 116 | 1 | 1 | 1 | 1 | 1 | 1 | 1 | 1 | 1 |
| 117 | 1 | 1 | 1 | 1 | 1 | 1 | 1 | 1 | 1 |
| 118 | 0 | 0 | 0 | 0 | 0 | 1 | 0 | 1 | 0 |
| 119 | 1 | 0 | 0 | 0 | 0 | 1 | 0 | 1 | 0 |
| 120 | 0 | 1 | 1 | 0 | 1 | 1 | 0 | 1 | 0 |
| 121 | 0 | 0 | 0 | 0 | 0 | 1 | 1 | 1 | 1 |
| 122 | 1 | 0 | 0 | 0 | 0 | 1 | 1 | 1 | 1 |
| 123 | 0 | 0 | 0 | 0 | 0 | 1 | 1 | 0 | 1 |
| 124 | 0 | 1 | 0 | 0 | 1 | 1 | 1 | 1 | 1 |
| 125 | 0 | 0 | 0 | 0 | 0 | 1 | 1 | 0 | 1 |

**TABLEAU 10B :**

| Transition numéro | VIR45 | ID6 | ID7 | ID8 | ID9 | ID10 | VIR10 | VIR19 | VIR28 |
|---|---|---|---|---|---|---|---|---|---|
| 1 | 1 | 1 | 1 | 1 | 1 | 1 | 0 | 0 | 0 |
| 2 | 1 | 1 | 1 | 1 | 1 | 1 | 0 | 0 | 0 |
| 3 | 1 | 1 | 1 | 1 | 1 | 1 | 0 | 0 | 0 |
| 4 | 1 | 1 | 1 | 1 | 1 | 1 | 0 | 0 | 0 |
| 5 | 1 | 1 | 1 | 1 | 1 | 1 | 0 | 0 | 0 |
| 6 | 1 | 1 | 1 | 1 | 1 | 1 | 0 | 0 | 0 |
| 7 | 1 | 1 | 1 | 1 | 1 | 1 | 0 | 0 | 0 |
| 8 | 1 | 1 | 1 | 1 | 1 | 1 | 0 | 0 | 0 |
| 9 | 1 | 1 | 1 | 1 | 1 | 1 | 0 | 0 | 0 |
| 10 | 1 | 1 | 1 | 1 | 1 | 1 | 0 | 0 | 0 |
| 11 | 1 | 1 | 1 | 1 | 1 | 1 | 0 | 0 | 1 |
| 12 | 1 | 1 | 1 | 1 | 1 | 1 | 0 | 0 | 0 |
| 13 | 1 | 1 | 1 | 1 | 1 | 1 | 0 | 0 | 0 |
| 14 | 1 | 1 | 1 | 1 | 1 | 1 | 0 | 0 | 1 |
| 15 | 1 | 1 | 1 | 1 | 1 | 1 | 0 | 0 | 0 |
| 16 | 1 | 1 | 1 | 1 | 1 | 1 | 0 | 0 | 0 |
| 17 | 1 | 1 | 1 | 1 | 1 | 1 | 0 | 0 | 0 |
| 18 | 1 | 1 | 1 | 1 | 1 | 1 | 0 | 0 | 0 |
| 19 | 1 | 1 | 1 | 1 | 1 | 1 | 0 | 0 | 0 |
| 20 | 1 | 1 | 1 | 1 | 1 | 1 | 0 | 0 | 0 |
| 21 | 1 | 1 | 1 | 1 | 1 | 1 | 0 | 0 | 0 |
| 22 | 1 | 1 | 1 | 1 | 1 | 1 | 0 | 0 | 0 |
| 23 | 1 | 1 | 1 | 1 | 1 | 1 | 0 | 0 | 0 |
| 24 | 1 | 1 | 1 | 1 | 1 | 1 | 0 | 0 | 0 |
| 25 | 1 | 1 | 1 | 1 | 1 | 1 | 0 | 0 | 0 |
| 26 | 1 | 1 | 1 | 1 | 1 | 1 | 0 | 0 | 0 |
| 27 | 1 | 1 | 1 | 1 | 1 | 1 | 0 | 0 | 0 |
| 28 | 1 | 1 | 1 | 1 | 1 | 1 | 0 | 0 | 0 |
| 29 | 1 | 1 | 1 | 1 | 1 | 1 | 0 | 0 | 0 |
| 30 | 1 | 1 | 1 | 1 | 1 | 1 | 0 | 0 | 0 |
| 31 | 1 | 1 | 1 | 1 | 1 | 1 | 0 | 0 | 0 |
| 32 | 1 | 1 | 1 | 1 | 1 | 1 | 0 | 0 | 0 |
| 33 | 1 | 1 | 0 | 0 | 0 | 0 | 0 | 0 | 0 |
| 34 | 1 | 1 | 1 | 1 | 1 | 1 | 1 | 0 | 0 |
| 35 | 1 | 1 | 1 | 1 | 1 | 1 | 0 | 1 | 0 |
| 36 | 1 | 1 | 1 | 1 | 1 | 1 | 0 | 0 | 0 |
| 37 | 1 | 1 | 1 | 1 | 1 | 1 | 0 | 0 | 0 |
| 38 | 1 | 1 | 1 | 1 | 1 | 1 | 0 | 0 | 0 |
| 39 | 1 | 1 | 1 | 1 | 1 | 1 | 0 | 0 | 0 |
| 40 | 1 | 1 | 1 | 1 | 1 | 1 | 0 | 0 | 0 |
| 41 | 1 | 1 | 1 | 1 | 1 | 1 | 0 | 0 | 0 |
| 42 | 1 | 1 | 0 | 0 | 0 | 0 | 0 | 0 | 0 |
| 43 | 1 | 1 | 1 | 1 | 1 | 1 | 0 | 0 | 0 |
| 44 | 1 | 1 | 1 | 1 | 1 | 1 | 0 | 0 | 0 |
| 45 | 1 | 1 | 0 | 0 | 0 | 0 | 0 | 0 | 0 |
| 46 | 1 | 1 | 1 | 1 | 1 | 1 | 0 | 0 | 0 |
| 47 | 1 | 1 | 1 | 1 | 1 | 1 | 0 | 0 | 0 |
| 48 | 1 | 1 | 1 | 1 | 1 | 1 | 0 | 0 | 0 |
| 49 | 1 | 0 | 0 | 0 | 0 | 0 | 0 | 0 | 0 |
| 50 | 0 | 0 | 0 | 0 | 0 | 0 | 0 | 0 | 0 |
| 51 | 1 | 0 | 0 | 0 | 0 | 0 | 0 | 0 | 0 |
| 52 | 1 | 0 | 0 | 0 | 0 | 0 | 0 | 0 | 0 |
| 53 | 0 | 0 | 0 | 0 | 0 | 0 | 0 | 1 | 0 |
| 54 | 0 | 0 | 0 | 0 | 0 | 0 | 0 | 0 | 0 |
| 55 | 1 | 0 | 0 | 0 | 0 | 0 | 0 | 0 | 0 |
| 56 | 0 | 0 | 0 | 0 | 0 | 0 | 0 | 0 | 0 |
| 57 | 0 | 0 | 0 | 0 | 0 | 0 | 0 | 0 | 0 |
| 58 | 0 | 0 | 0 | 0 | 1 | 1 | 1 | 0 | 0 |
| 59 | 0 | 0 | 0 | 0 | 0 | 0 | 0 | 0 | 0 |
| 60 | 0 | 0 | 0 | 0 | 0 | 0 | 0 | 0 | 0 |
| 61 | 0 | 0 | 1 | 1 | 1 | 0 | 0 | 0 | 0 |
| 62 | 1 | 0 | 1 | 1 | 1 | 0 | 0 | 0 | 0 |
| 63 | 0 | 0 | 0 | 0 | 0 | 0 | 0 | 0 | 0 |
| 64 | 1 | 0 | 0 | 0 | 0 | 0 | 0 | 0 | 0 |
| 65 | 1 | 0 | 0 | 0 | 0 | 0 | 0 | 0 | 0 |
| 66 | 1 | 0 | 0 | 0 | 0 | 0 | 0 | 0 | 0 |
| 67 | 1 | 1 | 1 | 1 | 1 | 1 | 0 | 0 | 0 |
| 68 | 1 | 1 | 1 | 1 | 1 | 1 | 1 | 0 | 0 |
| 69 | 1 | 1 | 1 | 1 | 1 | 1 | 0 | 1 | 0 |
| 70 | 1 | 1 | 1 | 1 | 1 | 1 | 0 | 0 | 0 |
| 71 | 1 | 1 | 1 | 1 | 1 | 1 | 0 | 0 | 0 |
| 72 | 1 | 1 | 0 | 0 | 0 | 0 | 0 | 0 | 0 |
| 73 | 1 | 1 | 1 | 1 | 1 | 1 | 0 | 0 | 0 |
| 74 | 1 | 1 | 1 | 1 | 1 | 1 | 0 | 0 | 0 |
| 75 | 1 | 1 | 1 | 1 | 1 | 1 | 0 | 0 | 0 |
| 76 | 1 | 1 | 1 | 1 | 1 | 1 | 0 | 0 | 0 |
| 77 | 1 | 1 | 1 | 1 | 1 | 1 | 0 | 0 | 0 |
| 78 | 1 | 1 | 1 | 1 | 1 | 1 | 0 | 0 | 0 |
| 79 | 1 | 1 | 1 | 1 | 1 | 1 | 1 | 1 | 0 |
| 80 | 1 | 1 | 1 | 1 | 1 | 1 | 0 | 0 | 0 |
| 81 | 1 | 1 | 1 | 1 | 1 | 1 | 0 | 0 | 0 |
| 82 | 1 | 1 | 1 | 1 | 1 | 1 | 0 | 0 | 0 |
| 83 | 1 | 1 | 1 | 1 | 1 | 1 | 0 | 0 | 0 |
| 84 | 1 | 1 | 1 | 1 | 1 | 1 | 0 | 0 | 0 |
| 85 | 1 | 1 | 1 | 1 | 1 | 1 | 0 | 0 | 0 |
| 86 | 1 | 1 | 1 | 1 | 1 | 1 | 0 | 0 | 0 |
| 87 | 1 | 1 | 0 | 0 | 0 | 0 | 0 | 0 | 0 |
| 88 | 1 | 0 | 1 | 1 | 1 | 1 | 0 | 0 | 0 |
| 89 | 1 | 0 | 1 | 1 | 1 | 1 | 0 | 0 | 0 |
| 90 | 1 | 1 | 1 | 1 | 1 | 1 | 0 | 0 | 0 |
| 91 | 1 | 1 | 1 | 1 | 1 | 1 | 1 | 0 | 0 |
| 92 | 1 | 1 | 1 | 1 | 1 | 1 | 0 | 0 | 0 |
| 93 | 1 | 1 | 1 | 1 | 1 | 1 | 0 | 0 | 0 |
| 94 | 0 | 1 | 1 | 1 | 1 | 1 | 0 | 0 | 0 |
| 95 | 1 | 1 | 1 | 1 | 1 | 1 | 0 | 0 | 0 |
| 96 | 1 | 1 | 1 | 1 | 1 | 1 | 0 | 0 | 0 |
| 97 | 1 | 1 | 1 | 1 | 1 | 1 | 0 | 0 | 0 |
| 98 | 1 | 0 | 1 | 0 | 1 | 0 | 0 | 0 | 0 |
| 99 | 0 | 0 | 0 | 0 | 0 | 0 | 0 | 0 | 0 |
| 100 | 1 | 1 | 1 | 1 | 1 | 1 | 0 | 0 | 0 |
| 101 | 1 | 1 | 1 | 1 | 1 | 1 | 1 | 0 | 0 |
| 102 | 1 | 1 | 1 | 1 | 1 | 1 | 0 | 0 | 0 |
| 103 | 1 | 1 | 1 | 1 | 1 | 1 | 0 | 0 | 0 |
| 104 | 1 | 1 | 1 | 1 | 1 | 1 | 0 | 0 | 0 |
| 105 | 1 | 1 | 1 | 1 | 1 | 1 | 0 | 0 | 0 |
| 106 | 1 | 1 | 1 | 1 | 1 | 1 | 0 | 0 | 0 |
| 107 | 1 | 1 | 1 | 1 | 1 | 1 | 0 | 0 | 0 |
| 108 | 1 | 1 | 1 | 1 | 1 | 1 | 0 | 0 | 0 |
| 109 | 1 | 1 | 1 | 1 | 1 | 1 | 0 | 0 | 0 |
| 110 | 1 | 1 | 1 | 1 | 1 | 1 | 0 | 0 | 0 |
| 111 | 1 | 1 | 1 | 1 | 1 | 1 | 0 | 0 | 0 |
| 112 | 1 | 1 | 1 | 1 | 1 | 1 | 0 | 0 | 0 |
| 113 | 1 | 1 | 1 | 1 | 1 | 1 | 0 | 0 | 0 |
| 114 | 1 | 0 | 0 | 0 | 0 | 0 | 0 | 0 | 0 |
| 115 | 1 | 1 | 1 | 1 | 1 | 1 | 0 | 0 | 0 |
| 116 | 1 | 1 | 1 | 1 | 1 | 1 | 0 | 0 | 0 |
| 117 | 1 | 1 | 1 | 1 | 1 | 1 | 0 | 0 | 0 |
| 118 | 0 | 0 | 0 | 0 | 0 | 0 | 0 | 0 | 0 |
| 119 | 0 | 0 | 0 | 0 | 0 | 0 | 0 | 0 | 0 |
| 120 | 0 | 0 | 0 | 0 | 0 | 0 | 0 | 0 | 0 |
| 121 | 0 | 0 | 0 | 0 | 0 | 0 | 0 | 0 | 0 |
| 122 | 0 | 1 | 0 | 1 | 1 | 1 | 0 | 0 | 0 |
| 123 | 1 | 0 | 0 | 0 | 0 | 0 | 0 | 0 | 0 |
| 124 | 1 | 0 | 0 | 0 | 0 | 0 | 0 | 0 | 0 |
| 125 | 0 | 0 | 0 | 0 | 0 | 0 | 0 | 0 | 0 |

**TABLEAU 11 :**

| Souches | Espèces | Transitions I | Transitions R | Transitions V |
|---|---|---|---|---|
| ASTI | *E. coli* | 48 | 1 | 2 |
| AST2 | *E. coli* | 48 | 15 | 2 |
| AST3 | *E. coli* | 47 | 15 | 1 |
| AST4 | *E. coli* | 47 | 15 | 0 |
| AST5 | *E. coli* | 47 | 15 | 2 |
| VIR41 | *E. coli* | 47 | 3 | 8 |
| VIR42 | *E. coli* | 47 | 4 | 5 |
| VIR43 | *E. coli* | 47 | 5 | 6 |
| VIR44 | *E. coli* | 47 | 6 | 5 |
| VIR45 | *E. coli* | 48 | 5 | 2 |
| ID6 | *E. coli* | 48 | 0 | 1 |
| ID7 | *E. coli* | 45 | 2 | 0 |
| ID8 | *E. coli* | 45 | 2 | 1 |
| ID9 | *E. coli* | 45 | 3 | 1 |
| ID10 | *E. coli* | 45 | 1 | 1 |
| VIR10 | *S. aureus* | 1 | 1 | 0 |
| VIR19 | *S. pneumoniae* | 1 | 1 | 0 |
| VIR28 | *C. difficile* | 2 | 0 | 0 |

Tous les échantillons d'*E. coli* présentent plus de 44 transitions positives dans la catégorie I. Tous ces échantillons sont donc confirmés comme appartenant bien à l'espèce *E. coli.*

En revanche, les échantillons VIR10, VIR19 et VIR28 présentent moins de 3 transitions positives dans la catégorie I, ces échantillons sont donc confirmés comme n'appartenant pas à l'espèce *E. coli.*

Les souches AST2, AST3, AST4 et AST5 de *E. coli* présentent plus de 14 transitions positives pour la catégorie R, elles expriment donc la pénicillinase plasmidique TEM-2, ce qui est synonyme d'un mécanisme de résistance aux Pénicillines.

En revanche, les souches AST1, VIR41, VIR42, VIR43, VIR44, VIR45, ID6, ID7, ID8, ID9 et ID10 de *E. coli* présentent moins de 7 transitions positives pour la catégorie R, elles n'expriment donc pas la pénicillinase plasmidique TEM-2. Ces souches sont donc sensibles aux Pénicillines, notamment aminopénicillines ou pénicillines A (ampicilline), carboxypénicillines ou pénicillines C (ticarcilline) et uréidopénicilline ou pénicilline U (pipéracilline).

Les souches VIR10, VIR19 et VIR28 qui n'appartiennent pas à l'espèce *E. coli* présentent moins de 2 transitions pour la catégorie R, elles n'expriment donc pas TEM-2, ce qui confirme la spécificité de la méthode.

Les échantillons VIR41, VIR42, VIR43 et VIR44 de *E. coli* présentent plus de 4 transitions positives dans la catégorie V, elles expriment donc les toxines Shigatoxine 1 ou 2 (STX1 ou STX2). Plus précisément, pour VIR41 les transitions 118 à 125 sont positives , VIR41 exprime donc simultanément Shigatoxine 1 et Shigatoxine 2. VIR42 et VIR44 sont positives pour les transitions 121 à 125, elles expriment donc Shigatoxine 2. Il en est de même pour VIR45 qui présente les transitions 123 et 124. VIR43 qui présente les transitions 118 à 122 exprime Shigatoxine 2.En revanche, les souches AST1, AST2, AST3, AST4, AST5, VIR45, ID6, ID7, ID8, ID9 et ID10 de *E. coli* présentent moins de 3 transitions positives, elles n'expriment donc pas de Shigatoxine. Ces souches n'ont donc pas les propriétés de virulence en lien avec les Shigatoxines.

Les souches VIR10, VIR19 et VIR28 qui n'appartiennent pas à l'espèce *E. coli* présentent également moins de 3 transitions pour la catégorie V, elles n'expriment donc pas stx1 ou stx2, ce qui confirme la spécificité de la méthode.

Pour le typage, les transitions de la catégorie T de chaque souche sont comparées aux transitions des autres souches considérées comme des souches de référence. En pratique une valeur 0 est attribuée lorsque les transitions entre les 2 souches se classent dans la même catégorie (positive ou négative) et une valeur de 1 est attribuée lorsque les transitions entre les 2 souches se classent dans des catégories différentes (une transition positive et une transition négative). Les valeurs sont sommées pour toutes les transitions de la catégorie T de chaque couple de souche afin d'établir un score. Les scores figurent dans le **TABLEAU 12 :**

**TABLEAU 12 :**

| | AST1 | AST2 | AST3 | AST4 | AST5 | VIR41 | VIR42 | VIR43 | VIR44 | VIR45 | ID6 | ID7 | ID8 | ID9 | ID10 | VIR10 | VIR19 | VIR28 |
|---|---|---|---|---|---|---|---|---|---|---|---|---|---|---|---|---|---|---|
| AST1 | 0 | 19 | 18 | 18 | 19 | 17 | 12 | 15 | 17 | 16 | 3 | 11 | 12 | 11 | 9 | 92 | 92 | 95 |
| AST2 | 19 | 0 | 3 | 3 | 2 | 24 | 21 | 18 | 22 | 19 | 22 | 22 | 23 | 22 | 22 | 109 | 109 | 112 |
| AST3 | 18 | 3 | 0 | 0 | 1 | 25 | 22 | 21 | 23 | 20 | 19 | 19 | 20 | 19 | 19 | 106 | 106 | 109 |
| AST4 | 18 | 3 | 0 | 0 | 1 | 25 | 22 | 21 | 23 | 20 | 19 | 19 | 20 | 19 | 19 | 106 | 106 | 109 |
| AST5 | 19 | 2 | 1 | 1 | 0 | 24 | 23 | 20 | 24 | 21 | 20 | 20 | 21 | 20 | 20 | 107 | 107 | 110 |
| VIR41 | 17 | 24 | 25 | 25 | 24 | 0 | 5 | 6 | 12 | 13 | 16 | 22 | 23 | 20 | 20 | 105 | 105 | 108 |
| VIR42 | 12 | 21 | 22 | 22 | 23 | 5 | 0 | 7 | 7 | 8 | 13 | 19 | 20 | 17 | 17 | 102 | 102 | 105 |
| VIR43 | 15 | 18 | 21 | 21 | 20 | 6 | 7 | 0 | 10 | 9 | 16 | 20 | 21 | 18 | 20 | 105 | 105 | 108 |
| VIR44 | 17 | 22 | 23 | 23 | 24 | 12 | 7 | 10 | 0 | 5 | 18 | 22 | 23 | 22 | 22 | 103 | 103 | 106 |
| VIR45 | 16 | 19 | 20 | 20 | 21 | 13 | 8 | 9 | 5 | 0 | 17 | 19 | 20 | 19 | 19 | 102 | 102 | 105 |
| ID6 | 3 | 22 | 19 | 19 | 20 | 16 | 13 | 16 | 18 | 17 | 0 | 10 | 11 | 10 | 8 | 91 | 91 | 94 |
| ID7 | 11 | 22 | 19 | 19 | 20 | 22 | 19 | 20 | 22 | 19 | 10 | 0 | 1 | 4 | 4 | 89 | 89 | 92 |
| ID8 | 12 | 23 | 20 | 20 | 21 | 23 | 20 | 21 | 23 | 20 | 11 | 1 | 0 | 2 | 3 | 90 | 92 | 92 |
| ID9 | 11 | 22 | 19 | 19 | 20 | 20 | 17 | 18 | 22 | 19 | 10 | 4 | 2 | 0 | 3 | 90 | 94 | 94 |
| ID10 | 9 | 22 | 19 | 19 | 20 | 20 | 17 | 20 | 22 | 19 | 8 | 4 | 3 | 3 | 0 | 87 | 91 | 91 |
| VIR10 | 92 | 109 | 106 | 106 | 107 | 105 | 102 | 105 | 103 | 102 | 91 | 89 | 90 | 90 | 87 | ∼ | ∼ | ∼ |
| VIR19 | 92 | 109 | 106 | 106 | 107 | 105 | 102 | 105 | 103 | 102 | 91 | 89 | 92 | 94 | 91 | ∼ | ∼ | ∼ |
| VIR28 | 95 | 112 | 109 | 109 | 110 | 108 | 105 | 108 | 106 | 105 | 94 | 92 | 92 | 94 | 91 | ∼ | ∼ | ∼ |

Les souches qui ont un score inférieur ou égal à 4 sont de même type, les souches qui ont un score strictement supérieur à 4 sont de type différent.
Ainsi, les souches AST2, AST3, AST4, AST5 sont de même type. Il en est de même respectivement pour les souches AST1 et ID6 et pour les souches ID7, ID8, ID9 et ID10. Toutes les autres souches prises deux à deux sont de types différents. Il faut noter les sommes élevées obtenues entre les souches VIR10, VIR19 et VIR28, qui ne sont pas des *S. Aureus,* et toutes les autres souches qui sont des *S. Aureus.* Ces résultats confirment la spécificité de la méthode.

Les souches VIR10, VIR19 et VIR28 sont d'espèces différentes. Ces souches ne peuvent donc pas être comparées entre elles et aucune valeur n'est reportée dans le TABLEAU 12.
De façon extrêmement intéressante, des scores supérieurs à 20, comme par exemple entre AST4 etVIR41, traduisent une grande divergence entre souches. Des scores entre 12 et 20, comme entre ID7 et VIR42, traduisent une divergence modérée et des scores entre 4 et 12, comme entre ID10 et AST1, une divergence faible.

La méthode ainsi mise en oeuvre permet donc non seulement d'établir si deux souches sont de même type, ce qui est important pour identifier un foyer infectieux commun, mais également d'estimer la proximité de deux souches, ce qui est extrêmement important pour les études épidémiologiques.

Cet exemple montre que, de façon très avantageuse, la présente invention permet en un temps inférieur à une heure, ce qui est très court, de confirmer l'identité d'une espèce telle que *E. coli* et de déterminer, simultanément au sein de la même analyse, les propriétés de typage, de résistance potentielle à au moins un antibiotique et d'établir l'existence de facteur de virulence. Ces propriétés ont été établies avec le même instrument, ce qui facilite grandement l'analyse et le rendu des résultats. Enfin, les caractéristiques des bactéries sont établies à l'aide de protéines bactériennes, ce qui traduit l'existence de microorganismes vivants et viables, contrairement aux caractérisations à partir d'ADN bactérien qui peuvent être faussées par l'existence de bactéries mortes.

### Exemple 8 : Protocole de digestion de microorganismes en présence de méthanol

Classiquement le protocole suivant est mis en oeuvre en 17 étapes :
1. Prélèvement d'une colonie de microorganisme, obtenue selon l'exemple 1, et mise en suspension dans 400µl d'une solution de bicarbonate d'ammonium 50 mM, pH=8,0.
2. Ajout de 600µl de méthanol
Etapes 3 à 7 : idem étapes 2 à 6 de l'exemple 4.
Etapes 8 à 17 : idem étapes 8 à 17 de l'exemple 6.

### Exemple 9 : Caractérisation d'échantillons de C. albicans:

Après avoir établi la ou les espèces des échantillons selon l'une quelconque des méthodes décrites dans les exemples 1 à 3, les espèces listées ci-dessous sont analysées. Dix-sept souches de C. *albicans* sont analysées pour confirmer leur identification et établir leurs caractéristiques :

| | |
|---|---|
| ATF1 | ATF2 |
| ATF3 | ATF4 |
| ATF5 | ATF6 |
| ATF7 | VIR31 |
| VIR32 | VIR33 |
| VIR34 | VIR35 |
| VIR36 | VIR37 |
| VIR38 | VIR39 |
| CA16 | |

La même méthode d'analyse est appliquée à des espèces n'appartenant pas à l'espèce C. *albicans* pour servir de témoin négatif :
- *E. coli* VIR43
- *S. aureus* VIR5
- *E*. *faecium* AST-VAN8

Chaque échantillon est traité selon l'exemple 8, puis un volume de 5µl de protéines digérées est injecté et analysé selon les mêmes conditions que dans l'exemple 7.
- Les peptides, issues de la digestion des protéines du microorganisme, sont analysés par le spectromètre de masse en mode MRM. Seuls les peptides indiqués dans le **TABLEAU 13** sont détectés. Pour cela, le ou les fragments de première génération indiqué dans le **TABLEAU 13** sont détectés. L'application, à laquelle permet de répondre chaque transition, c'est à dire chaque peptide associé à son fragment de première génération, est précisée dans la colonne intérêt clinique du **TABLEAU 14** avec les lettres I, T, R et V. I désigne la confirmation de l'identification du microorganisme, T le typage, R la résistance à au moins un antibiotique et V la détection de facteurs de virulence.

**TABLEAU 13 :**

| Transition numéro | Proteine | Peptide | Ion fragment de première génération | Intérêt clinique |
|---|---|---|---|---|
| 1 | ALF | ADEEFFAK | y5 monochargé | I+T |
| 2 | ALF | ADEEFFAK | y6 monochargé | I+T |
| 3 | ALF | ADEEFFAK | y7 monochargé | I+T |
| 4 | HSP75 | AFDDESVQK | y6 monochargé | I+T |
| 5 | HSP75 | AFDDESVQK | y7 monochargé | I+T |
| 6 | HSP75 | AFDDESVQK | y8 monochargé | I+T |
| 7 | ENO1 | AKIDWDQAK | y6 monochargé | I+T |
| 8 | ENO1 | AKIDWDQAK | y7 monochargé | I+T |
| 9 | ENO1 | AKIDWDQAK | y8 monochargé | I+T |
| 10 | RL13 | AVEVPEQTAYR | y6 monochargé | I+T |
| 11 | RL13 | AVEVPEQTAYR | y7 monochargé | I+T |
| 12 | RL13 | AVEVPEQTAYR | y8 monochargé | I+T |
| 13 | RS22 | CGVIQPR | y5 monochargé | I+T |
| 14 | RS22 | CGVIQPR | y6 monochargé | I+T |
| 15 | RS22 | CGVIQPR | y4 monochargé | I+T |
| 16 | RLA4 | DLQELIAEGNTK | y8 monochargé | I+T |
| 17 | RLA4 | DLQELIAEGNTK | y9 monochargé | I+T |
| 18 | RLA4 | DLQELIAEGNTK | y7 monochargé | I+T |
| 19 | PPIA | FADENFVKR | y6 monochargé | I+T |
| 20 | PPIA | FADENFVKR | y7 monochargé | I+T |
| 21 | PPIA | FADENFVKR | y8 monochargé | I+T |
| 22 | TPIS | FALDTGVK | y6 monochargé | I+T |
| 23 | TPIS | FALDTGVK | y7 monochargé | I+T |
| 24 | TPIS | FALDTGVK | y5 monochargé | I+T |
| 25 | RL28 | GRLPEVPVIVK | y8 monochargé | I+T |
| 26 | RL28 | GRLPEVPVIVK | y5 monochargé | I+T |
| 27 | ALF | IAEALDIFHTK | y6 monochargé | I+T |
| 28 | ALF | IAEALDIFHTK | y7 monochargé | I+T |
| 29 | ALF | IAEALDIFHTK | y8 monochargé | I+T |
| 30 | ENO1 | IDWDQAK | y5 monochargé | I+T |
| 31 | ENO1 | IDVVDQAK | y6 monochargé | I+T |
| 32 | ENO1 | IDWDQAK | y7 monochargé | I+T |
| 33 | ENO1 | IEEELGSEAIYAGKDFQK | y8 monochargé | I+T |
| 34 | ENO1 | IEEELGSEAIYAGKDFQK | y9 monochargé | I+T |
| 35 | PPIA | IESFGSGSGATSK | y9 monochargé | I+T |
| 36 | PPIA | IESFGSGSGATSK | y10 monochargé | I+T |
| 37 | PPIA | IESFGSGSGATSK | y11 monochargé | I+T |
| 38 | NTF2 | LASLPFQK | y5 monochargé | I+T |
| 39 | NTF2 | LASLPFQK | y6 monochargé | I+T |
| 40 | NTF2 | LASLPFQK | y7 monochargé | I+T |
| 41 | MBF1 | LDATDDWAVK | y7 monochargé | I+T |
| 42 | MBF1 | LDATDDVVAVK | y8 monochargé | I+T |
| 43 | MBF1 | LDATDDWAVK | y9 monochargé | I+T |
| 44 | PGK | NVEHLVEK | y6 monochargé | I+T |
| 45 | PGK | NVEHLVEK | y7 monochargé | I+T |
| 46 | PGK | NVEHLVEK | y5 monochargé | I+T |
| 47 | RL36 | SGIAAGVNK | y5 monochargé | I+T |
| 48 | RL36 | SGIAAGVNK | y6 monochargé | I+T |
| 49 | RL36 | SGIAAGVNK | y7 monochargé | I+T |
| 50 | G3P | SGVDYVIESTGVFTK | y9 monochargé | I+T |
| 51 | G3P | SGVDYVIESTGVFTK | y10 monochargé | I+T |
| 52 | G3P | SGVDYVIESTGVFTK | y8 monochargé | I+T |
| 53 | EF1B | SLNEFLADK | y6 monochargé | I+T |
| 54 | EF1B | SLNEFLADK | y7 monochargé | I+T |
| 55 | RL 13 | SQETFDANVAR | y7 monochargé | I+T |
| 56 | RL13 | SQETFDANVAR | b8 monochargé | I+T |
| 57 | RL13 | SQETFDANVAR | y9 monochargé | I+T |
| 58 | CS111 | SSSSTTKK | y6 monochargé | I+T |
| 59 | CS111 | SSSSTTKK | y7 monochargé | I+T |
| 60 | HSP71 | STLDPVGK | y6 monochargé | I+T |
| 61 | HSP71 | STLDPVGK | y7 monochargé | I+T |
| 62 | HSP71 | STLDPVGK | y5 monochargé | I+T |
| 63 | PGK | SVELFQQAVAK | y7 monochargé | I+T |
| 64 | PGK | SVELFQQAVAK | y8 monochargé | I+T |
| 65 | PGK | SVELFQQAVAK | y6 monochargé | I+T |
| 66 | KPYK | TANDVLELR | y5 monochargé | I+T |
| 67 | KPYK | TANDVLELR | y6 monochargé | I+T |
| 68 | KPYK | TANDVLELR | y7 monochargé | I+T |
| 69 | ENO1 | VGDKIQIVGDDLTVTNPTR | y7 monochargé | I+T |
| 70 | ENO1 | VGDKIQIVGDDLTVTNPTR | y8 monochargé | I+T |
| 71 | ENO1 | VGDKIQIVGDDLTVTNPTR | y9 monochargé | I+T |
| 72 | ADH1 | WAIDGGDEK | y7 monochargé | I+T |
| 73 | ADH1 | WAIDGGDEK | y8 monochargé | I+T |
| 74 | ADH1 | WAIDGGDEK | y9 monochargé | I+T |
| 75 | RS22 | WTDNLLPAR | y6 monochargé | I+T |
| 76 | RS22 | WTDNLLPAR | y7 monochargé | I+T |
| 77 | RS22 | WTDNLLPAR | y8 monochargé | I+T |
| 78 | G3P | YKGEVTASGDDLVIDGHK | y10 monochargé | I+T |
| 79 | G3P | YKGEVTASGDDLVIDGHK | y11 monochargé | I+T |
| 80 | G3P | YKGEVTASGDDLVIDGHK | y9 monochargé | I+T |
| 81 | ADH1 | YVLDTSK | y5 monochargé | I+T |
| 82 | ADH1 | YVLDTSK | y6 monochargé | I+T |
| 83 | ADH1 | YVLDTSK | y4 monochargé | I+T |
| 84 | CP51 | AVIYDCPNSR | y6 monochargé | R+T |
| 85 | CP51 | AVIYDCPNSR | y7 monochargé | R+T |
| 86 | CP51 | GHYVLVFPGYAHTSER | 10 monochargé | R+T |
| 87 | CP51 | GHYVLVFPGYAHTSER | y9 monochargé | R+T |
| 88 | CP51 | GHYVLVSPGYAHTSER | y9 monochargé | R+T |
| 89 | CP51 | GHYVLVSPGYAHTSER | 10 monochargé | R+T |
| 90 | CP51 | GVIYDCPNSR | y6 monochargé | R+T |
| 91 | CP51 | GVIYDCPNSR | y7 monochargé | R+T |
| 92 | CP51 | GVIYDCPNSR | y8 monochargé | R+T |
| 93 | CP51 | GVSSPYLPFGGGR | y9 monochargé | R+T |
| 94 | CP51 | GVSSPYLPFGGGR | y6 monochargé | R+T |
| 95 | CP51 | GVSSPYLPFSGGK | y9 dichargé | R+T |
| 96 | CP51 | GVSSPYLPFSGGK | y9 monochargé | R+T |
| 97 | CP51 | GVSSPYLPFSGGK | y6 monochargé | R+T |
| 98 | CP51 | GVSSPYLPFSGGR | y9 dichargé. | R+T |
| 99 | CP51 | GVSSPYLPFSGGR | y9 monochargé | R+T |
| 100 | CP51 | GVSSPYLPFSGGR | y6 monochargé | R+T |
| 101 | LIPASE8 | AAVGDILQSR | y7 monochargé | V+T |
| 102 | LIPASE8 | AAVGDILQSR | y8 monochargé | V+T |
| 103 | LIPASE8 | ITPDDLR | y5 monochargé | V+T |
| 104 | LIPASE8 | ITPDDLR | y6 monochargé | V+T |
| 105 | LIPASE8 | ITPDDLR | y4 monochargé | V+T |
| 106 | LIPASE8 | TGWDILK | y5 monochargé | V+T |
| 107 | LIPASE8 | TGWDILK | y6 monochargé | V+T |
| 108 | LIPASE8 | TGWDILK | y4 monochargé | V+T |

L'état de charge du peptide précurseur, son temps de rétention et les transitions, c'est-à-dire les rapports (m/z)₁ en Q1 et (m/z)₂ en Q3, sont indiqués dans le **TABLEAU 14**. L'énergie de collision utilisée pour fragmenter l'ion précurseur est également indiquée dans le **TABLEAU 14**.

**TABLEAU 14 :**

| Transition numéro | Etat de charge du précurseur | Temps de rétention | (m/z) filtré en Q1 | (m/z) filtré en Q3 | Energie de collision |
|---|---|---|---|---|---|
| 1 | 2 | 11.9 | 478.72 | 641.33 | 26 |
| 2 | 2 | 11.9 | 478.72 | 770.37 | 26 |
| 3 | 2 | 11.9 | 478.72 | 885.4 | 26 |
| 4 | 2 | 7.8 | 519.74 | 705.34 | 28 |
| 5 | 2 | 7.8 | 519.74 | 820.37 | 28 |
| 6 | 2 | 7.8 | 519.74 | 967.44 | 28 |
| 7 | 2 | 8.5 | 543.81 | 659.37 | 29 |
| 8 | 2 | 8.5 | 543.81 | 774.4 | 29 |
| 9 | 2 | 8.5 | 543.81 | 887.48 | 29 |
| 10 | 2 | 11 | 631.82 | 767.37 | 33 |
| 11 | 2 | 11 | 631.82 | 864.42 | 33 |
| 12 | 2 | 11 | 631.82 | 963.49 | 33 |
| 13 | 2 | 6.4 | 415.22 | 612.38 | 23 |
| 14 | 2 | 6.4 | 415.22 | 669.4 | 23 |
| 15 | 2 | 6.4 | 415.22 | 513.31 | 23.3 |
| 16 | 2 | 15.5 | 665.85 | 845.47 | 34 |
| 17 | 2 | 15.5 | 665.85 | 974.52 | 34 |
| 18 | 2 | 15.5 | 665.85 | 732.39 | 34.3 |
| 19 | 2 | 10.5 | 563.29 | 792.44 | 30 |
| 20 | 2 | 10.5 | 563.29 | 907.46 | 30 |
| 21 | 2 | 10.5 | 563.29 | 978.5 | 30 |
| 22 | 2 | 12.5 | 425.74 | 632.36 | 24 |
| 23 | 2 | 12.5 | 425.74 | 703.4 | 24 |
| 24 | 2 | 12.5 | 425.74 | 519.28 | 23.7 |
| 25 | 2 | 14.8 | 603.88 | 880.55 | 32 |
| 26 | 2 | 14.8 | 603.88 | 555.39 | 31.6 |
| 27 | 2 | 17.8 | 629.35 | 760.4 | 33 |
| 28 | 2 | 17.8 | 629.35 | 873.48 | 33 |
| 29 | 2 | 17.8 | 629.35 | 944.52 | 33 |
| 30 | 2 | 9 | 444.25 | 560.3 | 25 |
| 31 | 2 | 9 | 444.25 | 659.37 | 25 |
| 32 | 2 | 9 | 444.25 | 774.4 | 25 |
| 33 | 3 | 18.6 | 676.34 | 956.48 | 38 |
| 34 | 3 | 18.6 | 676.34 | 1069.57 | 38 |
| 35 | 2 | 8.4 | 614.3 | 751.36 | 32 |
| 36 | 2 | 8.4 | 614.3 | 898.43 | 32 |
| 37 | 2 | 8.4 | 614.3 | 985.46 | 32 |
| 38 | 2 | 14.1 | 452.27 | 632.38 | 25 |
| 39 | 2 | 14.1 | 452.27 | 719.41 | 25 |
| 40 | 2 | 14.1 | 452.27 | 790.45 | 25 |
| 41 | 2 | 12.1 | 573.31 | 745.41 | 30 |
| 42 | 2 | 12.1 | 573.31 | 846.46 | 30 |
| 43 | 2 | 12.1 | 573.31 | 917.49 | 30 |
| 44 | 2 | 7 | 484.26 | 754.41 | 26 |
| 45 | 2 | 7 | 484.26 | 853.48 | 26 |
| 46 | 2 | 7 | 484.26 | 625.37 | 26.3 |
| 47 | 2 | 4.5 | 408.73 | 488.28 | 23 |
| 48 | 2 | 4.5 | 408.73 | 559.32 | 23 |
| 49 | 2 | 4.5 | 408.73 | 672.4 | 23 |
| 50 | 2 | 18 | 801.41 | 981.53 | 40 |
| 51 | 2 | 18 | 801.41 | 1080.59 | 40 |
| 52 | 2 | 18 | 801.41 | 868.44 | 40.3 |
| 53 | 2 | 14.3 | 518.77 | 722.37 | 28 |
| 54 | 2 | 14.3 | 518.77 | 836.41 | 28 |
| 55 | 2 | 10.1 | 619.29 | 792.4 | 32 |
| 56 | 2 | 10.1 | 619.29 | 893.36 | 32 |
| 57 | 2 | 10.1 | 619.29 | 1022.49 | 32 |
| 58 | 2 | 10.6 | 413.22 | 651.37 | 23 |
| 59 | 2 | 10.6 | 413.22 | 738.4 | 23 |
| 60 | 2 | 8.7 | 408.73 | 628.37 | 23 |
| 61 | 2 | 8.7 | 408.73 | 729.41 | 23 |
| 62 | 2 | 8.7 | 408.73 | 515.28 | 23 |
| 63 | 2 | 14.6 | 610.34 | 791.44 | 32 |
| 64 | 2 | 14.6 | 610.34 | 904.53 | 32 |
| 65 | 2 | 14.6 | 610.34 | 644.37 | 31.9 |
| 66 | 2 | 12.9 | 515.78 | 629.4 | 28 |
| 67 | 2 | 12.9 | 515.78 | 744.43 | 28 |
| 68 | 2 | 12.9 | 515.78 | 858.47 | 28 |
| 69 | 3 | 15.3 | 681.04 | 788.43 | 38 |
| 70 | 3 | 15.3 | 681.04 | 901.51 | 38 |
| 71 | 3 | 15.3 | 681.04 | 1016.54 | 38 |
| 72 | 2 | 7.7 | 501.76 | 733.34 | 27 |
| 73 | 2 | 7.7 | 501.76 | 804.37 | 27 |
| 74 | 2 | 7.7 | 501.76 | 903.44 | 27 |
| 75 | 2 | 14.9 | 543.29 | 683.42 | 29 |
| 76 | 2 | 14.9 | 543.29 | 798.45 | 29 |
| 77 | 2 | 14.9 | 543.29 | 899.49 | 29 |
| 78 | 2 | 11.4 | 952.47 | 1068.53 | 47 |
| 79 | 2 | 11.4 | 952.47 | 1155.56 | 47 |
| 80 | 2 | 11.4 | 952.47 | 1011.51 | 46.9 |
| 81 | 2 | 8.5 | 413.22 | 563.3 | 23 |
| 82 | 2 | 8.5 | 413.22 | 662.37 | 23 |
| 83 | 2 | 8.5 | 413.22 | 450.22 | 23.2 |
| 84 | 2 | 9.3 | 597.78 | 748.3 | 31 |
| 85 | 2 | 9.3 | 597.78 | 911.37 | 31 |
| 86 | 2 | 14.3 | 916.96 | 1164.54 | 45 |
| 87 | 2 | 14.3 | 916.96 | 1017.47 | 45.3 |
| 88 | 2 | 11 | 886.94 | 1017.47 | 44 |
| 89 | 2 | 11 | 886.94 | 1104.51 | 44 |
| 90 | 2 | 9.4 | 590.77 | 748.3 | 31 |
| 91 | 2 | 9.4 | 590.77 | 911.37 | 31 |
| 92 | 2 | 9.4 | 590.77 | 1024.45 | 31 |
| 93 | 2 | 16.1 | 647.33 | 963.5 | 33 |
| 94 | 2 | 16.1 | 647.33 | 590.3 | 33.5 |
| 95 | 2 | 15.8 | 648.34 | 483.26 | 34 |
| 96 | 2 | 15.8 | 648.34 | 965.51 | 34 |
| 97 | 2 | 15.8 | 648.34 | 592.31 | 33.5 |
| 98 | 2 | 16 | 662.34 | 497.26 | 34 |
| 99 | 2 | 16 | 662.34 | 993.52 | 34 |
| 100 | 2 | 16 | 662.34 | 620.32 | 34.1 |
| 101 | 2 | 12.75 | 515.29 | 788.43 | 28 |
| 102 | 2 | 12.75 | 515.29 | 887.49 | 28 |
| 103 | 2 | 9.83 | 415.22 | 615.31 | 23 |
| 104 | 2 | 9.83 | 415.22 | 716.36 | 23 |
| 105 | 2 | 9.83 | 415.22 | 518.26 | 23.3 |
| 106 | 2 | 15.81 | 416.73 | 674.39 | 23 |
| 107 | 2 | 15.81 | 416.73 | 731.41 | 23 |
| 108 | 2 | 15.81 | 416.73 | 488.31 | 23.3 |

Les autres paramètres machine utilisés sont les mêmes que dans l'exemple 7. Les aires obtenues pour chacune des transitions et pour chacun des microorganismes étudiés ont été mesurées. Toutes les transitions dont l'aire est supérieure ou égale à 2000 (unité arbitraire) sont considérées comme positives et ont été notées « 1 » dans les **tableaux 15A et 15B.** Toutes les transitions dont l'aire est inférieure à 2000, sont considérées comme négatives et ont été notées 0 dans les tableaux 15A et 15B. Lorsqu'on n'a observé aucun pic de signal, on a noté la transition comme négative.

**TABLEAU 15A :**

| Transition Numéro | ATF1 | ATF2 | ATF3 | ATF4 | VIR31 | ATF5 | ATF6 | ATF7 | VIR32 | VIR33 |
|---|---|---|---|---|---|---|---|---|---|---|
| 1 | 1 | 1 | 1 | 1 | 1 | 1 | 1 | 1 | 1 | 1 |
| 2 | 1 | 1 | 1 | 1 | 1 | 1 | 1 | 1 | 1 | 1 |
| 3 | 1 | 1 | 1 | 1 | 1 | 1 | 1 | 1 | 1 | 1 |
| 4 | 1 | 1 | 1 | 1 | 1 | 1 | 1 | 1 | 1 | 1 |
| 5 | 1 | 1 | 1 | 1 | 1 | 1 | 1 | 1 | 1 | 1 |
| 6 | 1 | 1 | 1 | 1 | 1 | 1 | 1 | 1 | 1 | 1 |
| 7 | 1 | 1 | 1 | 1 | 1 | 1 | 1 | 1 | 1 | 1 |
| 8 | 1 | 1 | 1 | 1 | 1 | 1 | 1 | 1 | 1 | 1 |
| 9 | 1 | 1 | 1 | 1 | 1 | 1 | 1 | 1 | 1 | 1 |
| 10 | 1 | 1 | 1 | 1 | 0 | 1 | 1 | 1 | 0 | 0 |
| 11 | 1 | 1 | 1 | 1 | 0 | 1 | 1 | 1 | 0 | 1 |
| 12 | 1 | 1 | 1 | 1 | 0 | 1 | 1 | 1 | 0 | 0 |
| 13 | 1 | 1 | 1 | 1 | 0 | 1 | 1 | 1 | 1 | 1 |
| 14 | 1 | 1 | 1 | 1 | 0 | 1 | 1 | 1 | 1 | 1 |
| 15 | 1 | 1 | 1 | 1 | 1 | 1 | 1 | 1 | 1 | 1 |
| 16 | 1 | 1 | 1 | 1 | 1 | 1 | 1 | 1 | 1 | 1 |
| 17 | 1 | 1 | 1 | 1 | 1 | 1 | 1 | 1 | 1 | 1 |
| 18 | 1 | 1 | 1 | 1 | 1 | 1 | 1 | 1 | 1 | 1 |
| 19 | 1 | 1 | 1 | 1 | 0 | 1 | 1 | 1 | 0 | 1 |
| 20 | 1 | 1 | 1 | 1 | 0 | 1 | 1 | 1 | 1 | 1 |
| 21 | 1 | 1 | 1 | 1 | 0 | 1 | 1 | 1 | 0 | 1 |
| 22 | 1 | 1 | 1 | 1 | 1 | 1 | 1 | 1 | 1 | 1 |
| 23 | 1 | 1 | 1 | 1 | 1 | 1 | 1 | 1 | 1 | 1 |
| 24 | 1 | 1 | 1 | 1 | 1 | 1 | 1 | 1 | 1 | 1 |
| 25 | 1 | 1 | 1 | 1 | 1 | 1 | 1 | 1 | 1 | 1 |
| 26 | 1 | 1 | 1 | 1 | 1 | 1 | 1 | 1 | 1 | 1 |
| 27 | 0 | 0 | 0 | 0 | 1 | 0 | 0 | 0 | 1 | 1 |
| 28 | 0 | 0 | 0 | 0 | 1 | 0 | 0 | 0 | 1 | 1 |
| 29 | 0 | 0 | 0 | 0 | 0 | 0 | 0 | 0 | 0 | 1 |
| 30 | 1 | 1 | 1 | 1 | 1 | 1 | 1 | 1 | 1 | 1 |
| 31 | 1 | 1 | 1 | 1 | 1 | 1 | 1 | 1 | 1 | 1 |
| 32 | 1 | 1 | 1 | 1 | 1 | 1 | 1 | 1 | 1 | 1 |
| 33 | 0 | 0 | 0 | 0 | 1 | 0 | 0 | 0 | 1 | 1 |
| 34 | 0 | 0 | 0 | 0 | 0 | 0 | 0 | 0 | 0 | 1 |
| 35 | 1 | 1 | 1 | 1 | 1 | 1 | 1 | 1 | 1 | 1 |
| 36 | 1 | 1 | 1 | 1 | 1 | 1 | 1 | 1 | 1 | 1 |
| 37 | 1 | 1 | 1 | 1 | 1 | 1 | 1 | 1 | 1 | 1 |
| 38 | 1 | 1 | 1 | 1 | 0 | 1 | 1 | 1 | 0 | 0 |
| 39 | 1 | 1 | 1 | 1 | 0 | 1 | 1 | 1 | 0 | 1 |
| 40 | 1 | 1 | 1 | 1 | 0 | 1 | 1 | 1 | 0 | 0 |
| 41 | 1 | 1 | 1 | 1 | 1 | 1 | 1 | 1 | 1 | 1 |
| 42 | 1 | 1 | 1 | 1 | 1 | 1 | 1 | 1 | 1 | 1 |
| 43 | 1 | 1 | 1 | 1 | 1 | 1 | 1 | 1 | 1 | 1 |
| 44 | 1 | 1 | 1 | 1 | 1 | 1 | 1 | 1 | 1 | 1 |
| 45 | 1 | 1 | 1 | 1 | 1 | 1 | 1 | 1 | 1 | 1 |
| 46 | 1 | 1 | 1 | 1 | 1 | 1 | 1 | 1 | 1 | 1 |
| 47 | 1 | 1 | 1 | 1 | 1 | 1 | 1 | 1 | 1 | 1 |
| 48 | 1 | 1 | 1 | 1 | 1 | 1 | 1 | 1 | 1 | 1 |
| 49 | 0 | 0 | 0 | 0 | 1 | 0 | 0 | 0 | 1 | 1 |
| 50 | 1 | 1 | 1 | 1 | 1 | 1 | 1 | 1 | 1 | 1 |
| 51 | 1 | 1 | 1 | 1 | 1 | 1 | 1 | 1 | 1 | 1 |
| 52 | 1 | 1 | 1 | 1 | 1 | 1 | 1 | 1 | 1 | 1 |
| 53 | 1 | 1 | 1 | 1 | 0 | 1 | 1 | 1 | 1 | 1 |
| 54 | 1 | 1 | 1 | 1 | 1 | 1 | 1 | 1 | 1 | 1 |
| 55 | 1 | 1 | 1 | 1 | 0 | 1 | 1 | 1 | 0 | 0 |
| 56 | 1 | 1 | 1 | 1 | 0 | 1 | 1 | 1 | 0 | 0 |
| 57 | 1 | 1 | 1 | 1 | 0 | 1 | 1 | 1 | 0 | 0 |
| 58 | 0 | 0 | 0 | 0 | 1 | 0 | 0 | 0 | 1 | 1 |
| 59 | 0 | 0 | 0 | 0 | 1 | 0 | 0 | 0 | 1 | 1 |
| 60 | 1 | 1 | 1 | 1 | 1 | 1 | 1 | 1 | 1 | 1 |
| 61 | 1 | 1 | 1 | 1 | 0 | 1 | 1 | 1 | 0 | 1 |
| 62 | 1 | 1 | 1 | 1 | 1 | 1 | 1 | 1 | 1 | 1 |
| 63 | 1 | 1 | 1 | 1 | 1 | 1 | 1 | 1 | 1 | 1 |
| 64 | 1 | 1 | 1 | 1 | 0 | 1 | 1 | 1 | 0 | 0 |
| 65 | 1 | 1 | 1 | 1 | 0 | 1 | 1 | 1 | 1 | 1 |
| 66 | 1 | 1 | 1 | 1 | 1 | 1 | 1 | 1 | 1 | 1 |
| 67 | 1 | 1 | 1 | 1 | 1 | 1 | 1 | 1 | 1 | 1 |
| 68 | 1 | 1 | 1 | 1 | 1 | 1 | 1 | 1 | 1 | 1 |
| 69 | 1 | 1 | 1 | 1 | 1 | 1 | 1 | 1 | 1 | 1 |
| 70 | 1 | 1 | 1 | 1 | 1 | 1 | 1 | 1 | 1 | 1 |
| 71 | 1 | 1 | 1 | 1 | 0 | 1 | 1 | 1 | 1 | 1 |
| 72 | 1 | 1 | 1 | 1 | 1 | 1 | 1 | 1 | 1 | 1 |
| 73 | 1 | 1 | 1 | 1 | 1 | 1 | 1 | 1 | 1 | 1 |
| 74 | 1 | 1 | 1 | 1 | 1 | 1 | 1 | 1 | 1 | 1 |
| 75 | 1 | 1 | 1 | 1 | 1 | 1 | 1 | 1 | 1 | 1 |
| 76 | 1 | 1 | 1 | 1 | 1 | 1 | 1 | 1 | 1 | 1 |
| 77 | 1 | 1 | 1 | 1 | 1 | 1 | 1 | 1 | 1 | 1 |
| 78 | 1 | 1 | 1 | 1 | 1 | 1 | 1 | 1 | 1 | 1 |
| 79 | 1 | 1 | 1 | 1 | 1 | 1 | 1 | 1 | 1 | 1 |
| 80 | 1 | 1 | 1 | 1 | 0 | 1 | 1 | 1 | 1 | 1 |
| 81 | 1 | 1 | 1 | 1 | 1 | 1 | 1 | 1 | 1 | 1 |
| 82 | 1 | 1 | 1 | 1 | 1 | 1 | 1 | 1 | 1 | 1 |
| 83 | 1 | 1 | 1 | 1 | 1 | 1 | 1 | 1 | 1 | 1 |
| 84 | 0 | 0 | 0 | 0 | 0 | 0 | 1 | 1 | 0 | 0 |
| 85 | 0 | 0 | 0 | 0 | 0 | 0 | 1 | 1 | 0 | 0 |
| 86 | 0 | 0 | 1 | 0 | 0 | 0 | 0 | 0 | 0 | 0 |
| 87 | 0 | 1 | 1 | 0 | 0 | 0 | 0 | 0 | 0 | 0 |
| 88 | 0 | 0 | 0 | 1 | 0 | 0 | 0 | 0 | 0 | 0 |
| 89 | 0 | 0 | 0 | 1 | 0 | 0 | 0 | 0 | 0 | 0 |
| 90 | 1 | 1 | 0 | 1 | 0 | 1 | 1 | 0 | 0 | 1 |
| 91 | 1 | 1 | 0 | 1 | 0 | 1 | 1 | 0 | 0 | 0 |
| 92 | 0 | 0 | 0 | 1 | 0 | 0 | 0 | 0 | 0 | 0 |
| 93 | 1 | 1 | 1 | 1 | 0 | 0 | 1 | 0 | 0 | 0 |
| 94 | 1 | 1 | 1 | 1 | 0 | 0 | 1 | 1 | 0 | 0 |
| 95 | 0 | 0 | 0 | 0 | 1 | 1 | 0 | 0 | 1 | 1 |
| 96 | 0 | 0 | 0 | 0 | 0 | 1 | 0 | 0 | 0 | 0 |
| 97 | 1 | 0 | 0 | 1 | 1 | 1 | 0 | 1 | 1 | 1 |
| 98 | 0 | 0 | 0 | 0 | 0 | 0 | 0 | 1 | 0 | 1 |
| 99 | 0 | 0 | 0 | 0 | 0 | 0 | 0 | 1 | 0 | 0 |
| 100 | 0 | 0 | 0 | 0 | 0 | 0 | 0 | 1 | 0 | 0 |
| 101 | 0 | 0 | 0 | 0 | 1 | 0 | 0 | 0 | 0 | 0 |
| 102 | 0 | 0 | 0 | 0 | 1 | 0 | 0 | 0 | 0 | 0 |
| 103 | 0 | 0 | 0 | 0 | 1 | 0 | 0 | 0 | 1 | 1 |
| 104 | 0 | 0 | 0 | 1 | 1 | 0 | 0 | 0 | 1 | 1 |
| 105 | 0 | 0 | 0 | 0 | 1 | 0 | 0 | 0 | 1 | 0 |
| 106 | 0 | 0 | 0 | 0 | 1 | 0 | 0 | 0 | 1 | 0 |
| 107 | 0 | 0 | 0 | 0 | 1 | 0 | 0 | 0 | 1 | 0 |
| 108 | 0 | 0 | 0 | 0 | 1 | 0 | 0 | 0 | 1 | 0 |

**TABLEAU 15B :**

| Transition Numéro | VIR34 | VIR35 | VIR36 | VIR37 | VIR38 | VIR39 | CA16 | VIR43 | VIR5 | AST-VAN8 |
|---|---|---|---|---|---|---|---|---|---|---|
| 1 | 1 | 1 | 1 | 1 | 1 | 1 | 1 | 0 | 0 | 0 |
| 2 | 1 | 1 | 1 | 1 | 1 | 1 | 1 | 0 | 0 | 0 |
| 3 | 1 | 1 | 1 | 1 | 1 | 1 | 1 | 0 | 0 | 0 |
| 4 | 1 | 1 | 1 | 1 | 1 | 1 | 1 | 0 | 0 | 0 |
| 5 | 1 | 1 | 1 | 1 | 1 | 1 | 1 | 0 | 0 | 0 |
| 6 | 1 | 1 | 1 | 1 | 1 | 1 | 1 | 0 | 0 | 0 |
| 7 | 1 | 1 | 1 | 1 | 1 | 1 | 1 | 1 | 0 | 0 |
| 8 | 1 | 1 | 1 | 1 | 1 | 1 | 1 | 0 | 0 | 0 |
| 9 | 1 | 1 | 1 | 1 | 1 | 1 | 1 | 0 | 0 | 0 |
| 10 | 0 | 0 | 0 | 0 | 0 | 0 | 1 | 0 | 0 | 0 |
| 11 | 1 | 1 | 1 | 1 | 1 | 1 | 1 | 0 | 0 | 0 |
| 12 | 0 | 0 | 0 | 0 | 0 | 0 | 1 | 1 | 0 | 0 |
| 13 | 1 | 1 | 1 | 1 | 1 | 0 | 1 | 0 | 0 | 0 |
| 14 | 1 | 1 | 0 | 1 | 1 | 1 | 1 | 0 | 0 | 0 |
| 15 | 1 | 1 | 1 | 1 | 1 | 1 | 1 | 0 | 0 | 0 |
| 16 | 1 | 1 | 1 | 1 | 1 | 1 | 1 | 0 | 0 | 0 |
| 17 | 1 | 1 | 1 | 1 | 1 | 1 | 1 | 0 | 0 | 0 |
| 18 | 1 | 1 | 1 | 1 | 1 | 1 | 1 | 0 | 0 | 0 |
| 19 | 1 | 1 | 1 | 1 | 1 | 1 | 1 | 0 | 0 | 0 |
| 20 | 1 | 1 | 1 | 1 | 1 | 1 | 1 | 1 | 0 | 0 |
| 21 | 1 | 1 | 1 | 1 | 1 | 1 | 1 | 0 | 0 | 0 |
| 22 | 1 | 1 | 1 | 1 | 1 | 1 | 1 | 0 | 0 | 0 |
| 23 | 1 | 1 | 1 | 1 | 1 | 1 | 1 | 1 | 0 | 0 |
| 24 | 1 | 1 | 1 | 1 | 1 | 1 | 1 | 1 | 0 | 0 |
| 25 | 1 | 1 | 1 | 1 | 1 | 1 | 1 | 0 | 0 | 0 |
| 26 | 1 | 1 | 1 | 1 | 1 | 1 | 1 | 0 | 0 | 0 |
| 27 | 1 | 1 | 1 | 1 | 1 | 1 | 1 | 0 | 0 | 0 |
| 28 | 1 | 1 | 1 | 1 | 1 | 1 | 1 | 0 | 0 | 0 |
| 29 | 1 | 1 | 1 | 1 | 1 | 1 | 1 | 0 | 0 | 0 |
| 30 | 1 | 1 | 1 | 1 | 1 | 1 | 1 | 0 | 0 | 0 |
| 31 | 1 | 1 | 1 | 1 | 1 | 1 | 1 | 0 | 0 | 0 |
| 32 | 1 | 1 | 1 | 1 | 1 | 1 | 1 | 1 | 1 | 1 |
| 33 | 1 | 1 | 1 | 1 | 1 | 1 | 1 | 0 | 0 | 0 |
| 34 | 1 | 1 | 0 | 1 | 1 | 1 | 1 | 0 | 0 | 0 |
| 35 | 1 | 1 | 1 | 1 | 1 | 1 | 1 | 0 | 0 | 0 |
| 36 | 1 | 1 | 1 | 1 | 1 | 1 | 1 | 0 | 0 | 0 |
| 37 | 1 | 1 | 1 | 1 | 1 | 1 | 1 | 0 | 0 | 0 |
| 38 | 0 | 0 | 0 | 0 | 0 | 0 | 1 | 0 | 0 | 0 |
| 39 | 1 | 1 | 1 | 0 | 0 | 1 | 1 | 0 | 0 | 0 |
| 40 | 0 | 0 | 0 | 0 | 0 | 0 | 1 | 0 | 0 | 0 |
| 41 | 1 | 1 | 1 | 1 | 1 | 1 | 1 | 0 | 0 | 0 |
| 42 | 1 | 1 | 1 | 1 | 1 | 1 | 1 | 0 | 0 | 0 |
| 43 | 1 | 1 | 1 | 1 | 1 | 1 | 1 | 0 | 0 | 0 |
| 44 | 1 | 1 | 1 | 1 | 1 | 1 | 1 | 0 | 0 | 0 |
| 45 | 1 | 1 | 1 | 1 | 1 | 1 | 1 | 0 | 0 | 0 |
| 46 | 1 | 1 | 1 | 1 | 1 | 1 | 1 | 0 | 0 | 0 |
| 47 | 1 | 1 | 1 | 1 | 1 | 1 | 1 | 0 | 0 | 0 |
| 48 | 1 | 1 | 1 | 1 | 1 | 1 | 1 | 0 | 0 | 0 |
| 49 | 1 | 1 | 1 | 1 | 1 | 1 | 0 | 0 | 0 | 0 |
| 50 | 1 | 1 | 1 | 1 | 1 | 1 | 1 | 0 | 0 | 0 |
| 51 | 0 | 1 | 1 | 1 | 1 | 1 | 1 | 0 | 0 | 0 |
| 52 | 1 | 1 | 1 | 1 | 1 | 1 | 1 | 0 | 0 | 0 |
| 53 | 1 | 1 | 1 | 1 | 1 | 1 | 1 | 0 | 0 | 0 |
| 54 | 1 | 1 | 1 | 1 | 1 | 1 | 1 | 0 | 0 | 0 |
| 55 | 0 | 0 | 0 | 0 | 0 | 0 | 1 | 0 | 0 | 0 |
| 56 | 0 | 0 | 0 | 0 | 0 | 0 | 1 | 0 | 0 | 0 |
| 57 | 0 | 0 | 0 | 0 | 0 | 0 | 1 | 0 | 0 | 0 |
| 58 | 1 | 1 | 1 | 1 | 1 | 1 | 1 | 0 | 0 | 0 |
| 59 | 1 | 1 | 1 | 1 | 1 | 1 | 1 | 0 | 0 | 0 |
| 60 | 1 | 1 | 1 | 1 | 1 | 1 | 1 | 0 | 0 | 0 |
| 61 | 1 | 0 | 0 | 0 | 1 | 1 | 0 | 0 | 0 | 0 |
| 62 | 1 | 1 | 1 | 1 | 1 | 1 | 1 | 1 | 0 | 0 |
| 63 | 0 | 1 | 1 | 1 | 1 | 1 | 1 | 1 | 0 | 0 |
| 64 | 0 | 1 | 1 | 1 | 1 | 1 | 1 | 0 | 0 | 0 |
| 65 | 0 | 1 | 1 | 1 | 1 | 1 | 1 | 1 | 0 | 0 |
| 66 | 1 | 1 | 1 | 1 | 1 | 1 | 1 | 0 | 0 | 0 |
| 67 | 1 | 1 | 1 | 1 | 1 | 1 | 1 | 0 | 0 | 0 |
| 68 | 1 | 1 | 1 | 1 | 1 | 1 | 1 | 0 | 0 | 0 |
| 69 | 1 | 1 | 1 | 1 | 1 | 1 | 1 | 0 | 0 | 0 |
| 70 | 1 | 1 | 1 | 1 | 1 | 1 | 1 | 1 | 0 | 0 |
| 71 | 1 | 1 | 1 | 1 | 1 | 1 | 1 | 0 | 0 | 0 |
| 72 | 1 | 1 | 1 | 1 | 1 | 1 | 1 | 0 | 0 | 0 |
| 73 | 1 | 1 | 1 | 1 | 1 | 1 | 1 | 0 | 0 | 0 |
| 74 | 1 | 1 | 1 | 1 | 1 | 1 | 1 | 0 | 0 | 0 |
| 75 | 1 | 1 | 1 | 1 | 1 | 1 | 1 | 0 | 0 | 0 |
| 76 | 1 | 1 | 1 | 1 | 1 | 1 | 1 | 1 | 0 | 0 |
| 77 | 1 | 1 | 1 | 1 | 1 | 1 | 1 | 0 | 0 | 0 |
| 78 | 1 | 1 | 1 | 1 | 1 | 1 | 1 | 0 | 0 | 0 |
| 79 | 1 | 1 | 1 | 1 | 1 | 1 | 1 | 0 | 0 | 0 |
| 80 | 1 | 1 | 1 | 1 | 1 | 1 | 0 | 0 | 0 | 0 |
| 81 | 1 | 1 | 1 | 1 | 1 | 1 | 1 | 0 | 1 | 0 |
| 82 | 1 | 1 | 1 | 1 | 1 | 1 | 1 | 1 | 0 | 0 |
| 83 | 1 | 1 | 1 | 1 | 1 | 1 | 1 | 0 | 0 | 0 |
| 84 | 0 | 0 | 0 | 0 | 0 | 0 | 0 | 0 | 0 | 0 |
| 85 | 0 | 0 | 0 | 0 | 0 | 0 | 0 | 0 | 0 | 0 |
| 86 | 0 | 0 | 0 | 0 | 0 | 0 | 0 | 0 | 0 | 0 |
| 87 | 0 | 0 | 0 | 0 | 0 | 0 | 0 | 0 | 0 | 0 |
| 88 | 0 | 0 | 0 | 0 | 0 | 0 | 0 | 0 | 0 | 0 |
| 89 | 0 | 0 | 0 | 0 | 0 | 0 | 0 | 0 | 0 | 0 |
| 90 | 0 | 0 | 0 | 0 | 0 | 0 | 0 | 0 | 0 | 0 |
| 91 | 0 | 0 | 0 | 0 | 0 | 0 | 0 | 0 | 0 | 0 |
| 92 | 0 | 0 | 0 | 0 | 0 | 0 | 0 | 1 | 0 | 0 |
| 93 | 0 | 0 | 0 | 0 | 0 | 0 | 0 | 0 | 0 | 0 |
| 94 | 1 | 0 | 0 | 0 | 0 | 0 | 0 | 0 | 0 | 0 |
| 95 | 0 | 1 | 1 | 1 | 1 | 1 | 1 | 0 | 0 | 0 |
| 96 | 0 | 0 | 0 | 0 | 0 | 0 | 0 | 0 | 0 | 0 |
| 97 | 1 | 0 | 0 | 1 | 1 | 1 | 0 | 0 | 0 | 0 |
| 98 | 1 | 1 | 1 | 1 | 1 | 0 | 0 | 0 | 0 | 0 |
| 99 | 0 | 0 | 0 | 0 | 0 | 0 | 0 | 0 | 0 | 0 |
| 100 | 0 | 0 | 0 | 0 | 0 | 0 | 0 | 0 | 0 | 0 |
| 101 | 0 | 0 | 0 | 0 | 0 | 0 | 0 | 0 | 0 | 0 |
| 102 | 0 | 0 | 0 | 0 | 0 | 0 | 0 | 0 | 0 | 0 |
| 103 | 0 | 1 | 1 | 0 | 0 | 1 | 0 | 1 | 0 | 0 |
| 104 | 0 | 1 | 1 | 1 | 1 | 0 | 1 | 0 | 0 | 0 |
| 105 | 0 | 0 | 0 | 0 | 0 | 0 | 0 | 0 | 0 | 0 |
| 106 | 0 | 0 | 0 | 0 | 0 | 0 | 0 | 0 | 0 | 0 |
| 107 | 0 | 0 | 0 | 0 | 0 | 0 | 0 | 0 | 0 | 0 |
| 108 | 0 | 0 | 0 | 0 | 0 | 0 | 0 | 0 | 0 | 0 |

Le nombre de transitions positives est ensuite sommé pour les applications I, R et V et reporté dans le **TABLEAU 16 :**

**TABLEAU 16 :**

| Souches | Espèces | Transitions I |
|---|---|---|
| ATF1 | *C. albicans* | 75 |
| ATF2 | *C. albicans* | 75 |
| ATF3 | *C. albicans* | 75 |
| ATF4 | *C. albicans* | 75 |
| VIR31 | *C. albicans* | 61 |
| ATF5 | *C. albicans* | 75 |
| ATF6 | *C. albicans* | 75 |
| ATF7 | *C. albicans* | 75 |
| VIR32 | *C. albicans* | 68 |
| VIR33 | *C. albicans* | 75 |
| VIR34 | *C. albicans* | 72 |
| VIR35 | *C. albicans* | 75 |
| VIR36 | *C. albicans* | 73 |
| VIR37 | *C. albicans* | 74 |
| VIR38 | *C. albicans* | 75 |
| VIR39 | *C. albicans* | 75 |
| CA16 | *C. albicans* | 80 |
| VIR43 | *E coli* | 12 |
| VIR5 | S. *aureus* | 2 |
| AST-VAN8 | *E. faecium* | 1 |

Tous les échantillons de *C. albicans* présentent plus de 60 transitions positives dans la catégorie I. Tous ces échantillons sont donc confirmés comme appartenant bien à l'espèce *C. albicans.*

En revanche, les échantillons VIR43, VIR5 et AST-VAN8 présentent moins de 13 transitions positives dans la catégorie I, ces échantillons sont donc confirmés comme n'appartenant pas à l'espèce *C. albicans.*
L'observation des transitions 84 et 85 indiquent la présence du peptide muté et traduit la résistance des souches ATF6 et ATF7.
L'observation des transitions 86 et 87 indiquent la présence du peptide muté et traduit la résistance des souches ATF2 et ATF3.
L'observation de la transition 96 indiquent la présence du peptide muté et traduisent la résistance de la souche ATF5.
L'observation des transitions 99 et 100 indiquent la présence du peptide muté et traduit la résistance de la souche ATF7.
L'observation des transitions 88 et 89 indiquent la présence du peptide natif et traduit la sensibilité de la souche ATF4.
L'observation des transitions 93 et 94 indiquent la présence du peptide natif et traduit la sensibilité des souches ATF1 et ATF4.
L'ensemble des aires des transitions I sont sommées pour conduire à la somme SI. L'ensemble des aires des transitions V sont sommées pour conduire à la somme SV. Le rapport SV/SI est ensuite calculé et multiplié par un facteur multiplicatif FM. Les résultats obtenus figurent dans le **TABLEAU 17.**

**TABLEAU 17 :**

| Souches | Somme (SI) des aires des transitions I | Somme (SV) des aires des transitions V | Rapport des sommes SV/SI multiplié par le facteur multiplicatif (FM) | Facteur multiplicatif (FM) |
|---|---|---|---|---|
| ATF1 | 1.8E+07 | 1.6E+03 | 1.9E+03 | 2.0E+07 |
| ATF2 | 1.3E+07 | 1.6E+03 | 2.4E+03 | |
| ATF3 | 2.0E+07 | 2.3E+03 | 2.3E+03 | |
| ATF4 | 2.9E+07 | 3.3E+03 | 2.3E+03 | |
| VIR31 | 1.6E+07 | 1.4E+06 | 1.8E+06 | |
| ATF5 | 1.7E+07 | 2.3E+03 | 2.6E+03 | |
| ATF6 | 2.4E+07 | 4.8E+03 | 4.0E+03 | |
| ATF7 | 2.9E+07 | 2.4E+03 | 1.7E+03 | |
| VIR32 | 2.9E+07 | 5.3E+05 | 3.6E+05 | |
| VIR33 | 1.9E+07 | 4.2E+04 | 4.4E+04 | |
| VIR34 | 4.2E+07 | 1.8E+03 | 8.9E+02 | |
| VIR35 | 1.1E+07 | 8.8E+03 | 1.5E+04 | |
| VIR36 | 8.3E+06 | 3.5E+04 | 8.5E+04 | |
| VIR37 | 2.3E+07 | 3.9E+03 | 3.4E+03 | |
| VIR38 | 2.4E+07 | 3.4E+03 | 2.8E+03 | |
| VIR39 | 1.6E+07 | 9.2E+03 | 1.1E+04 | |
| CA16 | 1.2E+07 | 5.4E+03 | 9.1E+03 | |

Les souches VIR31 et VIR32 qui ont un rapport (SV/SI)xFM supérieur à 9.10⁴ surexpriment la lipase 8, ces souches sont donc virulentes. Toutes les autres souches ont un rapport inférieur à 9.10⁴, elles ne surexpriment pas la lipase 8 et ne sont donc pas virulentes. De façon intéressante le rapport (SV/SI)xFM étant plus élevé pour la souche VIR31 que pour la souche VIR32, la souche VIR31 est caractérisée comme plus virulente que la souche VIR32.

Pour le typage, les transitions de la catégorie T de chaque souche sont comparées aux transitions des autres souches considérées comme des souches de référence. En pratique une valeur 0 est attribuée lorsque les transitions entre les 2 souches se classent dans la même catégorie (positive ou négative) et une valeur de 1 est attribuée lorsque les transitions entre les 2 souches se classent dans des catégories différentes (une transition positive et une transition négative). Les valeurs sont sommées pour toutes les transitions de la catégorie T de chaque couple de souche afin d'établir un score. Les scores figurent dans le **TABLEAU 18 :**

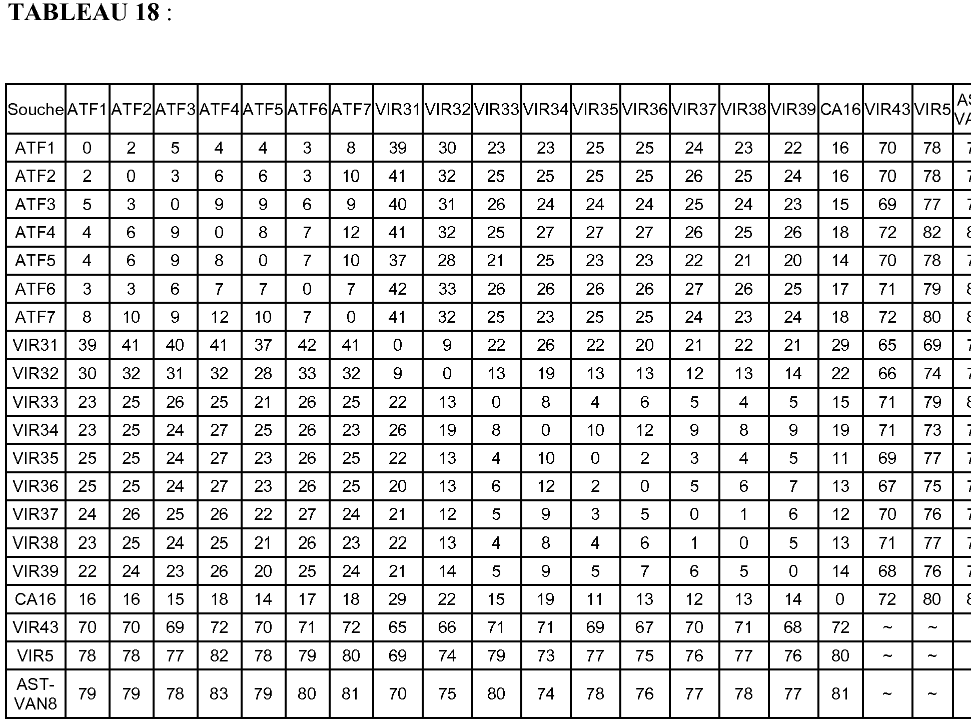

Les souches qui ont un score inférieur ou égal à 2 sont de même type, les souches qui ont un score strictement supérieur à 2 sont de type différent.
Ainsi, les souches ATF1 et ATF2, VIR35 et VIR36, VIR37 et VIR38 sont respectivement de même type. Toutes les autres souches prises deux à deux sont de types différents. Il faut noter les sommes élevées obtenues entre les souches VIR43, VIR5 et AST-VAN8, qui ne sont pas des *S. Aureus,* et toutes les autres souches qui sont des *S. Aureus.* Ces résultats confirment la spécificité de la méthode.

Les souches VIR43, VIR5 et AST-VAN8 sont d'espèces différentes. Ces souches ne peuvent donc pas être comparées entre elles et aucune valeur n'est reportée dans le **TABLEAU 18.**
De façon extrêmement intéressante, des scores supérieurs à 20, comme par exemple entre ATF1 et VIR39, traduisent une grande divergence entre souches. Des scores entre 14 et 20, comme entre CA16 et ATF1, traduisent une divergence modérée et des scores entre 4 et 14, comme entre ATF1 et ATF7, une divergence faible.

La méthode ainsi mise en oeuvre permet donc non seulement d'établir si 2 souches sont de même type, ce qui est important pour identifier un foyer infectieux commun, mais également d'estimer la proximité de 2 souches, ce qui est extrêmement important pour les études épidémiologiques.

Cet exemple montre que, de façon très avantageuse, la présente invention permet en un temps inférieur à une heure, ce qui est très court, de confirmer l'identité d'une espèce telle que *C*. *albicans* et de déterminer, simultanément au sein de la même analyse, les propriétés de typage, de résistance potentielle à au moins un antibiotique et d'établir l'existence de facteur de virulence. La présente invention permet en outre un dosage quantitatif, ce qui est particulièrement avantageux lorsque les propriétés de résistance à au moins un antibiotique ou de virulence sont liées au taux d'expression d'une protéine ou d'un métabolite. C'est ce que nous illustrons ici avec le dosage quantitatif de la lipase 8. Ces propriétés ont été établies avec le même instrument, ce qui facilite grandement l'analyse et le rendu des résultats. Enfin, les caractéristiques des levures sont établies à l'aide de protéines fongiques, ce qui traduit l'existence de microorganismes vivants et viables, contrairement aux caractérisations à partir d'ADN fongique qui peuvent être faussées par l'existence de levures mortes.

### Exemple 10 : Protocole de digestion de microorganismes en présence de méthanol

### adapté au dosage d'au moins un métabolite

Classiquement le protocole suivant est mis en oeuvre en 19 étapes :
1. Prélèvement de cinq colonies de microorganisme, obtenue selon l'exemple 1, et mise en suspension dans 100µl d'une solution d'hydrochlorure de guanidine 6M, 50 mM Tris-HCl, pH=8,0.
2. Centrifuger à 15000g pendant 5 minutes
3. Le culot est repris dans 400µl d'une solution de bicarbonate d'ammonium 50 mM, pH=8,0.
4. Ajout de 600µl de méthanol
Etapes 5 à 9 : idem étapes 2 à 6 de l'exemple 4.
Etapes 10 à 19 : idem étapes 8 à 17 de l'exemple 6.

### Exemple 11 : Caractérisation d'échantillons de C. albicans par analyse simultanée de protéines et de métabolites:

Après avoir établi la ou les espèces des échantillons selon l'une quelconque des méthodes décrites dans les exemples 1 à 3, les espèces listées ci-dessous sont analysées. Cinq souches de C. *albicans* sont analysées pour confirmer leur identification et établir leurs caractéristiques :

| | |
|---|---|
| ATF1 | ATF2 |
| ATF3 | ATF4 |
| ATF6 | |

Chaque échantillon est traité selon l'exemple 10, puis un volume de 20µl de protéines digérées est injecté et analysé selon les mêmes conditions que dans l'exemple 7.
Les tableaux 13 et 14 sont identiques à une exception près. La transition numéro 109 est ajouté à la méthode. Elle correspond à la molécule, ergostérol, à l'intérêt clinique I et R.
L'état de charge du précurseur est de 1, le temps de rétention est de 36.3 minutes, le m/z filtré en Q1 est de 379.4, le m/z filtré en Q3 est de 69.2, l'énergie de collision est de 47.
Les paramètres de masse sont identiques à ceux de l'exemple 7 à l'exception de ceux-ci :

| | |
|---|---|
| Gaz rideau: | 25,00 psi |
| Température de source: | 450,00 °C |
| Gaz chauffant: | 50,00 psi |
| Potentiel d'entrée avant Q0 (EP): | 4,00 V |

Pour la transition 109, des paramètres diffèrent :

| | |
|---|---|
| Potentiel d'orifice : | 200, 00 V |
| (en anglais declustering potential (DP)) | |
| Potentiel d'entrée avant Q0 (EP): | 10,00 V |
| Potentiel en sortie de cellule de collision : | 8 V |
| (en anglais cell exit potential (CXP)) | |

L'ensemble des transitions 1 à 108 sont analysées de façon identique à l'exemple 9. L'ensemble des aires des transitions I sont sommées pour conduire à la somme SI. L'aire de la transition 109 est nommé A109. Le rapport A109/SI est ensuite calculé et multiplié par un facteur multiplicatif FM. Les résultats obtenus figurent dans le tableau 19.

**TABLEAU 19 :**

| Souches | Aire de la transition 109 (A109) | Somme (SI) des aires des transitions I | Rapport A109/SI multiplié par le facteur multiplicatif (FM) | Facteur multiplicatif (FM) |
|---|---|---|---|---|
| ATF1 | 1.7E+03 | 9.3E+07 | 1.6E+03 | 9.0E+07 |
| ATF2 | 7.9E+03 | 1.1E+08 | 6.4E+03 | |
| ATF3 | 3.9E+03 | 5.7E+07 | 6.3E+03 | |
| ATF4 | 5.7E+03 | 2.9E+08 | 1.8E+03 | |
| ATF6 | 6.4E+03 | 6.8E+07 | 8.5E+03 | |

Les souches ATF2, ATF3 et ATF6 qui ont un rapport (A109/SI) x FM supérieur à 2.10³ surexpriment l'ergostérol, ces souches sont donc résistantes. Toutes les autres souches ont un rapport inférieur à 2.10³, elles ne surexpriment pas l'ergostérol et ne sont donc pas résistantes.

Cet exemple montre que, de façon très avantageuse, la présente invention permet de doser simultanément au sein de la même analyse, en un temps inférieur à une heure, ce qui est très court, et de façon quantitative, différents composés tels que des protéines, des peptides ou des métabolites. De façon encore plus avantageuse que le dosage d'une protéine, le dosage quantitatif d'un métabolite issu de l'action de cette protéine caractérise la présence d'une protéine fonctionnelle et plus largement d'une voie de synthèse fonctionnelle. Ainsi la quantification de l'ergostérol permet ici d'établir l'existence d'un mécanisme de résistance au fluconazole lié à l'existence de lanostérol déméthylase fonctionnelle et fortement active.

### Exemple 12 : Caractérisation de microorganismes présents dans des échantillons urinaires :

Après avoir établi la ou les espèces des échantillons selon l'une quelconque des méthodes décrites dans les exemples 1 à 3, cinq échantillons d'urine contaminée par E. coli (Urine 1 à 5) sont analysés. Une sixième urine non contaminée (Urine 6) est également analysée pour servir de témoin négatif.

Le protocole suivant est mis en oeuvre en 29 étapes (les étapes 5 à 12 sont optionnelles et pourraient être omises sans altérer significativement les résultats) :
1. Centrifugation de 5 mL d'urine contaminée à 2000g pendant 30 secondes
2. Récupération du surnageant
3. Centrifugation à 15000g pendant 5 minutes
4. Elimination du surnageant
5. Lavage du culot avec 3 mL d'eau distillée par remise en suspension
6. Centrifugation à 15000g pendant 5 minutes
7. Elimination du surnageant
8. Mettre le culot en présence de solvant en dilution au 1/10
9. Laisser 1 heure à -20°C
10. Centrifugation à 15000g pendant 5 minutes
11. Elimination du surnageant
12. Mettre le culot en présence de solvant en dilution au 1/10
13. Laisser 1 heure à -20°C
14. Centrifugation à 15000g pendant 5 minutes
15. Elimination du surnageant
12. Mise en suspension du culot dans 10 à 100µl d'une solution d'hydrochlorure de guanidine 6M, 50 mM Tris-HCl, pH=8,0.
13. Etapes 14 à 18 : idem étapes 2 à 6 de l'exemple 4.
19. Etapes 19 à 28 : idem étapes 8 à 17 de l'exemple 6.
29. Injecter 100µL d'éluât acidifié sur la chaine chromatographique et le spectromètre de masse, selon le protocole décrit dans l'exemple 7.

Les aires obtenues pour chacune des transitions et pour chacun des microorganismes étudiés ont été mesurées. Les échantillons d'urine ayant une charge bactérienne très différente d'un échantillon à l'autre, le seuil utilisé dans l'exemple 7 pour déclarer une transition positive, ne peut pas être utilisé ici. Il doit être adapté à la quantité de bactéries présentes. Dans les exemples précédents, le prélèvement d'une colonie conduisait à une quantité comparable de microorganismes. Ce n'est plus le cas dans cet exemple. Aussi, la quantité de bactéries est estimée en sommant l'aire de l'ensemble des transitions I. La somme des aires des transitions I est reportée dans le **TABLEAU 20.**

**TABLEAU 20 :**

| Urine 1 | Urine 2 | Urine 3 | Urine 4 | Urine 5 | Urine 6 |
|---|---|---|---|---|---|
| 1 509 251,3 | 828 079,5 | 5 349 271,0 | 11 918 946,9 | 1 054 480,6 | 0,0 |

Ainsi l'échantillon 4 comporte une charge bactérienne plus élevée que l'échantillon 3, lui-même plus chargé que les échantillons 1 et 5, eux-mêmes plus chargés que l'échantillon 2. Les échantillons 1 et 5 ont une charge bactérienne comparable. L'échantillon 6 ne comporte pas d'*E. coli* et présente une charge nulle, ce qui démontre la spécificité de la technique.
L'aire de chaque transition est ensuite normalisée en la divisant par l'aire de l'ensemble des transitions I. Toutes les transitions dont le ratio est supérieur ou égal à 0.00015 (ratio adimensionnel) sont considérées comme positives et ont été notées « 1 » dans le **TABLEAU 21.** Toutes les transitions dont le ratio est inférieur à 0.00015, sont considérées comme négatives et ont été notées 0 dans le **TABLEAU 21.** Lorsqu'on n'a observé aucun pic de signal, on a noté la transition comme négative.

**TABLEAU 21 :**

| Transition numéro | Urine 1 | Urine 2 | Urine 3 | Urine 4 | Urine 5 | Urine 6 |
|---|---|---|---|---|---|---|
| 1 | 1 | 1 | 1 | 1 | 1 | 0 |
| 2 | 1 | 1 | 1 | 1 | 1 | 0 |
| 3 | 1 | 1 | 1 | 1 | 1 | 0 |
| 4 | 0 | 0 | 1 | 1 | 1 | 0 |
| 5 | 0 | 0 | 1 | 1 | 1 | 0 |
| 6 | 0 | 0 | 0 | 1 | 1 | 0 |
| 7 | 0 | 0 | 0 | 0 | 0 | 0 |
| 8 | 0 | 0 | 0 | 0 | 0 | 0 |
| 9 | 0 | 0 | 0 | 0 | 0 | 0 |
| 10 | 1 | 1 | 1 | 1 | 1 | 0 |
| 11 | 1 | 1 | 1 | 1 | 1 | 0 |
| 12 | 1 | 1 | 1 | 1 | 1 | 0 |
| 13 | 1 | 1 | 1 | 1 | 1 | 0 |
| 14 | 1 | 1 | 1 | 1 | 1 | 0 |
| 15 | 1 | 1 | 1 | 1 | 1 | 0 |
| 16 | 1 | 1 | 1 | 1 | 1 | 0 |
| 17 | 1 | 1 | 1 | 1 | 1 | 0 |
| 18 | 1 | 1 | 1 | 1 | 0 | 0 |
| 19 | 1 | 1 | 1 | 1 | 0 | 0 |
| 20 | 1 | 0 | 1 | 0 | 0 | 0 |
| 21 | 1 | 1 | 1 | 1 | 1 | 0 |
| 22 | 1 | 1 | 1 | 1 | 1 | 0 |
| 23 | 1 | 1 | 1 | 1 | 1 | 0 |
| 24 | 1 | 1 | 1 | 1 | 1 | 0 |
| 25 | 1 | 1 | 0 | 1 | 1 | 0 |
| 26 | 1 | 1 | 0 | 1 | 1 | 0 |
| 27 | 1 | 1 | 0 | 1 | 1 | 0 |
| 28 | 1 | 1 | 1 | 1 | 1 | 0 |
| 29 | 1 | 1 | 1 | 1 | 1 | 0 |
| 30 | 1 | 1 | 1 | 1 | 1 | 0 |
| 31 | 0 | 1 | 1 | 1 | 1 | 0 |
| 32 | 0 | 0 | 1 | 1 | 1 | 0 |
| 33 | 0 | 0 | 0 | 1 | 0 | 0 |
| 34 | 1 | 1 | 1 | 1 | 1 | 0 |
| 35 | 1 | 1 | 1 | 1 | 1 | 0 |
| 36 | 1 | 1 | 1 | 1 | 1 | 0 |
| 37 | 1 | 1 | 1 | 1 | 1 | 0 |
| 38 | 1 | 1 | 1 | 1 | 1 | 0 |
| 39 | 1 | 1 | 1 | 1 | 1 | 0 |
| 40 | 1 | 1 | 1 | 1 | 1 | 0 |
| 41 | 1 | 1 | 1 | 1 | 1 | 0 |
| 42 | 1 | 1 | 1 | 1 | 1 | 0 |
| 43 | 1 | 1 | 1 | 1 | 1 | 0 |
| 44 | 1 | 1 | 1 | 1 | 1 | 0 |
| 45 | 1 | 1 | 1 | 1 | 1 | 0 |
| 46 | 0 | 0 | 1 | 1 | 1 | 0 |
| 47 | 0 | 0 | 1 | 1 | 1 | 0 |
| 48 | 0 | 0 | 1 | 1 | 1 | 0 |
| 49 | 0 | 0 | 0 | 0 | 0 | 0 |
| 50 | 0 | 0 | 0 | 0 | 0 | 0 |
| 51 | 0 | 0 | 0 | 0 | 0 | 0 |
| 52 | 0 | 0 | 0 | 0 | 0 | 0 |
| 53 | 0 | 0 | 0 | 0 | 0 | 0 |
| 54 | 0 | 0 | 0 | 0 | 0 | 0 |
| 55 | 1 | 1 | 1 | 0 | 1 | 0 |
| 56 | 1 | 1 | 1 | 0 | 1 | 0 |
| 57 | 1 | 1 | 1 | 0 | 1 | 0 |
| 58 | 1 | 1 | 1 | 0 | 1 | 0 |
| 59 | 1 | 1 | 1 | 0 | 1 | 0 |
| 60 | 1 | 1 | 1 | 0 | 0 | 0 |
| 61 | 0 | 0 | 0 | 0 | 0 | 0 |
| 62 | 0 | 0 | 0 | 0 | 0 | 0 |
| 63 | 0 | 0 | 0 | 0 | 0 | 0 |
| 64 | 0 | 0 | 0 | 0 | 0 | 0 |
| 65 | 0 | 0 | 0 | 0 | 0 | 0 |
| 66 | 1 | 0 | 0 | 0 | 0 | 0 |
| 67 | 1 | 1 | 1 | 1 | 1 | 0 |
| 68 | 1 | 1 | 1 | 1 | 1 | 0 |
| 69 | 1 | 1 | 1 | 1 | 1 | 0 |
| 70 | 1 | 0 | 0 | 1 | 1 | 0 |
| 71 | 1 | 0 | 0 | 1 | 1 | 0 |
| 72 | 0 | 0 | 0 | 0 | 0 | 0 |
| 73 | 1 | 0 | 1 | 1 | 1 | 0 |
| 74 | 1 | 0 | 1 | 1 | 1 | 0 |
| 75 | 1 | 0 | 1 | 1 | 1 | 0 |
| 76 | 1 | 1 | 1 | 1 | 1 | 0 |
| 77 | 1 | 1 | 1 | 1 | 1 | 0 |
| 78 | 1 | 1 | 1 | 1 | 1 | 0 |
| 79 | 1 | 1 | 1 | 1 | 1 | 0 |
| 80 | 1 | 1 | 1 | 1 | 1 | 0 |
| 81 | 1 | 1 | 1 | 1 | 1 | 0 |
| 82 | 1 | 1 | 1 | 1 | 1 | 0 |
| 83 | 1 | 1 | 1 | 1 | 1 | 0 |
| 84 | 1 | 1 | 1 | 1 | 1 | 0 |
| 85 | 1 | 1 | 1 | 1 | 1 | 0 |
| 86 | 1 | 1 | 1 | 1 | 1 | 0 |
| 87 | 1 | 1 | 1 | 1 | 1 | 0 |
| 88 | 1 | 1 | 1 | 1 | 1 | 0 |
| 89 | 1 | 1 | 1 | 1 | 1 | 0 |
| 90 | 1 | 1 | 1 | 1 | 1 | 0 |
| 91 | 1 | 0 | 1 | 1 | 1 | 0 |
| 92 | 1 | 1 | 1 | 1 | 1 | 0 |
| 93 | 1 | 0 | 1 | 1 | 1 | 0 |
| 94 | 0 | 0 | 0 | 0 | 0 | 0 |
| 95 | 0 | 0 | 0 | 0 | 0 | 0 |
| 96 | 0 | 0 | 0 | 0 | 0 | 0 |
| 97 | 0 | 0 | 0 | 0 | 0 | 0 |
| 98 | 0 | 0 | 0 | 0 | 0 | 0 |
| 99 | 0 | 0 | 0 | 0 | 0 | 0 |
| 100 | 0 | 0 | 0 | 0 | 0 | 0 |
| 101 | 0 | 0 | 0 | 0 | 0 | 0 |
| 102 | 0 | 1 | 0 | 0 | 0 | 0 |
| 103 | 1 | 1 | 1 | 1 | 1 | 0 |
| 104 | 1 | 1 | 1 | 1 | 1 | 0 |
| 105 | 1 | 1 | 1 | 1 | 1 | 0 |
| 106 | 1 | 1 | 1 | 1 | 1 | 0 |
| 107 | 1 | 1 | 1 | 1 | 1 | 0 |
| 108 | 1 | 1 | 1 | 1 | 1 | 0 |
| 109 | 1 | 1 | 1 | 1 | 1 | 0 |
| 110 | 1 | 1 | 1 | 1 | 1 | 0 |
| 111 | 1 | 1 | 1 | 1 | 1 | 0 |
| 112 | 1 | 1 | 1 | 1 | 1 | 0 |
| 113 | 1 | 1 | 1 | 1 | 1 | 0 |
| 114 | 0 | 0 | 0 | 1 | 1 | 0 |
| 115 | 1 | 1 | 1 | 1 | 1 | 0 |
| 116 | 1 | 1 | 1 | 1 | 1 | 0 |
| 117 | 1 | 1 | 1 | 1 | 1 | 0 |
| 118 | 0 | 0 | 0 | 0 | 0 | 0 |
| 119 | 0 | 0 | 0 | 0 | 0 | 0 |
| 120 | 0 | 0 | 0 | 0 | 0 | 0 |
| 121 | 1 | 1 | 1 | 0 | 0 | 0 |
| 122 | 0 | 0 | 0 | 0 | 0 | 0 |
| 123 | 0 | 0 | 0 | 0 | 0 | 0 |
| 124 | 0 | 0 | 0 | 0 | 0 | 0 |
| 125 | 0 | 0 | 0 | 0 | 0 | 0 |

Le nombre de transitions positives est ensuite sommé pour les applications I, R et V et reporté dans le **TABLEAU 22 :**

**TABLEAU 22 :**

| | Urine 1 | Urine 2 | Urine 3 | Urine 4 | Urine 5 | Urine 6 |
|---|---|---|---|---|---|---|
| I | 36 | 36 | 40 | 44 | 41 | 60 |
| R | 6 | 6 | 6 | 0 | 5 | 0 |
| V | 1 | 1 | 1 | 0 | 0 | 0 |

De même que le seuil de positivité des transitions a du être modifié pour tenir compte de la concentration des échantillons, le nombre de peptides nécessaires pour caractériser une souche doit être adapté à la concentration totale en bactérieq. Certains peptides faiblement détectés dans l'exemple 7 peuvent être sous la limite de détection si la quantité de bactéries est inférieure à une colonie.

Les urines 1 à 5 présentent plus de 30 transitions positives dans la catégorie I. Tous ces échantillons sont donc confirmés comme contaminé par l'espèce *E. coli.*

En revanche, l'urine 6 ne présentent aucune transition positive dans la catégorie I. Elle est donc confirmée comme n'étant pas contaminée par l'espèce *E. coli.*

Les urines 1, 2, 3 et 5 présentent au moins 5 transitions positives pour la catégorie R, elles expriment donc la pénicillinase plasmidique TEM-2, ce qui est synonyme d'un mécanisme de résistance aux Pénicillines, notamment aminopénicillines ou pénicillines A (ampicilline), carboxypénicillines ou pénicillines C (ticarcilline) et uréidopénicilline ou pénicilline U (pipéracilline).

En revanche, les urines 4 et 6 ne présentent pas de transitions positives pour la catégorie R, elles n'expriment donc pas la pénicillinase plasmidique TEM-2. Ces souches sont donc sensibles aux Pénicillines.

Ces résultats de résistance à l'ampicilline, la ticarcilline et la pipéracilline ont été confirmés avec l'automate VITEK®2 commercialisé par la demanderesse et les cartes AST-EXN et AST-N103. De façon moins rapide que pour la présente invention, la confirmation a pris un temps de 6 à 8 heures sur l'automate VITEK®2.

Les urines 1 à 6 ne présentent pas plus d'une transition positive pour la catégorie V. Ces urines ne sont donc pas contaminées par des toxines de type Shigatoxines.

Pour le typage, les transitions de la catégorie T de chaque souche sont comparées aux transitions des autres souches considérées comme des souches de référence. En pratique une valeur 0 est attribuée lorsque les transitions entre les 2 souches se classent dans la même catégorie (positive ou négative) et une valeur de 1 est attribuée lorsque les transitions entre les 2 souches se classent dans des catégories différentes (une transition positive et une transition négative). Les valeurs sont sommées pour toutes les transitions de la catégorie T de chaque couple de souche afin d'établir un score. Les scores figurent dans le **TABLEAU 23 :**

**TABLEAU 23 :**

| | Urine 1 | Urine 2 | Urine 3 | Urine 4 | Urine 5 |
|---|---|---|---|---|---|
| Urine 1 | 0 | 11 | 13 | 19 | 15 |
| Urine 2 | 11 | 0 | 16 | 24 | 20 |
| Urine 3 | 13 | 16 | 0 | 16 | 12 |
| Urine 4 | 19 | 24 | 16 | 0 | 8 |
| Urine 5 | 15 | 20 | 12 | 8 | 0 |

Aucune urine n'a un score inférieur ou égal à 4. Les souches infectant les urines 1 à 5 sont donc de type différents. Ces échantillons urinaires ont été collectés dans un laboratoire d'analyse médicale de ville où les infections surviennent souvent dans des environnements différents. Dans ces conditions, il est peut fréquent d'observer des infections urinaires liées à la même souche.

Cet exemple montre que, de façon très avantageuse, la présente invention permet directement à partir de l'échantillon primaire, ce qui est très avantageux de confirmer l'identité d'une espèce telle que *E. coli* et de déterminer, simultanément au sein de la même analyse, les propriétés de typage, et de résistance potentielle à au moins un antibiotique.

### Références bibliographiques

[1] J. Anhalt & C. Fenselau, 1975, Anal. Chem., 47(2) :219-225.
[2] A. Fox et al, ed., 1990, Analytical microbiology methods : chromatography and mass spectrometry, Plenum Press, New York, N.Y.
[3] M. Claydon et al, 1996, Nature Biotech. 14 :1584-1586.
[4] T. Krishnamurthy & P. Ross, 1996, Rapid Com. Mass Spec., 10 :1992-1996.
[5] P. Seng et al. 2009, Clin. Infect. Dis., 49 :543-551.
[6] C. Fenselau et al., 2008, Appl. Environ. Microbiol., 904-906.
[7] D. Ding et al. 2009, J. Pharm. Biomed. Anal. 50:79-85.
[8] R. Everley et al., 2009, J. Microbiol. Methods, 77:152-158.
[9] S. Hofstadler et al., 2005, Int. .J Mass Spectrom., 242:23-41.
[10] D. Ecker, 2008, Nat. Rev. Microbiol., 6(7):553-558.
[11] W.-J. Chen et al., 2008, Anal. Chem., 80 : 9612-9621
[12] D. Lopez-Ferrer et al., 2008, Anal. Chem., 80 :8930-8936
[13] D. Lopez-Ferrer et al., 2005, J. Proteome res., 4(5) : 1569-1574
[14] T. Fortin et al., 2009, Mol. Cell Proteomics, 8(5) : 1006-1015.
[15] H. Keshishian et al., 2007, Mol. Cell Proteomics, 2212-2229.
[16] J. Stal-Zeng et al., 2007, Mol. Cell Proteomics, 1809-1817.
[17] Gaskell, Electrospray: principles and practise, 1997, J. Mass Spectrom., 32, 677-688).
[18] V. Fusaro et al., 2009, Nature Biotech. 27, 190-198.
[19] J. Mead et al., 15 nov 2008, Mol. Cell Proteomics, E-pub.
[20] F. Desiere et al., 2006, Nucleic Acids Res., 34(database issue) : D655-8).
L. Anderson & C. Hunter, 2006, Mol. Cell Proteomics, 573-588).
B. Han & R. Higgs, 2008, Brief Funct Genomic Proteomic.,7(5):340-54).
K.-Y. Wang et al., 2008, Anal Chem, 80(16) 6159-6167).
J. Bundy & C. Fenselau, 1999, Anal. Chem. 71 : 1460-1463.
K-C Ho et al., 2004, Anal. Chem. 76 : 7162-7268.
Y.S. Lin et al., 2005, Anal. Chem., 77 : 1753-1760.
S. Vaidyanathan et al., 2001, Anal. Chem., 73 :4134-4144.
P. Seng et al., 2009, Clin. Infect. Dis., 49 :543-551.
Manes N. et al., 2007, Mol. & Cell. Proteomics, 6(4): 717-727.
R. Nandakumar et al., 2009, Oral Microbiology Immunology, 24 :347-352).
L. Hernychova et al., 2008, Anal. Chem., 80 :7097-7104.
J.-M. Pratt et al., 2006, Nat. Protoc., 1:1029-1043.
V. Brun et al., 2007, Mol. Cell Proteomics, 2139-2149.
V. Lange et al., Molecular and Cellular Proteomics, 7 : 1489-1500

## Revendications

1. Procédé de caractérisation d'au moins un microorganisme issu d'un échantillon, comprenant l'identification dudit au moins un microorganisme et la détermination des propriétés de typage, résistance potentielle à au moins un antimicrobien et facteur de virulence, **caractérisé en ce que** l'identification dudit au moins un microorganisme et la détermination des propriétés de typage, résistance à au moins un antimicrobien et facteur de virulence pour ledit au moins un microorganisme sont mises en oeuvre par spectrométrie de masse de type MS/MS en mode MRM, en utilisant des protéines, peptides et/ou métabolites en tant que marqueurs desdites propriétés de typage, résistance à au moins un antimicrobien et facteur de virulence et **en ce que** la détermination des propriétés de typage, résistance à au moins un antimicrobien et facteur de virulence est mise en oeuvre simultanément, dans le même appareil de spectrométrie de masse.

2. Procédé de caractérisation d'au moins un microorganisme selon la revendication précédente, **caractérisé en ce qu'**il comprend une étape supplémentaire de confirmation de l'identification dudit au moins un microorganisme, laquelle étape de confirmation est mise en oeuvre par spectrométrie de masse.

3. Procédé de caractérisation d'au moins un microorganisme selon la revendication 2, **caractérisé en ce que** la spectrométrie de masse de l'étape de confirmation est un spectrométrie de masse de type MS/MS.

4. Procédé de caractérisation d'au moins un microorganisme selon la revendication 3, **caractérisé en ce que** la spectrométrie de masse de l'étape de confirmation est une MRM.

5. Procédé de caractérisation d'au moins un microorganisme selon l'une quelconque des revendications précédentes, **caractérisé en ce que** les marqueurs desdites propriétés de typage, résistance à au moins un antimicrobien et facteur de virulence sont des protéines dudit au moins un microorganisme.

6. Procédé de caractérisation d'au moins un microorganisme selon la revendication 5, **caractérisé en ce que** les protéines dudit microorganisme sont digérées en peptides.

7. Procédé de caractérisation d'au moins un microorganisme selon la revendication 6, **caractérisé en ce que** la digestion est réalisée par une enzyme.

8. Procédé de caractérisation d'au moins un microorganisme selon la revendication 7, **caractérisé en ce que** l'enzyme est de la trypsine.

9. Procédé de caractérisation d'au moins un microorganisme selon les revendications précédentes, **caractérisé en ce que** ledit au moins un microorganisme est une bactérie et l'antimicrobien est un antibiotique.

10. Procédé de caractérisation d'au moins un microorganisme selon la revendication 9, **caractérisé en ce que** la bactérie est *Staphylococcus aureus.*

11. Procédé de caractérisation d'au moins un microorganisme selon la revendication 10, **caractérisé en ce que** la détermination des propriétés de typage met en oeuvre au moins un peptide de séquence SEQ ID N°2, 3, 4, 5, 6, 7, 8, 10, 11, 12, 13, 14, 15, 16, 17, 19, 20, 21, 23, 24, 26, 27, 29, 30, 32, 34, 35, 37, 38, 40, 42, 44 ou 45.

12. Procédé de caractérisation d'au moins un microorganisme selon la revendication 10 ou 11, **caractérisé en ce que** la détermination des propriétés de résistance potentielle à au moins un antibiotique met en oeuvre au moins un peptide de séquence SEQ ID N°10 à 17.

13. Procédé de caractérisation d'au moins un microorganisme selon l'une quelconque des revendications 10 à 12, **caractérisé en ce que** la détermination de la virulence met en oeuvre au moins un peptide de séquence SEQ ID N°19, 20, 21, 23 ou 24.

14. Procédé de caractérisation d'au moins un microorganisme selon l'une quelconque des revendications 10 à 13, **caractérisé en ce que** l'identification met en oeuvre au moins un peptide de séquence SEQ ID N°26, 27, 29, 30, 32, 34, 35, 37, 38, 40, 42, 44 et 45.

15. Procédé de caractérisation d'au moins un microorganisme selon la revendication 10, **caractérisé en ce que** la bactérie est *Escherichia coli.*

16. Procédé de caractérisation d'au moins un microorganisme selon la revendication 15, **caractérisé en ce que** la détermination des propriétés de typage met en oeuvre au moins un peptide de séquence SEQ ID N°46 à 87.

17. Procédé de caractérisation d'au moins un microorganisme selon la revendication 15 ou 16, **caractérisé en ce que** la détermination des propriétés de résistance potentielle à au moins un antibiotique met en oeuvre au moins un peptide de séquence SEQ ID N°85, 86 et 87.

18. Procédé de caractérisation d'au moins un microorganisme selon l'une quelconque des revendications 15 à 17, **caractérisé en ce que** la détermination de la virulence met en oeuvre au moins un peptide de séquence SEQ ID N° 62 à 66.

19. Procédé de caractérisation d'au moins un microorganisme selon l'une quelconque des revendications 15 à 18, **caractérisé en ce que** l'identification met en oeuvre au moins un peptide de séquence SEQ ID N°46 à 61.

20. Procédé de caractérisation d'au moins un microorganisme selon les revendications 1 à 8, **caractérisé en ce que** ledit au moins un microorganisme est une levure et l'antimicrobien est un antifongique.

21. Procédé de caractérisation d'au moins un microorganisme selon la revendication 20, **caractérisé en ce que** la levure est *Candida albicans.*

22. Procédé de caractérisation d'au moins un microorganisme selon la revendication 21, **caractérisé en ce que** la détermination des propriétés de typage met en oeuvre au moins un peptide de séquence SEQ ID N°88 à 125 et 177.

23. Procédé de caractérisation d'au moins un microorganisme selon la revendication 21 ou 22, **caractérisé en ce que** la détermination des propriétés de résistance potentielle à au moins un antifongique met en oeuvre au moins un peptide de séquence SEQ ID N° 117 à 122 et 177.

24. Procédé de caractérisation d'au moins un microorganisme selon l'une quelconque des revendications 21 à 23, **caractérisé en ce que** la détermination de la virulence met en oeuvre au moins un peptide de séquence SEQ ID N°123 à 125.

25. Procédé de caractérisation d'au moins un microorganisme selon l'une quelconque des revendications 21 à 24, **caractérisé en ce que** l'identification met en oeuvre au moins un peptide de séquence SEQ ID N°88 à 116.

26. Procédé de caractérisation d'au moins un microorganisme selon l'une quelconque des revendications précédentes, **caractérisé en ce que** le métabolite est l'ergostérol.

27. Peptide de séquence SEQ ID N°2.

## Patentansprüche

1. Verfahren zum Charakterisieren von mindestens einem aus einer Probe stammenden Mikroorganismus, umfassend das Identifizieren des mindestens einen Mikroorganismus und die Bestimmung von Eigenschaften der Typisierung, möglicher Resistenz gegen mindestens ein antimikrobielles Mittel und eines Virulenzfaktors, **dadurch gekennzeichnet, dass** das Identifizieren des mindestens einen Mikroorganismus und die Bestimmung von Eigenschaften der Typisierung, einer Resistenz gegen mindestens ein antimikrobielles Mittel und eines Virulenzfaktors für den mindestens einen Mikroorganismus mittels Massenspektrometrie des MS/MS-Typs im MRM-Modus durchgeführt werden, und zwar unter Verwendung von Proteinen, Peptiden und/oder Metaboliten als Marker für diese Eigenschaften der Typisierung, eine Resistenz gegen mindestens ein antimikrobielles Mittel und einen Virulenzfaktor, und dass die Bestimmung der Eigenschaften der Typisierung, einer Resistenz gegen mindestens ein antimikrobielles Mittel und eines Virulenzfaktors gleichzeitig in demselben Massenspektrometer erfolgt.

2. Verfahren zum Charakterisieren von mindestens einem Mikroorganismus nach dem vorhergehenden Anspruch, **dadurch gekennzeichnet, dass** es einen zusätzlichen Schritt der Bestätigung der Identifizierung des mindestens einen Mikroorganismus umfasst, wobei der Bestätigungsschritt mittels Massenspektrometrie durchgeführt wird.

3. Verfahren zum Charakterisieren von mindestens einem Mikroorganismus nach Anspruch 2, **dadurch gekennzeichnet, dass** die Massenspektrometrie des Bestätigungsschritts eine Massenspektrometrie des MS/MS-Typs ist.

4. Verfahren zum Charakterisieren von mindestens einem Mikroorganismus nach Anspruch 3, **dadurch gekennzeichnet, dass** die Massenspektrometrie des Bestätigungsschritts ein MRM ist.

5. Verfahren zum Charakterisieren von mindestens einem Mikroorganismus nach einem der vorhergehenden Ansprüche, **dadurch gekennzeichnet, dass** die Marker der Typisierungseigenschaften, der Resistenz gegen mindestens ein antimikrobielles Mittel und des Virulenzfaktors Proteine des mindestens einen Mikroorganismus sind.

6. Verfahren zum Charakterisieren von mindestens einem Mikroorganismus nach Anspruch 5, **dadurch gekennzeichnet, dass** die Proteine des Mikroorganismus zu Peptiden verdaut werden.

7. Verfahren zum Charakterisieren von mindestens einem Mikroorganismus nach Anspruch 6, **dadurch gekennzeichnet, dass** das Verdauen mittels eines Enzyms erfolgt.

8. Verfahren zum Charakterisieren von mindestens einem Mikroorganismus nach Anspruch 7, **dadurch gekennzeichnet, dass** es sich bei dem Enzym um Trypsin handelt.

9. Verfahren zum Charakterisieren von mindestens einem Mikroorganismus nach den vorhergehenden Ansprüchen, **dadurch gekennzeichnet, dass** der mindestens eine Mikroorganismus ein Bakterium ist und das antimikrobielle Mittel ein Antibiotikum ist.

10. Verfahren zum Charakterisieren von mindestens einem Mikroorganismus nach Anspruch 9, **dadurch gekennzeichnet, dass** das Bakterium *Staphylococcus aureus* ist.

11. Verfahren zum Charakterisieren von mindestens einem Mikroorganismus nach Anspruch 10, **dadurch gekennzeichnet, dass** bei der Bestimmung der Typisierungseigenschaften mindestens ein Peptid der Sequenz SEQ. ID. Nr. 2, 3, 4, 5, 6, 7, 8, 10, 11, 12, 13, 14, 15, 16, 17, 19, 20, 21, 23, 24, 26, 27, 29, 30, 32, 34, 35, 37, 38, 40, 42, 44 oder 45 eingesetzt wird.

12. Verfahren zum Charakterisieren von mindestens einem Mikroorganismus nach Anspruch 10 oder 11, **dadurch gekennzeichnet, dass** bei der Bestimmung von Eigenschaften einer möglichen Resistenz gegen mindestens ein Antibiotikum mindestens ein Peptid der Sequenz SEQ. ID. Nr. 10 bis 17 eingesetzt wird.

13. Verfahren zum Charakterisieren von mindestens einem Mikroorganismus nach einem der Ansprüche 10 bis 12, **dadurch gekennzeichnet, dass** bei der Bestimmung der Virulenz mindestens ein Peptid der Sequenz SEQ. ID. Nr. 19, 20, 21, 23 oder 24 eingesetzt wird.

14. Verfahren zum Charakterisieren von mindestens einem Mikroorganismus nach einem der Ansprüche 10 bis 13, **dadurch gekennzeichnet, dass** bei dem Identifizieren mindestens ein Peptid der Sequenz SEQ. ID. Nr. 26, 27, 29, 30, 32, 34, 35, 37, 38, 40, 42, 44 und 45 eingesetzt wird.

15. Verfahren zum Charakterisieren von mindestens einem Mikroorganismus nach Anspruch 10, **dadurch gekennzeichnet, dass** es sich bei dem Bakterium um *Escherichia coli* handelt.

16. Verfahren zum Charakterisieren von mindestens einem Mikroorganismus nach Anspruch 15, **dadurch gekennzeichnet, dass** bei der Bestimmung von Eigenschaften der Typisierung mindestens ein Peptid der Sequenz SEQ. ID. Nr. 46 bis 87 eingesetzt wird.

17. Verfahren zum Charakterisieren von mindestens einem Mikroorganismus nach Anspruch 15 oder 16, **dadurch gekennzeichnet, dass** bei der Bestimmung der Eigenschaften einer möglichen Resistenz gegen mindestens ein Antibiotikum mindestens ein Peptid der Sequenz SEQ. ID. Nr. 85, 86 und 87 eingesetzt wird.

18. Verfahren zum Charakterisieren von mindestens einem Mikroorganismus nach einem der Ansprüche 15 bis 17, **dadurch gekennzeichnet, dass** bei der Bestimmung der Virulenz mindestens ein Peptid der Sequenz SEQ. ID. Nr. 62 bis 66 eingesetzt wird.

19. Verfahren zum Charakterisieren von mindestens einem Mikroorganismus nach einem der Ansprüche 15 bis 18, **dadurch gekennzeichnet, dass** bei dem Identifizieren mindestens ein Peptid der Sequenz SEQ. ID. Nr. 46 bis 61 eingesetzt wird.

20. Verfahren zum Charakterisieren von mindestens einem Mikroorganismus nach den Ansprüchen 1 bis 8, **dadurch gekennzeichnet, dass** es sich bei dem mindestens einen Mikroorganismus um eine Hefe und bei dem antimikrobiellen Mittel um ein antifungales Mittel handelt.

21. Verfahren zum Charakterisieren von mindestens einem Mikroorganismus nach Anspruch 20, **dadurch gekennzeichnet, dass** es sich bei der Hefe um *Candida albicans* handelt.

22. Verfahren zum Charakterisieren von mindestens einem Mikroorganismus nach Anspruch 21, **dadurch gekennzeichnet, dass** bei der Bestimmung der Eigenschaften der Typisierung mindestens ein Peptid der Sequenz SEQ. ID. Nr. 88 bis 125 und 177 eingesetzt wird.

23. Verfahren zum Charakterisieren von mindestens einem Mikroorganismus nach Anspruch 21 oder 22, **dadurch gekennzeichnet, dass** bei der Bestimmung der Eigenschaften der möglichen Resistenz gegen mindestens ein antifungales Mittel mindestens ein Peptid der Sequenz SEQ. ID. Nr. 117 bis 122 und 177 eingesetzt wird.

24. Verfahren zum Charakterisieren von mindestens einem Mikroorganismus nach einem der Ansprüche 21 bis 23, **dadurch gekennzeichnet, dass** bei der Bestimmung der Virulenz mindestens ein Peptid der Sequenz SEQ. ID. Nr. 123 bis 125 eingesetzt wird.

25. Verfahren zum Charakterisieren von mindestens einem Mikroorganismus nach einem der Ansprüche 21 bis 24, **dadurch gekennzeichnet, dass** bei dem Identifizieren mindestens ein Peptid der Sequenz SEQ. ID. Nr. 88 bis 116 eingesetzt wird.

26. Verfahren zum Charakterisieren von mindestens einem Mikroorganismus nach einem der vorhergehenden Ansprüche, **dadurch gekennzeichnet, dass** es sich bei dem Metaboliten um Ergosterol handelt.

27. Peptid der Sequenz SEQ. ID. Nr. 2.

## Claims

1. A method for characterizing at least one microorganism from a sample, which comprises identifying said at least one microorganism and determining the properties of typing, potential resistance to at least one antimicrobial, and virulence factor, **characterized in that** the identifying said at least one microorganism and the determining of the properties of typing, resistance to at least one antimicrobial, and virulence factor for said at least one microorganism are implemented by mass spectrometry of MS/MS type in MRM mode, using proteins, peptides and/or metabolites as markers of said properties of typing, resistance to at least one antimicrobial, and virulence factor and **in that** the determining of the properties of typing, resistance to at least one antimicrobial, and virulence factor for said at least one microorganism is implemented simultaneously, in the same mass spectrometry apparatus.

2. The method for characterizing at least one microorganism as claimed in any one of the preceding claims, **characterized in that** it comprises an additional step of confirming the identification of said at least one microorganism, which confirmation step is implemented by mass spectrometry.

3. The method for characterizing at least one microorganism as claimed in claim 2, **characterized in that** the mass spectrometry of the confirmation step is mass spectrometry of MS/MS type.

4. The method for characterizing at least one microorganism as claimed in claim 3, **characterized in that** the mass spectrometry of the confirmation step is an MRM.

5. The method for characterizing at least one microorganism as claimed in any one of the preceding claims, **characterized in that** the markers of said properties of typing, resistance to at least one antimicrobial, and virulence factor are proteins of said at least one microorganism.

6. The method for characterizing at least one microorganism as claimed in claim 5, **characterized in that** the proteins of said microorganism are digested into peptides.

7. The method for characterizing at least one microorganism as claimed in claim 6, **characterized in that** the digestion is carried out with an enzyme.

8. The method for characterizing at least one microorganism as claimed in claim 7, **characterized in that** the enzyme is trypsin.

9. The method for characterizing at least one microorganism as claimed in the preceding claims, **characterized in that** said at least one microorganism is a bacterium and the antimicrobial is an antibiotic.

10. The method for characterizing at least one microorganism as claimed in claim 9, **characterized in that** the bacterium is *Staphylococcus aureus.*

11. The method for characterizing at least one microorganism as claimed in claim 10, **characterized in that** the determining of the typing properties uses at least one peptide having the sequence SEQ ID No.2, 3, 4, 5, 6, 7, 8, 10, 11, 12, 13, 14, 15, 16, 17, 19, 20, 21, 23, 24, 26, 27, 29, 30, 32, 34, 35, 37, 38, 40, 42, 44 or 45.

12. The method for characterizing at least one microorganism as claimed in claim 10 or 11, **characterized in that** the determining of the properties of potential resistance to at least one antibiotic uses at least one peptide having the sequence SEQ ID No. 10 to 17.

13. The method for characterizing at least one microorganism as claimed in any one of claims 10 to 12, **characterized in that** the determining of the virulence uses at least one peptide having the sequence SEQ ID No.19, 20, 21, 23 or 24.

14. The method for characterizing at least one microorganism as claimed in any one of claims 10 to 13, **characterized in that** the identifying uses at least one peptide having the sequence SEQ ID No.26, 27, 29, 30, 32, 34, 35, 37, 38, 40, 42, 44 and 45.

15. The method for characterizing at least one microorganism as claimed in claim 10, **characterized in that** the bacterium is *Escherichia coli.*

16. The method for characterizing at least one microorganism as claimed in claim 15, **characterized in that** the determining of the typing properties uses at least one peptide having the sequence SEQ ID No.46 to 87.

17. The method for characterizing at least one microorganism as claimed in claim 15 or 16, **characterized in that** the determining of the properties of potential resistance to at least one antibiotic uses at least one peptide having the sequence SEQ ID No.85, 86 and 87.

18. The method for characterizing at least one microorganism as claimed in any one of claims 15 to 17, **characterized in that** the determining of the virulence uses at least one peptide having the sequence SEQ ID No. 62 to 66.

19. The method for characterizing at least one microorganism as claimed in any one of claims 15 to 18, **characterized in that** the identifying uses at least one peptide having the sequence SEQ ID No.46 to 61.

20. The method for characterizing at least one microorganism as claimed in claims 1 to 8, **characterized in that** said at least one microorganism is a yeast and the antimicrobial is an antifungal.

21. The method for characterizing at least one microorganism as claimed in claim 20, **characterized in that** the yeast is *Candida albicans.*

22. The method for characterizing at least one microorganism as claimed in claim 21, **characterized in that** the determining of the typing properties uses at least one peptide having the sequence SEQ ID No.88 to 125 and 177.

23. The method for characterizing at least one microorganism as claimed in claim 21 or 22, **characterized in that** the determining of the properties of potential resistance to at least one antifungal uses at least one peptide having the sequence SEQ ID No. 117 to 122 and 177.

24. The method for characterizing at least one microorganism as claimed in any one of claims 21 to 23, **characterized in that** the determining of the virulence uses at least one peptide having the sequence SEQ ID No. 123 to 125.

25. The method for characterizing at least one microorganism as claimed in any one of claims 21 to 24, **characterized in that** the identifying uses at least one peptide having the sequence SEQ ID No.88 to 116.

26. The method for characterizing at least one microorganism as claimed in any one of the preceding claims, **characterized in that** the metabolite is ergosterol.

27. A peptide having the sequence SEQ ID No.2.
